# EUROPEAN PATENT SPECIFICATION

(11) **EP 0 512 352 B1**
(45) Date of publication and mention of the grant of the patent: **27.03.1996**
(21) Application number: 92107135.3
(22) Date of filing: 27.04.1992
(51) Int. Cl.: C07C 59/90, A61K 31/19, C07C 65/40

(54) **Substituted carboxylic acid derivatives**
Substituierte Carbonsäurederivate
Dérivés substitués d'acides carboxyliques

(30) Priority: 09.05.1991 US 698014; 13.03.1992 US 850620
(43) Date of publication of application: 11.11.1992
(73) Proprietor: F. HOFFMANN-LA ROCHE AG, CH-4002 Basel (CH)
(72) Inventor: Guthrie, Robert William, Saddle Brook, NJ 07662 (US); Heathers, Guy Phillip, Morristown, NJ 07960 (US); Higgins, Alan John, Upper Montclair, NJ 07043 (US); Kachensky, David Francis, Milford, Pennsylvania 18337 (US); Kierstead, Richard Wightmann, North Caldwell, NJ 07006 (US); LeMahieu, Ronald Andrew, North Caldwell, NJ 07006 (US); Mullin, John Guilfoyle, Jr., Hawthorne, NJ 07512 (US); Tilley, Jefferson Wright, North Caldwell, NJ 07006 (US)
(74) Representative: Kellenberger, Marcus, Dr.

(56) References cited:
- EP-A- 0 331 085
- DE-A- 2 950 608
- PATENT ABSTRACTS OF JAPAN vol. 12, no. 333 (C-526)(3180) 8 September 1988 & JA-A-63 096 152

## Description

The present invention relates to compounds of the general formula 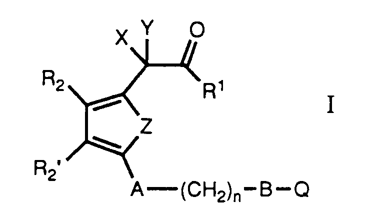
wherein R₁ is hydroxy, OR₃ or 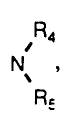
wherein one of R₄ and R₅ is hydrogen, lower alkyl or hydroxy-lower alkyl, and the other is hydrogen, hydroxy, lower alkyl or lower alkoxy, and wherein R₃ is (̵CH₂CH₂O)̵ₘH, 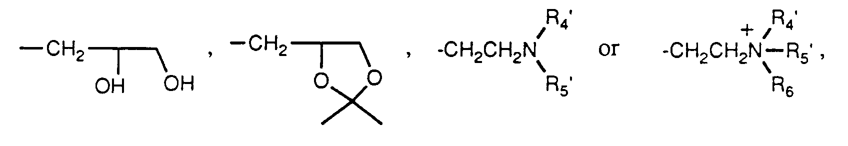
and wherein R₄', R₅', and R₆ are, independently, hydrogen , lower alkyl or hydroxy-lower alkyl, and wherein m is an integer from 1 to 4;
R₂ and R₂', independently, are hydrogen, lower alkyl, aryl, aryl-lower alkyl, lower alkoxy, hydroxy, amino, lower alkylamino, di-lower-alkylamino, cyano, halogen, mercapto, lower alkylthio, lower alkylsulfinyl, lower alkylsulfonyl, trihalo-lower alkyl, acyl or nitro;
A is a bond,
―O―, ―R₇―, ―S―, 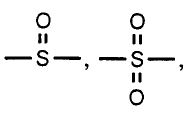
―C≡C―, ―HC=CH―, ―CH₂CH₂―, 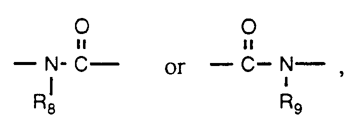
wherein R₇ is hydrogen, lower alkyl or acyl and R₈ and R₉ are, independently, hydrogen or lower alkyl; n is an integer from 0 to 10;
B is a bond, ―O―, ―NR₇, ―S―, 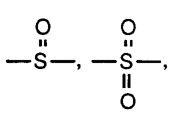
―C≡C―, ―HC=CH―, 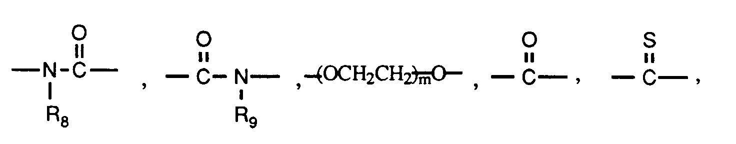 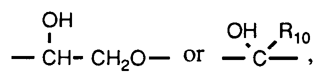
wherein R₇, R₈, R₉ and m are as defined above and R₁₀ is hydrogen or lower alkyl;
Z is ―O―, ―S―, ―CR₂ = CR₂'―, ―N=CR₂―, ―CR₂ = N― or 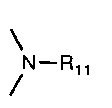
wherein R₁₁ is hydrogen or lower alkyl;
X and Y taken together are 0= , S=, hydroxyimino, alkyloxyimino, alkenyloxyimino, arylalkoxyimino, hydrazono, mono-lower alkyl hydrazono, di-lower alkyl hydrazono or semicarbazono, or, independently, when one of X and Y is halogen, the other is hydrogen, halogen, lower alkyl or aryl-lower alkyl; or when one of X and Y is amino, lower alkylamino or di-lower alkylamino, the other is hydrogen, lower alkyl or aryl-lower alkyl; or when one of X and Y is hydroxy, alkoxy or aryl-lower alkoxy, the other is hydrogen, hydroxy, lower alkyl, lower alkoxy or aryl-lower alkoxy; Q is a cycloalkyl, aryl or heterocyclic radical; provided that, when A is oxygen (O) and B is a bond, sulfur (S) or oxygen (O), then n is 2-10; and, when appropriate, in the form of enantiomers, racemates, diastereomers or mixtures thereof or geometric isomers or mixtures thereof, and, when a basic or acidic group is present, pharmaceutically acceptable salts thereof.

These compounds inhibit the enzyme carnitine acyltransferase 1 (CAT-1) and are therefore useful in the prevention of injury to ischemic tissue, and can limit infarct size, improve cardiac function and prevent arrhythmias during and following a myocardial infarction.

Objects of the present invention are the compounds of formula I and their pharmaceutically acceptable salts per se and for use as therapeutically active substances, the manufacture of these compounds, medicaments containing these and the manufacture of such medicaments, as well as the use of compounds of formula I and their pharmaceutically acceptable salts in the control or prevention of illnesses or in the improvement of health, especially in the control or prevention of injury to ischemic tissue and arryhthmias during and following a myocardial infarction.

The following definitions of the general terms used in the present description apply irrespective of whether the terms in question appear alone or in combination.

As used herein, the term "alkyl" denotes a straight or branched chain saturated hydrocarbon containing 1 to 16 carbon atoms, preferably from 1 to 10 carbon atoms, for example, methyl, ethyl, propyl, isopropyl, butyl, t-butyl, neopentyl, pentyl, hexyl, heptyl, octyl, nonyl, decyl, undecyl, dodecyl, tridecyl, tetradecyl, pentadecyl, and hexadecyl. The term "lower alkyl" denotes a straight or branched chain saturated hydrocarbon containing 1 to 7 carbon atoms. In the term "hydroxyalkyl", the alkyl group is as described above. The term "alkoxy" denotes an alkyl ether group in which the alkyl group is as described above, for example, methoxy, ethoxy, propoxy, pentoxy, hexoxy, heptoxy, octoxy, nonoxy, decoxy, undecoxy, tridecoxy, tetradecoxy, and hexadecoxy. The term "lower alkoxy" denotes an alkyl ether group in which the alkyl group contains 1 to 7 carbon atoms. In the term "hydroxyalkoxy", the alkoxy group is as described above. In the term "alkoxyalkyl", the alkoxy and alkyl groups are as described above. The term "lower alkylthio" denotes a lower alkylthio ether group in which the lower alkyl group is as described, for example, methylthio, ethylthio, isopropylthio, propylthio, butylthio, pentylthio, and heptylthio. The term "alkenyl" denotes a straight or branched olefinic hydrocarbon containing 2 to 12 carbon atoms, preferably from 2 to 6 carbon atoms, for example, vinyl, allyl, butenyl, pentenyl, nonenyl, and dodecenyl. The term "alkenyloxy" denotes an alkenyl ether group, wherein the alkenyl group is as previously described, for example, allyloxy, pentenyloxy, and octenyloxy. The term "aryl" preferably denotes a mono-, bi- or tricyclic aromatic hydrocarbon radical, for example, phenyl, naphthyl, 1,1'-biphenyl, anthracenyl, phenanthrenyl, 5,6,7,8-tetrahydro-1- naphthalenyl, 5,6,7,8-tetrahydro-2-naphthalenyl, and 1,2,3,4-tetrahydro-1-naphthalenyl, 1,2,3,4-tetrahydro-2-naphthalenyl, which radical may be unsubstituted or mono-, di- or tri-substituted, independently, by a radical selected from alkyl, alkoxy, acyloxy, halogen, acyloxyalkyl, alkoxyalkyl, arylalkyl, arylalkoxy, aryloxyalkyl, aryloxyalkoxy, alkoxyalkoxy, hydroxy, hydroxyalkyl, hydroxyalkoxy, amino, lower alkylamino or di-lower alkylamino, cyano, nitro, mercapto, alkylthio, alkylsulfinyl, alkylsulfonyl, trihaloalkyl, sulfamoyl, N-alkylsulfamoyl, N,N-dialkylsulfamoyl, carboxycarbonyl, alkoxalyl (which has the formula R₁₂O-C(O)-C(O)-, wherein R₁₂ is alkyl as described earlier), phenyl or phenyl mono-, di- or tri-substituted, independently, by a radical selected from lower alkyl, lower alkoxy, halogen and trifluoromethyl. Exemplary of such aryl radicals are unsubstituted phenyl, naphthyl or 1,1'-biphenyl or, mono-, di-or tri-substituted independently, by a radical select from halogen, alkyl, alkoxy, acyloxyalkyl, phenyl and substituted phenyl, and trihaloalkyl. The term "aryloxy" denotes an unsubstituted or substituted aromatic hydrocarbon ether, in which the aryl group is defined as above. In the terms "arylalkyl", "arylalkoxy", "aryloxyalkyl" and "aryloxyalkoxy", the aryl, aryloxy, alkyl and alkoxy groups are as described above. The term "halogen" denotes the four halogens bromine, chlorine, fluorine and iodine.

The term "acyl" denotes an "alkanoyl" group, derived from an aliphatic carboxylic acid of 1 to 7 carbon atoms, for example, formyl, acetyl, propionyl and the like; or an "aroyl" group derived from an aromatic carboxylic acid, such as, benzoyl and the like. The term "acyloxy" denotes an "alkanoyloxy" group, derived from an aliphatic carboxylic acid of 1 to 7 carbon atoms, for example, formyloxy, acetyloxy, and propionyloxy. In the term "acyloxyalkyl", the terms acyloxy and alkyl are as described above. The term "cycloalkyl" denotes a cyclic radical of 3 to 10 carbon atoms, for example, cyclopropyl, cyclobutyl, cyclohexyl, cycloheptyl, cyclooctyl, and adamantyl. The term "heterocyclic radical" denotes a monocyclic 5-,6-or 7-membered heterocyclic or bi- or tricyclic heterocyclic radical containing one or more hetero atoms selected from nitrogen, oxygen or sulfur, which radical may be unsubstituted or substituted, independently, by one or more groups selected from alkyl, alkoxy, acyloxy, halogen, acyloxyalkyl, alkoxyalkyl, arylalkyl, arylalkoxy, aryloxyalkyl, aryloxyalkoxy, alkoxyalkoxy, hydroxy, hydroxyalkyl, hydroxyalkoxy, amino, lower alkylamino or di-lower alkylamino, cyano, nitro, mercapto, alkylthio, alkylsulfinyl, alkylsulfonyl, trihaloalkyl, sulfamoyl, N-alkylsulfamoyl, N,N-dialkylsulfamoyl, carboxycarbonyl, alkoxalyl (which has the formula R₁₂O-C(O)-C(O)-, wherein R₁₂ is alkyl, as described earlier), phenyl or phenyl mono-, di- or tri-substituted, independently, by a radical selected from lower alkyl lower alkoxy, halogen and trifluoromethyl. Exemplary of such heterocyclic radicals are pyridinyl, pyrimidyl, imidazolinyl, piperidinyl, morpholinyl, thienyl, furanyl, quinolyl, isoquinolyl, benzothienyl, indolyl, benzofuranyl, carbazolyl, chromanyl, isochromanyl, chromenyl, isochromenyl, naphthothienyl, phenothiazinyl, and xanthenyl.

A particular groups of compounds of formula I is one wherein one of R₄ and R₅ is hydrogen, lower alkyl or hydroxy-lower alkyl and the other is hydrogen or lower alkyl, X and Y taken together are O=, S= or, independently, when one of X and Y is halogen, the other is hydrogen, halogen, lower alkyl or aryl-lower alkyl; or when one of X and Y is amino, lower alkylamino or di-lower alkylamino, the other is hydrogen, lower alkyl or aryl-lower alkyl; or when one of X and Y is hydroxy, alkoxy or aryl-lower alkoxy, the other is hydrogen, hydroxy, lower alkyl, lower alkoxy or aryl-lower alkoxy; and the remaining symbols are as previously described.
A preferred group of compounds of formula **I** is one wherein R₁ is as previously described;
R₂ and R₂', independently, are hydrogen, lower alkyl, aryl, lower alkoxy, hydroxy, halogen, acyl or nitro;
A is a bond,
―O―, ―NR₇―, ―S―, 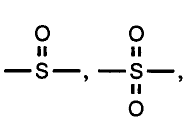
―C≡C―, ―HC=CH―, ―CH₂CH₂―, 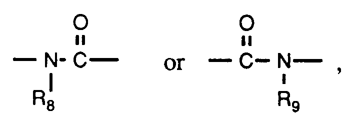
wherein R₇ is hydrogen, lower alkyl or acyl and R₈ and R₉ are, independently, hydrogen or lower alkyl;
B is a bond, ―O―, ―NR₇―, ―S―, ―C≡C― , ―HC=CH― , 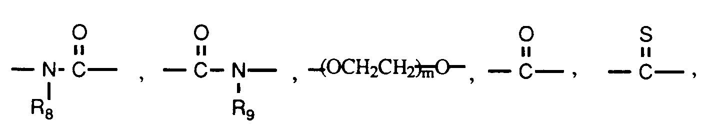 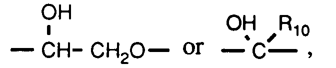
wherein R₇, R₈, R₉ and m are as previously described and R₁₀ is hydrogen;
n is an integer from 1 to 6, whereby when A is a bond or one of the following groups,
―O―, ―NR₇―, ―S―, 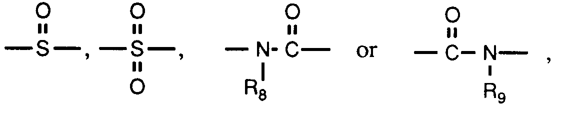
and B is a bond or one of the following groups
―O―, ―NR₇―, ―S―, 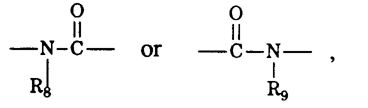
then n is different from the integer 1;
Z is ―S―, ―CR₂ = CR₂'―,―N=CR₂― or ―CR₂ =N―;
X and Y taken together are 0=, hydroxyimino, alkyloxyimino, alkenyloxyimino, arylalkoxyimino, hydrazono, mono-lower alkyl hydrazono, di-lower alkyl hydrazono or semicarbazono, or, independently, when one of X and Y is halogen, the other is hydrogen or halogen; or when one of X and Y is amino, the other is hydrogen; or when one of X and Y is hydroxy or alkoxy, the other is hydrogen or hydroxy;
Q is a cycloalkyl, aryl or heterocyclic radical, for example, phenyl, cyclohexyl, cyclooctyl, pyridinyl, adamantyl, 1,1'-biphenyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoinolyl, which radical can be substituted by one or more of the following groups: alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl, carboxycarbonyl or alkoxalyl.
A more preferred group of compounds of formula **I** is one wherein R₁ is as previously described; R₂ and R₂', independently, are hydrogen, lower alkyl, lower alkoxy, hydroxy or halogen;
A is a bond,
―O―, ―NR₇―, ―S―, 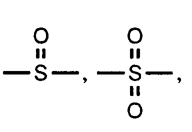
―C≡C―, ―HC=CH―, ―CH₂CH₂―, 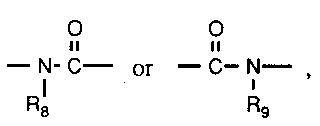
wherein R₇ is hydrogen or lower alkyl and R₈ and R₉ are hydrogen;
B is a bond,
―O―, ―S―, ―C≡C―, ―HC=CH―, 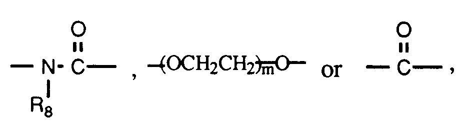
wherein m is 1 or 2 and R₈ is hydrogen;
n is an integer from 1 to 6, whereby when A is a bond or one of the following groups,
―O―, ―NR₇―, ―S―, 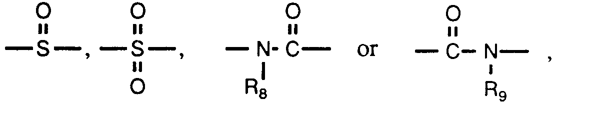
and B is a bond or one of the following groups
―O―, ―S― or 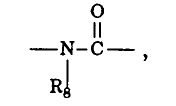
then n is different from the integer 1;
Z is ―S―, or ―CR₂ = CR₂'―,
X and Y taken together are 0= , hydroxyimino, alkyloxyimino or alkenyloxyimino, or, independently, when one of X and Y is halogen, the other is halogen; or when one of X and Y is amino, the other is hydrogen; or when one of X and Y is hydroxy , the other is hydrogen or hydroxy;
Q is phenyl, cyclohexyl, cyclooctyl, pyridinyl, adamaptyl, 1,1'-biphenyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, which radical can be substituted by one or more of the following groups; alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl carboxycarbonyl or alkoxalyl.

A still more preferred group of compounds of formula I is one wherein R₁ is hydroxy or 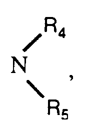
wherein one of R₄ and R₅ is hydrogen, lower alkyl or hydroxy-lower alkyl, and the other is hydroxy;
R₂ and R₂' are hydrogen;
A is ―O―, ―NR₇― or ―S―, wherein R₇ is hydrogen;
B is a bond,
―O―, ―S―, ―C≡C―, ―HC=CH―, 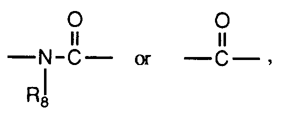
wherein R₈ is hydrogen;
n is an integer from 1 to 4, whereby when B is a bond or one of the following groups,
―O―, ―S― or 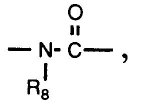
then n is different from the integer 1;
Z is ―S― or ―CR₂ = CR'₂―;
X and Y taken together are 0=, hydroxyimino, alkyloxyimino or alkenyloxyimino, or, independently, when one of X and Y is fluoro, the other is fluoro; or when one of X and Y is amino, the other is hydrogen; or when one of X and Y is hydroxy , the other is hydroxy; Q is phenyl, cyclohexyl, cyclooctyl, adamantyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, of which phenyl or naphthalenyl can be substituted by one or more of the following groups, lower alkyl, phenyl, acyloxy, acyloxyalkyl or hydroxyalkyl.
Preferred compounds of formula I are:
(S)-alpha-amino-4-[[2-(cyclooctyloxy)ethyl]oxy]benzeneacetic acid hydrochloride;
3-fluoro-4-[2-(2-naphthalenyloxy)ethoxy]-alpha-oxobenzeneacetic acid;
4-[2-(1-naphthalenyl)-2-oxoethoxy]-alpha-oxobenzeneacetic acid;
4-[[2-(2-naphthalenyloxy)ethyl]oxy]-alpha-oxobenzeneacetic acid 2-(dimethylamino)ethyl ester;
(E)-4-[3-[2-[(2,2-dimethyl-1-oxobutoxy)methyl]-6-methylphenyl]-2-propenyloxy]-alpha-oxobenzeneacetic acid(1:1) morpholine salt;
4-[[2-(2-naphthalenyloxy)ethyl]oxy]-alpha-oxobenzeneacetic acid;
4-[[2-(2-naphthalenyloxy)ethyl]thio]-alpha-oxobenzeneacetic acid;
4-[[2-(2-naphthalenylthio)ethyl]oxy]-alpha-oxobenzeneacetic acid;
4-[[2-cyclooctyloxy)ethyl]oxy]-alpha-oxobenzeneacetic acid;
4-[3-(4-acetyl-3-hydroxy-2-propylphenoxy)propoxy]-alpha-oxobenzeneacetic acid;
N-hydroxy-N-methyl-4-[2-(2-naphthalenyloxy)ethoxy]-alpha-oxobenzeneacetamide;
(Z)-alpha-(hydroxyimino)-5-[2-(2-naphthalenyloxy)ethoxy]-2-thiopheneacetic acid;
5-[[2-(2-naphthalenyloxy)ethyl]oxy]-alpha-oxo-2-thiopheneacetic acid;
alpha-oxo-4-[[2-(phenoxy)ethyl]oxy]benzeneacetic acid;
(Z)-alpha-(ethoxyimino)-5-[2-(2-naphthalenyloxy)ethoxy]-2-thiopheneacetic acid;
rac.-5-[[2-(2-naphthalenyloxy)ethyl]oxy]-alpha-oxo-2-thiopheneacetic acid 2,3-dihydroxypropyl ester;
(S)-alpha-amino-4-[2-[2-[(2,2-dimethyl-1-oxobutoxy)methyl]-6-methylphenoxy]ethoxy]benzeneacetic acid;
(E)-4-[[3-(2-naphthalenyl)-2-propenyl]oxy]-alpha-oxobenzeneacetic acid;
4-[[2-[2-[(2,2-dimethyl-1-oxobutoxy)methyl]-6-methylphenoxy]ethyl]oxy]-alpha-oxobenzeneacetic acid(2:1) hydrate; and
4-[[4-(2-naphthalenyloxy)butyl]oxy]alpha-oxobenzeneacetic acid.
Exemplary of other compounds of formula I of the invention are:
4-[2-(6-hydroxy-2-naphthalenyloxy)ethoxy]-alpha-oxobenzeneacetic acid;
4-[2-(8-hydroxy-2-naphthalenyloxy)ethoxy]-alpha-oxobenzeneacetic acid;
6-[2-(2-naphthalenyloxy)ethoxy]-alpha-oxo-3-pyridineacetic acid;
4-[2-[2,4-dichloro-6-[(2,2-dimethyl-1-oxobutoxy)methyl]phenoxy]ethoxy]-alpha-oxobenzeneacetic acid;
4-[2-[2,4-dimethyl-6-[(2,2-dimethyl-1-oxobutoxy)methyl]phenoxy]ethoxy]-alpha-oxobenzeneacetic acid;
4-[2-[2-[[[(4-fluorophenyl)carbonyl]oxy]methyl]phenoxy]ethoxy]alpha-oxobenzeneacetic acid;
5-[2-[2-[[[(4-fluorophenyl)carbonyl]oxy]methyl]phenoxy]ethoxy]-alpha-oxo-2-thiopheneacetic acid; and
5-[2-[2-[(methoxycarbonyl)-6-methyl]phenoxy]ethoxy]-alpha-oxo-2-thiopheneacetic acid.
Further exemplary of other compounds of formula I of the invention are:
(E)-4-[[3-(4-bromophenyl)-2-propenyl]oxy]-alpha-oxo-3-pyridineacetic acid;
(E)-4-[[4-(4-fluorophenyl)-2-butenyl]oxy]-alpha-oxobenzeneacetic acid;
(E)-alpha-oxo-4-[(3-phenyl-2-propenyl)oxy]thiopheneacetic acid;
(S)-alpha-amino-4-[[2-(2-naphthalenyloxy)ethyl]oxy]benzeneacetic acid;
(S)-alpha-amino-4-[[2-(phenoxy)ethyl]oxy]benzeneacetic acid hydrochloride;
(S)-alpha-amino-alpha-methyl-4[[2-(2-naphthalenyloxy)ethyl]oxy]benzeneacetic acid;
(Z)-alpha-oxo-5-[(3-phenyl-2-propenyl)oxy]-2-thiopheneacetic acid;
(E)-4-[[3-(1-naphthalenyl)-2-propenyl]oxy]-alpha-oxobenzeneacetic acid;
alpha-oxo-5-[4-(3-pyridinyl)butoxy]-2-thiopheneacetic acid;
alpha-oxo-4-[2-(4-pyridinyl)ethoxy]benzeneacetic acid;
alpha-oxo-4-[[7-(phenoxy)heptyl]oxy]benzeneacetic acid;
alpha-oxo-4-[[8-(phenoxy)octyl]oxy]benzeneacetic acid;
alpha-oxo-5-[[2-(4-phenoxyphenoxy)ethyl]oxy]-2-furanacetic acid;
alpha-oxo-4-[[3-(4-quinolyloxy)propyl]oxy]benzeneacetic acid;
2-[[4-[[4-(1-naphthalenyloxy)butyl]oxy]-alpha-oxobenzeneacetyl]oxy]-N,N,N-trimethylethanaminium iodide;
5-[3-(2-naphthalenyloxy)propyl]-alpha-oxo-2-thiopheneacetic acid;
4-[[4-(1-naphthalenyloxy)butyl]oxy]-alpha-oxobenzeneacetic acid;
4-[[2-(2-chlorophenoxy)ethyl]oxy]-alpha-oxobenzeneacetic acid;
4-[[2-(2-fluorophenoxy)ethyl]oxy]-alpha-oxo-3-pyridineacetic acid;
4-[[2-(2-naphthalenyl)ethyl]amino]-alpha-oxobenzeneacetic acid;
5-[[2-(2-naphthalenyloxy)ethyl]oxy]-alpha-oxo-2-thiopheneacetamide;
4-[[3-(2-naphthalenyloxy)propyl]sulfonyl]-alpha-oxobenzeneacetic acid;
4-[[3-(2-naphthalenylthio)propyl]oxy]-alpha-oxobenzeneacetic acid;
5-[[2-(3,4,5-trimethoxyphenoxy)ethyl]oxy]-alpha-oxo-2-furanacetic acid;
4-[[2-(6-methoxy-2-naphthalenyloxy)ethyl]oxy]alpha-oxobenzeneacetic acid;
4-[[2-(3-naphtho[2,3-b]thienyloxy)ethyl]oxy]-alpha-oxobenzeneacetic acid;
4-[[2-(7-isoquinolyl)ethyl]oxy]-alpha-oxobenzeneacetic acid;
4-[[3-(cyclohexyl)propyl]oxy]-alpha-oxobenzeneacetic acid;
4-[[3-(cyclooctyloxy)propyl]oxy]-alpha-oxobenzeneacetic acid;
4-[[2-[2-(hydroxymethyl)phenoxy]ethyl]oxy]-alpha-oxobenzeneacetic acid;
4-[[2-[2-[(2,2-dimethyl-1-oxopropoxy)methyl]-6-methylphenoxy] ethyl]oxy]-alpha-oxobenzeneacetic acid;
4-[[2-[4-(methylaminosulfonyl)phenoxy]ethyl]oxy]-alpha-oxobenzeneacetic acid
4-[[2-[8-(2,2-dimethyl-1-oxopropoxy)-2-naphthalenyloxy]ethyl]oxy]-alpha-oxobenzeneacetic acid;
4-[[3-(2-dimethylaminophenyl)propyl]oxy]-alpha-oxobenzeneacetic acid:
5-[[4-(2-naphthalenyl)butyl]oxy]-alpha-oxo-2-thiopheneacetic acid;
4-[[3-(4-cyanophenyl)propyl]oxy]-alpha-oxobenzeneacetic acid
4-[[3-(4-nitrophenoxy)propyl]oxy]-alpha-oxobenzeneacetic acid;
4-[[3-[2(trifluoromethyl)phenyl]propyl]oxy]-alpha-oxobenzeneacetic acid;
4-[[3-[4-(N,N-dimethylsulfamoyl)phenoxy]ethyl]oxy]-alpha-oxobenzeneacetic acid;
4-[[3-[4-(N-ethylsulfamoyl)phenoxy]ethyl]oxy]-alpha-oxobenzeneacetic acid;
alpha-oxo-4-[[4-(phenylthio)butyl]oxy]benzeneacetic acid;
alpha-oxo-4-[[4-(2-pyrimidyl)butyl]oxy]benzeneacetic acid;
4-[[4-(3-fluorophenoxy)butyl]oxy]-alpha-oxobenzeneacetic acid;
4-[[5-(4-fluorophenoxy)pentyl]oxy]-alpha-oxobenzeneacetic acid;
4-methyl-5-[[2-(2-naphthalenyloxy)ethyl]oxy]-alpha-oxo-2-thiopheneacetic acid;
5-[(phenylmethyl)oxy]-alpha-oxo-2-furanacetic acid;
5-[[2-(2-naphthalenyloxy)ethyl]oxy]-alpha-oxo-2-thiopheneacetic acid 2-(dimethylamino)ethyl ester;
5-[[2-(3-carbazolyloxy)ethyl]oxy]-alpha-oxo-2-thiopheneacetic acid;
5-[[2-(cyclooctyloxy)ethyl]thio]-alpha-oxo-2-thiopheneacetic acid;
5-[[3-(cycloheptyloxy)propyl]oxy]-alpha-oxo-2-thiopheneacetic acid potassium salt;
alpha, alpha-difluoro-4[[2-(3-methylphenoxy)ethyl]oxy]benzeneacetic acid;
alpha, alpha-difluoro-4[[2-(3-naphtho[2,3-b]thienyloxy)ethyl]oxy]benzeneacetic acid;
alpha, alpha-dimethoxy-4-[[2-(phenoxy)ethyl]oxy]benzeneacetic acid
alpha-oxo-4-[(3-phenyl-2-propynyl)oxy]thiopheneacetic acid;
alpha-oxo-4-[[2-(2-xanthenyloxy)ethyl]oxy]benzeneacetic acid (1:1) diethanolamine salt;
alpha-oxo-4-[[2-(4-trifluoromethylphenoxy)ethyl]oxy]-2-furanacetic acid;
alpha-oxo-4-[[6-(phenoxy)hexyl]oxy]benzeneacetic acid;
4-[[2-(3-benzo[b]thienyloxy)ethyl]oxy]-alpha-oxobenzeneacetic acid
rac.-5-[[2-(3-indolyloxy)ethyl]oxy]-alpha-oxo-2-thiopheneacetic acid 2,3-dihydroxypropyl ester;
rac.-alpha-chloro-alpha-methyl-4-[[2-(phenoxy)ethyl]oxy]benzeneacetic acid;
rac.-alpha-ethoxy-4-[(2-phenylethyl)oxy]benzeneacetic acid;
rac.-alpha-oxo-4-[[2-(1,2,3,4-tetrahydro-2-naphthalenyloxy)ethyl]oxy]benzeneacetic acid and the like.

In accordance with the present invention, the compounds of formula I and their pharmaceutically acceptable salts can be prepared by a process which comprises
(a) for the manufacture of compounds of formula I wherein R₁ is hydroxy and the remaining symbols are as previously described, saponifying a compound of the general formula 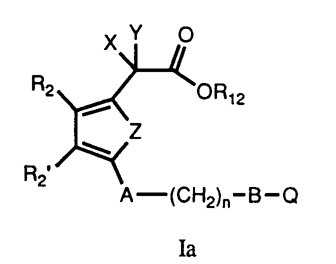 wherein A, B, Q, R₂, R₂', X, Y, Z and n are as previously described and R₁₂ is lower alkyl,
   or
(b) for the manufacture of compounds of formula I wherein R₁ is OR₃ and the remaining symbols are as previously described, treating a compound of the general formula 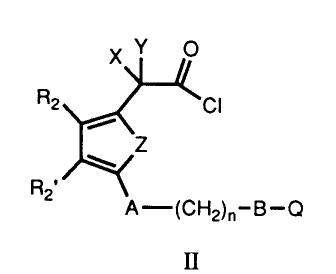 wherein A, B, Q, R₂, R₂', X, Y, Z and n are as previously described, with an alcohol of the general formula

   HO-R₃ IV

   wherein R₃ is as previously described,
   or
(c) for the manufacture of compounds of formula I wherein R₁ is 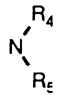 and the remaining symbols are as previously described, treating a compound of the general formula 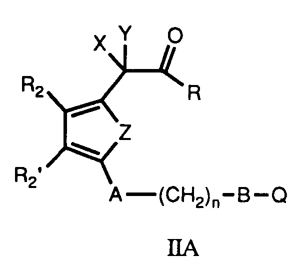 wherein A, B, Q, R₂, R₂', X, Y, Z and n are as previously described and R is chloro or lower alkoxy,
   with an amine of the general formula 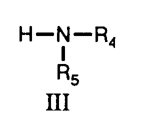 wherein R₄ and R₅ are as previously described,
   or
(d) for the manufacture of compounds of formula I wherein R₁ is hydroxy, A is -O- or -S-, X and Y taken together are O= and Z is -CR₂=CR₂'- and the remaining symbols are as previously described, reacting a compound of the general formula 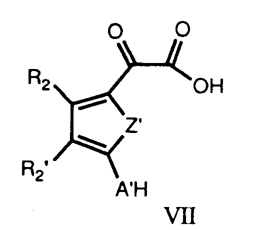 wherein R₂ and R₂' are as previously described and A' is -O- or -S- and Z' is -CR₂=CR₂'-
   with a compound of the general formula 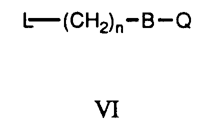 wherein B, Q and n are as previously described and L is a leaving group,
   or
(e) for the manufacture of compounds of formula I wherein R₁ is hydroxy, A is -O- or -S-, X and Y taken together are O= and Z is -CR₂=CR₂'- and the remaining symbols are as previously described, oxidizing a compound of the general formula 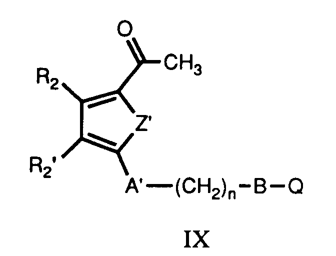 wherein A', B, Q, R₂, R₂', Z' and n are as previously described,
   or
(f) for the manufacture of compounds of formula I wherein R₁ is hydroxy, A is 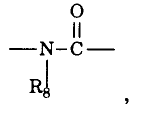 X and Y taken together are O= and Z is -CR₂=CR₂'- and the remaining symbols are as previously described, oxidizing a compound of the general formula 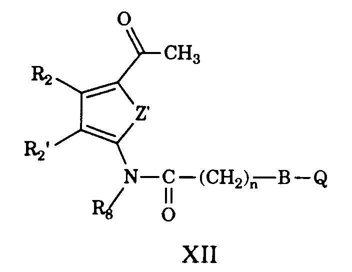 wherein B, Q, R₂, R₂', R₈, Z' and n are as previously described,
   or
(g) for the manufacture of compounds of formula I wherein R₁ is hydroxy, A is -O-, X is amino and Z is -CR₂=CR₂'- and the remaining symbols are as previously described, splitting off the amino acid O-protecting group from a compound of the general formula 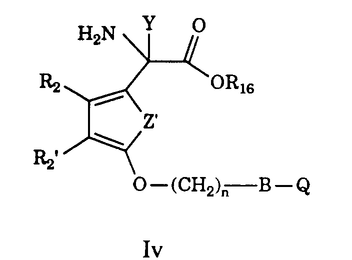 wherein B, Q, R₂, R₂', Y and n are as previously described and R₁₆ is an amino acid O-protecting group,
   and, if desired,
(h) converting a compound obtained into a pharmaceutically acceptable salt.

The reaction conditions for the above process variants are described in more detail hereinafter in Reaction Schemes I, III, IV, V and XII. The starting materials can be prepared as described in Reaction Schemes I to XV or in an analogous manner. The compounds of formulae III, IV, XV, XVI, XVIII and XX are known compounds or can be prepared in an analogous manner as the known compounds. 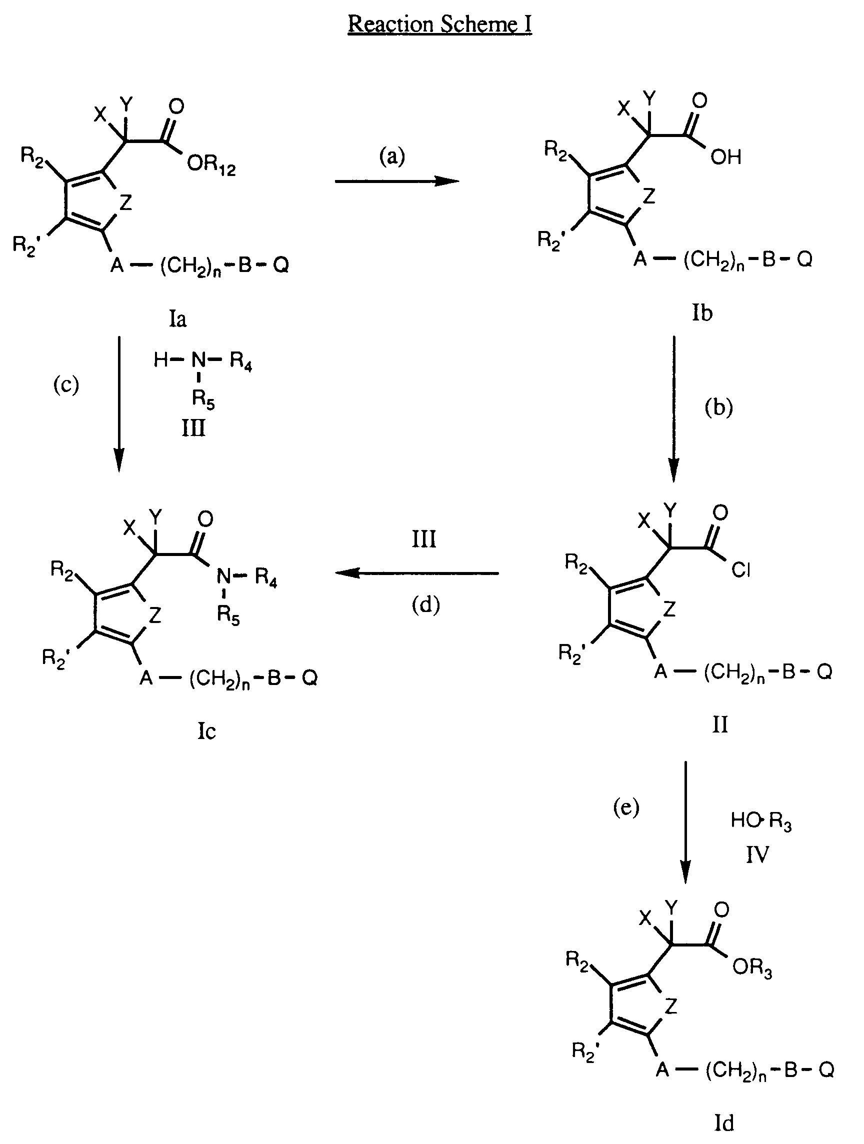
wherein A, B, Q, R₂, R₂', R₃, R₄, R₅, R₁₂, X, Y, Z and n are as previously described.

In Reaction Scheme I, step (a), an ester of formula Ia, which is prepared by methods hereinafter described, which includes as appropriate, its enantiomeric, diastereoisomeric or geometric isomers and/or mixtures thereof is reacted with an excess of an alkali metal hydroxide in a solvent mixture, preferably methanol-water or methanol-tetrahydrofuran-water, at a temperature of from 0 °C to reflux. The resulting carboxylic acid of formula Ib which includes as appropriate, its enantiomeric, diastereoisomeric or geometric isomers and/or mixtures thereof may be isolated utilizing conventional methods such as crystallization, crystallization of salts, chromatography and the like.

In step (b), a carboxylic acid of formula Ib, which includes as appropriate, its enantiomeric, diastereoisomeric or geometric isomers and/or mixtures thereof, is dissolved in a suitably inert solvent, for example, dichloromethane or toluene, optionally containing a catalytic amount of dimethylformamide, and treated with a carboxylic acid halide forming reagent such as oxalyl chloride at a temperature of from -80 °C to the reflux temperature of the mixture. The resulting carboxylic acid chloride of formula II which includes as appropriate, its enantiomeric, diastereoisomeric or geometric isomers and/or mixtures thereof, may be isolated utilizing conventional methods such as distillation, crystallization and the like, but, most conveniently, after removal of the reaction solvent, the crude product of formula II can be used per se in subsequent steps.

In step (c), a carboxylic acid ester of formula Ia, which includes as appropriate, its enantiomeric, diastereoisomeric or geometric isomers and/or mixtures thereof, is reacted with an excess of the amine of formula III, optionally in a mixture of solvents, preferably a lower alkanol-tetrahydrofuran, such as methanol-tetrahydrofuran, at a temperature of from room temperature to the reflux temperature of the mixture. The resulting carboxylic acid amide of formula Ic which includes as appropriate, its enantiomeric or diastereoisomeric isomers or mixture thereof or geometric isomers or mixture thereof may be isolated utilizing conventional methods such as crystallization, crystallization of salts, chromatography and the like.

Alternatively, in step (d), a solution of the carboxylic acid chloride of formula II, which includes as appropriate, its enantiomeric, diastereoisomeric or geometric isomers and/or mixtures thereof in an inert solvent, for example, a chlorinated hydrocarbon, such as dichloromethane, is added to a solution of an excess of the amine of formula III in an inert solvent, for example, dichloromethane, optionally in the presence of a proton acceptor, for example triethylamine, at a temperature in the range of from -78 °C to room temperature. The resulting carboxylic acid amide of formula Ic which includes as appropriate, its enantiomeric, diastereoisomeric or geometric isomers and/or mixtures thereof may be isolated utilizing conventional methods such as crystallization, crystallization of salts, chromatography and the like.

In step (e), a solution of the carboxylic acid chloride of formula II, which includes as appropriate, its enantiomeric, diastereoisomeric or geometric isomers and/or mixtures thereof in an inert solvent, for example dichloromethane, is added to a solution of an excess of the alcohol of formula IV, an inert solvent, for example, dichloromethane, in the presence of a proton acceptor, for example, triethylamine, at a temperature of from -78 °C to room temperature. The resulting carboxylic acid ester of formula Id which includes as appropriate, its enantiomeric, diastereoisomeric or geometric isomers and/or mixtures thereof may be isolated utilizing conventional methods such as crystallization, crystallization of salts, chromatography and the like. 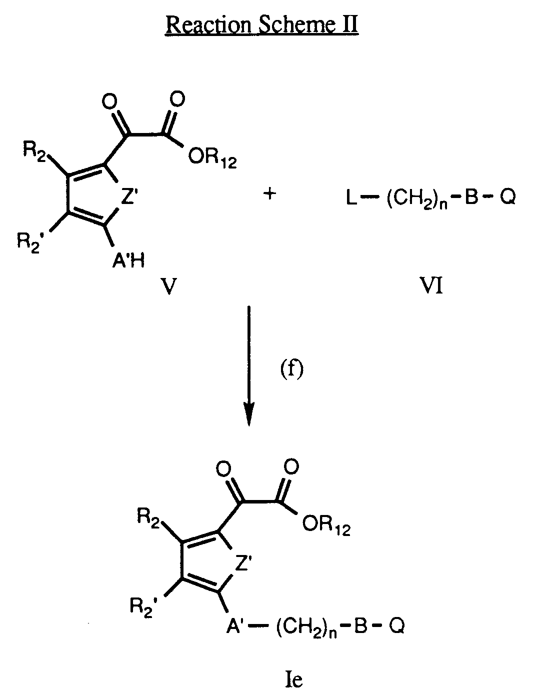
wherein n, B, Q, R₂, R₂' and R₁₂ are as previously described, A' is O or S and Z' is -CR₂=CR₂'- and L is a leaving group such as chloro, bromo, iodo, alkylsulfonyloxy or arylsulfonyloxy.

In Reaction Scheme II, step (f), a phenol or thiophenol of formula V which is known or can be made by known methods, is reacted with a compound of formula VI, which is known or can be made by known methods, and which includes as appropriate, its enantiomeric, diastereoisomeric or geometric isomers and/or mixtures thereof, in the presence of an alkali metal hydride, for example, sodium hydride, in an inert solvent, preferably dimethylformamide or dimethylsulfoxide, at a temperature of from 0 °C to 100 °C. The resulting compound of formula Ie which includes its enantiomeric or geometric isomers and/or mixtures thereof may be isolated utilizing conventional methods such as crystallization, chromatography and the like. 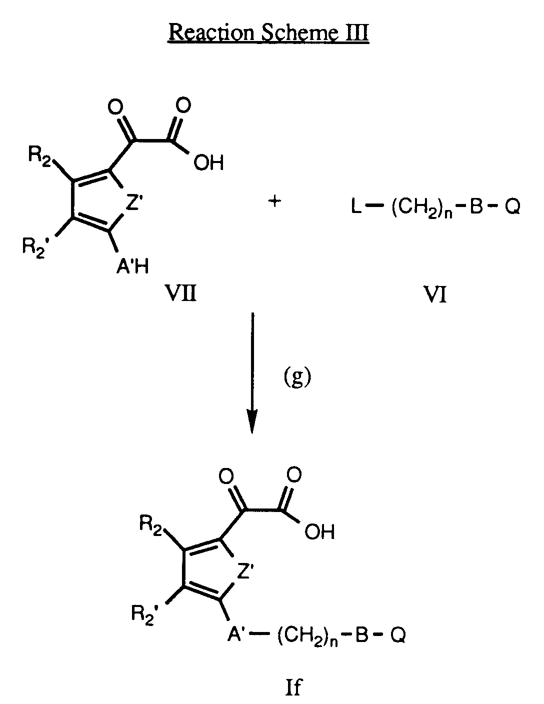
wherein n, R₂, R₂', A', Z', B, Q, and L are as previously described.

In Reaction Scheme III, step (g), a phenol or thiophenol of formula VII which is known or can be made by known methods, is reacted with a compound of formula VI, in the presence of an alkali metal hydroxide, for example sodium hydroxide, in an inert solvent mixture, for example dimethylsulfoxide-water, at a temperature of from 0 °C to 100 °C. The resulting compound of formula If which includes as appropriate, its enantiomeric, diastereoisomeric or geometric isomers and/or mixtures thereof, may be isolated utilizing conventional methods such as crystallization, crystallization of salts, chromatography and the like. 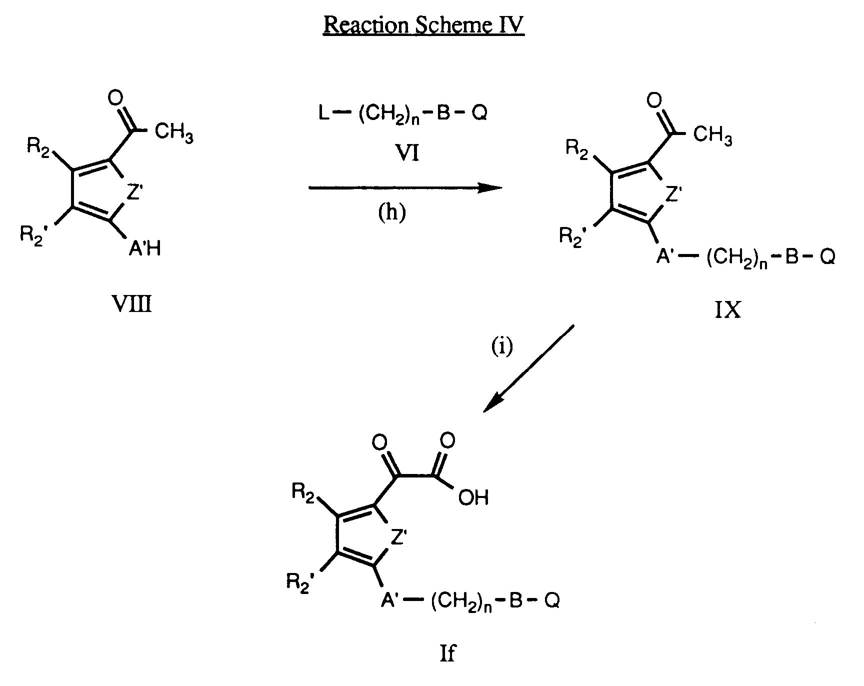
wherein A', n, B, Q, R₂, R₂', Z' and L are as previously described.

In Reaction Scheme IV , step (h), a phenol or thiophenol of formula VIII, which is known or can be made by known methods, is reacted with an alkylating agent of formula VI, in the presence of an alkali metal hydride in an inert solvent, for example dimethylformamide, at a temperature of from 0 °C to 100 °C. The resulting compound of formula IX which includes as appropriate, its enantiomers, diastereoisomers or geometric isomers and/or mixtures thereof, may be isolated utilizing conventional methods such as crystallization, chromatography and the like.

In step (i), an aryl methyl ketone of formula IX is reacted with an excess of an oxidizing agent, preferably selenium dioxide in the presence of a tertiary amine, preferably pyridine, at a temperature of from 60 °C to 100 °C to give the alpha-ketocarboxylic acid of formula If which includes as appropriate, its enantiomeric, diastereoisomeric or geometric isomers and/or mixtures thereof. The compound may be isolated utilizing conventional methods such as crystallization, crystallization of salts, chromatography and the like. 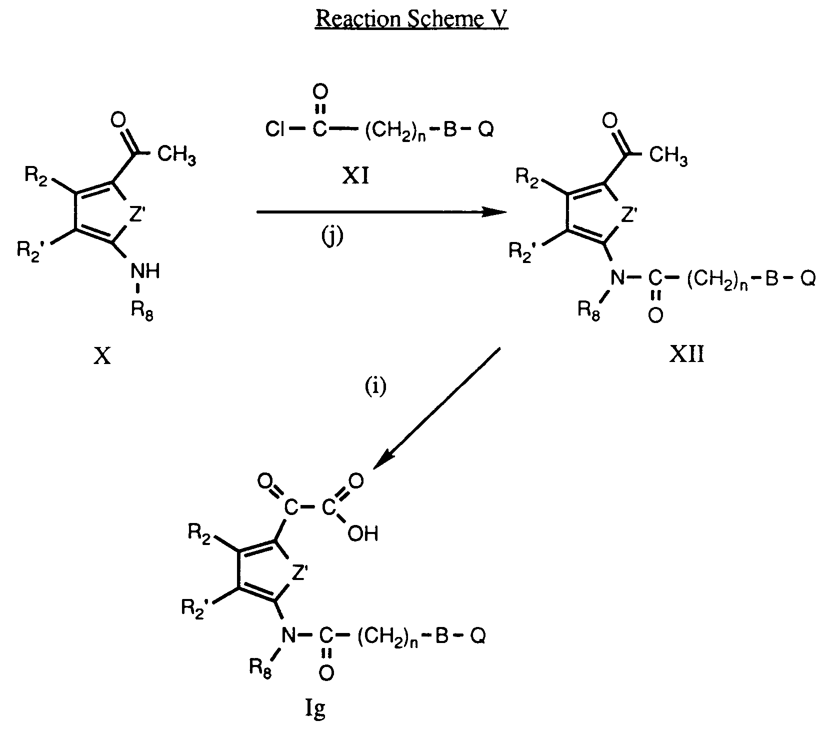
wherein n, B, Q, R₂, R₂', R₈ and Z' are as previously described.

In Reaction Scheme V, step (j), an amine of formula X, which is known or can be prepared by known methods, is reacted with an excess of the carboxylic acid chloride of formula XI, which is known or can be prepared by known methods and which includes as appropriate, its enantiomeric, diastereoisomeric or geometric isomers and/or mixtures thereof, in the presence of a proton acceptor, preferably pyridine, or triethylamine, in a suitably inert solvent such as dichloromethane at a temperature of from -78 °C to ambient temperature. The resulting carboxamide of formula XII which includes as appropriate, its enantiomeric, diastereoisomeric or geometric isomers and/or mixtures thereof, may be isolated utilizing conventional methods such as crystallization, chromatography and the like.

The compound of formula XII is transformed under conditions previously described in step (i) of Reaction Scheme IV into the alpha-ketocarboxylic acid of formula Ig. The resulting compound of formula Ig which includes as appropriate, its enantiomeric, diastereoisomeric or geometric isomers and/or mixtures thereof, may be isolated utilizing conventional methods such as crystallization, crystallization of salts, chromatography and the like. 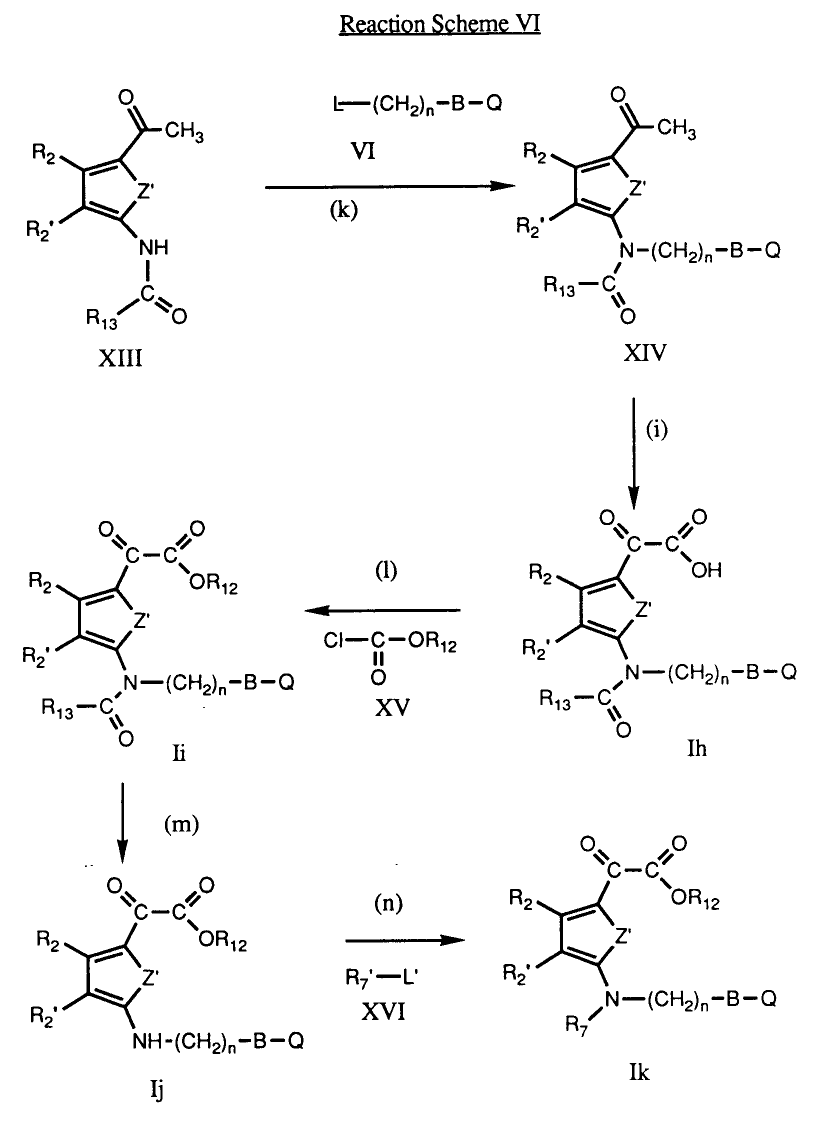
wherein n, B, L, Q, R₂, R₂', R₁₂ and Z' are as previously described, L' is chloro or bromo, R₇' is lower alkyl and R₁₃ is hydrogen, lower alkyl or arylalkyl.

In Reaction Scheme VI, step (k), an N-acylated compound of formula XIII, which is known or can be prepared by known methods, is reacted with a compound of formula VI, in the presence of an alkali metal hydride in an inert solvent, preferably dimethylformamide, at a temperature of from 0 °C to 100 °C. The resulting compound of formula XIV which includes as appropriate its enantiomeric, diastereoisomeric or geometric isomers and/or mixtures thereof may be isolated utilizing conventional methods such as crystallization, crystallization of salts, chromatography and the like.

The compound of formula XIV is transformed under conditions previously described in step (i) of Reaction Scheme IV into a alpha-keto carboxylic acid of formula Ih. The resulting compound of formula Ih which includes as appropriate, its enantiomeric, diastereoisomeric or geometric isomers and/or mixtures thereof, may be isolated utilizing conventional methods such as crystallization, crystallization of salts, chromatography and the like. Optionally, the reaction mixture containing the alpha-ketocarboxylic acid of formula Ih may be treated in step (l) directly with an aryl or alkyl chloroformate (XV), for example, methyl chloroformate. The resulting alpha-ketocarboxylic acid ester of formula Ii which includes as appropriate, its enantiomeric, diastereoisomeric or geometric isomers and/or mixtures thereof, may be isolated utilizing conventional methods such as crystallization, chromatography and the like.

In step (m), the compound of formula Ii is deacylated by treatment with an excess of aqueous mineral acid, for example, hydrochloric acid or sulfuric acid optionally in the presence of an inert solvent, such as methanol or glyme, at a temperature of from 50 °C to 100 °C. The resulting amine of formula Ij which includes as appropriate, its enantiomeric, diastereoisomeric or geometric isomers and/or mixtures thereof, may be isolated utilizing conventional methods such as crystallization, crystallization of salts, chromatography and the like.

In step (n), the secondary amine of formula Ij is reacted with an alkylating agent XVI, for example, methyl iodide, in the presence of an inert solvent, for example, diethyl ether or methanol, at a temperature of from room temperature to reflux. The resulting tertiary amines of formula Ik which includes as appropriate, its enantiomeric, diastereoisomeric or geometric isomers and/or mixtures thereof, may be isolated utilizing conventional methods such as crystallization, crystallization of salts,chromatography and the like. 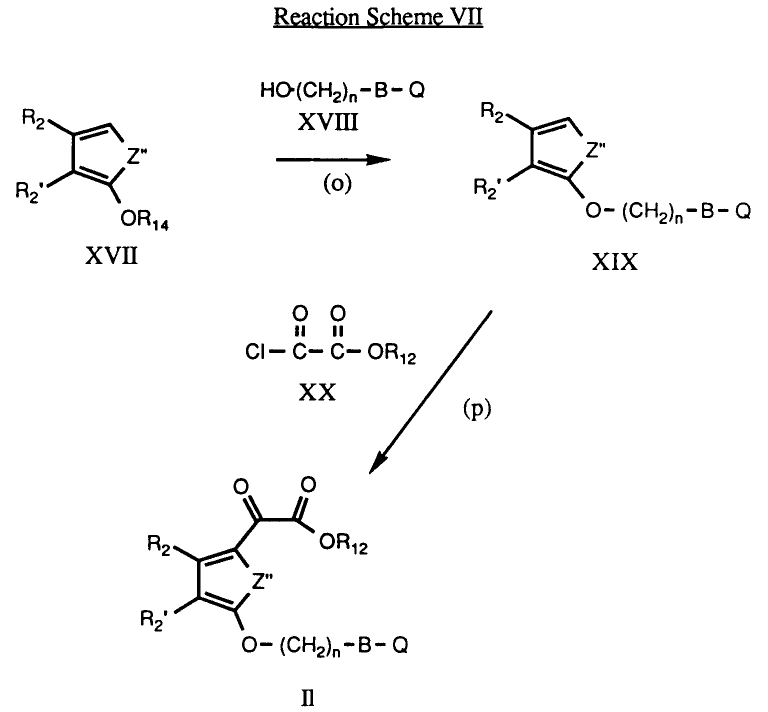
wherein n, B, Q, R₂, R₂' and R₁₂ are as previously described, R₁₄ is methyl or ethyl and Z'' is O or S.

In Reaction Scheme VII, step (o), a 2-furanyl or 2-thienyl ether, unsubstituted in position 5, of formula XVII, which is known or can be prepared by known methods, is reacted with an alcohol of formula XVIII, which is known or can be prepared by known methods, and which includes as appropriate, its enantiomeric, diastereoisomeric or geometric isomers or mixture thereof, in an inert solvent, for example, dichloromethane or benzene in the presence of a catalytic amount of an aryl- or alkylsulfonic acid, for example, p-toluenesulfonic acid and in the presence of an agent that will absorb lower alkanols from the reaction mixture as they are formed, for example, 4Å or 5Å molecular sieves, at a temperature of from room temperature to reflux. The resulting ether of formula XIX, which includes as appropriate, its enantiomeric, diastereoisomeric or geometric isomers and/or mixtures thereof, may be isolated utilizing conventional methods such as crystallization, chromatography and the like.

In step (p), the compound of formula XIX is reacted with an excess of an oxalyl chloride ester of formula XX, in the presence of a tertiary amine base, preferably pyridine, in a suitably inert solvent ,such as, dichloromethane at a temperature of from O °C to reflux. The resulting compound of formula Il, which includes as appropriate, its enantiomeric, diastereoisomeric or geometric isomer or mixture thereof, may be isolated utilizing conventional methods such as crystallization, chromatography and the like.

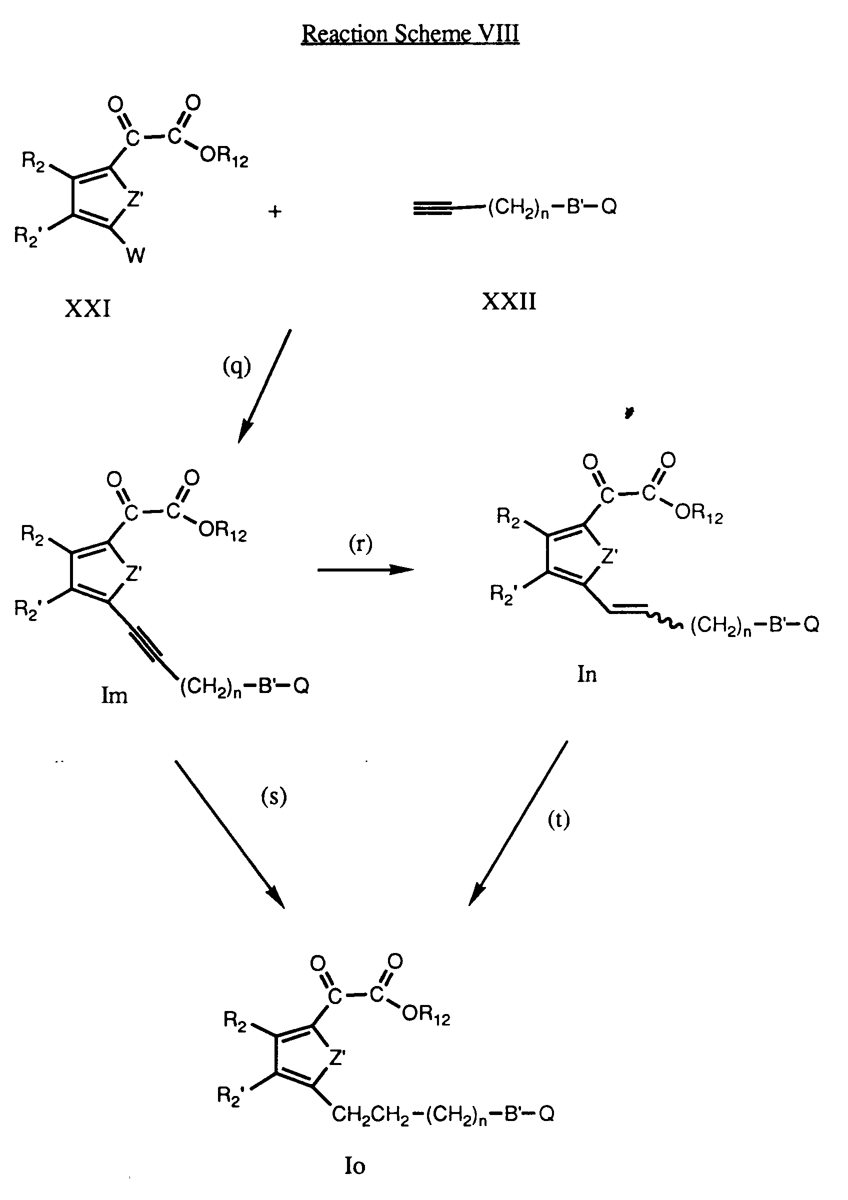

wherein n, Q, R₂, R₂', R₁₂ and Z' are as previously described, and B' is other than -C≡C- or -HC=CH- and W is bromo, iodo or a perfluoroalkylsulfonate.

In Reaction Scheme VIII, step (q), a compound of formula XXI, which is known or can be prepared by known methods, is reacted with an acetylene of formula XXII, a compound which is known or can be prepared by known methods and which includes, as appropriate, its enantiomeric, diastereoisomeric or geometric isomers and/or mixtures thereof, in the presence of an excess of a proton acceptor, for example triethylamine, and a suitable palladium catalyst, for example bis(triphenylphosphine)palladium dichloride, optionally in an inert solvent, for example, dichloromethane or dimethylformamide, at a temperature of from room temperature to 100 °C. The resulting compound of formula Im, which includes as appropriate, its enantiomeric, diastereoisomeric or geometric isomers and/or mixtures thereof, may be isolated utilizing conventional methods such as crystallization, chromatography and the like.

In step (r), the acetylene of formula Im, is dissolved in an inert solvent, for example, benzene or tetrahydrofuran, and hydrogenated over a suitably deactivated palladium catalyst, for example, Lindlar catalyst or the like, at ambient temperature and at from one to three atmospheres pressure until one equivalent of hydrogen is absorbed. The resulting olefin of formula In, which includes as appropriate, its enantiomeric, diastereoisomeric or geometric isomers and/or mixtures thereof, may be isolated utilizing conventional methods such as crystallization, chromatography and the like.

In step (s), the acetylene of formula Im, is dissolved in an inert solvent, for example methanol or tetrahydrofuran, and hydrogenated over a suitable catalyst, for example, palladium on carbon or platinum oxide, at from one to five atmospheres pressure, preferably at ambient temperature until the uptake of hydrogen had ceased. The resulting compound of formula Io, which includes as appropriate, its enantiomeric, diastereoisomeric or geometric isomers and/or mixtures thereof, may be isolated utilizing conventional methods such as crystallization, chromatography and the like.

In step (t), the olefin of formula In, which includes as appropriate, its enantiomeric, diastereoisomeric or geometric isomers and/or mixtures thereof, is dissolved in an inert solvent, for example, methanol or tetrahydrofuran, and hydrogenated over a suitable catalyst, for example, palladium on carbon or platinum oxide, at from one to five atmospheres pressure, preferably at ambient temperature until the uptake of hydrogen had ceased. The resulting compound of formula Io may be isolated utilizing conventional methods such as crystallization, chromatography and the like. 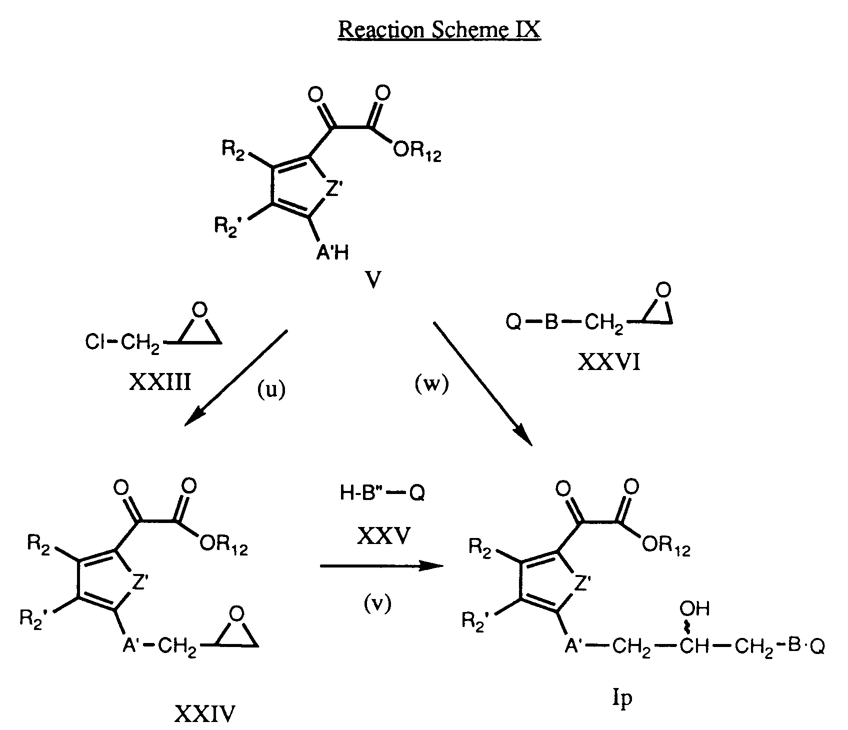
wherein A', B, Q, R₂, R₂', R₁₂ and Z' are as previously described and B'' is O, S or NR₇ wherein R₇ is as previously described.

In Reaction Scheme IX, step (u) a phenol or thiophenol of formula V is reacted with the compound of formula XXIII, epichlorohydrin, which includes its enantiomers and/or mixtures thereof, in the presence of an alkali metal hydride, for example sodium hydride in an inert solvent, for example, dimethylformamide, at a temperature of from room temperature to 100 °C. The resulting epoxide of formula XXIV, which includes its enantiomers and/or mixtures thereof, may be isolated utilizing conventional methods such as crystallization, chromatography and the like.

In step (v), the compound of formula XXIV is optionally dissolved in an inert solvent and reacted with a compound of formula XXV, which is known or can be prepared by known methods, and which includes, as appropriate, its enantiomeric, diastereoisomeric or geometric isomers or mixtures thereof, in the presence of a proton acceptor, preferably a tertiary amine, for example pyridine, at a temperature of from 40 °C to 100 °C. The resulting compound of formula Ip, which includes its enantiomers, and, as appropriate, its diastereoisomeric or geometric isomers and/or mixtures thereof, may be isolated utilizing conventional methods such as crystallization, crystallization of salts, chromatography and the like.

Alternatively, in step (w), a phenol or thiophenol of formula V is reacted with an epoxide of formula XXVI, which is known or can be prepared by known methods, which includes, as appropriate, its enantiomeric, diastereoisomeric or geometric isomers and/or mixtures thereof, in the presence of a catalytic amount of an alkali metal hydroxide, for example potassium hydroxide, or an alkali metal alkoxide, for example sodium methylate, in an inert solvent, for example dimethylformamide, at a temperature of from room temperature to 100 °C. The resulting compound of formula Ip, which includes, as appropriate its enantiomeric, diastereoisomeric or geometric isomers and/or mixtures thereof, may be isolated utilizing conventional methods such as crystallization, chromatography and the like. 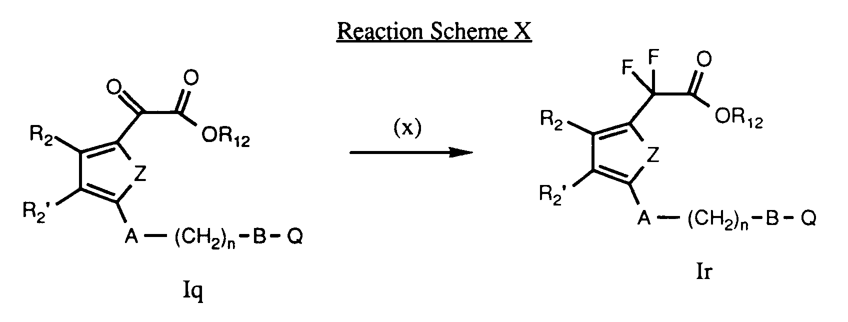
wherein A, B, Q, R₂, R₂', R₁₂, Z and n are as previously described.

In Reaction Scheme X, step (x), an alpha-ketocarboxylic acid ester of formula Iq, which is or can be prepared according to one of the methods described herein, for example, in Reaction Scheme II, VI, VII, VIII or IX, and which includes, as appropriate, its enantiomeric, diastereoisomeric or geometric isomers and/or mixtures thereof, is reacted with an excess of diethylaminosulfur trifluoride in an inert solvent for example 1,2-dichloroethane, at a temperature of from room temperature to reflux. The compound of formula Ir which includes its diastereoisomeric or geometric isomers and/or mixtures thereof, may be isolated utilizing conventional methods such as crystallization, crystallization of salts, chromatography and the like. 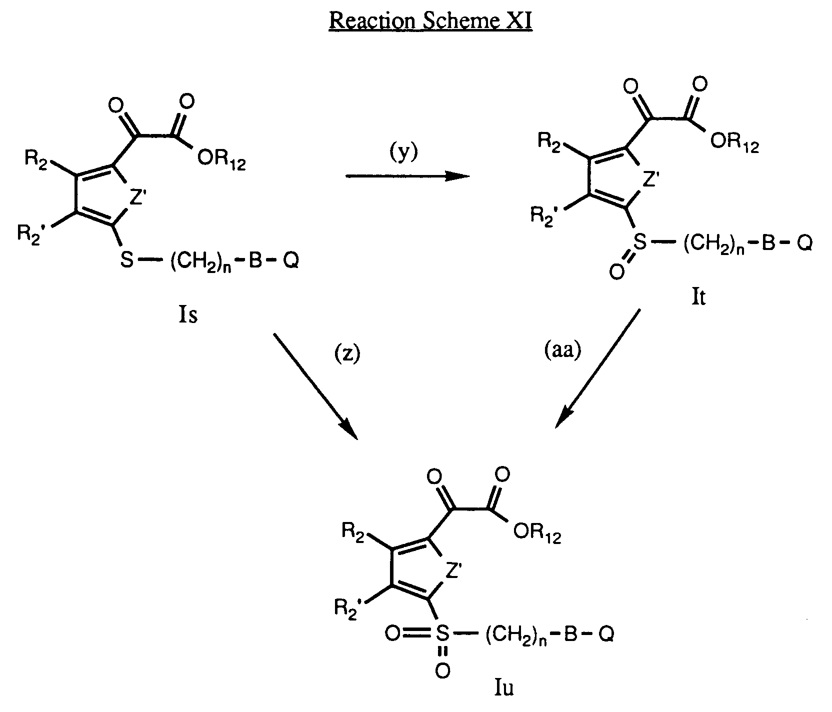
wherein B, Q, R₂, R₂', R₁₂, Z' and n are as previously described.

In Reaction Scheme XI, step (y), a sulfide of formula Is, which is or can be prepared according to the methods described herein, for example, Scheme II which includes, as appropriate, its enantiomeric, diastereoisomeric or geometric isomers and/or mixtures thereof, is reacted with one equivalent of a peracid, preferably m-chloroperbenzoic acid in an inert solvent, for example dichloromethane, at a temperature of from -20 °C to 0 °C. The resulting sulfoxide of formula It, which includes, as appropriate, its enantiomeric, diastereoisomeric or geometric isomers and/or mixtures thereof, may be isolated utilizing conventional methods such as crystallization, crystallization of salts, chromatography and the like.

In step (z), a sulfide of formula Is, which includes, as appropriate, its enantiomeric, diastereoisomeric or geometric isomers and/or mixtures thereof, is reacted with an excess i.e. > two equivalents of a peracid, preferably m-chloroperbenzoic acid in an inert solvent, for example dichloromethane, at a temperature of from 0 °C to room temperature. The resulting sulfone of formula Iu, which includes, as appropriate, its enantiomeric, diastereoisomeric or geometric isomers and/or mixtures thereof, may be isolated utilizing conventional methods such as crystallization, crystallization of salts, chromatography and the like.

Alternatively, in step (aa) a sulfoxide of formula It, is reacted with an excess of a peracid, preferably m-chloroperbenzoic acid in an inert solvent, for example, dichloromethane at a temperature of from 0 °C to room temperature to give the sulfone of formula Iu. 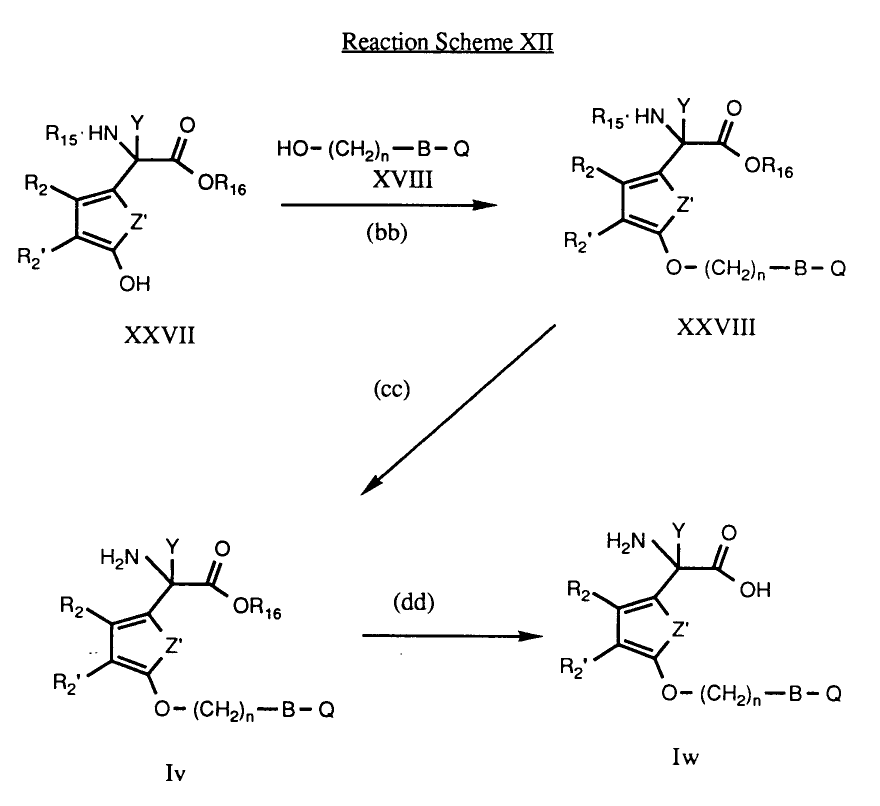
wherein B, Q, R₂, R₂', Y, Z' and n are as previously described, R₁₅ is a suitable amino acid N-protecting group and R₁₆ is a suitable amino acid O-protecting group.

In Reaction Scheme XII, step (bb) an appropriately O,N-diprotected amino acid of formula XXVII, which is known or can be prepared by known methods, and which includes its enantiomers and/or mixtures thereof, is reacted with an alcohol of formula XVIII, in the presence of a triaryl or trialkylphosphine, for example, triphenylphosphine and a coupling reagent, for example diethyl azodicarboxylate, in an inert solvent, for example tetrahydrofuran, at a temperature of from 0 °C to room temperature. The resulting compound of formula XXVIII, which includes, as appropriate, its enantiomeric, diastereoisomeric or geometric isomers and/or mixtures thereof, may be isolated utilizing conventional methods such as crystallization, crystallization of salts, chromatography and the like.

In step (cc), the N-protected amino carboxylic acid ester of formula XXVIII, preferably N-tert-butyloxycarbonylamino carboxylic acid ester, which includes as appropriate, its enantiomeric, diastereoisomeric or geometric isomers and/or mixtures thereof, is treated with a strong acid, for example trifluoroacetic acid, in a suitably inert solvent, for example dichloromethane, to give the resulting N-deprotected amino carboxylic acid ester of formula Iv, which includes as appropriate, its enantiomeric, diastereoisomeric or geometric isomers and/or mixtures thereof. A compound of formula Iv may be isolated utilizing conventional methods such as crystallization, chromatography and the like but is generally used without further purification in the next step.

In step (dd), the aminocarboxylic acid ester of formula Iv , preferably an amino carboxylic acid benzyl ester, is dissolved in a solvent, preferably a lower alcohol, optionally containing an acid, preferably acetic acid, and hydrogenolyzed at ambient temperature and at a hydrogen pressure of from one to five atmospheres over a noble metal catalyst, for example, palladium on carbon. The resulting amino acid of formula Iw, which includes as appropriate, its enantiomeric, diastereoisomeric or geometric isomers and/or mixtures thereof, may be isolated utilizing conventional methods such as crystallization, crystallization of acid addition salts and the like. 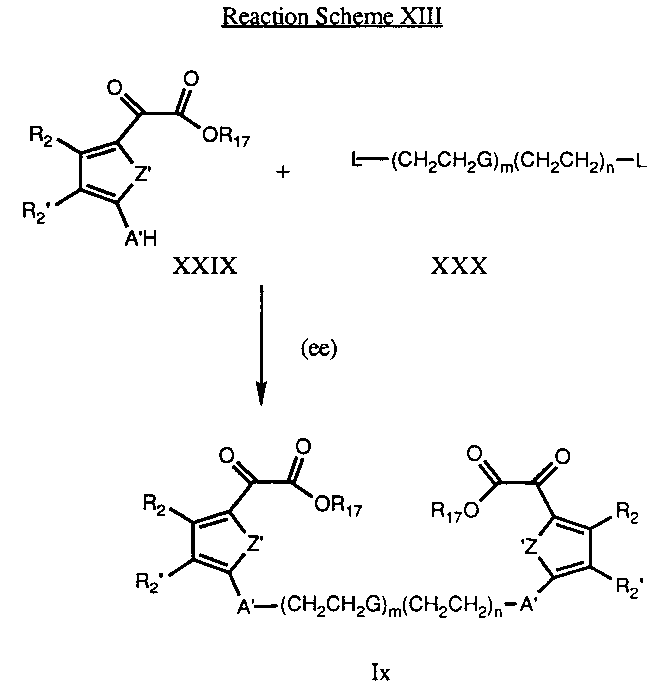
wherein m, n, A', L, R₂, R₂' and Z' are as previously described, R₁₇ is hydrogen or lower alkyl and G is O, S or a bond.

In Reaction Scheme XIII, step (ee) a phenol or thiophenol carboxylic acid ester of formula XXIX, which is known or can be prepared by known methods, and which includes as appropriate, its enantiomers and/or mixtures thereof, is reacted with a dialkylating agent of formula XXX, which is known or can be made by known methods, in the presence of an alkali metal hydride, for example, sodium hydride, in an inert solvent, preferably dimethylformamide, at a temperature of from 0 °C to 100 °C. Alternatively, a phenol or thiophenol carboxylic acid of formula XXIX may be reacted with a dialkylating agent of formula XXX, in the presence of an alkali metal hydroxide, for example sodium hydroxide, in an inert solvent mixture, preferably dimethylsulfoxide-water, at a temperature of from 0 °C to 100 °C. The resulting compound of formula Ix which includes, as appropriate, its diastereoisomeric isomers and/or mixtures thereof may be isolated utilizing conventional methods such as crystallization, crystallization of salts, chromatography and the like. 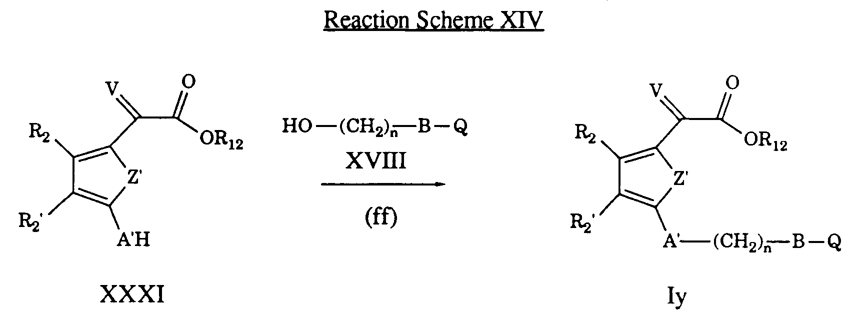
wherein A', B, Q, R₂, R₂', R₁₂, Z' and n are as previously described and V is hydroxyimino, alkyloxyimino, alkenyloxyimino, arylalkoxyimino, hydrazono, mono-lower alkyl hydrazono, di-lower alkyl hydrazono or semicarbazono.

In Reaction Scheme XIV, step (ff) a phenol or thiophenol carboxylic acid ester of formula XXXI, which includes as appropriate, all geometric forms and/or mixtures, is reacted with an alcohol of formula XVIII, which includes as appropriate, all enantiomeric, diastereoisomeric or geometric isomers and/or mixtures thereof, in the presence of a triaryl or trialkylphosphine, for example, triphenylphosphine and a coupling reagent, for example diethyl azodicarboxylate, in an inert solvent, for example tetrahydrofuran, at a temperature of from 0 °C to room temperature. The resulting compound of formula Iy which includes all diastereoisomeric or geometric isomers and/or mixtures thereof may be isolated utilizing conventional methods such as crystallization, crystallization of salts, chromatography and the like.

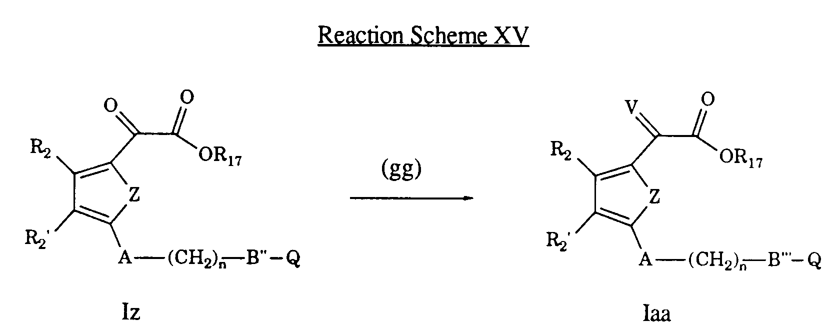

wherein A, Q, R₂, R₂', R₁₇, V, Z and n are as previously described and B''' is other than

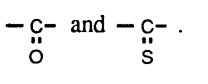

In Reaction Scheme XV, step (gg), an alpha-ketocarboxylic acid ester of formula Iz, which is or can be prepared according to one of the methods described herein, for example, in Reaction Scheme II, VI, VII, VIII or IX, and which includes, as appropriate, its enantiomeric, diastereoisomeric or geometric isomers and/or mixtures thereof, is reacted with an excess of an O-alkyloxy- or O-alkenyloxy- or O-arylalkyloxyamine hydrochloride, or alternatively semicarbazide hydrochloride, in an inert basic solvent for example pyridine, at a temperature of from room temperature to 50 °C. The compound of formula Iaa which includes its diastereoisomeric or geometric isomers and/or mixtures thereof, may be isolated utilizing conventional methods such as crystallization, crystallization of salts, chromatography and the like.

Alternatively, in step (gg), an alpha-ketocarboxylic acid ester of formula Iz, is reacted with an excess of an N-alkyl or N,N-dialkylhydrazine and a catalytic amount of an acid, preferably acetic acid, in an inert solvent for example methanol, optionally containing tetrahydrofuran, at a temperature of from room temperature to reflux. The compound of formula Iaa which includes its diastereoisomeric or geometric isomers and/or mixtures thereof, may be isolated utilizing conventional methods such as crystallization, crystallization of salts, chromatography and the like.
Alternatively, in step (gg), an alpha-ketocarboxylic acid of formula Iz, is reacted with a hydroxylamine in an inert solvent for example dimethylformamide at a temperature of from room temperature to 100 °C. The compound of formula Iaa which includes its diastereoisomeric or geometric isomers and/or mixtures thereof, may be isolated utilizing conventional methods such as crystallization, crystallization of salts, chromatography and the like.

The invention also relates to salts of the compounds of formula I when they contain a basic or acidic functionality which lends itself for salt formation with either an acid or base. Salts of the compounds of formula I which have a carboxy group are prepared by the reaction with a base having a non-toxic, pharmacologically acceptable cation. In general, any base which will form a salt with a carboxylic acid and whose pharmacological properties will not cause an adverse physiological effect is within the scope of this invention.

Suitable bases thus include, for example, the alkali metal and alkaline earth metal hydroxides, carbonates, and the like, for example, calcium hydroxide, sodium hydroxide, sodium carbonate, potassium carbonate and the like, ammonia, primary, secondary and tertiary amines, such as monoalkylamines, dialkylamines, trialkylamines, for example, methylamine, diethylamine, triethylamine and the like, nitrogen containing heterocyclic amines, for example, piperidine and the like. A salt thus produced is the functional equivalent of the corresponding compound of formula I wherein R is hydrogen and one skilled in the art will appreciate that the variety of salts embraced by the invention is limited only by the criterion that a base employed in forming the corresponding salts be both non-toxic and physiologically acceptable.

Salts of the compounds of formula I which have a basic functionality, for example, amino, pyridyl, amino lower alkyl, or the like are prepared by the reaction of an appropriate compound of formula I with a non-toxic pharmacologically or pharmaceutically acceptable acid. In general, the referred to compounds of formula I form pharmaceutically acceptable addition salts with, for example, both pharmaceutically acceptable organic and inorganic acids, such as, acetic acid, succinic acid, formic acid, methanesulfonic acid, p-toluenesulfonic acid, hydrochloric acid, nitric acid, phosphoric acid, sulfuric acid and the like.

The compounds of formula I exhibit activity as carnitine acyl transferase 1 (CAT-1) inhibitors and as such are therefore useful in disease states characterized by excessive production of the product of the reaction catalyzed by CAT-1. CAT-1 resides on the inner face of the outer mitochondrial membrane and is responsible for formation of long chain acyl carnitines from carnitine and the corresponding acyl CoA esters. These acylcarnitines may cross the inner mitochondrial membrane where they are reconverted to CoA esters through the action of a second carnitine acyl transferase (CAT-II). CAT-I is thought to represent the rate limiting step in mitochondrial fatty acid oxidation. Myocardial ischemia and the accompanying decrease in oxidative metabolism leads to an increase in cytosolic long chain acyl carnitines which, in turn, can cause electrophysiological disturbances and arrhythmias leading to sudden death. Thus, potent and cardioselective inhibitors of CAT-1 would prevent this increase in long chain acyl carnitines and are therefore useful in the improvement of cardiac function, in limiting infarct size and in the prevention of arrhythmias during and following infarction.

The useful activity of the compounds of formula I can be demonstrated by the following procedures:

### 1. Primary Screen - Inhibitors of CAT-1 in Mitochondria

### (a). Preparation of Mitochondria

The heart is rapidly removed from anesthetized rat (sodium pentabarbitol) and placed in ice-cold homogenization buffer (sucrose, 250 mM; Tris, 10 mM; EDTA, 1 mM; BSA, 0.01%; pH=7.4). The atria and extraneous tissue are removed and the remainder is chopped with scissors. The pieces are rinsed in homogenization buffer before being homogenized in 10 ml of the same buffer for 5-7 seconds. The homogenate is then diluted with homogenization buffer (40 ml total volume) and centrifuged at 200g for 20 minutes. The supernatant is withdrawn and centrifuged at 7000g for 20 minutes and the resulting pellet is isolated, resuspended in 40 ml of homogenization buffer and centrifuged once more at 7000g for 20 minutes. The isolated pellet is resuspended in 10 ml of homogenization buffer and stored at
4° C until use. This preparation is used as the isolated mitochondrial preparation.

### (b). Inhibition of CAT-1 assay

Isolated mitochondria (20 µl) are incubated with 60 µl of Tris/sucrose buffer (sucrose, 500 mM; Tris, 20 mM; KC1, 100 mM; EDTA, 2 mM; pH=7.0 plus 83 µM palmitoyl CoA, and BSA , 0.8 mg/mL and 10⁻⁴ to 10⁻¹⁰ M of the drug to be tested. After 10 minutes at 30° C, the reaction is started by addition of 10 µl of ³H-carnitine (final concentration = 250 µM). After 5 minutes, the reaction is terminated by the addition of ice cold 70% perchloric acid (PCA). The precipitated solids so formed, containing the insoluble product of the enzyme palmitoyl carnitine, are captured on filter paper. The filter paper is washed with 7% PCA, then dried and placed in scintillation vials with 10 ml of scintillant (Aquasol, New England Nuclear) and counted for radioactivity. The rate of the reaction is assessed by the difference in radioactivity between zero time and five minutes in the absence of test compound. The ability of test compounds at selected concentrations to inhibit CAT-1 is determined by their ability to reduce the formation of the radioactive product. The IC₅₀ of the test compound is the concentration of test compound that reduces the formation of radioactive product by 50%.

Results of this assay are reported in Table I, under the heading "Primary Screen".

### 2. Second Screen - Inhibition of CAT-1 in intact Cardiac Myocytes

### (a) Preparation of Isolated Rat Cardiac Myocytes

Four hearts are excised from anesthetized rats and perfused retrogradly via the Langendorf technique. Each heart receives 100 ml of Krebs (NaC1, 118.4 ; KCL, 4.6 mM; NaH₂PO₄, 1.0mM; NaHCO₃, 20 mM; MgSO₄, 2mM; CaC1₂, 50 µM; glucose, 11.0mM), 100 ml of Ca++ free Krebs and finally 100 ml of 0.5% collagenase (Boehringer Mannheim, type 1) in Hepes (NaC1, 115 ml; KC1, 5mM; sucrose, 35 mM; glucose, 10 mM; Hepes, 10 mM; taurine, 4mM; MgSO₄, 2mM; CaC1₂, 50 µM). After these perfusions, the hearts are chopped into pieces, approx. 0.5 cm X 0.5 cm and placed in four 125 ml plastic bottles along with 25 ml of collagenase/Hepes situated in a water bath maintained at 35 °C. The pieces of cardiac tissue in each bottle are agitated by using a Harvard respirator connected to four glass syringes, which sucks the tissue up and down each tube during each respiratory cycle. After fifteen minutes, the collagenase/Hepes is removed and replaced with fresh collegenase/Hepes and the process repeated. The collegenase/Hepes from the subsequent fifteen minute harvests are centrifuged at 500g to pellet the cells which are then resuspended in Hepes buffer. After six harvests, all cells are pelleted, resuspended in 2 x 26.5 ml of Hepes plus BSA (350 mg) and layered on top of 2 x percoll gradients (21.15 ml percoll, 2.35 ml NaC1, 6 drops acetic acid). This is centrifuged at 3000g for eight minutes forming a percoll gradient which results in viable cells being separated from damaged cells. The viable cells are removed, washed with Hepes and finally resuspended in 25 ml of Hepes. The concentration of calcium is slowly increased to 500 µM by small additions of CaC1₂ over two hours.

### (b). Cellular Assay

The isolated cardiac myocytes (2ml; approximately 1 x 10⁶ cells) are separated into six glass pods. Into four of these are placed selected concentrations of the compound to be tested, and the cells are incubated for twenty minutes, after which the pods are centrifuged at 300g for three minutes. 300 µl of normoxic Hepes is placed in one of the pods not containing compound and then all pods are placed in a hypoxic chamber (N₂ atmosphere). Oxygen is fed via small bore steel tubes to the pod containing the normoxic buffer, while the other five pods receive pre-purified argon (99.998% pure). After two minutes, Hepes (pre-gassed with argon for at least twelve hours) is fed into the five hypoxic pods. This hypoxic enviornment (pO₂ < 10mm Hg) is maintained for twenty minutes, after which the cells and pods are frozen by contact with a metal block precooled in liquid N₂. The pods are then removed from the chamber and stored at -70° C. The carnitine assay is performed, as described below in (c), to determine the amount of long-chain acylcarnitine in the cells from the normoxic, hypoxic and hypoxic plus compound samples. The hypoxic condition causes the long-chain acylcarnitines to increase threefold. The presence of the CAT-1 inhibitors inhibit this increase if they penetrate the cell membrane. Thus the IC₅₀ in this screen is the concentration of test compound which produces a 50% inhibition of the hypoxia-induced increase in long-chain acylcarnitines.

Results of this assay are reported in Table I, under the heading "Secondary Screen".

### (c) Long-chain Acylcarnitine Assay.

The frozen cell samples are thawed, mixed and homogenized with 40 µl of 70% PCA. The homogenate is transferred to eppendorf tubes while the homogenization tube and the glass pod is rinsed with 500 µl of 7% PCA, with the rinse being added to the eppendorf tube. This is then centrifuged at 1000g for two minutes to pellet the precipitate (containing the long-chain acylcarnitine) and the supernatant is discarded. The pellet is washed twice with 500 µL of 7% PCA, the supenatants being discarded. The pellet is then hydrolyzed (to separate the acyl unit from carnitine to yield free carnitine) by the addition of 200 µl of deionized H₂0 and 50 µl of 5N KOH and incubated at 70° C for ninety minutes. Following hydrolysis, the samples are neutralized by the addition of 500 µl of 0.83M Hepes (pH = 7.4) and 10 µl of 70% PCA. After the tubes are centrifuges, the supernatants are transferred into eppendorf tubes. The pellets are washed with 250 µl of 0.83M Hepes and the washes are added to the eppendorf tube. To measure the level of carnitine in the samples, 300 µl of the supernatant in the eppendorf tubes is incubated with 300 µl of deionized H₂0, 400 µl of (0.83M Hepes; 50mM EDTA: 10mM N-ethylmaleimide; 10 µM ³H-acetyl CoA; 10 µl carnitine acyltransferase, Sigma, 45 units). The tubes are vortexed and incubated for ninety minutes at 35 ° C. Standards are also incubated containing known amounts of carnitine (from 5 to 500 pmoles). The ³H-acetyl carnitine so formed is separated from the ³H-acetyl CoA by passing the samples through a Dowex 1-8x anion exchange resin (100-200 mesh, chloride form) and the eluate (containing the ³H-acetyl carnitine) is captured in mini-vials. The resin is washed with 300 µl of deionized H₂0 and the eluate is again captured in the vial, 5ml of scintillant (Aquasol 2, New England Nuclear) is added and the amount of radioactivity is determined. The amount of carnitine in each sample and hence LCA is determined from comparison with the standard curve.

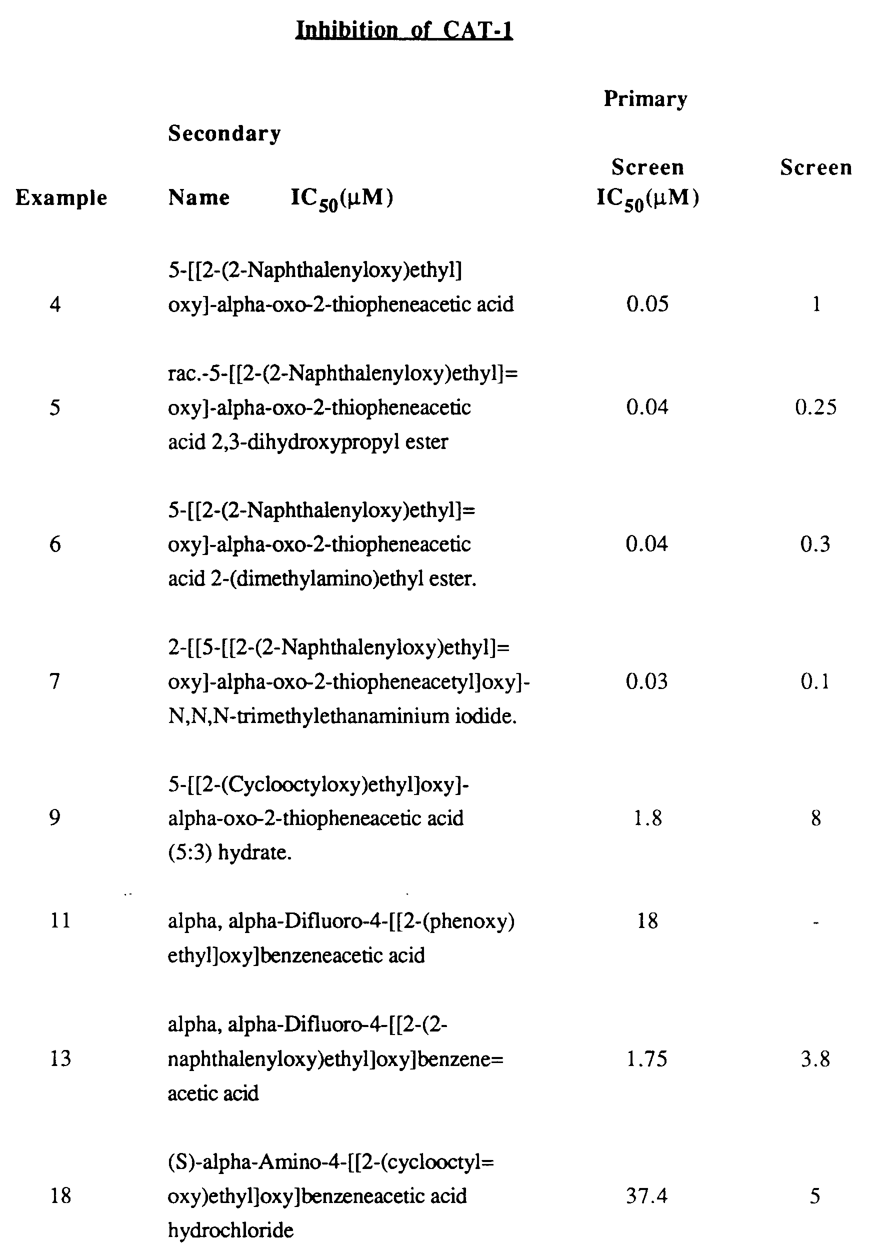

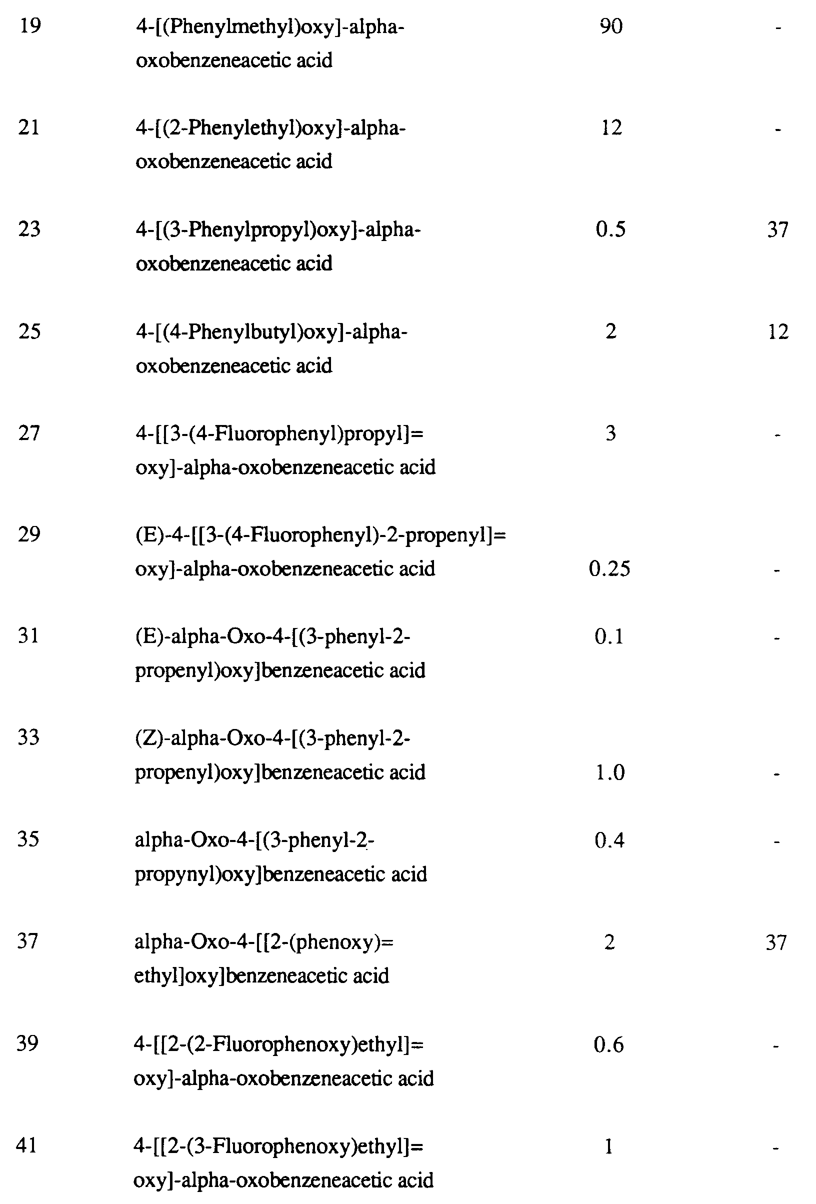

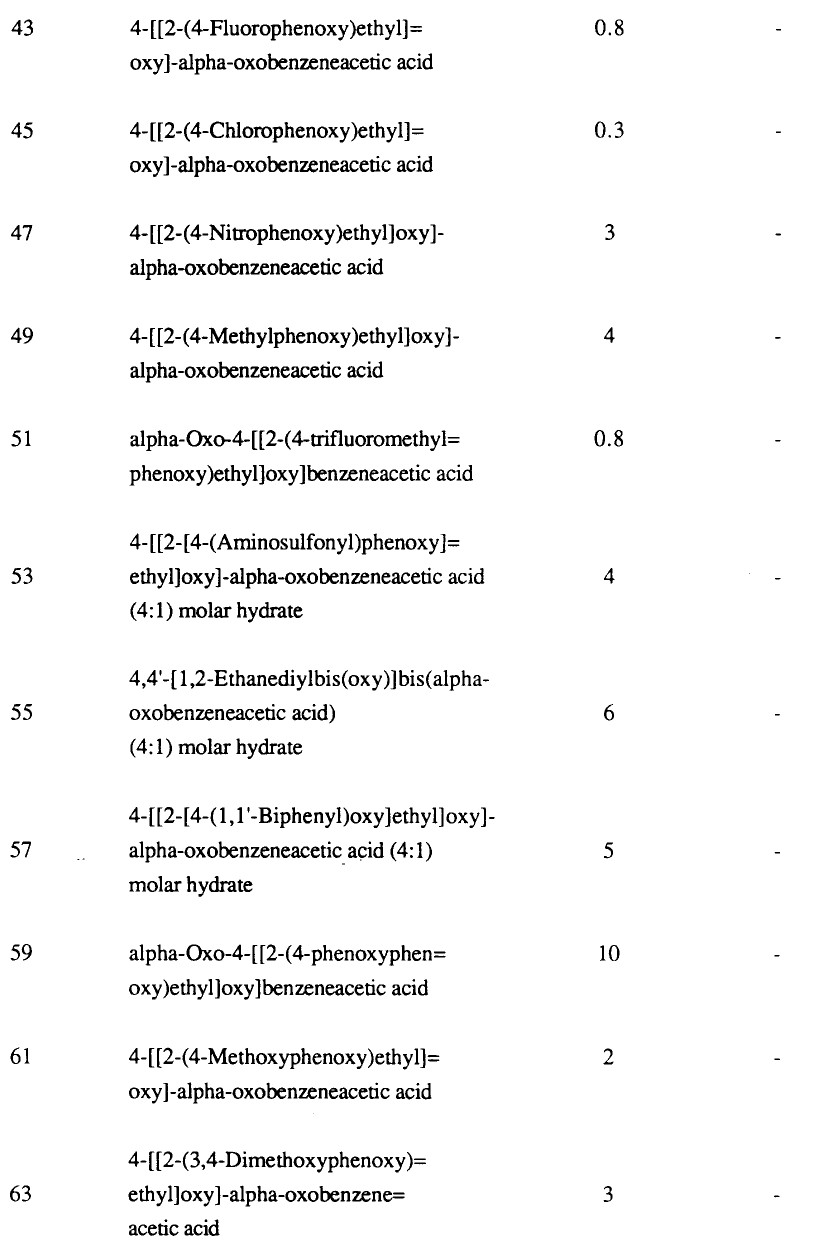

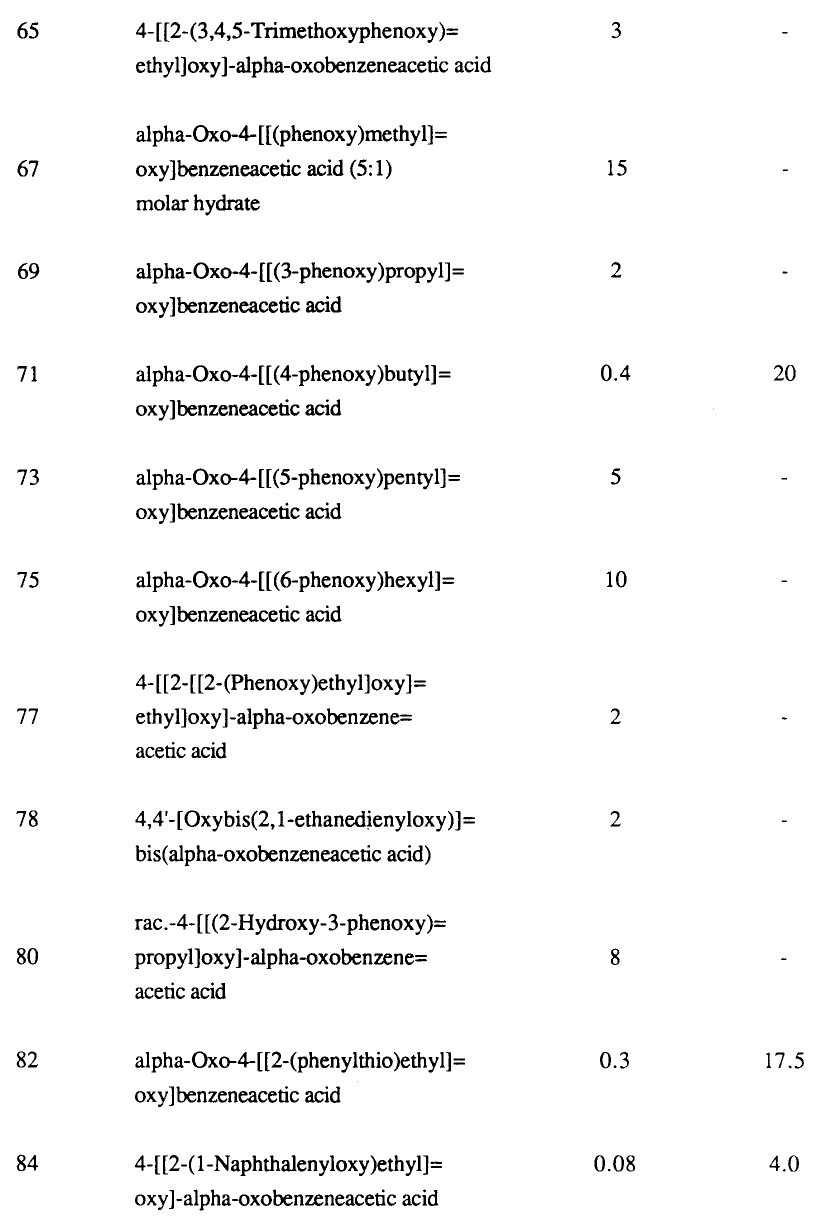

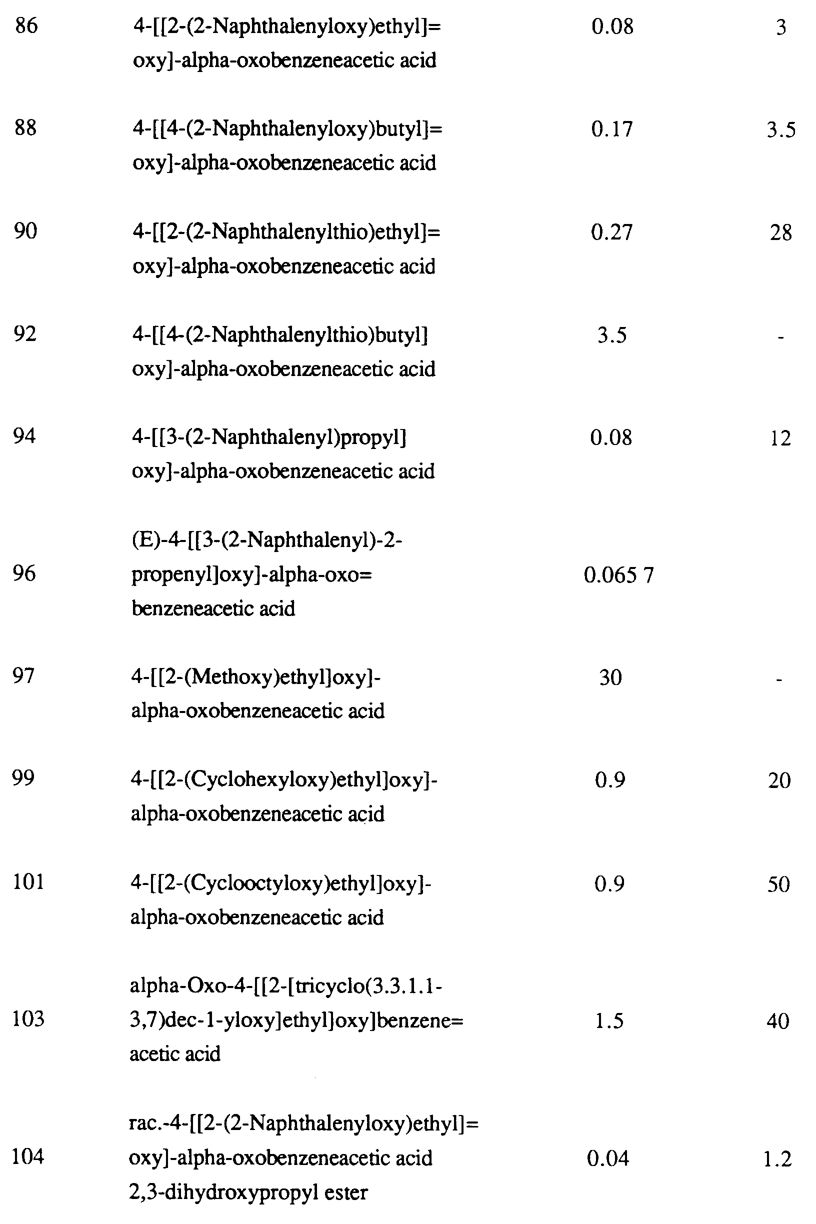

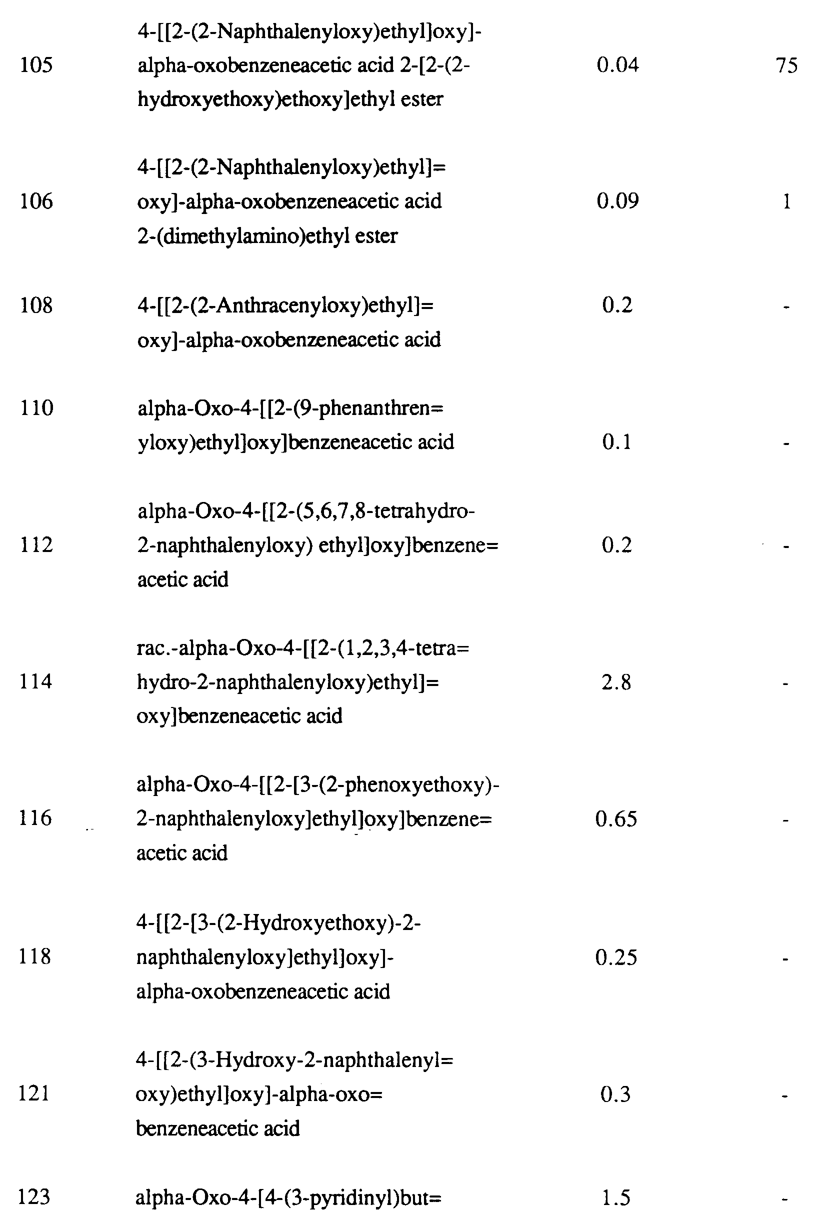

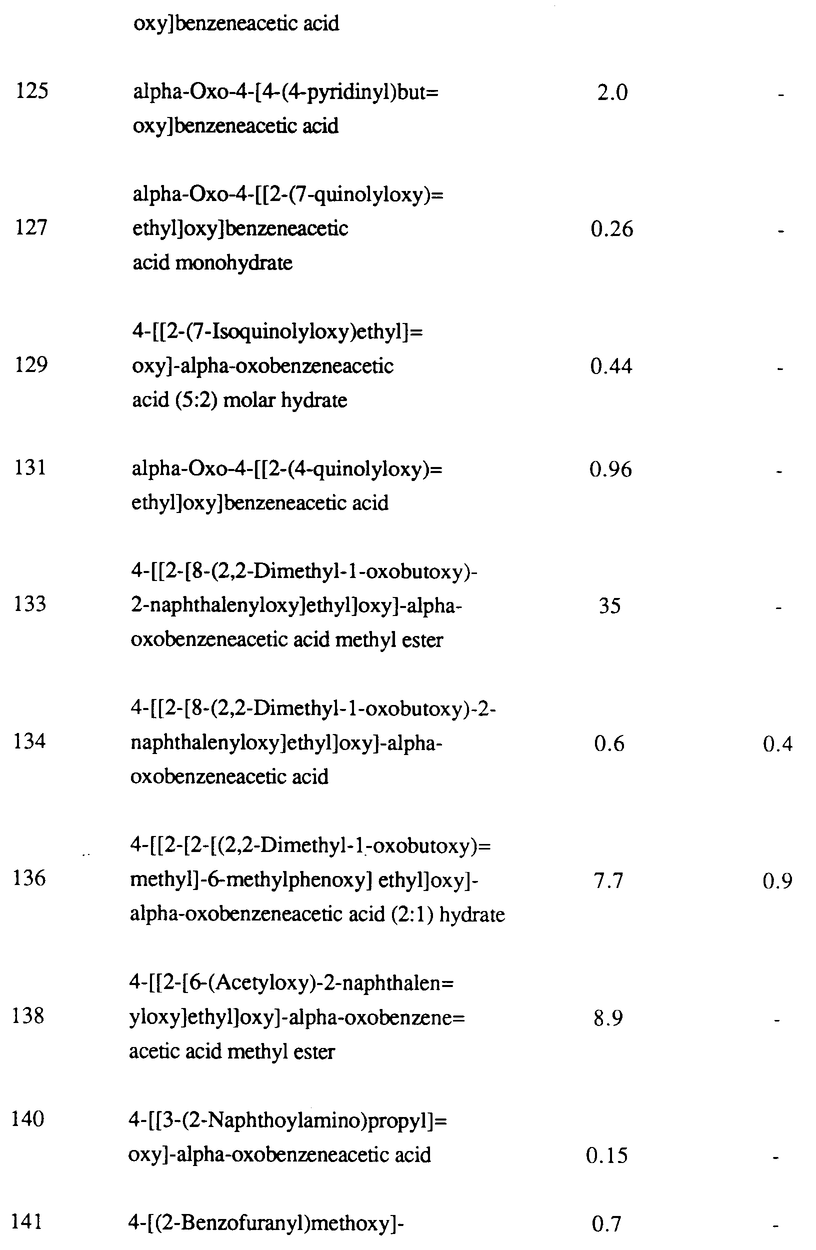

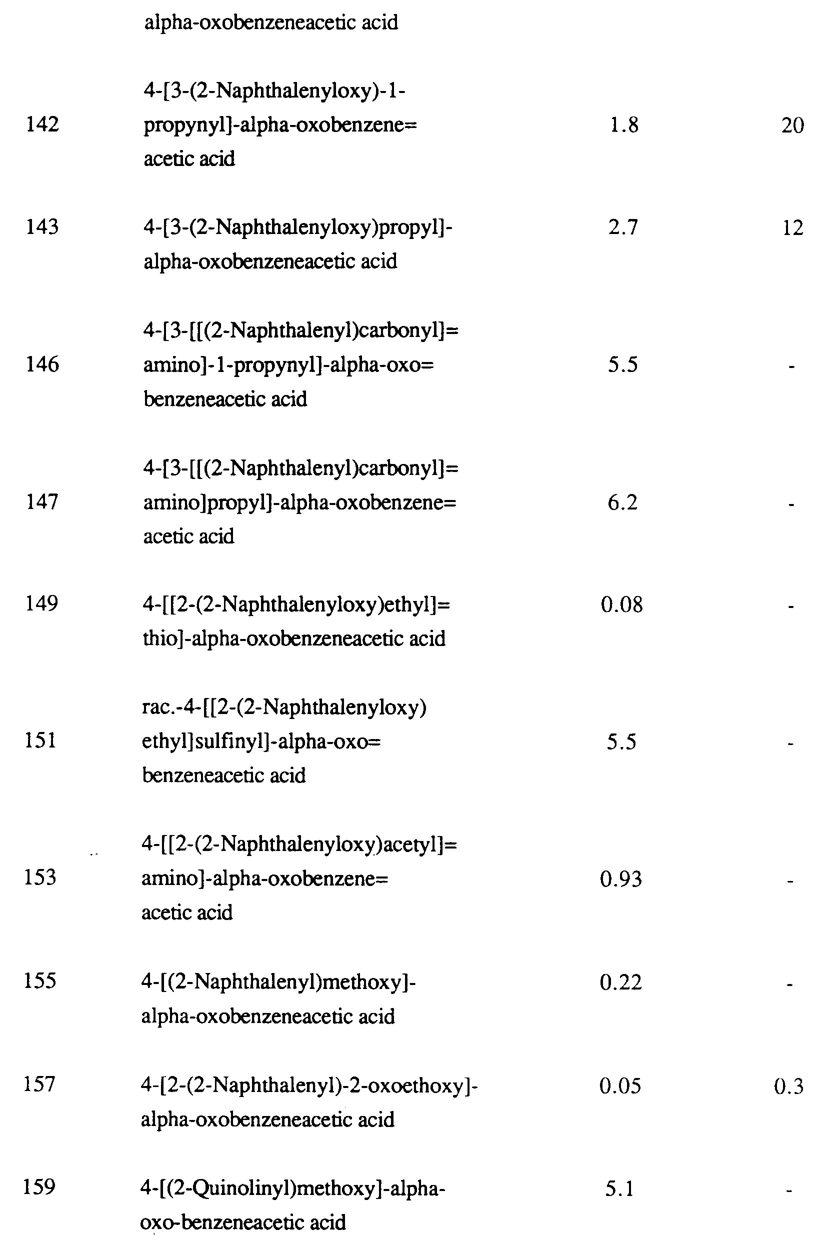

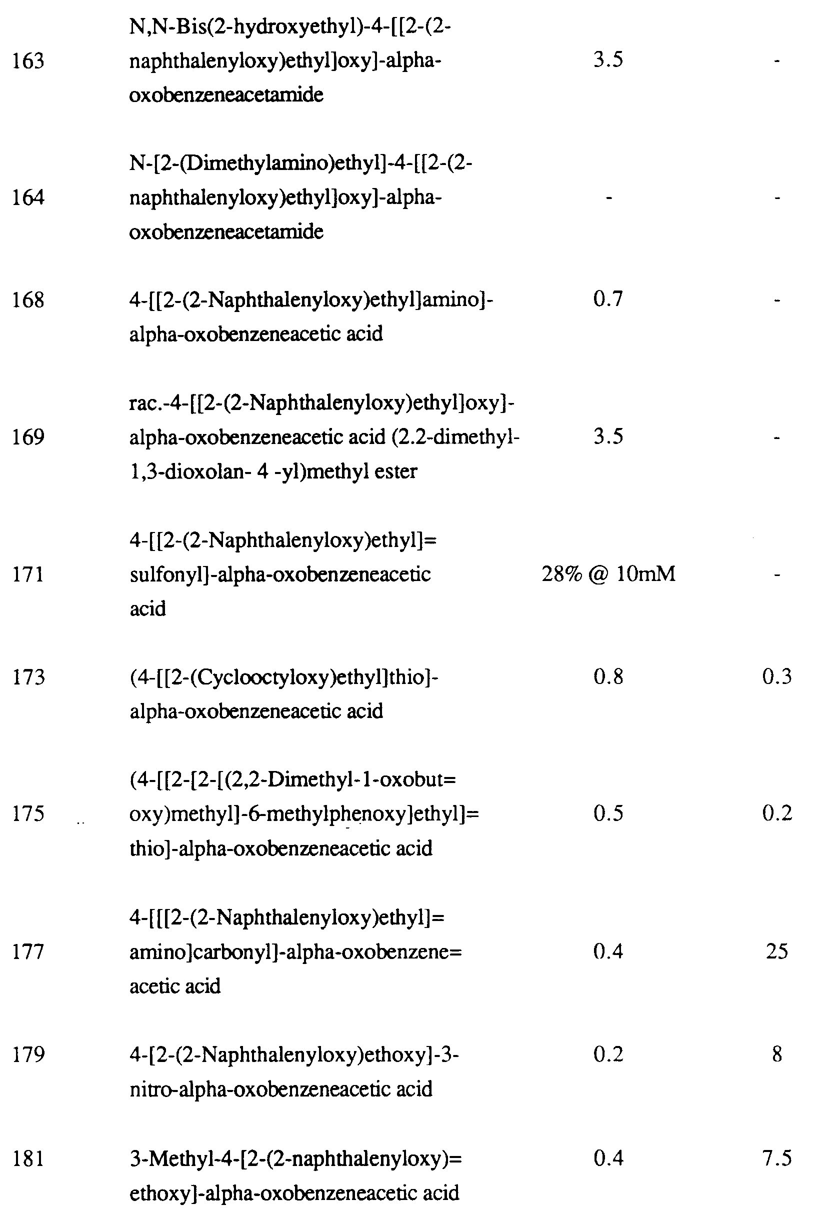

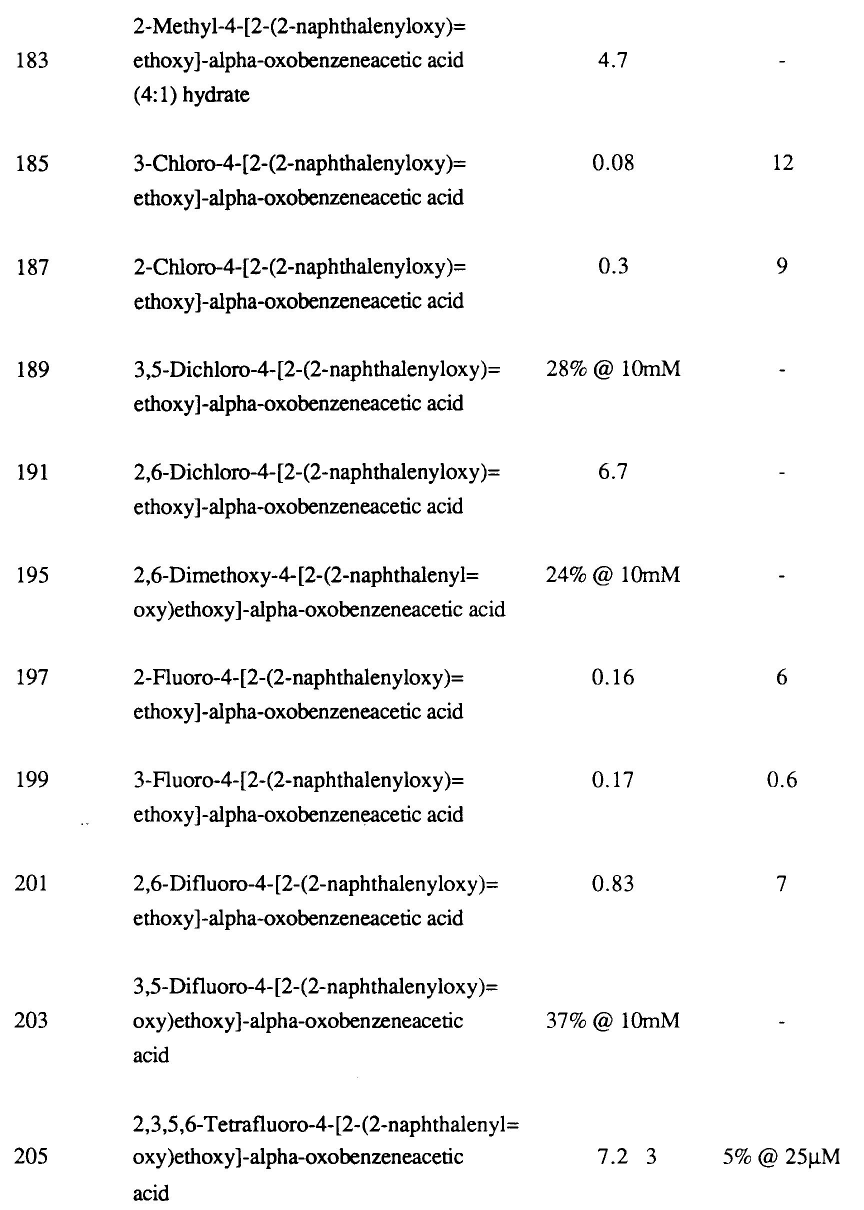

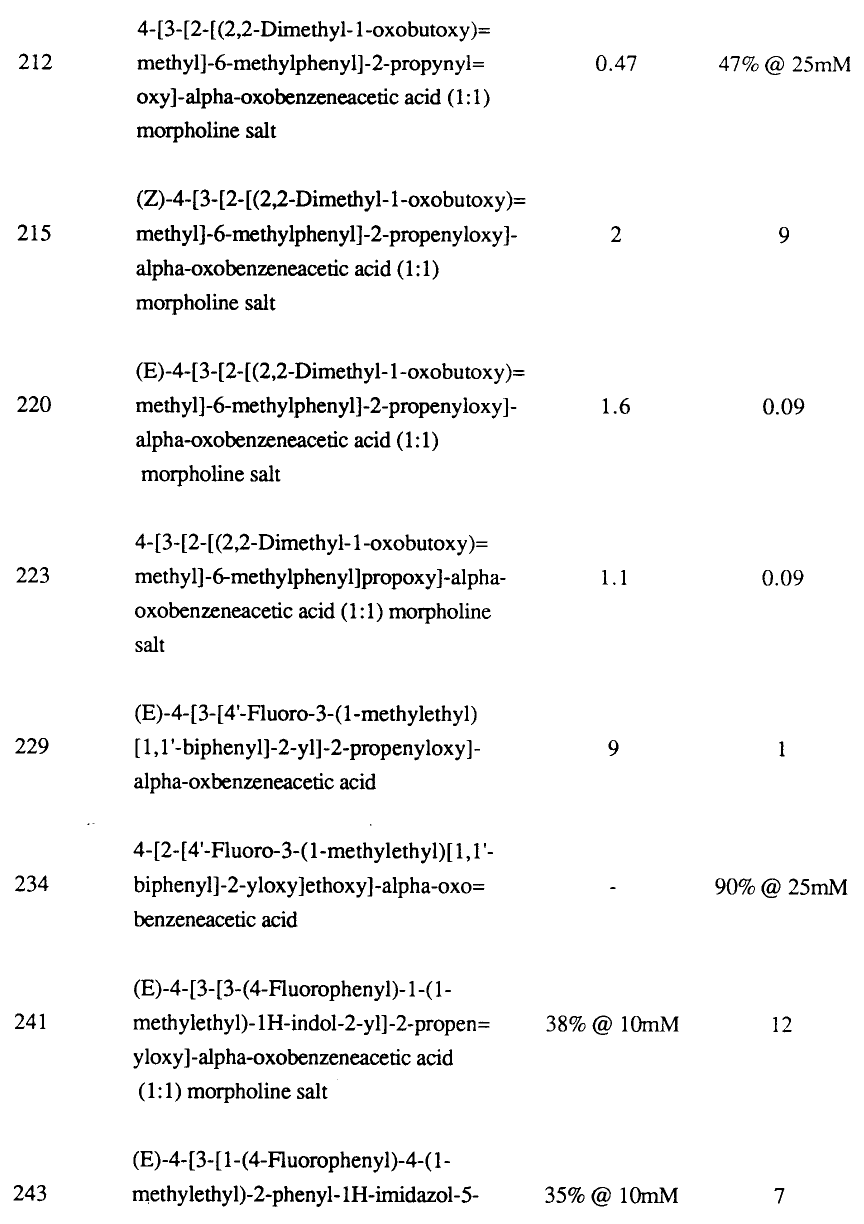

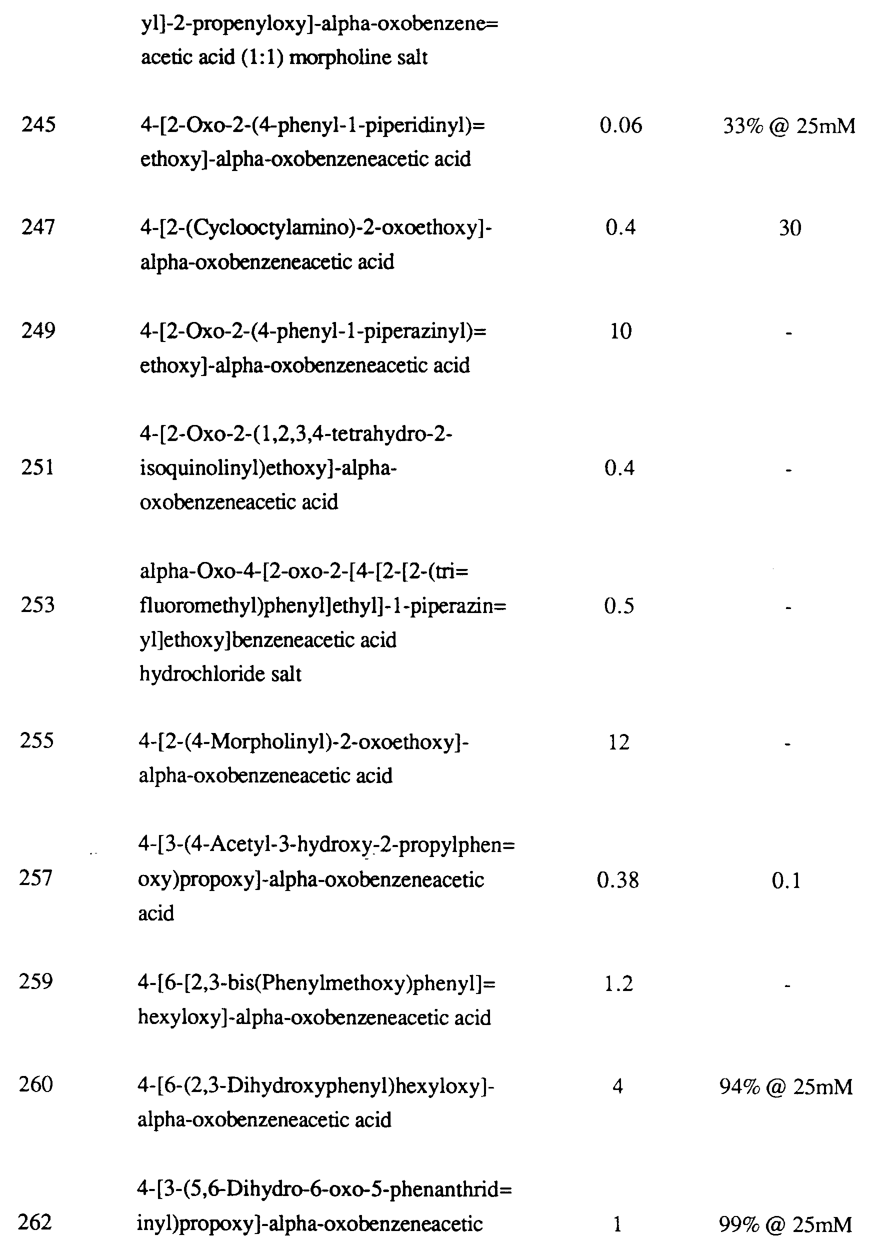

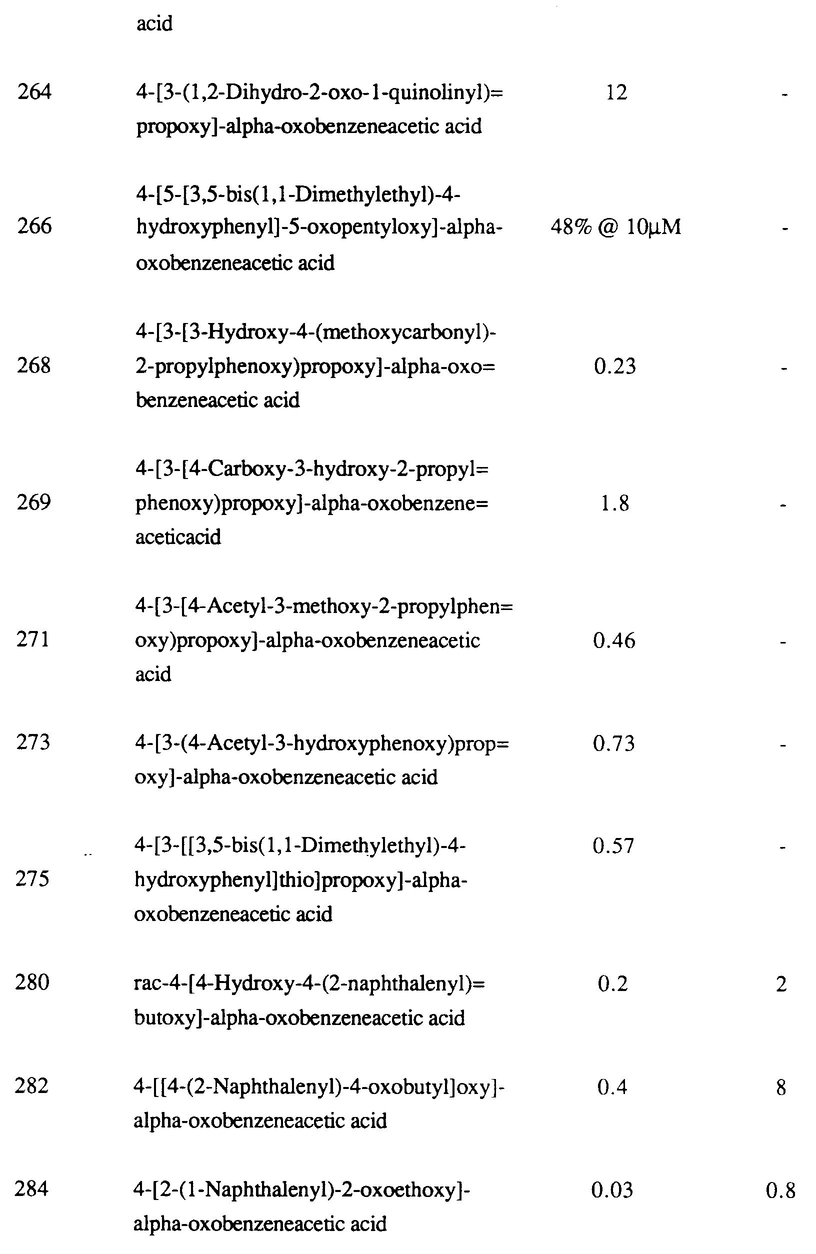

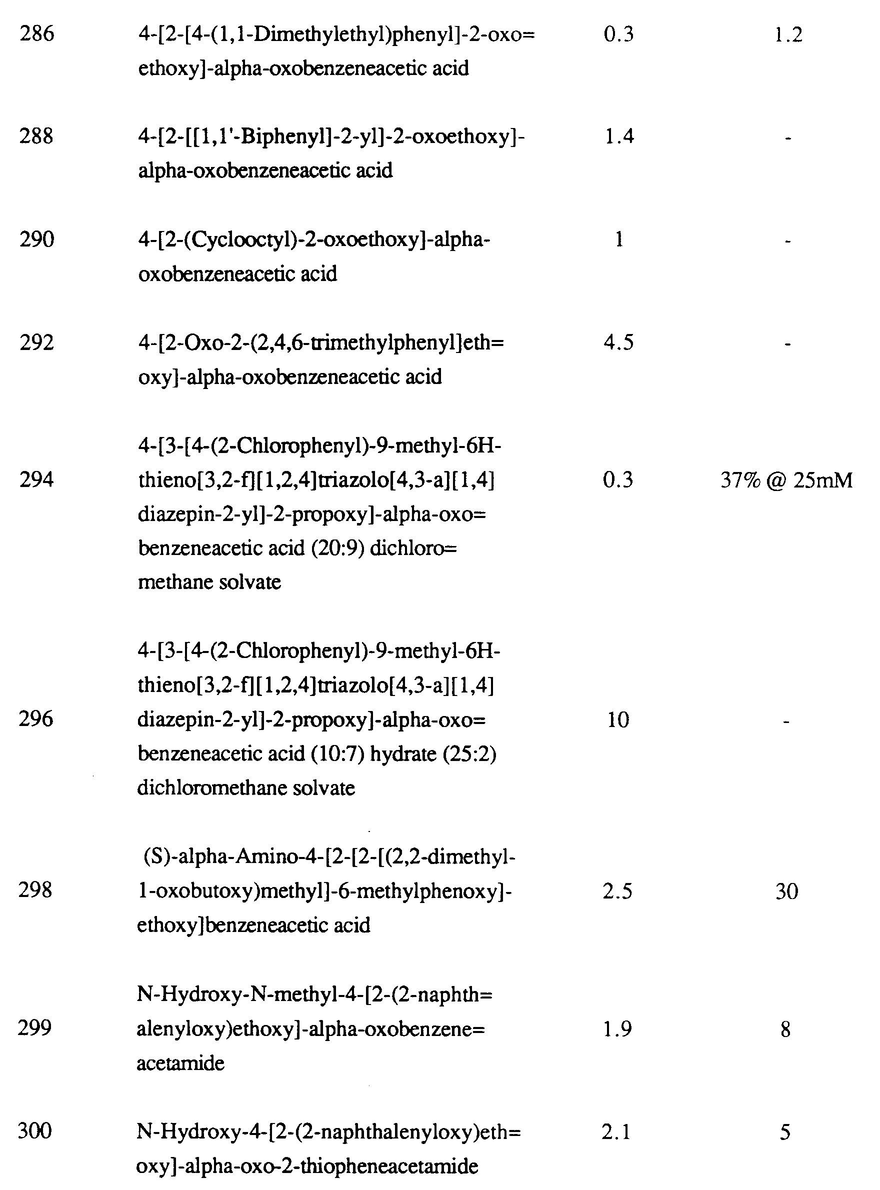

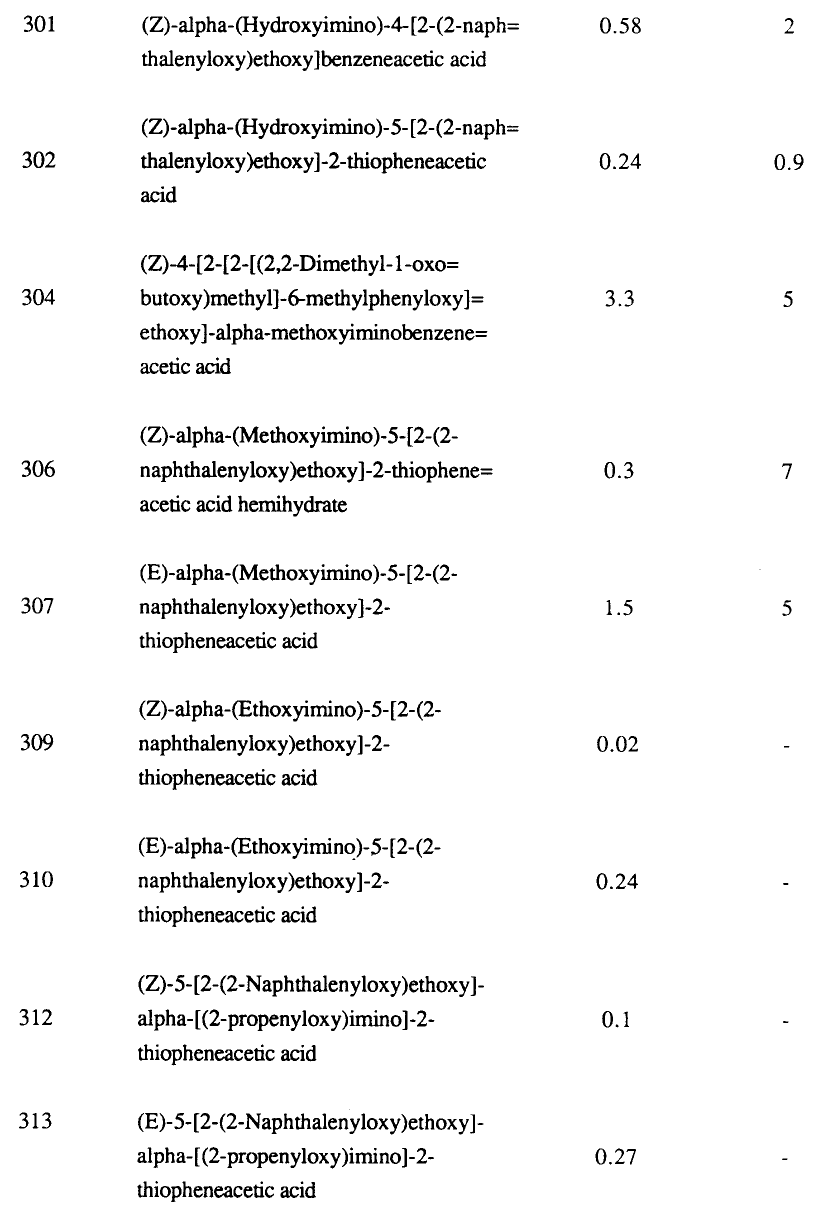

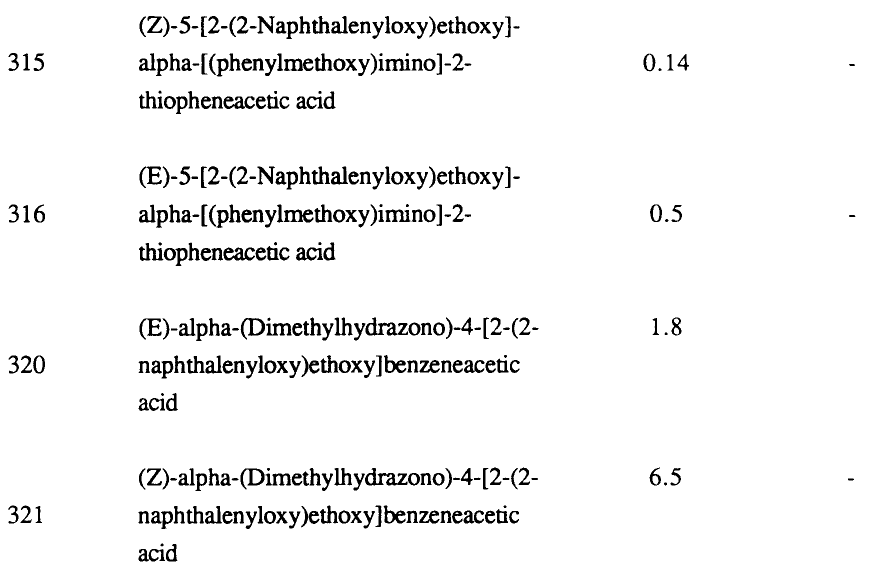

A compound of formula I, an enantiomer thereof or a salt thereof or a composition containing a therapeutically effective amount of a compound of formula I or a salt thereof can be administered by methods well known in the art. Thus, a compound of formula I or a salt thereof can be administered either singly or with other pharmaceutical agents, orally, parenterally, rectally, or by inhalation, for example, in the form of an aerosol, micropulverized powder or nebulized solution. For oral administration the described compound can be administered in the form of tablets, capsules, for example, in admixture with talc, starch, milk sugar or other inert ingredients, that is, pharmaceutically acceptable carriers, in the form of aqueous solutions, suspensions, elixirs or aqueous alcoholic solutions, for example, in admixture with sugar or other sweetening agents, flavoring agents, colorants, thickeners and other conventional pharmaceutical excipients, or beadlets for oral administration. For parenteral administration, the desired compound can be administered in solutions or suspension, for example, as an aqueous or peanut oil solution or suspension using excipients and carriers conventional for this mode of administration. For administration as aerosols, they can be dissolved in a suitable pharmaceutically acceptable solvent, for example, ethyl alcohol or combinations of miscible solvents, and mixed with a pharmaceutically acceptable propellant. Such aerosol compositions are packaged for use in pressurized container fitted with an aerosol valve suitable for release of the pressurized composition. Preferably, the aerosol valve is a metered valve, that is one which on activation releases a predetermined effective dose of the aerosol composition. For rectal administration, the desired compound can be administered in the form of suppositories utilizing an inert carrier material cocoa butter and the like. For topical administration, the compounds of formula I can be incorporated into ointments, creams, lotions, gels, and the like. In general, the solutions, ointments and creams which are useful in accordance with this invention include formulations having absorbable, water soluble or emulsion-type bases, such as petrolatum, lanolin, polyethylene glycols, or the like.

Suitable solutions will contain the compounds of formula I dissolved in a pharmaceutically acceptable solvent, such as polyethylene glycol, or the like.

Suitable lotions include, true solutions to aqueous or hydroalcoholic formulations containing finely divided particles. Lotions can contain suspending or dispersing agents such as cellulose derivatives, for example, methyl cellulose, ethyl cellulose, or the like. Gels will typically be semi-solid preparations made by gelling a solution or suspension of a compound of formula I in a suitable hydrous or anhydrous vehicle, using a gelling agent such as carboxy polymethylene, or the like, and thereafter neutralizing it to proper consistency with an alkali metal hydroxide, for example, sodium hydroxide, and an amine, for example, polyethylenecocoamine. Topical pharmaceutical compositions containing a compound of formula I can also be formulated to include conventional ingredients such as preservatives, stabilizers, wetting agents, emulsifying agents, buffers, and the like, in conventional amounts adjusted for particular requirements and which are readily determinable by those skilled in the art.

In the practice of the invention, the dose of a compound of formula I or a salt thereof to be administered and the frequency of administration will be dependent on the potency and duration of activity of the particular compound of formula I or salt to be administered and on the route of administration, as well as the severity of the condition, age of the mammal to be treated and the like. Oral doses of a compound of formula I or a salt thereof contemplated for use in practicing the invention are in the range of from about 1 to about 2000 mg per day, preferably about 25 to about 500 mg either as a single dose or in divided doses.

The compounds of formula I of the invention may possess one or more asymmetric carbon atoms, they can thus be obtained as enantiomers or as racemic mixtures. The resolution of racemates into the optically active isomers can be carried out by known procedures. Some racemic mixtures can be precipitated as eutectics and can thereafter be separated. Chemical resolution of the starting material is, however, preferred if an enantiomer of formula I is to be prepared. By this method, diastereomeric salts are formed from the racemic mixture, for example, of a precursor of a compound of formula VI, with an optically active resolving agent, for example, an optically active base, such as R-(+)-α-methylbenzylamine, which can be reacted with a carboxyl group. The formed diastereomers are separated by selective crystallization and converted to the corresponding optical isomer. Thus, the invention covers the racemates of the compounds of formula I as well a their optically active isomers (enantiomers).

The examples that follow also further illustrate the invention Examples 1, 2, 97, 132, 144, 206, 207, 208, 209, 210, 213, 216, 217, 218, 221, 224, 225, 226, 227, 230, 231, 232, 237, 238, 239, 276, 277, 278, 278 and 297 are preparation examples. The other examples describe compounds according to the present invention. Melting points of compounds were determined on a Thomas-Hoover capillary melting point apparatus and are uncorrected. The proton NMR spectra were recorded on a Varian XL-100, XL-200 or XL-400 spectrometer, IR spectra were obtained on a Beckman IR-9 or IR-12 spectrometer. NMR and IR spectra were recorded for each new compound reported herein and were consistent with the assigned structures. Preparative high-pressure liquid chromatography (HPLC) was performed on silica gel Prep-Pak 500 cartridges with a Waters Associates Prep LC 500A instrument. Flash chromatography was performed with a loading ratio most often in the range of 50-100 g of sorbent to 1 g of compound, using a column pressure of 3-5 psi on Kieselgel 60,230-400 mesh, obtained from the same supplier. Column chromatography was accomplished on Kieselgel 60, 35-70 mesh, from E. Merck, Darmstadt. Kieselgel 60 F₂₅₄ plates from E. Merck were used for TLC, and compounds were visualized with UV light or iodine vapor. Dimethylformamide was dried over Linde 3A sieves.

### Example 1

### Preparation of 2-[[2-(2-naphthalenyloxy)ethyl]oxy]thiophene.

A solution of 2-methoxythiophene (2.28 g) and 2-(2-naphthalenyloxy)ethanol (4.0 g) in benzene (35 ml) containing para-toluenesulfonic acid (0.02 g) was stirred at reflux for 3 days in a flask equipped with a Soxhlet extractor charged with 4Å molecular sieves. The mixture was cooled, washed with 0.5N sodium hydroxide solution, dried over anhydrous magnesium sulfate (MgSO₄) and evaporated. Crystallization of the residual material from ethanol (30 ml) provided 2.68 g of 2-[[2-(2-naphthalenyloxy)ethyl]oxy]thiophene as colorless flakes, mp 129-130 °C.

| | | | |
|---|---|---|---|
| Analysis Calculated for C₁₆H₁₄O₂S: | C, 71.09; | H, 5.22; | S, 11.86 |
| Found: | C, 70.85; | H, 5.14; | S, 11.73 |

### Example 2

### Preparation of 2-[[2-(cyclooctyloxy)ethyl]oxy]thiophene.

As in Example 1, a solution of 2-methoxythiophene (11.4 g) and 2-(cyclooctyloxy) ethanol (13.78 g) in benzene (200 ml) containing para-toluenesulfonic acid (0.1 g) was stirred at reflux for 3 days in a flask equipped with a Soxhlet extractor charged with 4 Å molecular sieves. After the reaction was worked up in the usual manner, the crude product was purified by HPLC (hexane-diethyl ether; 24:1) to give 12.6 g of 2-[[2-(cyclooctyloxy) ethyl]oxy] thiophene as a colorless oil. A small portion was distilled in a Kugelrohr apparatus (∼130 °C/0.02 mm) to furnish the analytical specimen.

| | | | |
|---|---|---|---|
| Analysis Calculated for C₁₄H₂₂O₂S: | C, 66.10; | H, 8.72; | S, 12.60 |
| Found: | C, 66.30; | H, 8.72; | S, 12.40 |

### Example 3

### Preparation of 5-[[2-(2-naphthalenyloxy)ethyl]oxy]-alpha-oxo-2-thiopheneacetic acid ethyl ester.

Ethyl oxalyl chloride (3 ml) was added dropwise with stirring to a chilled solution of 2-[[2-(2-naphthalenyloxy)ethyl]oxy]thiophene (2.027 g) in dichloromethane (15 ml) containing pyridine (0.75 ml). After the solution was stirred at 35 °C for 5 hours, it was then cooled and poured into a saturated aqueous sodium bicarbonate solution. The mixture was stirred for 10 minutes , then the phases were separated and the organic layer was washed in turn with brine, 0.5 N hydrochloric acid and brine. The aqueous layer and washes were back-extracted in turn with dichloromethane, then the combined extracts were dried (MgSO₄) and evaporated. The residual material was crystallized from dichloromethane-diethyl ether to provide 1.96 g of 5-[[2-(2-naphthalenyloxy)ethyl]oxy]-alpha-oxo-2-thiopheneacetic acid ethyl ester as a yellow crystalline solid, mp 98-100 °C. A sample was recrystallized from the same solvents to give the analytical specimen, mp 98-100 °C.

| | | | |
|---|---|---|---|
| Analysis Calculated for C₂₀H₁₈O₅S: | C, 64.85; | H, 4.90; | S, 8.65 |
| Found: | C, 65.00; | H, 4.83; | S, 8.46 |

### Example 4

### Preparation of 5-[[2-(2-naphthalenyloxy)ethyl]oxy]-alpha-oxo-2-thiopheneacetic acid.

1N potassium hydroxide solution (3.1 ml) was added dropwise with stirring to a chilled solution of 5-[[2-(2-naphthalenyloxy)ethyl]oxy]-alpha-oxo-2-thiopheneacetic acid ethyl ester (1.13 g) in methanol (7 ml). Almost immediately the potassium salt precipitated from the solution as a colorless solid which was filtered off and washed with methanol. The solid was partitioned between dichloromethane (100 ml) and 0.1N hydrochloric acid (35 ml) and after all solids had dissolved, the organic layer was washed with water, dried over anhydrous sodium sulfate (Na₂SO₄) and evaporated to yield 0.94 g of 5-[[2-(2-naphthalenyloxy)ethyl]oxy]-alpha-oxo-2-thiopheneacetic acid as a yellow-orange solid. Crystallization of the acid from benzene-hexane furnished the title compound as a yellow solid, mp 152-154 °C.

| | | | |
|---|---|---|---|
| Analysis Calculated for C₁₈H₁₄O₅S: | C, 63.15; | H, 4.12; | S, 9.36 |
| Found: | C, 63.18; | H, 4.11; | S, 9.09 |

### Example 5

### Preparation of rac.-5-[[2-(2-naphthalenyloxy)ethyl]oxy]-alpha-oxo-2-thiopheneacetic acid 2,3-dihydroxypropyl ester.

A solution of the acid chloride, prepared from 1.027 g of 5-[[2-(2-naphthalenyloxy) ethyl]oxy]-alpha-oxo-2-thiopheneacetic acid as described in Example 6, in tetrahydrofuran -dimethylformamide (3:1; 35 ml) was added dropwise with stirring to a chilled (-70 °C) solution of glycerol (1.824g) and triethylamine (0.47 ml) in tetrahydrofurandimethylformamide (3:1; 60 ml). The cooling bath was removed and the reaction was stirred at room temperature for 2 hours, then was diluted with dichloromethane and washed in turn with saturated sodium bicarbonate solution and brine. The aqueous layers were backwashed with dichloromethane, then the combined organic extracts were dried (Na₂SO₄) and evaporated. The resulting solid was crystallized from dichloromethane to furnish 0.67 g of rac.-5-[[2-(2-naphthalenyloxy)ethyl]oxy]-alpha-oxo-2-thiopheneacetic acid 2,3-dihydroxypropyl ester, mp 113-115 °C.

| | | | |
|---|---|---|---|
| Analysis Calculated for C₂₁H₂₀O₇S: | C, 60.57; | H, 4.84; | S, 7.70 |
| Found: | C, 60.29; | H, 4.80; | S, 7.57 |

### Example 6

### Preparation of 5-[[2-(2-naphthalenyloxy)ethyl]oxy]-alpha-oxo-2-thiopheneacetic acid 2-(dimethylamino)ethyl ester.

Oxalyl chloride (1 ml) was added dropwise with stirring to a chilled (-70 °C) solution of 2-[[2-(2-naphthalenyloxy)ethyl]oxy]-alpha-oxo-2-thiopheneacetic acid (1.027 g) in dichloromethane (20 ml) containing a catalytic amount of dimethylformamide. After the addition was complete, the cooling bath was removed and the mixture was stirred at room temperature for 1 hour. The solvent was then removed in vacuo to give the crude acid chloride as a yellow-orange solid.

A solution of the above acid chloride in dichloromethane (15 ml) was added dropwise with stirring to a chilled (-70 °C) solution of 2-(dimethylamino)ethanol (0.357 g) and triethylamine (0.47 ml) in dichloromethane (10 ml). The cooling bath was removed and the reaction was stirred at room temperature for 2 hours, then was diluted with dichloromethane and washed in turn with saturated sodium bicarbonate solution and with brine twice. After the dried (Na₂SO₄) organic layer was evaporated, the gummy residue was triturated with diethyl ether, filtered to remove some insolubles, and the filtrate concentrated to dryness. The resulting solid was crystallized from ethyl acetate-hexane to yield 0.7 g of 5-[[2-(2-naphthalenyloxy)ethyl]oxy]-alpha-oxo-2-thiopheneacetic acid 2-(dimethylamino)ethyl ester as an off-white crystalline solid, mp 76-77 °C.

| | | | | |
|---|---|---|---|---|
| Analysis Calculated for C₂₂H₂₃NO₅S: | C, 63.91; | H, 5.61; | N, 3.39; | S, 7.75 |
| Found: | C, 63.62; | H, 5.57; | N, 3.46; | S, 7.58 |

### Example 7

### Preparation of 2-[[5-[[2-(2-naphthalenyloxy)ethyl]oxy)-alpha-oxo-2-thiopheneacetyl]oxy]-N,N,N-trimethylethanaminium iodide.

A solution of 5-[[2-(2-naphthalenyloxy)ethyl]oxy]-alpha-oxo-2-thiopheneacetic acid 2-(dimethylamino)ethyl ester (0.4 g) and methyl iodide (0.15 g) in acetone (7 ml) was stirred overnight at room temperature. The resulting yellow crystalline solid was filtered off, washed with acetone and then crystallized from methanol to afford 0.36 g of 2-[[5-[[2-(2-naphthalenyloxy)ethyl]oxy]-alpha-oxo-2-thiopheneacetyl]oxy]-N,N,N-trimethylethanaminium iodide, mp 169-170 °C.

| | | | | | |
|---|---|---|---|---|---|
| Analysis Calculated for C₂₃H₂₆INO₅S: | C, 49.74; | H, 4.72; | I, 22.85; | N, 2.52; | S, 5.77 |
| Found: | C, 49.68; | H, 4.60; | I, 22.67; | N, 2.57; | S, 6.06 |

### Example 8

### Preparation of 5-[[2-(cyclooctyloxy)ethyl]oxy]-alpha-oxo-2-thiopheneacetic acid ethyl ester.

As described in Example 3, ethyl oxalyl chloride (16 ml) was added dropwise with stirring to a chilled solution of 2-[[2-(cyclooctyloxy)ethyl]oxy]thiophene (10.18 g) in dichloromethane (80 ml) containing pyridine (4.0 ml). After the solution was stirred at 35 °C for 5 hours, the product was isolated in the usual way and was purified by HPLC (hexane-diethyl ether; 3:1) to give 9.7 g of 5-[[2-(cyclooctyloxy)ethyl]oxy]-alpha-oxo-2-thiopheneacetic acid ethyl ester as a yellow liquid. A sample was distilled in a Kugelrohr apparatus (∼210 °C/0.02 mm) to furnish the analytical sample.

| | | | |
|---|---|---|---|
| Analysis Calculated for C₁₈H₂₆O₅S: | C, 60.99; | H, 7.39; | S, 9.05 |
| Found: | C, 60.80; | H, 7.40; | S, 8.86 |

### Example 9

### Preparation of 5-[[2-(cyclooctyloxy)ethyl]oxy]-alpha-oxo-2-thiopheneacetic acid (5:3) hydrate.

1N Potassium hydroxide solution (10 ml) was added dropwise with stirring to a chilled solution of 5-[[2-(cyclooctyloxy)ethyl]oxy]-alpha-oxo-2-thiopheneacetic acid ethyl ester (4.43 g) in methanol (50 ml). After the reaction was stirred at 0-5 °C for 15 minutes, the product was isolated as described in Example 6 to furnish 3.9 g of 5-[[2-(cyclooctyloxy)ethyl]oxy]-alpha-oxo-2-thiopheneacetic acid as a yellow-orange oil. Crystallization of the product from wet benzene-hexane furnished 3.1 g of the hydrated acid as a pale yellow crystalline solid, mp 60-62 °C.

| | | | | |
|---|---|---|---|---|
| Analysis Calculated for C₁₆H₂₂O₅S.0.6H₂O: | C, 56.99; | H, 6.93; | S, 9.50; | H₂O, 3.20 |
| Found:. | C, 58.73; | H, 6.89; | S, 9.45; | H₂O, 3.11 |

### Example 10

### Preparation of alpha, alpha-difluoro-4-[[2-(phenoxy)ethyl]oxy]benzeneacetic acid methyl ester

To a solution of alpha-oxo-4-[(2-phenoxy)ethyl]oxy]benzeneacetic acid methyl ester (0.7 g) in 1,2-dichloroethane (30 ml) was added diethylaminosulfur trifluoride (3.08 ml) dropwise. The reaction mixture was heated to a bath temperature of 60-65 °C for 18 hours and was cooled in an ice bath. The mixture was poured carefully on to a mixture of ice and water (40 ml) and the layers were separated. The aqueous layer was extracted with dichloromethane (3 x 20ml) and the combined organic layers were washed with brine (2 x 25 ml) and dried (MgSO₄). Concentration of the extracts afforded 0.99 g of crude product which was purified by chromatography over 100 g of silica gel (hexane-dichloromethane; 3:7) followed by crystallization to give 0.39 g of alpha, alpha-difluoro-4-[[2-(phenoxy)ethyl]oxy]benzeneacetic acid methyl ester, mp 76-78 °C.

| | | | |
|---|---|---|---|
| Analysis Calculated for C₁₇H₁₆F₂O₄: | C, 63.35; | H, 5.00; | F, 11.79 |
| Found: | C, 63.19; | H, 5.02; | F, 11.73 |

### Example 11

### Preparation of alpha, alpha-difluoro-4-[[2-(phenoxy)ethyl]oxy]benzeneacetic acid

A solution of alpha, alpha-difluoro-4-[[2-(phenoxy)ethyl]oxy]benzeneacetic acid methyl ester (0.35 g) in methanol (8 ml) was treated with 1N sodium hydroxide (2.5 ml) and was heated on a steam bath for 10 minutes. The mixture was allowed to cool, then was acidified with excess 2N hydrochloric acid and extracted with ethyl acetate. The combined organic layers were washed with water and brine. Concentration of the dried (MgSO₄) extracts afforded 0.39 g of a solid which was crystallized from ethyl acetate-hexane to give 0.26 g of alpha, alpha-difluoro-4-[[2-(phenoxy)ethyl]oxy]benzeneacetic acid, mp 143-144 °C.

| | | | |
|---|---|---|---|
| Analysis Calculated for C₁₆H₁₄F₂O₄: | C, 62.33; | H, 4.58; | F, 12.33 |
| Found: | C, 62.12; | H, 4.55; | F, 12.27 |

### Example 12

### Preparation of alpha, alpha-difluoro-4-[[2-(2-naphthalenyloxy)ethyl]oxy]benzeneacetic acid methyl ester

To a solution of 4-[[2-(2-naphthalenyloxy)ethyl]oxy]-alpha-oxobenzeneacetic acid methyl ester (1.0 g) in 1,2-dichloroethane (35 ml) was added diethylaminosulfur trifluoride (3.77 ml) dropwise. The reaction mixture was heated to a bath temperature of 60-65 °C for 18 hours. After the cooled mixture was poured carefully onto 40 ml of ice and water, the layers were separated and the aqueous layer was extracted with 3 portions of dichloromethane. The combined organic layers were washed with brine, dried (MgSO₄) and evaporated to afford 1.26 g of a tan solid. The crude product was purified by chromatography over silica gel (100 g; hexane-dichloromethane,1:3) followed by crystallization from ethyl acetate to give 0.59 g of alpha, alpha-difluoro-4-[[2-(2-naphthalenyloxy)ethyl]oxy]benzeneacetic acid methyl ester, mp 123-125 °C. The mother liquors were diluted with hexane to give an additional 0.16 g mp 123-124.5 °C.

| | | | |
|---|---|---|---|
| Analysis Calculated for C₂₁H₁₈F₂O₄: | C, 67.73; | H, 4.87; | F, 10.21 |
| Found: | C, 67.64; | H, 5.11; | F, 10.50 |

### Example 13

### Preparation of alpha, alpha-difluoro-4-[[2-(2-naphthalenyloxy)ethyl]oxy]benzeneacetic acid

A suspension of alpha, alpha-difluoro-4-[[2-(2-naphthalenyloxy)ethyl]oxy] benzeneacetic acid methyl ester (0.65 g) in methanol (4 ml) and 1N sodium hydroxide (4 ml) was heated on a steam bath then methanol (150 ml), followed by acetone (350 ml) were added and the mixture was filtered hot. The resulting solid was taken up in 2N hydrochloric acid and was extracted with ethyl acetate. The organic layers were washed with brine and were dried over MgSO₄. The methanol-acetone filtrate from the reaction mixture was concentrated, acidified with excess 2N hydrochloric acid and was extracted with ethyl acetate as above. The combined organic layers from both extractions were concentrated and the residue crystallized from ethyl acetate-hexane to give 0.45 g of alpha, alpha-difluor-4-[[2-(2-naphthalenyloxy)ethyl]oxy] benzeneacetic acid, mp 173-175 °C.

| | | | |
|---|---|---|---|
| Analysis Calculated for C₂₀H₁₆F₂O₄: | C, 67.03; | H, 4.50; | F, 10.60 |
| Found: | C, 66.87; | H, 4.47; | F, 10.80 |

### Example 14

### Preparation of (S)-alpha-amino-N-[[(1,1-dimethylethyl)oxy]carbonyl]-4-hydroxybenzeneacetic acid benzyl ester

Di-*tert*-butyl dicarboxylate (31.44 g) was added dropwise to a stirred solution of (S)-4-hydroxyphenylglycine (24 g) and triethylamine (19.92 ml) in tetrahydrofuran-water (1:1; 720 ml). After the mixture was allowed to stir overnight at room temperature, ethyl acetate (600 ml) was added and the phases separated. The aqueous layer was adjusted to pH 3.0-3.5 by the addition of 6N hydrochloric acid, then was extracted with ethyl acetate (6 x 250 ml). The combined extracts were washed with brine, dried (MgSO₄), evaporated and the residual oil was triturated with hexane. The resulting solid was filtered off to give 35.9 g (S)-alpha-amino-N-[[(1,1-dimethylethyl)oxy]carbonyl]-4-hydroxybenzeneacetic acid.
A portion of the above acid (10 g) and benzyl bromide (6.4 g) was dissolved in dry dimethylformamide (150 ml). After the addition of potassium bicarbonate (3.75 g), the heterogeneous mixture was stirred overnight at room temperature and then the solvent was removed in vacuo. The residual material was partitioned between ethyl acetate and water, and the separated organic layer was washed in turn with saturated sodium bicarbonate solution, water (twice) and brine. Evaporation of the dried (MgSO₄) extract and trituration of the resulting residue with hexane furnished 11.7 g of (S)-alpha-amino-N-[[(1,1-dimethyethyl)oxy]carbonyl]-4-hydroxybenzeneacetic acid benzyl ester as a colorless solid, mp 104-106 °C. Crystallization of a portion from diethyl ether-hexane provided the analytical specimen, mp 105-106 °C, [α]_{D} +65.09° (c, 0.994, methanol).

| | | | |
|---|---|---|---|
| Analysis Calculated for C₂₀H₂₃NO₅: | C, 67.21; | H, 6.49; | N, 3.92 |
| Found: | C, 67.35; | H, 6.39; | N, 3.74 |

### Example 15

### Preparation of (S)-alpha-amino-N-[[(1,1-dimethylethyl)oxy]carbonyl]-4-[[2-(cyclohexyloxy)ethyl]oxy]benzeneacetic acid benzyl ester

A solution of diethyl azodicarboxylate (1.37 g) in dry tetrahydrofuran (10 ml) was added dropwise with stirring to a chilled (0-5 °C) solution of (S)-alpha-amino-N-[[(1,1-dimethyethyl)oxy]carbonyl]-4-hydroxybenzeneacetic acid benzyl ester (2.25 g), 2-(cyclohexyloxy)ethanol (1 g) and triphenylphosphine (2.07 g) in dry tetrahydrofuran (25 ml). After the reaction mixture was stirred at 0-5 °C for 4 hours then at room temperature overnight, the solvents were removed under reduced pressure and the residue triturated with ethyl acetate-hexane (1:3). The solids (triphenylphosphine oxide) were filtered off and the filtrate was evaporated. Purification of the residue by HPLC (ethyl acetate-hexane; 1:7) furnished 2.15 g of (S)-alpha-amino-N-[[(1,1-dimethylethyl)oxy]carbonyl]-4-[[2-(cyclohexyloxy)ethyl]oxy] benzeneacetic acid benzyl ester as an oil, [α]_{D} +48.07° (c, 1.19, methanol).

### Example 16

### Preparation of (S)-alpha-amino-N-[[(1,1-dimethylethyl)oxy]carbonyl]-4-[[2-(cyclooctyloxy)ethyl]oxy]benzeneacetic acid benzyl ester

As described in Example 15, a solution of diethyl azodicarboxylate (2.057 g) in dry tetrahydrofuran (10 ml) was added to a chilled (0-5 °C) solution of (S)-alpha-amino-N-[[(1,1-dimethylethyl)oxy]carbonyl]-4-hydroxybenzeneacetic acid benzyl ester (3.37 g), 2-(cyclooctyloxy)ethanol (1.77 g) and triphenylphosphine (3.105 g) in dry tetrahydrofuran (30 ml). After the reaction mixture was stirred at 0-5 °C for 4 hours then at room temperature overnight, the reaction was worked up in the usual manner. Purification of the crude product by HPLC (ethyl acetate-hexane; 1:7) followed by crystallization from hexane afforded 3.56 g of (S)-alpha-amino-N-[[(1,1-dimethylethyl)oxy]carbonyl]-4-[[2-(cyclooctyloxy)ethyl]oxy] benzeneacetic acid benzyl ester as a colorless solid, mp 71-73 °C; [a]_{D} +48.94° (c, 0.993, ethanol).

| | | | |
|---|---|---|---|
| Analysis Calculated for C₃₀H₄₁NO₆: | C, 70.42; | H, 8.08; | N, 2.74 |
| Found: | C, 70.33; | H, 8.12; | N, 2.82 |

### Example 17

### Preparation of (S)-alpha-amino-4-[[2-(cyclohexyloxy)ethyl]oxy]benzeneacetic acid

A solution of (S)-alpha-amino-N-[[(1,1-dimethylethyl)oxy]carbonyl]-4-[[2-(cyclohexyloxy)ethyl]oxy]benzeneacetic acid benzyl ester (2.15 g) in dichloromethane (15 mL) containing trifluoroacetic acid (15 mL) was stirred at room temperature for 1 hour. After the solvents were removed under reduced pressure, the residue was dissolved in ethyl acetate and the mixture was basified to ∼pH 9.5 with concentrated ammonium hydroxide. The organic layer was washed in turn with ammonium bicarbonate solution and brine, then was dried over anhydrous potassium carbonate (K₂CO₃) and evaporated to give 1.3 g of (S)-alpha-amino-4-[[2-(cyclohexyloxy)ethyl]oxy]benzeneacetic acid benzyl ester.
A solution of the crude ester (1.25 g) in methanol (50 mL) was hydrogenated over 10% Pd/C at ambient temperature and pressure until the uptake of hydrogen had ceased. The reaction was diluted with methanol (100 ml), heated to reflux for five minutes, then the catalyst was filtered off through a bed of Celite, and washed with hot methanol (3 x 30 mL). The combined filtrates were concentrated at reflux to ∼ 50 mL, then were cooled and the solids collected by filtration to yield 0.65 g of (S)-alpha-amino-4-[[2-(cyclohexyloxy)ethyl]oxy]benzeneacetic acid, mp 219-220 °C.

### Example 18

### Preparation of (S)-alpha-amino-4-[[2-(cyclooctyloxy)ethyl]oxy]benzeneacetic acid hydrochloride

As in Example 17, a solution of (S)-alpha-amino-N-[[(1,1-dimethylethyl)oxy] carbonyl]-4-[[2-(cyclooctyloxy)ethyl]oxy]benzeneacetic acid benzyl ester (3.5 g) in dichloromethane (12 ml) containing trifluoroacetic acid (12 ml) was stirred at room temperature for 45 minutes. The crude product was isolated in the previously described manner, was purified by HPLC (ethyl acetate-hexane; 1:1) to furnish 1.97 g of (S)-alpha-amino-4-[[2-(cyclooctyloxy)ethyl]oxy]benzeneacetic acid benzyl ester as an oil.
A solution of the benzyl ester (1.9 g) in methanol (50 ml) containing one drop of acetic acid was hydrogenated over 10% Pd/C (0.2 g) at ambient temperature and pressure for 16 hours. The reaction was diluted with methanol (100 ml), heated to reflux for five minutes, then the catalyst was filtered off through a bed of Celite, and washed with hot methanol (3 x 30 ml). The combined filtrates were evaporated to yield 1.4 g of (S)-alpha-amino-4-[[2-(cyclooctyloxy)ethyl]oxy]benzeneacetic acid as an off-white solid. A stirred slurry of the amino acid (0.35 g) in diethyl ether (6 ml) was treated with 3.5M ethanolic HCl (0.25 ml) to give 0.28 g of S)-alpha-amino-4-[[2-(cyclooctyloxy)ethyl]oxy]benzeneacetic acid hydrochloride as a colorless solid, mp 210 °C, [α]_{D} +83.21° (c, 1.092, methanol)

| | | | | |
|---|---|---|---|---|
| Analysis Calculated for C₁₈H₂₈NO₄.HCl: | C, 60.41; | H, 7.89; | Cl, 9.91; | N, 3.91 |
| Found: | C, 60.16; | H, 7.71; | Cl, 9.68; | N, 3.70 |

### Example 19

### Preparation of 4-[(phenylmethyl)oxy]-alpha-oxobenzeneacetic acid

A mixture of 4-[(phenylmethyl)oxy]-alpha-oxobenzeneacetic acid methyl ester (0.53 g) in methanol (5 ml) and 0.5N sodium hydroxide (8 ml) were heated on the steam bath for 0.5 hours while methanol was distilled out. The resulting mixture containing solid sodium salt was cooled, concentrated to remove the organic solvents, acidified with 1N hydrochloric acid (4 ml), and extracted with diethyl ether (3 x 25 ml). The organic layers were washed in turn with water (2 x 10 ml) and the combined organic layers were dried (Na₂SO₄), filtered, and evaporated to give 0.5 g of crude product. Crystallization from benzene-hexane provided 0.45 g of 4-[(phenylmethyl)oxy]-alpha-oxobenzeneacetic acid, mp 97-98 °C.

| | | |
|---|---|---|
| Analysis Calculated for C₁₅H₁₂O₄: | C, 70.31; | H, 4.72 |
| Found: | C, 70.44; | H, 4.64 |

### Example 20

### Preparation of 4-[(2-phenylethyl)oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride dispersion in mineral oil (0.175 g) and stirred for 15 minutes. The mesylate of phenethyl alcohol (1.28 g)was added and the mixture was stirred and heated under argon at 60 °C overnight. The cooled mixture was treated with glacial acetic acid (3 drops) and the volatiles were removed under vacuum. The residue was mixed with water, the product was extracted with diethyl ether (3 x 50 ml) and the organic layers were washed in turn with water. The combined organic layers were dried (Na₂SO₄), filtered, and evaporated to give crude product. The material was purified by HPLC (dichloromethane-hexane; 3:1) to provide 0.4 g of 4-[(2-phenylethyl)oxy]-alpha-oxobenzeneacetic acid methyl ester as a colorless oil.

| | | |
|---|---|---|
| Analysis Calculated for C₁₇H₁₆O₄: | C, 71.82; | H, 5.67 |
| Found: | C, 72.12; | H, 5.93 |

### Example 21

### Preparation of 4-[(2-phenylethyl)oxy]-alpha-oxobenzeneacetic acid

A mixture of 4-[(2-phenylethyl)oxy)]-alpha-oxobenzeneacetic acid methyl ester (0.4 g) in methanol and 0.5N sodium hydroxide (8 ml) was treated as in Example 19 and the crude product was crystallized from benzene-hexane to give 0.37 g of 4-[(2-phenylethyl)oxy]-alpha-oxobenzeneacetic acid as a colorless solid, mp 72-73 °C.

| | | |
|---|---|---|
| Analysis Calculated for C₁₆H₁₄O₄: | C, 71.10; | H, 5.22 |
| Found: | C, 70.86; | H, 5.12 |

### Example 22

### Preparation of 4-[(3-phenylpropyl)oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with 3-bromo-1-phenylpropane. The mixture was heated at 60 °C overnight and worked up as in Example 20. The material was purified by HPLC (dichloromethane-hexane; 4:1) to provide 0.65 g of 4-[(3-phenylpropyl)oxy]-alpha-oxobenzeneacetic acid methyl ester as a colorless oil.

| | | |
|---|---|---|
| Analysis Calculated for C₁₈H₁₈O₄: | C, 72.47; | H, 6.08 |
| Found: | C, 72.19; | H, 6.23 |

### Example 23

### Preparation of 4-[(3-phenylpropyl)oxy]-alpha-oxobenzeneacetic acid

A mixture of 4-[(3-phenylpropyl)oxy]-alpha-oxobenzeneacetic acid methyl ester (0.65 g) in methanol and 0.5N sodium hydroxide (8 ml) was treated as in Example 19. Extraction provided 0.6 g which solidified and was crystallized from benzene-hexane to give 0.45 g of colorless 4-[(3-phenylpropyl)oxy]-alpha-oxobenzeneacetic acid, mp 58-59 °C.

| | | |
|---|---|---|
| Analysis Calculated for C₁₇H₁₆O₄: | C, 71.82; | H, 5.67 |
| Found: | C, 71.66; | H, 5.78 |

### Example 24

### Preparation of 4-[(4-phenylbutyl)oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 4-phenylbutanol (1.37 g). The mixture was heated at 60 °C overnight and worked up as in Example 20. The material was purified by HPLC (dichloromethane-hexane; 2:1) to provide 0.64 g of 4-[(4-phenylbutyl)oxy]-alpha-oxobenzeneacetic acid methyl ester as a colorless oil.

| | | |
|---|---|---|
| Analysis Calculated for C₁₉H₂₀O₄: | C, 73.06; | H, 6.45 |
| Found: | C, 72.93; | H, 6.55 |

### Example 25

### Preparation of 4-[(4-phenylbutyl)oxy]-alpha-oxobenzeneacetic acid

A mixture of analytically pure 4-[(4-phenylbutyl)oxy]-alpha-oxobenzeneacetic acid methyl ester (0.575 g) in methanol and 0.5N sodium hydroxide (8 ml) was treated as in Example 19. Extraction provided 0.53 g of analytically pure 4-[(4-phenylbutyl)oxy]-alpha-oxobenzeneacetic acid which would not solidify.

| | | |
|---|---|---|
| Analysis Calculated for C₁₈H₁₈O₄: | C, 72.47; | H, 6.08 |
| Found: | C, 72.50; | H, 6.00 |

### Example 26

### Preparation of 4-[[3-(4-fluorophenyl)propyl]oxy]-alpha-oxobenzeneacetic acid methyl ester

A stirred mixture of 4-hydroxy-alpha-oxobenzeneacetic acid methyl ester (1.63 g) in dimethylformamide (20 ml) under argon was treated with 55% sodium hydride (0.394 g), stirred for 15 minutes and treated with 4-fluorophenylpropyl bromide (1.25 g). The mixture was heated at 60 °C overnight and worked up as in Example 20. The material was purified on HPLC (dichloromethane-hexane; 4:1) and crystallized from diethyl etherhexane to provide 0.67 g of 4-[[3-(4-fluorophenyl)propyl]oxy]-alpha-oxobenzeneacetic acid methyl ester, mp 42-43 °C.

| | | | |
|---|---|---|---|
| Analysis Calculated for C₁₈H₁₇FO₄: | C, 68.35; | H, 5.42; | F, 6.01 |
| Found: | C, 68.22; | H, 5.52; | F, 6.31 |

### Example 27

### Preparation of 4-[[3-(4-fluorophenyl)propyl]oxy]-alpha-oxobenzeneacetic acid

A mixture of 4-[[3-(4-fluorophenyl)propyl]oxy]-alpha-oxobenzeneacetic acid methyl ester (0.58 g) in methanol and 0.5N sodium hydroxide (8 ml) was treated as in Example 19. Extraction provided 0.55 g which solidified and was crystallized from diethyl ether-hexane to give 0.43 g of colorless 4-[[3-(4-fluorophenyl)propyl]oxy]-alpha-oxobenzeneacetic acid, mp 77-78 °C.

| | | | |
|---|---|---|---|
| Analysis Calculated for C₁₇H₁₅FO₄: | C, 67.54; | H, 5.00; | F, 6.28 |
| Found: | C, 67.32; | H, 5.07; | F, 6.53 |

### Example 28

### Preparation of (E)-4-[[3-(4-fluorophenyl)-2-propenyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with 1-(3-chloro-1-propenyl)-4-fluorobenzene (0.85 g). The mixture was heated at 60 °C overnight and worked up as in Example 20. Crystallization from diethyl ether-hexane provided 0.9 g of (E)-4-[[3-(4-fluorophenyl)-2-propenyl]oxy]-alpha-oxobenzeneacetic acid methyl ester, mp 84-85 °C.

| | | | |
|---|---|---|---|
| Analysis Calculated for C₁₈H₁₅FO₄: | C, 68.78; | H, 4.81; | F, 6.04 |
| Found: | C, 68.70; | H, 5.08; | F, 6.09 |

### Example 29

### Preparation of (E)-4-[[3-(4-fluorophenyl)-2-propenyl]oxy]-alpha-oxobenzeneacetic acid

A mixture of (E)-4-[[3-(4-fluorophenyl)-2-propenyl]oxy]-alpha-oxobenzeneacetic acid methyl ester (0.5 g) in methanol and 0.5N sodium hydroxide (8 ml) was treated as in Example 19. Extraction provided solids which were crystallized from diethyl ether-hexane to give 0.43 g of colorless (E)-4-[[3-(4-fluorophenyl)-2-propenyl]oxy]-alpha-oxobenzeneacetic acid, mp 132-133 °C.

| | | | |
|---|---|---|---|
| Analysis Calculated for C₁₇H₁₃FO₄: | C, 68.00; | H, 4.36; | F, 6.33 |
| Found: | C, 67.92; | H, 4.39; | F, 6.03 |

### Example 30

### Preparation of (E)-alpha-oxo-4-[(3-phenyl-2-propenyl)oxy]benzeneacetic acid methyl ester 0.1 molar hydrate

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (E)-(3-chloro-1-propenyl)benzene (0.763 g). The mixture was heated at 60 °C overnight and worked up as in Example 20. The material was purified by HPLC (dichloromethane-hexane; 3:1) and crystallized from diethyl ether-hexane to provide 0.8 g of (E)-alpha-oxo-4-[(3-phenyl-2-propenyl)oxy]benzeneacetic acid methyl ester 0.1 molar hydrate, mp 80-82 °C.

| | | |
|---|---|---|
| Analysis Calculated for C₁₈H₁₆O₄.0.1 H₂O: | C, 72.52; | H, 5.48 |
| Found: | C, 72.44; | H, 5.46 |

### Example 31

### Preparation of (E)-alpha-oxo-4-[(3-phenyl-2-propenyl)oxy]benzeneacetic acid

A mixture of (E)-alpha-oxo-4-[(3-phenyl-2-propenyl)oxy]benzeneacetic acid methyl ester 0.1 molar hydrate (0.7 g) in methanol and 0.5N sodium hydroxide (8 ml) was treated as in Example 19. Extraction provided solids which were crystallized from diethyl ether-hexane to give 0.55 g of colorless (E)-alpha-oxo-4-[(3-phenyl-2-propenyl)oxy] benzeneacetic acid, mp 111-112 °C.

| | | |
|---|---|---|
| Analysis Calculated for C₁₇H₁₄O₄: | C, 72.33; | H, 5.00 |
| Found: | C, 72.16; | H, 5.12 |

### Example 32

### Preparation of (Z)-alpha-oxo-4-[(3-phenyl-2-propenyl)oxy]benzeneacetic acid methyl ester

A stirred mixture of 4-hydroxy-alpha-oxobenzeneacetic acid methyl ester (1.79 g) in dimethylformamide (30 ml) under argon was treated with 55% sodium hydride (0.432 g), stirred for 15 minutes and treated with (Z)-(3-chloro-1-propenyl)benzene (1.9 g). The mixture was heated at 60 °C overnight and worked up as in Example 20. The crude dichloromethane extract was purified by HPLC (dichloromethane-hexane; 4:1) and the resulting 1.9 g of analytically pure (Z)-alpha-oxo-4-[(3-phenyl-2-propenyl)oxy] benzeneacetic acid methyl ester failed to crystallize.

| | | |
|---|---|---|
| Analysis Calculated for C₁₈H₁₆O₄: | C, 72.96; | H, 5.44 |
| Found: | C, 72.87; | H, 5.47 |

### Example 33

### Preparation of (Z)-alpha-oxo-4-[(3-phenyl-2-propenyl)oxy]benzeneacetic acid

A mixture of (Z)-alpha-oxo-4-[(3-phenyl-2-propenyl)oxy]benzeneacetic acid methyl ester (1 g) in methanol (15 ml) and 0.5N sodium hydroxide (10 ml) was treated as in Example 19. Extraction provided solids which were crystallized from diethyl ether-hexane to give 0.8 g of colorless (Z)-alpha-oxo-4-[(3-phenyl-2-propenyl)oxy]benzeneacetic acid, mp 103-105 °C.

| | | |
|---|---|---|
| Analysis Calculated for C₁₇H₁₄O₄: | C, 72.33; | H, 5.00 |
| Found: | C, 72.59; | H, 4.88 |

### Example 34

### Preparation of alpha-oxo-4-[(3-phenyl-2-propynyl)oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (3-chloro-1-propynyl)benzene (0.754 g). The mixture was heated at 60 °C overnight and worked up as in Example 20. The crude dichloromethane extract was purified by HPLC (dichloromethane-hexane; 4:1) and the resulting solids were crystallized from diethyl ether-hexane to give 0.8 g of alpha-oxo-4-[(3-phenyl-2-propynyl)oxy]benzeneacetic acid methyl ester mp 57-59 °C.

| | | |
|---|---|---|
| Analysis Calculated for C₁₈H₁₄O₄: | C, 73.46; | H, 4.79 |
| Found: | C, 73.13; | H, 4.82 |

### Example 35

### Preparation of alpha-oxo-4-[(3-phenyl-2-propynyl)oxy]benzeneacetic acid

A mixture of alpha-oxo-4-[(3-phenyl-2-propynyl)oxy]benzeneacetic acid methyl ester (0.7 g) in methanol (10 ml) and 0.5N sodium hydroxide (8 ml) was treated as in Example 19. Extraction provided solids which were crystallized from diethyl ether-hexane to give 0.55 g of colorless alpha-oxo-4-[(3-phenyl-2-propynyl)oxy]benzeneacetic acid, mp 97-99 °C.

| | | |
|---|---|---|
| Analysis Calculated for C₁₇H₁₂O₄: | C, 72.85; | H, 4.32 |
| Found: | C, 72.83; | H, 4.41 |

### Example 36

### Preparation of alpha-oxo-4-[[2-(phenoxy)ethyl]oxy]benzeneacetic acid methyl ester

A stirred mixture of 4-hydroxy-alpha-oxobenzeneacetic acid methyl ester (1.81 g) in dimethylformamide (25 ml) under argon was treated with 55% sodium hydride (0.437 g), stirred for 15 minutes and treated with the mesylate of 2-phenoxyethanol (2.38 g). The mixture was heated at 60 °C overnight and worked up as in Example 20. The crude residue in dichloromethane was passed through a plug of silica gel (5 g) and evaporated and the resulting solids were crystallized from diethyl ether-hexane to give 1.85 g of alpha-oxo-4-[[2-(phenoxy)ethyl]oxy]benzeneacetic acid methyl ester, mp 79-81 °C.

| | | |
|---|---|---|
| Analysis Calculated for C₁₇H₁₆O₅: | C, 67.99; | H, 5.37 |
| Found: | C, 67.84; | H, 5.30 |

### Example 37

### Preparation of alpha-oxo-4-[[2-(phenoxy)ethyl]oxy]benzeneacetic acid

A mixture of alpha-oxo-4-(2-phenoxyethoxy)benzeneacetic acid methyl ester (1.6 g) in methanol (10 ml) and 0.5N sodium hydroxide (30 ml )was treated as in Example 19. Extraction provided solids which were crystallized from diethyl ether-hexane to give 1.4 g of colorless alpha-oxo-4-[[2-(phenoxy)ethyl]oxy]benzeneacetic acid, mp 102-103 °C.

| | | |
|---|---|---|
| Analysis Calculated for C₁₆H₁₄O₅: | C, 67.13; | H, 4.93 |
| Found: | C, 66.90; | H, 4.90 |

### Example 38

### Preparation of 4-[[2-(2-fluorophenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(2-fluorophenoxy)ethanol (1.17 g). The mixture was heated at 60 °C overnight and worked up as in Example 20. The dichloromethane extract was evaporated and the residue was purified by HPLC (dichloromethane-hexane; 4:1) and the resulting solids were crystallized from dichloromethane-diethyl ether to give 0.8 g of 4-[[2-(2-fluorophenoxy)ethyl]oxy]-alpha-oxobenzeneacetic acid methyl ester, mp 112-113 °C.

| | | | |
|---|---|---|---|
| Analysis Calculated for C₁₇H₁₅FO₅: | C, 64.15; | H, 4.75; | F, 5.97 |
| Found: | C, 63.87; | H, 4.84; | F, 6.23 |

### Example 39

### Preparation of 4-[[2-(2-fluorophenoxy)ethyl]oxy]-alpha-oxobenzeneacetic acid

A mixture of 4-[[2-(2-fluorophenoxy)ethyl]oxy]-alpha-oxobenzeneacetic acid methyl ester (0.6 g) in methanol (20 ml) and 0.5N sodium hydroxide (8 ml) was treated as in Example 19. Extraction provided solids which were crystallized from diethyl ether-hexane to give 0.55 g of colorless 4-[[2-(2-fluorophenoxy)ethyl]oxy]-alpha-oxobenzeneacetic acid, mp 108-110 °C.

| | | | |
|---|---|---|---|
| Analysis Calculated for C₁₆H₁₃FO₅: | C, 63.16; | H, 4.31; | F, 6.24 |
| Found: | C, 63.07; | H, 4.42; | F, 6.20 |

### Example 40

### Preparation of 4-[[2-(3-fluorophenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(3-fluorophenoxy)ethanol (1.17 g). The mixture was heated at 60 °C overnight and worked up as in Example 20. The dichloromethane extract was evaporated and the residue was purified by HPLC (dichloromethane-hexane; 4:1) and the resulting solids were crystallized from diethyl ether-hexane to give 0.84 g of 4-[[2-(3-fluorophenoxy)ethyl]oxy]-alpha-oxobenzeneacetic acid methyl ester, mp 68-69 °C.

| | | | |
|---|---|---|---|
| Analysis Calculated for C₁₇H₁₅FO₅: | C, 64.15; | H, 4.75; | F, 5.97 |
| Found: | C, 63.79; | H, 4.76; | F, 6.06 |

### Example 41

### Preparation of 4-[[2-(3-fluorophenoxy)ethyl]oxy]-alpha-oxobenzeneacetic acid

A mixture of 4-[[2-(3-fluorophenoxy)ethyl]oxy]-alpha-oxobenzeneacetic acid methyl ester (0.62 g) in methanol (5 ml) and 0.5N sodium hydroxide (6 ml) was treated as in Example 19. Extraction provided solids which were crystallized from diethyl ether-hexane to give 0.5 g of colorless 4-[[2-(3-fluorophenoxy)ethyl]oxy]-alpha-oxobenzeneacetic acid, mp 108-109 °C.

| | | | |
|---|---|---|---|
| Analysis Calculated for C₁₆H₁₃FO₅: | C, 63.16; | H, 4.31; | F, 6.24 |
| Found: | C, 62.90; | H, 4.30; | F, 6.22 |

### Example 42

### Preparation of 4-[[2-(4-fluorophenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of ethyleneglycol monoparafluorophenyl ether (1.4 g). The mixture was heated at 60 °C overnight and worked up as in Example 20. The material was purified by HPLC (dichloromethane-hexane-ethyl acetate; 25:25:1) and crystallized from dichloromethane-hexane to provide 0.77 g of 4-[[2-(4-fluorophenoxy)ethyl]oxy]-alpha-oxobenzeneacetic acid methyl ester, mp 77-79 °C.

| | | | |
|---|---|---|---|
| Analysis Calculated for C₁₇H₁₅FO₅: | C, 64.15; | H, 4.75; | F, 5.97 |
| Found: | C, 63.90; | H, 4.77; | F, 5.70 |

### Example 43

### Preparation of 4-[[2-(4-fluorophenoxy)ethyl]oxy]-alpha-oxobenzeneacetic acid

A mixture of 4-[[2-(4-fluorophenoxy)ethyl]oxy]-alpha-oxobenzeneacetic acid methyl ester (0.65 g) in methanol and 0.5N sodium hydroxide (8 ml) was treated as in Example 19. Extraction with dichloromethane-diethyl ether provided solids which were crystallized from dichloromethane-hexane to give 0.53 g of colorless 4-[[2-(4-fluorophenoxy) ethyl]oxy]-alpha-oxobenzeneacetic acid, mp 97-98 °C.

| | | | |
|---|---|---|---|
| Analysis Calculated for C₁₆H₁₃FO₅: | C, 63.16; | H, 4.31; | F, 6.24 |
| Found: | C, 62.86; | H, 4.24; | F, 6.21 |

### Example 44

### Preparation of 4-[[2-(4-chlorophenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(4-chlorophenoxy)ethanol (1.25 g). The mixture was heated at 60°C overnight and worked up as in Example 20. The material from dichloromethane extraction was purified by HPLC (dichloromethane-hexane; 4:1) and crystallized from dichloromethane-hexane to provide 0.815 g of 4-[[2-(4-chlorophenoxy)ethyl]oxy]-alpha-oxobenzeneacetic acid methyl ester, mp 103-105 °C.

| | | | |
|---|---|---|---|
| Analysis Calculated for C₁₇H₁₅ClO₅: | C, 61.00; | H, 4.52; | Cl, 10.59 |
| Found: | C, 60.65; | H, 4.62; | Cl, 10.32 |

### Example 45

### Preparation of 4-[[2-(4-chlorophenoxy)ethyl]oxy]-alpha-oxobenzeneacetic acid

A mixture of 4-[[2-(4-chlorophenoxy)ethyl]oxy]-alpha-oxobenzeneacetic acid methyl ester (0.72 g) in methanol and 0.5N sodium hydroxide (8 ml) was treated as in Example 19. Extraction provided solids which were crystallized from diethyl ether-hexane to give 0.63 g of colorless 4-[[2-(4-chlorophenoxy)ethyl]oxy]-alpha-oxobenzeneacetic acid, mp 121-122 °C.

| | | | |
|---|---|---|---|
| Analysis Calculated for C₁₆H₁₃ClO₅: | C, 59.92; | H, 4.09; | Cl, 11.05 |
| Found: | C, 59.82; | H, 4.18; | Cl, 10.89 |

### Example 46

### Preparation of 4-[[2-(4-nitrophenoxy)ethyl]oxy]-alpha-oxobenzeneacetic acid methyl ester (4:1) molar hydrate

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(4-nitrophenoxy)ethanol (1.56 g). The mixture was heated at 60 °C overnight and worked up as in Example 20. The material was purified by HPLC (dichloromethane-hexane; 9:1) and crystallized from dichloromethane-hexane to provide 0.75 g of 4-[[2-(4-nitrophenoxy)ethyl]oxy]-alpha-oxobenzeneacetic acid methyl ester as a 4:1 molar hydrate, mp 122-123 °C.

| | | | |
|---|---|---|---|
| Analysis Calculated for C₁₇H₁₅NO₇.4:1H₂O: | C, 58.37; | H, 4.47; | N, 4.00 |
| Found: | C, 58.55; | H, 4.43; | N, 3.93 |

### Example 47

### Preparation of 4-[[2-(4-nitrophenoxy)ethyl]oxy]-alpha-oxobenzeneacetic acid

A mixture of 4-[[2-(4-nitrophenoxy)ethyl]oxy]-alpha-oxobenzeneacetic acid methyl ester (4:1) molar hydrate (0.65 g) in methanol and 0.5 N sodium hydroxide (8 ml) was treated as in Example 19. Extraction provided 0.6 g of crude product which solidified and was crystallized from dichloromethane-hexane to give 0.55 g of colorless 4-[[2-(4-nitrophenoxy)ethyl]oxy]-alpha-oxobenzeneacetic acid, mp 129-130 °C.

| | | | |
|---|---|---|---|
| Analysis Calculated for C₁₆H₁₃NO₇: | C, 58.01; | H, 3.96; | N, 4.23 |
| Found: | C, 58.19; | H, 3.93; | N, 4.18 |

### Example 48

### Preparation of 4-[[2-(4-methylphenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(4-methylphenoxy)ethanol (1.15 g). The mixture was heated at 60 °C overnight and worked up as in Example 20. The material was purified by HPLC (dichloromethane-hexane; 4:1) and crystallized from diethyl ether-hexane to provide 0.83 g of 4-[[2-(4-methylphenoxy)ethyl]oxy]-alpha-oxobenzeneacetic acid methyl ester, mp 105-107 °C.

| | | |
|---|---|---|
| Analysis Calculated for C₁₈H₁₈O₅: | C, 68.78; | H, 5.77 |
| Found: | C, 68.78; | H, 5.89 |

### Example 49

### Preparation of 4-[[2-(4-methylphenoxy)ethyl]oxy]-alpha-oxobenzeneacetic acid

A mixture of 4-[[2-(4-methylphenoxy)ethyl]oxy]-alpha-oxobenzeneacetic acid methyl ester (0.68 g) in methanol and 0.5N sodium hydroxide (8 ml) was treated as in Example 19. Extraction provided solids which were crystallized from diethyl ether-hexane to give 0.6 g of colorless 4-[[2-(4-methylphenoxy)ethyl]oxy]-alpha-oxobenzeneacetic acid, mp 135-136 °C.

| | | |
|---|---|---|
| Analysis Calculated for C₁₇H₁₆O₅: | C, 67.99; | H, 5.37 |
| Found: | C, 67.79; | H, 5.33 |

### Example 50

### Preparation of alpha-oxo-4-[[2-(4-trifluoromethylphenoxy)ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the tosylate of 2-(4-trifluoromethylphenoxy)ethanol (1.8 g). The mixture was heated at 60 °C overnight and worked up as in Example 20. The material was purified by HPLC (dichloromethane-hexane; 4:1) and crystallized from diethyl ether-hexane to provide 1 g of alpha-oxo-4-[[2-(4-trifluoromethylphenoxy)ethyl]oxy] benzeneacetic acid methyl ester, mp 96-98 °C.

| | | | |
|---|---|---|---|
| Analysis Calculated for C₁₈H₁₅F₃O₅: | C, 58.70; | H, 4.11; | F, 15.47 |
| Found: | C, 58.45; | H, 4.17; | F, 15.17 |

### Example 51

### Preparation of alpha-oxo-4-[[2-(4-trifluoromethylphenoxy)ethyl]oxy]benzeneacetic acid

A mixture of alpha-oxo-4-[[2-(4-trifluoromethylphenoxy)ethyl]oxy]benzeneacetic acid methyl ester (0.55 g) in methanol and 0.5N sodium hydroxide (8 ml) was treated as in Example 19. Extraction provided 0.5 g of material which solidified and was crystallized from diethyl ether-hexane to give 0.45 g of colorless alpha-oxo-4-[[2-(4-trifluoromethyl phenoxy)ethyl]oxy]benzeneacetic acid, mp 121-123 °C.

| | | | |
|---|---|---|---|
| Analysis Calculated for C₁₇H₁₃F₃O₅: | C, 57.63; | H, 3.70; | F, 16.09 |
| Found: | C, 57.53; | H, 3.76; | F, 16.00 |

### Example 52

### Preparation of 4-[[2-[4-(aminosulfonyl)phenoxy]ethyl]oxy]-alpha-oxobenzeneacetic acid methyl ester

A stirred mixture of 4-hydroxy-alpha-oxobenzeneacetic acid methyl ester (0.905 g) in dimethylformamide (10 ml) under argon was treated with 55% sodium hydride (0.218 g), stirred for 15 minutes and treated with the tosylate of 2-(4-aminosulfonylphenoxy)ethanol (1.67 g). The mixture was heated at 60 °C overnight and worked up as in Example 20. The material from ethyl acetate extraction was purified by HPLC (dichloromethane-ethyl acetate; 4:1) and crystallized from dichloromethane-hexane to provide 0.9 g of 4-[[2-[4-(amino-sulfonyl)phenoxy]ethyl]oxy]-alpha-oxobenzeneacetic acid methyl ester, mp 136-137 °C.

| | | | | |
|---|---|---|---|---|
| Analysis Calculated for C₁₇H₁₇NO₇S: | C, 53.82; | H, 4.52; | N, 3.69; | S, 8.45 |
| Found: | C, 53.65; | H, 4.63; | N, 3.66; | S, 8.26 |

### Example 53

### Preparation of 4-[[2-[4-(aminosulfonyl)phenoxy]ethyl]oxy]-alpha-oxobenzeneacetic acid (4:1) molar hydrate

A mixture of 4-[[2-[4-(aminosulfonyl)phenoxy]ethyl]oxy]-alpha-oxobenzeneacetic acid methyl ester (0.3 g) and 0.5N sodium hydroxide (6 ml) was warmed on the steam bath for 5 minutes and allowed to cool over 10 minutes. The clear solution was acidified with 1N hydrochloric acid (4 ml) and the resulting mixture chilled, filtered, and the solids washed with water. The solids were dried over phosphorus pentoxide at 70 °C and 0.1 mm to give 0.27 g of colorless 4-[[2-[4-(aminosulfonyl)phenoxy]ethyl]oxy]-alpha-oxobenzeneacetic acid as a 4:1 molar hydrate, mp 178-179 °C with decomposition.

| | | | | | |
|---|---|---|---|---|---|
| Analysis Calculated for C₁₆H₁₅NO₇S . 0.25 H₂O: | C, 51.96; | H, 4.22; | N, 3.79; | S, 8.67; | H₂O, 1.22 |
| Found: | C, 51.62; | H, 4.01; | N, 3.72; | S, 8.73; | H₂O, 1.37 |

### Example 54

### Preparation of 4,4'-[1,2-ethanediylbis(oxy)]bis(alpha-oxobenzeneacetic acid) dimethyl ester (4:1) molar hydrate

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with ethylene glycol di-p-tosylate (0.74 g). The mixture was heated at 60 °C overnight and worked up as in Example 20. Extraction with dichloromethane and crystallization from dichloromethane-hexane provided 0.46 g of 4,4'-[1,2-ethanediylbis(oxy)]bis(alpha-oxobenzeneacetic acid) dimethyl ester as an 0.25 molar hydrate, mp 144-145 °C.

| | | |
|---|---|---|
| Analysis Calculated for C₂₀H₁₈O₈ . 4:1 H₂O: | C, 61.45; | H, 4.77 |
| Found: | C, 61.66; | H, 4.78 |

### Example 55

### Preparation of 4,4'-[1,2-ethanediylbis(oxy)]bis(alpha-oxobenzeneacetic acid) (4:1) molar hydrate

A mixture of 4,4'-[1,2-ethanediylbis(oxy)bis(alpha-oxobenzeneacetic acid) dimethyl ester (4:1) molar hydrate (0.385 g) in methanol and 0.5N sodium hydroxide (8 ml) was treated as in Example 19. Extraction with tetrahydrofuran provided solids which were crystallized from acetonitrile to give 0.315 g of colorless 4,4'-[1,2-ethanediylbis(oxy)] bis(alpha-oxobenzene-acetic acid) as an 0.25 molar hydrate, mp 212-213 °C.

| | | |
|---|---|---|
| Analysis Calculated for C₁₈H₁₄O₈ . 0.25 H₂O: | C, 59.59; | H, 4.03 |
| Found: | C, 59.42; | H, 4.09 |

### Example 56

### Preparation of 4-[[2-[4-(1,1'-biphenyl)oxy]ethyl]oxy]-alpha-oxobenzeneacetic acid methyl ester (4:1) molar hydrate

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(4-phenylphenoxy)ethanol (1.4 g). The mixture was heated at 60 °C overnight and worked up as in Example 20. The crude dichloromethane extract was purified by HPLC (dichloromethane-hexane; 4:1) and the resulting solids were crystallized from dichloromethane-diethyl ether to provide 0.45 g of 4-[[2-[4-(1,1'-biphenyl)oxy]ethyl]oxy]-alpha-oxobenzeneacetic acid methyl ester as an 0.25 molar hydrate, mp 143-145 °C.

| | | |
|---|---|---|
| Analysis Calculated for C₂₃H₂₀O₅ . 0.25 H₂O: | C, 72.52; | H, 5.42 |
| Found: | C, 72.84; | H, 5.46 |

### Example 57

### Preparation of 4-[[2-[4-(1,1'-biphenyl)oxy]ethyl]oxy]-alpha-oxobenzeneacetic acid (4:1) molar hydrate

A mixture of 4-[[2-[4-(1,1'-biphenyl)oxy]ethyl]oxy]-alpha-oxobenzeneacetic acid methyl ester (4:1) molar hydrate (0.35 g) in methanol and 0.5N sodium hydroxide (8 ml) was treated as in Example 19. Dichloromethane extraction provided 0.3 g of the acid which solidified and was crystallized from dichloromethane-diethyl ether to give 0.25 g of colorless 4-[[2-[4-(1,1'-biphenyl)oxy]ethyl]oxy]-alpha-oxobenzeneacetic acid as an 0.25 molar hydrate, mp 179-180 °C.

| | | |
|---|---|---|
| Analysis Calculated for C₂₂H₁₈O₅ . 0.25 H₂O: | C, 72.02; | H, 5.08 |
| Found: | C, 72.25; | H, 5.20 |

### Example 58

### Preparation of alpha-oxo-4-[[2-(4-phenoxyphenoxy)ethyl]oxy]-benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(4-phenoxyphenoxy)ethanol (1.5 g). The mixture was heated at 60 °C overnight and worked up as in Example 20. The material from dichloromethane extraction was purified by HPLC (dichloromethane-hexane; 4:1) and crystallized from diethyl ether-hexane to provide 1.1 g of alpha-oxo-4-[[2-(4-phenoxy-phenoxy)ethyl]oxy]benzeneacetic acid methyl ester, mp 97-98 °C.

| | | |
|---|---|---|
| Analysis Calculated for C₂₃H₂₀O₆: | C, 70.40; | H, 5.14 |
| Found: | C, 70.02; | H, 5.30 |

### Example 59

### Preparation of alpha-oxo-4-[[2-(4-phenoxyphenoxy)ethyl]oxy]benzeneacetic acid

A mixture of 4-[[2-[4-(1,1'-biphenyl)oxy]ethyl]oxy]-alpha-oxobenzeneacetic acid methyl ester (0.8 g) in methanol (20 ml) and 0.5N sodium hydroxide (8 ml) was treated as in Example 19. Extraction provided solids which were crystallized from diethyl ether-hexane to give 0.55 g of colorless alpha-oxo-4-[[2-(4-phenoxyphenoxy)ethyl]oxy] benzeneacetic acid, mp 124-126 °C.

| | | |
|---|---|---|
| Analysis Calculated for C₂₂H₁₈O₆: | C, 69.84; | H, 4.79 |
| Found: | C, 69.83; | H, 4.93 |

### Example 60

### Preparation of 4-[[2-(4-Methoxyphenoxy]ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of ethyleneglycol monoparamethoxyphenyl ether (1.5 g). The mixture was heated at 60 °C overnight and worked up as in Example 20. The material from dichloromethane extraction was purified by HPLC (dichloromethane-ethyl acetate; 50:1) and crystallized from dichloromethane-hexane to provide 0.875 g of 4-[[2-(4-methoxyphenoxy)ethyl]oxy]-alpha-oxobenzeneacetic acid methyl ester, mp 114-115 °C.

| | | |
|---|---|---|
| Analysis Calculated for C₁₈H₁₈O₆: | C, 65.45; | H, 5.49 |
| Found: | C, 65.84; | H, 5.56 |

### Example 61

### Preparation of 4-[[2-(4-methoxyphenoxy)ethyl]oxy]-alpha-oxobenzeneacetic acid

A mixture of 4-[[2-(4-methoxyphenoxy)ethyl]oxy]-alpha-oxobenzeneacetic acid methyl ester (0.77 g) in methanol and 0.5N sodium hydroxide (8 ml) was treated as in Example 19. Extraction with dichloromethane provided material which solidified and was crystallized from diethyl ether-hexane to give 0.65 g of colorless 4-[[2-(4-methoxyphenoxy)ethyl]oxy]-alpha-oxobenzeneacetic acid, mp 138-139 °C.

| | | |
|---|---|---|
| Analysis Calculated for C₁₇H₁₆O₆: | C, 64.55; | H, 5.10 |
| Found: | C, 64.45; | H, 5.04 |

### Example 62

### Preparation of 4-[[2-(3,4-dimethoxyphenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(3,4-dimethoxyphenoxy)ethanol (1.65 g). The mixture was heated at 60 °C overnight and worked up as in Example 20. The material was purified by HPLC (dichloromethane-diethyl ether; 99:1) and crystallized from dichloromethane-hexane to provide 0.9 g of 4-[[2-(3,4-dimethoxyphenoxy)ethyl] oxy]-alpha-oxobenzeneacetic acid methyl ester, mp 93-94 °C.

| | | |
|---|---|---|
| Analysis Calculated for C₁₉H₂₀O₇: | C, 63.33; | H, 5.59 |
| Found: | C, 63.05; | H, 5.51 |

### Example 63

### Preparation of 4-[[2-(3,4-dimethoxyphenoxy)ethyl]oxy]-alpha-oxobenzeneacetic acid

A mixture of 4-[[2-(3,4-dimethoxyphenoxy)ethyl]oxy]-alpha-oxobenzeneacetic acid methyl ester (0.7 g) in methanol and 0.5N sodium hydroxide (8 ml) was heated on the steam bath for 0.5 hours and cooled. 2N hydrochloric acid (5 ml) was added and the resulting solids were filtered and washed with water. The solids were dried by boiling in benzene (20 ml), filtered, hexane (5 ml) was added, and the resulting mixture was chilled to give 0.63 g of colorless 4-[[2-(3,4-dimethoxyphenoxy)ethyl]oxy]-alpha-oxobenzeneacetic acid, mp 140-141 °C.

| | | |
|---|---|---|
| Analysis Calculated for C₁₈H₁₈O₇: | C, 62.42; | H, 5.24 |
| Found: | C, 62.70; | H, 4.90 |

### Example 64

### Preparation of 4-[[2-(3,4,5-trimethoxyphenoxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(3,4,5-trimethoxyphenoxy)ethanol (1.8 g). The mixture was heated at 60 °C overnight and worked up as in Example 20. The material form dichloromethane extraction was purified by HPLC (dichloromethane-diethyl ether; 50:1) and crystallized from dichloromethane-hexane to provide 1 g of 4-[[2-(3,4,5-trimethoxyphenoxy)ethyl]oxy]-alpha-oxobenzeneacetic acid methyl ester, mp 82-83 °C.

| | | |
|---|---|---|
| Analysis Calculated for C₂₀H₂₂O₈: | C, 61.53; | H, 5.68 |
| Found: | C, 60.82; | H, 5.71 |

### Example 65

### Preparation of 4-[[2-(3,4,5-trimethoxyphenoxy)ethyl]oxy]-alpha-oxobenzeneacetic acid

A mixture of 4-[[2-(3,4,5-trimethoxyphenoxy)ethyl]oxy]-alpha-oxobenzeneacetic acid methyl ester (0.9 g) in methanol and 0.5N sodium hydroxide (8 ml) was heated on the steam bath for 0.5 hours, chilled, and 2N hydrochloric acid (5 ml) was added. The resulting solids were filtered, washed with water and then boiled in benzene to dry. Filtration, evaporation and crystallization from diethyl ether-hexane provided 0.625 g of colorless 4-[[2-(3,4,5-trimethoxyphenoxy)ethyl]oxy]-alpha-oxobenzeneacetic acid, mp 118-119 °C.

| | | |
|---|---|---|
| Analysis Calculated for C₁₉H₂₀O₈: | C, 60.64; | H, 5.36 |
| Found: | C, 60.53; | H, 5.30 |

### Example 66

### Preparation of alpha-oxo-4-[[(phenoxy)methyl]oxy]benzeneacetic acid methyl ester

A stirred mixture of 4-hydroxy-alpha-oxobenzeneacetic acid methyl ester (1.0 g) in dimethylformamide (10 ml) under argon was treated with 55% sodium hydride (0.262 g), stirred for 15 minutes and treated with chloromethoxybenzene (1.1 g). The mixture was heated at 60 °C for 1.5 hours and worked up as in Example 20. The material was crystallized from diethyl ether-hexane to provide 1 g of alpha-oxo-4-[[(phenoxy)methyl] oxy]benzeneacetic acid methyl ester, mp 52-54 °C.

| | | |
|---|---|---|
| Analysis Calculated for C₁₆H₁₄O₅: | C, 67.13; | H, 4.93 |
| Found: | C, 67.20; | H, 4.90 |

### Example 67

### Preparation of alpha-oxo-4-[[(phenoxy)methyl]oxy]benzeneacetic acid (5:1) molar hydrate

A mixture of alpha-oxo-4-[[(phenoxy)methyl]oxy]benzeneacetic acid methyl ester (0.43 g) in methanol and 0.5N sodium hydroxide (4 ml) was treated as in Example 19. Extraction provided 0.415 g which solidified and was crystallized from benzene-hexane to give 0.32 g of colorless alpha-oxo-4-[[(phenoxy)methyl]oxy]benzeneacetic acid as an 0.2 molar hydrate, mp 72-74 °C.

| | | | |
|---|---|---|---|
| Analysis Calculated for C₁₅H₁₂O₅ . 0.2 H₂O: | C, 65.31; | H, 4.53; | H₂O, 1.31 |
| Found: | C, 65.28; | H, 4.74; | H₂O, 1.24 |

### Example 68

### Preparation of alpha-oxo-4-[[(3-phenoxy)propyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (3-bromopropoxy)benzene (1.3 g). The mixture was heated at 60 °C overnight and worked up as in Example 20. The material from dichloromethane extraction was purified by HPLC (dichloromethane-hexane; 3:1) and crystallized from dichloromethane-hexane to provide 0.7 g of alpha-oxo-4-[[(3-phenoxy) propyl]oxy]benzeneacetic acid methyl ester, mp 61-62 °C.

| | | |
|---|---|---|
| Analysis Calculated for C₁₈H₁₈O₅: | C, 68.78; | H, 5.77 |
| Found: | C, 68.83; | H, 5.73 |

### Example 69

### Preparation of alpha-oxo-4-[[(3-phenoxy)propyl]oxy]benzeneacetic acid

A mixture of alpha-oxo-4-[[(3-phenoxy)propyl]oxy]benzeneacetic acid methyl ester (0.63 g) in methanol and 0.5N sodium hydroxide (8 ml) was treated as in Example 19. Extraction with dichloromethane provided material which was crystallized from diethyl ether-hexane to give 0.34 g of colorless alpha-oxo-4-[[(3-phenoxy)propyl]oxy] benzeneacetic acid, mp 50-52 °C.

| | | |
|---|---|---|
| Analysis Calculated for C₁₇H₁₆O₅: | C, 67.99; | H, 5.37 |
| Found: | C, 67.92; | H, 5.35 |

### Example 70

### Preparation of alpha-oxo-4-[[(4-phenoxy)butyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (4-bromobutoxy)benzene (1.37 g). The mixture was heated at 60 °C overnight and worked up as in Example 20. The material from dichloromethane extraction was purified by HPLC (dichloromethane-hexane; 3:1) and crystallized from dichloromethane-hexane to provide 0.85 g of alpha-oxo-4-[[(4-phenoxy)butyl]oxy]benzeneacetic acid methyl ester, mp 57-59 °C.

| | | |
|---|---|---|
| Analysis Calculated for C₁₉H₂₀O₅: | C, 69.50; | H, 6.14 |
| Found: | C, 69.38; | H, 6.00 |

### Example 71

### Preparation of alpha-oxo-4-[[(4-phenoxy)butyl]oxy]benzeneacetic acid

A mixture of alpha-oxo-4-[[(4-phenoxy)butyl]oxy]benzeneacetic acid methyl ester (0.85 g) in methanol and 0.5N sodium hydroxide (8 ml) was treated as in Example 19. Extraction provided material which was crystallized from diethyl ether-hexane to give 0.7 g of colorless alpha-oxo-4-[[(4-phenoxy)butyl]oxy]benzeneacetic acid, mp 103-105 °C.

| | | |
|---|---|---|
| Analysis Calculated for C₁₈H₁₈O₅: | C, 68.78; | H, 5.77 |
| Found: | C, 68.95; | H, 5.91 |

### Example 72

### Preparation of alpha-oxo-4-[[(5-phenoxy)pentyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (5-bromopentoxy)benzene (1.4 g). The mixture was heated at 60 °C overnight and worked up as in Example 20. The material from dichloromethane extraction was purified by HPLC (dichloromethane-hexane; 4:1) to provide 0.9 g of analytically pure alpha-oxo-4-[[(5-phenoxy)pentyl]oxy]benzeneacetic acid methyl ester which failed to crystallize.

| | | |
|---|---|---|
| Analysis Calculated for C₂₀H₂₂O₅: | C, 70.16; | H, 6.48 |
| Found: | C, 70.01; | H, 6.24 |

### Example 73

### Preparation of alpha-oxo-4-[[(5-phenoxy)pentyl]oxy]benzeneacetic acid

A mixture of alpha-oxo-4-[[(5-phenoxy)pentyl]oxy]benzeneacetic acid methyl ester (0.9 g) in methanol and 0.5N sodium hydroxide (10 ml) was treated as in Example 19. Extraction provided material which was crystallized from benzene-hexane to give 0.77 g of colorless alpha-oxo-4-[[(5-phenoxy)pentyl]oxy]benzeneacetic acid, mp 69-70 °C.

| | | |
|---|---|---|
| Analysis Calculated for C₁₉H₂₀O₅: | C, 69.50; | H, 6.14 |
| Found: | C, 69.49; | H, 6.12 |

### Example 74

### Preparation of alpha-oxo-4-[[(6-phenoxy)hexyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with (6-bromohexyloxy)benzene (1.5 g). The mixture was heated at 60 °C overnight and worked up as in Example 20. The material from dichloromethane extraction was purified by HPLC (dichloromethane-hexane; 5:1) and crystallized from diethyl ether-hexane to provide 0.95 g of alpha-oxo-4-[[(6-phenoxy)hexyl]oxy]benzeneacetic acid methyl ester, mp 50-52 °C.

| | | |
|---|---|---|
| Analysis Calculated for C₂₁H₂₄O₅: | C, 70.77; | H, 6.79 |
| Found: | C, 71.01; | H, 6.62 |

### Example 75

### Preparation of alpha-oxo-4-[[(6-phenoxy)hexyl]oxy]benzeneacetic acid

A mixture of alpha-oxo-4-[[(6-phenoxy)hexyl]oxy]benzeneacetic acid methyl ester (0.75 g) in methanol and 0.5N sodium hydroxide (8 ml) was treated as in Example 19. Extraction provided solids which were crystallized from diethyl ether-hexane to give 0.5 g of colorless alpha-oxo-4-[[(6-phenoxy)hexyl]oxy]benzeneacetic acid, mp 121-122 °C.

| | | |
|---|---|---|
| Analysis Calculated for C₂₀H₂₂O₅: | C, 70.16; | H, 6.48 |
| Found: | C, 70.29; | H, 6.43 |

### Example 76

### Preparation of 4-[[2-[[2-(phenoxy)ethyl]oxy]ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of diethyleneglycol monophenyl ether (1.56 g). The mixture was heated at 60 °C overnight and worked up as in Example 20. The material was purified by HPLC (dichloromethane-ethyl acetate; 50:1) and crystallized from dichloromethane-hexane to provide 0.9 g of 4-[[2-[[2-(phenoxy)ethyl]oxy] ethyl]oxy]-alpha-oxobenzeneacetic acid methyl ester, mp 75-76 °C.

| | | |
|---|---|---|
| Analysis Calculated for C₁₉H₂₀O₆: | C, 66.27; | H, 5.85 |
| Found: | C, 66.09; | H, 5.75 |

### Example 77

### Preparation of 4-[[2-[[2-(phenoxy)ethyl]oxy]ethyl]oxy]-alpha-oxobenzeneacetic acid

A mixture of 4-[[2-[[2-(phenoxy)ethyl]oxy]ethyl]oxy]-alpha-oxobenzeneacetic acid methyl ester (0.7 g) in methanol and 0.5N sodium hydroxide (8 ml) was treated as in Example 19. Extraction provided solids which were crystallized from benzene-hexane to give 0.6 g of colorless 4-[[2-[[2-(phenoxy)ethyl]oxy]ethyl]oxy]-alpha-oxobenzeneacetic acid, mp 58-59 °C.

| | | |
|---|---|---|
| Analysis Calculated for C₁₈H₁₈O₆: | C, 65.45; | H, 5.49 |
| Found: | C, 65.32; | H, 5.47 |

### Example 78

### Preparation of 4,4'-[oxybis(2,1-ethanedienyloxy)]bis(alpha-oxobenzeneacetic acid)

A stirred mixture of 4-hydroxybenzoylformic acid sodium salt (1.13 g) in dimethylsulfoxide (10 ml) under argon was treated with 4N sodium hydroxide (1.5 ml), stirred for 15 minutes and treated with the bis-mesylate of diethyleneglycol (0.786 g). The mixture was heated at 60 °C overnight and poured into excess hydrochloric acid. The product was extracted with ethyl acetate, washed with water, dried (Na₂SO₄), filtered, and evaporated to give 1.1 g of crude solid. Crystallization from ethyl acetate-hexane and recrystallization from acetonitrile provided 0.2 g of 4,4'-[oxybis(2,1-ethanedienyloxy)] bis(alpha-oxobenzeneacetic acid) as a yellow solid, mp 158-160 °C.

| | | |
|---|---|---|
| Analysis Calculated for C₂₀H₁₈O₉: | C, 59.70; | H, 4.51 |
| Found: | C, 59.55; | H, 4.59 |

### Example 79

### Preparation of rac.-4-[[(2-hydroxy-3-phenoxy)propyl]oxy]-alpha-oxobenzeneacetic acid methyl ester

A stirred mixture of 4-hydroxy-alpha-oxobenzeneacetic acid methyl ester (2.71 g) in dimethylformamide (20 ml) under argon was treated with 55% sodium hydride (0.65 g), stirred for 15 minutes and treated with epichlorohydrin (12 ml). The mixture was heated at 60 °C for four hours and worked up as in Example 20. The material was purified by HPLC (dichloromethane-diethyl ether; 50:1) to provide 2.4 g of 4-(oxiranylmethoxy)-alpha-oxobenzeneacetic acid methyl ester, as an oil.

| | | |
|---|---|---|
| Analysis Calculated for C₁₂H₁₂O₅: | C, 61.02; | H, 5.12 |
| Found: | C, 60.84; | H, 5.29 |

A mixture of 4-(oxiranylmethoxy)-alpha-oxobenzeneacetic acid methyl ester (0.944 g), phenol (0.94 g) and pyridine (1 drop) were heated at 100 °C under argon for 3 hours and cooled. The product was purified by HPLC (dichloromethane-diethyl ether; 20:1) to give 1 g of analytically pure rac.-4-[[(2-hydroxy-3-phenoxy)propyl]oxy]-alpha-oxobenzeneacetic acid methyl ester as an oil.

| | | |
|---|---|---|
| Analysis Calculated for C₁₈H₁₈O₆: | C, 65.45; | H, 5.49 |
| Found: | C, 65.19; | H, 5.32 |

### Example 80

### Preparation of rac.-4-[[(2-hydroxy-3-phenoxy)propyl]oxy]-alpha-oxobenzeneacetic acid

A mixture of rac.-4-[[(2-hydroxy-3-phenoxy)propyl]oxy]-alpha-oxobenzeneacetic acid methyl ester (0.8 g) in methanol and 0.5N sodium hydroxide (8 ml) was treated as in Example 19. Extraction with dichloromethane provided material which was boiled in benzene to dry and was crystallized from dichloromethane to give 0.52 g of colorless rac.-4-[[(2-hydroxy-3-phenoxy)propyl]oxy]-alpha-oxobenzeneacetic acid, mp 132-133 °C.

| | | |
|---|---|---|
| Analysis Calculated for C₁₇H₁₆O₆: | C, 64.55; | H, 5.10 |
| Found: | C, 63.84; | H, 4.65 |

### Example 81

### Preparation of alpha-oxo-4-[[2-(phenylthio)ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-phenylthioethanol (1.16 g). The mixture was heated at 60 °C for five hours and worked up as in Example 20. The material was purified by HPLC (dichloromethane-hexane; 4:1) and crystallized from diethyl ether-hexane to provide 0.5 g of alpha-oxo-4-[[2-(phenylthio)ethyl]oxy]benzeneacetic acid methyl ester, mp 49-51 °C.

| | | | |
|---|---|---|---|
| Analysis Calculated for C₁₇H₁₆O₄S: | C, 64.54; | H, 5.10; | S, 10.13 |
| Found: | C, 64.58; | H, 5.33; | S, 9.99 |

### Example 82

### Preparation of alpha-oxo-4-[[2-(phenylthio)ethyl]oxy]benzeneacetic acid

A mixture of alpha-oxo-4-[[2-(phenylthio)ethyl]oxy]benzeneacetic acid methyl ester (0.5 g) in methanol and 0.5N sodium hydroxide (6 ml) was treated as in Example 19. Extraction provided material which was crystallized from benzene-hexane to give 0.43 g of colorless alpha-oxo-4-[[2-(phenylthio)ethyl]oxy]benzeneacetic acid, mp 72-73 °C.

| | | | |
|---|---|---|---|
| Analysis Calculated for C₁₆H₁₄O₄S: | C, 63.56; | H, 4.67; | S, 10.60 |
| Found: | C, 63.39; | H, 4.69; | S, 10.50 |

### Example 83

### Preparation of 4-[[2-(1-naphthalenyloxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(1-naphthyloxy)ethanol (1.37 g). The mixture was heated at 60 °C overnight and worked up as in Example 20. The material was purified by HPLC (dichloromethane-hexane; 4:1) and crystallized from diethyl ether-hexane to provide 0.8 g of 4-[2-(1-naphthalenyloxy)ethyl]oxy]-alpha-oxobenzeneacetic acid methyl ester, mp 87-91 °C.

| | | |
|---|---|---|
| Analysis Calculated for C₂₁H₁₈O₅: | C, 71.99; | H, 5.18 |
| Found: | C, 72.05; | H, 5.25 |

### Example 84

### Preparation of 4-[[2-(1-naphthalenyloxy)ethyl]oxy]-alpha-oxobenzeneacetic acid

A mixture of 4-[[2-(1-naphthalenyloxy)ethyl]oxy]-alpha-oxobenzeneacetic acid methyl ester (0.7 g) in methanol and 0.5N sodium hydroxide(6 ml) was treated as in Example 19. Extraction with dichloromethane provided material which was crystallized from dichloromethane-diethyl ether to give 0.54 g of colorless 4-[[2-(1-naphthalenyloxy)ethyl]oxy]-alpha-oxobenzeneacetic acid, mp 124-125 °C after drying over phosphorus pentoxide at 60 °C and 0.1 mm.

| | | |
|---|---|---|
| Analysis Calculated for C₂₀H₁₆O₅: | C, 71.42; | H, 4.79 |
| Found: | C, 71.45; | H, 4.98 |

### Example 85

### Preparation of 4-[[2-(2-naphthalenyloxy)ethyl]oxy]-alpha-oxobenzeneacetic acid methyl ester

A stirred mixture of 4-hydroxy-alpha-oxobenzeneacetic acid methyl ester (3.62 g) in dimethylformamide (40 ml) under argon was treated with 55% sodium hydride (0.875 g), stirred for 15 minutes and treated with the mesylate of 2-(2-naphthyloxy)ethanol (5.48 g). The mixture was heated at 60 °C overnight and worked up as in Example 20. The material from dichloromethane extraction was crystallized from dichloromethane-hexane to provide 4.2 g of 4-[[2-(2-naphthalenyloxy)ethyl]oxy]-alpha-oxobenzeneacetic acid methyl ester, mp 155-157 °C.

| | | |
|---|---|---|
| Analysis Calculated for C₂₁H₁₈O₅: | C, 71.99; | H, 5.18 |
| Found: | C, 71.75; | H, 5.32 |

### Example 86

### Preparation of 4-[[2-(2-naphthalenyloxy)ethyl]oxy]-alpha-oxobenzeneacetic acid

A mixture of 4-[[2-(2-naphthalenyloxy)ethyl]oxy]-alpha-oxobenzeneacetic acid methyl ester (3.2 g) in 95% ethanol (700 ml) was heated at reflux and 1N sodium hydroxide (15 ml) was added dropwise. Heating was continued for five minutes and the resulting mixture was chilled in ice, filtered, washed with water and dried at 80 °C over phosphorus pentoxide at 0.1 mm to give 3.2 g of colorless 4-[[2-(2-naphthalenyloxy)ethyl]oxy]-alpha-oxobenzeneacetic acid sodium salt, mp > 295 °C with decomposition.

| | | | |
|---|---|---|---|
| Analysis Calculated for C₂₀H₁₅O₅Na: | C, 67.04; | H, 4.22; | Na, 6.42 |
| Found: | C, 66.76; | H, 4.35; | Na, 6.71 |

A mixture of 4-[[2-(2-naphthalenyloxy)ethyl]oxy]-alpha-oxobenzeneacetic acid sodium salt (3.0 g), dichloromethane (500 ml), and 1N hydrochloric acid (25 ml) was stirred for one hour until all solids dissolved. The layers were separated, the water layer was extracted 2 x 100 ml of dichloromethane and the organic layers were washed in turn with water. The combined organic layers were dried (Na₂SO₄), filtered and evaporated to give 2.8 g of crude product. Crystallization from dichloromethane-hexane provided 2.6 g of 4-[[2-(2-naphthalenyloxy)ethyl]oxy]-alpha-oxobenzeneacetic acid, mp 170-171 °C.

| | | |
|---|---|---|
| Analysis Calculated for C₂₀H₁₆O₅: | C, 71.42; | H, 4.79 |
| Found: | C, 71.08; | H, 4.94 |

Solid 4-[[2-(2-naphthalenyloxy)ethyl]oxy]-alpha-oxobenzeneacetic acid (1.5 g) was added to a stirred solution of diethanolamine (0.53 g) in anhydrous ethanol (30 ml) and the mixture was heated until all solids dissolved. The resulting solution was chilled and the solids were filtered, washed with cold ethanol and dried to give 1.8 g of 4-[[2-(2-naphthalenyloxy)ethyl] oxy]-alpha-oxobenzeneacetic acid (1:1) 2,2'-iminobis(ethanol) salt, mp 144-145 °C with decomposition.

| | | | |
|---|---|---|---|
| Analysis Calculated for C₂₀H₁₆O₅ .1:1 C₄H₁₁NO₂: | C, 65.29; | H, 6.16; | N, 3.17 |
| Found: | C, 64.99; | H, 6.31; | N, 3.11 |

### Example 87

### Preparation of 4-[[4-(2-naphthalenyloxy)butyl]oxy]-alpha-oxobenzeneacetic acid methyl ester

A stirred mixture of 4-hydroxy-alpha-oxobenzeneacetic acid methyl ester (0.724 g )in dimethylformamide (10 ml) under argon was treated with 55% sodium hydride (0.175 g), stirred for 15 minutes and treated with 2-(4-bromobutoxy)naphthlene (1.12 g). The mixture was stirred and heated under argon at 60 °C overnight and worked up as in Example 20. The material from dichloromethane extraction was crystallized from dichloromethane-diethyl ether to provide 1.1 g of 4-[[4-(2-naphthalenyloxy)butyl]oxy]-alpha-oxobenzeneacetic acid methyl ester, mp 93-95 °C.

| | | |
|---|---|---|
| Analysis Calculated for C₂₃H₂₂O₅: | C, 73.00; | H, 5.86 |
| Found: | C, 72.98; | H, 5.94 |

### Example 88

### Preparation of 4-[[4-(2-naphthalenyloxy)butyl]oxy]-alpha-oxobenzeneacetic acid

A mixture of 4-[[4-(2-naphthalenyloxy)butyl]oxy]-alpha-oxobenzeneacetic acid methyl ester (0.9 g) in methanol (15 ml), acetone (5 ml), and 0.5N sodium hydroxide (10 ml) was treated as in Example 19. Extraction with dichloromethane provided material which was crystallized from diethyl ether-hexane to give 0.43 g of colorless 4-[[4-(2-naphthalenyloxy)butyl]oxy]-alpha-oxobenzeneacetic acid, mp 133-135 °C.

| | | |
|---|---|---|
| Analysis Calculated for C₂₂H₂₀O₅: | C, 72.51; | H, 5.53 |
| Found: | C, 72.31; | H, 5.52 |

### Example 89

### Preparation of 4-[[2-(2-naphthalenylthio)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(2-naphthylthio)ethanol (1.4 g). The mixture was heated at 60 °C overnight and worked up as in Example 20. The material from dichloromethane extraction was purified by HPLC (dichloromethane-hexane; 4:1) and crystallized from diethyl ether-hexane to provide 0.55 g of 4-[[2-(2-naphthalenylthio)ethyl]oxy]-alpha-oxobenzeneacetic acid methyl ester, mp 63-65 °C.

| | | | |
|---|---|---|---|
| Analysis Calculated for C₂₁H₁₈O₄S: | C, 68.83; | H, 4.95; | S, 8.75 |
| Found: | C, 68.66; | H, 5.12; | S, 8.88 |

### Example 90

### Preparation of 4-[[2-(2-naphthalenylthio)ethyl]oxy]-alpha-oxobenzeneacetic acid

A mixture of 4-[[2-(2-naphthalenylthio)ethyl]oxy]-alpha-oxobenzeneacetic acid methyl ester (0.495 g) in methanol and 0.5N sodium hydroxide (5 ml) was treated as in Example 19. Extraction with dichloromethane provided material which was crystallized from diethyl ether-hexane to give 0.4 g of colorless 4-[[2-(2-naphthalenylthio)ethyl]oxy]-alpha-oxobenzeneacetic acid, mp 121-123 °C.

| | | | |
|---|---|---|---|
| Analysis Calculated for C₂₀H₁₆O₄S: | C, 68.17; | H, 4.58; | S, 9.10 |
| Found: | C, 67.87; | H, 4.41; | S, 9.36 |

### Example 91

### Preparation of 4-[[4-(2-naphthalenylthio)butyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate prepared from 4-(2-naphthylthio)butanol (1.55 g). The mixture was stirred and heated under argon at 60 °C overnight and worked up as in Example 20. The material from dichloromethane extraction was purified by HPLC (dichloromethane-hexane; 3:1) and crystallized from diethyl ether-hexane to provide 0.625 g of 4-[[4-(2-naphthalenylthio)butyl]oxy]-alpha-oxobenzeneacetic acid methyl ester, mp 64-65 °C.

| | | | |
|---|---|---|---|
| Analysis Calculated for C₂₃H₂₂O₄S: | C, 70.03; | H, 5.62; | S, 8.13 |
| Found: | C, 69.77; | H, 5.59; | S, 8.04 |

### Example 92

### Preparation of

### 4-[[4-(2-naphthalenylthio)butyl]oxy]-alpha-oxobenzeneacetic acid

A mixture of 4-[[4-(2-naphthalenylthio)butyl]oxy]-alpha-oxobenzeneacetic acid methyl ester (0.53 g) in methanol (5 ml), acetone (2 ml), and 0.5N sodium hydroxide (4 ml) was treated as in Example 19. Extraction with dichloromethane provided material which was crystallized from diethyl ether-hexane to give 0.48 g of colorless 4-[[4-(2-naphthalenylthio) butyl]oxy]-alpha-oxobenzeneacetic acid, mp 113-115 °C.

| | | | |
|---|---|---|---|
| Analysis Calculated for C₂₂H₂₀O₄S: | C, 69.45; | H, 5.30; | S, 8.43 |
| Found: | C, 69.24; | H, 5.24; | S, 8.13 |

### Example 93

### Preparation of 4-[[3-(2-naphthalenyl)propyl]oxy]-alpha-oxobenzeneacetic acid methyl ester

A stirred mixture of 4-hydroxy-alpha-oxobenzeneacetic acid methyl ester (1.01 g) in dimethylformamide (10 ml) under argon was treated with 55% sodium hydride (0.262 g), stirred for 15 minutes and treated with 2-(3-bromopropyl)naphthalene (1.7g). The mixture was heated at 60 °C overnight and worked up as in Example 20. The material from dichloromethane extraction was purified by HPLC (dichloromethane-hexane; 4:1) and crystallized from diethyl ether-hexane to provide 0.4 g of 4-[[3-(2-naphthalenyl) propyl]oxy]-alpha-oxobenzeneacetic acid methyl ester, mp 97-99 °C.

| | | |
|---|---|---|
| Analysis Calculated for C₂₂H₂₀O₄: | C, 75.84; | H, 5.79 |
| Found: | C, 75.84; | H, 5.80 |

### Example 94

### Preparation of 4-[[3-(2-naphthalenyl)propyl]oxy]-alpha-oxobenzeneacetic acid

A mixture of 4-[[3-(2-naphthalenyl)propyl]oxy]-alpha-oxobenzeneacetic acid methyl ester (0.6 g) in methanol (10 ml) and 0.5N sodium hydroxide (4 ml) was treated as in Example 19. Extraction with dichloromethane provided material which was crystallized from dichloromethane-hexane to give 0.5 g of colorless 4-[[3-(2-naphthalenyl)propyl]oxy]-alpha-oxobenzeneacetic acid, mp 123-124 °C.

| | | |
|---|---|---|
| Analysis Calculated for C₂₁H₁₈O₄: | C, 75.43; | H, 5.43 |
| Found: | C, 75.43; | H, 5.37 |

### Example 95

### Preparation of (E)-4-[[3-(2-naphthalenyl)-2-propenyl]oxy]-alpha-oxobenzeneacetic acid methyl ester

A stirred mixture of 4-hydroxy-alpha-oxobenzeneacetic acid methyl ester (1.01 g) in dimethylformamide (10 ml) under argon was treated with 55% sodium hydride (0.262 g), stirred for 15 minutes and treated with 2-(3-bromo-1-propenyl)naphthalene (1.7g). The mixture was heated at 60 °C overnight and worked up as in Example 20. The material from dichloromethane extraction was purified by HPLC (dichloromethane-hexane; 4:1) and crystallized from dichloromethane-diethyl ether to provide 0.95 g of (E)-4-[[3-(2-naphthalenyl)-2-propenyl]oxy]-alpha-oxobenzeneacetic acid methyl ester, mp 100-102 °C.

| | | |
|---|---|---|
| Analysis Calculated for C₂₂H₁₈O₄: | C, 76.29; | H, 5.24 |
| Found: | C, 76.24; | H, 5.19 |

### Example 96

### Preparation of (E)-4-[[3-(2-naphthalenyl)-2-propenyl]oxy]-alpha-oxobenzeneacetic acid

A mixture of (E)-4-[[3-(2-naphthalenyl)-2-propenyl]oxy]-alpha-oxobenzeneacetic acid methyl ester (0.85 g) in methanol (20 ml), acetone (5 ml), and 0.5N sodium hydroxide (6 ml) was treated as in Example 19. Extraction with dichloromethane provided material which was crystallized from dichloromethane-hexane to give 0.75 g of colorless (E)-4-[[3-(2-naphthalenyl)-2-propenyl]oxy]-alpha-oxobenzeneacetic acid, mp 142-143 °C.

| | | |
|---|---|---|
| Analysis Calculated for C₂₁H₁₆O₄: | C, 75.89; | H, 4.85 |
| Found: | C, 75.95; | H, 4.89 |

### Example 97

### Preparation of 4-[[2-(methoxy)ethyl]oxy]-alpha-oxobenzeneacetic acid

A stirred mixture of 4-hydroxy-alpha-oxobenzeneacetic acid (0.498 g) in dimethylsulfoxide (5 ml) under argon was treated with 4N sodium hydroxide (1.5 ml), stirred for 5 minutes and treated with the mesylate prepared from 2-methoxyethanol (0.462 g). The mixture was heated at 60 °C for 3 hours and poured into excess 1N hydrochloric acid. The product was extracted into diethyl ether (3 x 50 ml) and the organic layers were washed in turn with water (2 x 25 ml). The combined organic layers were dried (Na₂SO₄), filtered, and evaporated to give 0.56 g of crude product as a solid. Crystallizion from ethyl acetate-hexane provided 0.3 g of 4-[[2-(methoxy)ethyl]oxy]-alpha-oxobenzeneacetic acid, mp 129-130 °C.

| | | |
|---|---|---|
| Analysis Calculated for C₁₁H₁₂O₅: | C, 58.93; | H, 5.39 |
| Found: | C, 58.69; | H, 5.32 |

### Example 98

### Preparation of 4-[[2-(cyclohexyloxy)ethyl]oxy]-alpha-oxobenzeneacetic acid methyl ester

A stirred mixture of 4-hydroxy-alpha-oxobenzeneacetic acid methyl ester (0.724 ) in dimethylformamide (10 ml) under argon was treated with 55% sodium hydride (0.175 g), stirred for 15 minutes and treated with the tosylate prepared from 2-(cyclohexyloxy)ethanol (1.49 g). The mixture was heated at 60 °C overnight and worked up as in Example 20. The material from dichloromethane extraction was purified by HPLC (diethyl ether-hexane; 1:1) to provide 0.9 g of pure 4-[[2-(cyclohexyloxy)ethyl]oxy]-alpha-oxobenzeneacetic acid methyl ester as an oil.

| | | |
|---|---|---|
| Analysis Calculated for C₁₇H₂₂O₅: | C, 66.65; | H, 7.24 |
| Found: | C, 66.50; | H, 6.93 |

### Example 99

### Preparation of 4-[[2-(cyclohexyloxy)ethyl]oxy]-alpha-oxobenzeneacetic acid

A mixture of 4-[[2-(cyclohexyloxy)ethyl]oxy]-alpha-oxobenzeneacetic acid methyl ester (0.7 g) in methanol (10 ml) and 0.5N sodium hydroxide (7 ml) was treated as in Example 19. Extraction with diethyl ether provided material which was crystallized from diethyl ether-hexane to give 0.6 g of colorless 4-[[2-(cyclohexyloxy)ethyl]oxy]-alpha-oxobenzeneacetic acid, mp 96-98 °C.

| | | |
|---|---|---|
| Analysis Calculated for C₁₆H₂₀O₅: | C, 65.74; | H, 6.90 |
| Found: | C, 65.83; | H, 6.97 |

### Example 100

### Preparation of 4-[[2-(cyclooctyloxy)ethyl]oxy]-alpha-oxobenzeneacetic acid methyl ester

A stirred mixture of 4-hydroxy-alpha-oxobenzeneacetic acid methyl ester (1.27 g) in dimethylformamide (10 ml) under argon was treated with 55% sodium hydride (0.303 g), stirred for 15 minutes and treated with the tosylate prepared from 2-(cyclooctyloxy)ethanol (2.18 g). The mixture was heated at 60 °C overnight and worked up as in Example 20. The material from dichloromethane extraction was purified by HPLC (dichloromethane-hexane-ethyl acetate; 80:20:2) to provide 1.0 g of pure 4-[[2-(cyclooctyloxy)ethyl]oxy]-alpha-oxobenzeneacetic acid methyl ester as an oil.

| | | |
|---|---|---|
| Analysis Calculated for C₁₉H₂₆O₅: | C, 68.24; | H, 7.84 |
| Found: | C, 68.28; | H, 7.86 |

### Example 101

### Preparation of 4-[[2-(cyclooctyloxy)ethyl]oxy]-alpha-oxobenzeneacetic acid

A mixture of 4-[[2-(cyclooctyloxy)ethyl]oxy]-alpha-oxobenzeneacetic acid methyl ester (0.3 g) in methanol (10 ml) and 0.5N sodium hydroxide (4 ml) was treated as in Example 19. Extraction with dichloromethane provided material which was crystallized from diethyl ether-hexane to give 0.22 g of colorless 4-[[2-(cyclooctyloxy)ethyl]oxy]-alpha-oxobenzeneacetic acid, mp 64-65 °C.

| | | |
|---|---|---|
| Analysis Calculated for C₁₈H₂₄O₅: | C, 67.48; | H, 7.55 |
| Found: | C, 67.30; | H, 7.69 |

### Example 102

### Preparation of alpha-oxo-4-[[2-[tricyclo(3.3.1.1-3,7)dec-1-yloxy]ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the tosylate prepared from 2-(1-adamantyloxy) ethanol (1.5 g). The mixture was heated at 60 °C overnight and worked up as in Example 20. The material from dichloromethane extraction was purified by HPLC (dichloromethane-hexane-ethyl acetate; 80:20:2) and crystallized from diethyl ether-hexane to provide 0.77 g of alpha-oxo-4-[[2-[tricyclo(3.3.1.1-3,7)dec-1-yloxy]ethyl]oxy] benzeneacetic acid, mp 107-109 °C.

| | | |
|---|---|---|
| Analysis Calculated for C₂₁H₂₆O₅: | C, 70.37; | H, 7.31 |
| Found: | C, 70.29; | H, 7.31 |

### Example 103

### Preparation of alpha-oxo-4-[[2-[tricyclo(3.3.1.1-3,7)dec-1-yloxy]ethyl]oxy]benzeneacetic acid

A mixture of alpha-oxo-4-[[2-[tricyclo(3.3.1.1-3,7)dec-1-yloxy]ethyl]oxy] benzeneacetic acid methyl ester (0.685 g) in methanol (10 ml) was heated on the steam bath and sufficient acetone was added to dissolve the solids. Then 0.5N sodium hydroxide (8 ml) was added and the mixture was treated as in Example 19. Extraction with dichloromethane provided material which was crystallized from diethyl ether-hexane to give 0.524 g of colorless alpha-oxo-4-[[2-[tricyclo(3.3.1.1-3,7)dec-1-yloxy]ethyl]oxy]benzeneacetic acid, mp 155-156 °C.

| | | |
|---|---|---|
| Analysis Calculated for C₂₀H₂₄O₅: | C, 69.75; | H, 7.02 |
| Found: | C, 69.81; | H, 7.12 |

### Example 104

### Preparation of rac.-4-[[2-(2-naphthalenyloxy)ethyl]oxy]-alpha-oxobenzeneacetic acid 2,3-dihydroxypropyl ester

The acid chloride, prepared from 4-[[2-(2-naphthalenyloxy)ethyl]oxy]-alpha-oxobenzene-acetic acid (0.5 g) as described in Example 6, was dissolved in dichloromethane (10 ml) and added dropwise to a stirred, cold (<-50 °C) mixture of glycerine (0.9 ml) in tetrahydrofuran (10 ml). The cooling bath was removed and the mixture stirred for 1 hour at room temperature. The mixture was diluted with dichloromethane and washed once with saturated aqueous sodium bicarbonate, once with water, and the organic solution was dried (Na₂SO₄), filtered, and evaporated to give crude product. The material was purified by HPLC (ethyl acetate) to provide, after crystallization from ethyl acetate-hexane, 0.25 g of rac.-4-[[2-(2-naphthalenyloxy)ethyl]oxy]-alpha-oxobenzeneacetic acid 2,3-dihydroxypropyl ester as a colorless solid, mp 124-125 °C.

| | | |
|---|---|---|
| Analysis Calculated for C₂₃H₂₂O₇: | C, 67.31; | H, 5.40 |
| Found: | C, 67.11; | H, 5.36 |

### Example 105

### Preparation of 4-[[2-(2-naphthalenyloxy)ethyl]oxy]-alpha-oxobenzeneacetic acid 2-[2-(2-hydroxyethoxy)ethoxy]ethyl ester

The acid chloride, prepared from 4-[[2-(2-naphthalenyloxy)ethyl]oxy]-alpha-oxobenzene-acetic acid (0.5 g) as described in Example 6, was dissolved in dichloromethane (10 ml) and added dropwise to a stirred, cold (<-50 °C) mixture of triethylene glycol (0.9 g) in dichloro-methane (10 ml). The cooling bath was removed and the mixture stirred for 1 hour at room temperature. The mixture was diluted with dichloromethane and washed once with saturated aqueous sodium bicarbonate, once with water, and the organic solution was dried (Na₂SO₄), filtered, and evaporated to give crude product. The material was purified by HPLC (dichloro-methane-ethyl acetate; 2:1) to provide, after crystallization from ethyl acetate-hexane, 0.36 g of 4-[[2-(2-naphthalenyloxy)ethyl]oxy]-alpha-oxobenzeneacetic acid 2-[2-(2-hydroxyethoxy)ethoxy] ethyl ester as a colorless solid, mp 70-71 °C.

| | | |
|---|---|---|
| Analysis Calculated for C₂₆H₂₈O₈: | C, 66.66; | H, 6.02 |
| Found: | C, 66.35; | H, 6.01 |

### Example 106

### Preparation of 4-[[2-(2-naphthalenyloxy)ethyl]oxy]-alpha-oxobenzeneacetic acid 2-(dimethylamino)ethyl ester

The acid chloride, prepared from 4-[[2-(2-naphthalenyloxy)ethyl]oxy]-alpha-oxobenzeneacetic acid (0.5 g) as described in Example 6, was dissolved in dichloromethane (10 ml) and added dropwise to a stirred, cold (<-50 °C) mixture of 2-(dimethylamino)ethanol (0.178 g) in dichloromethane (10 ml). The cooling bath was removed and the mixture stirred for 1 hour at room temperature. The mixture was diluted with dichloromethane and washed once with saturated aqueous sodium bicarbonate, once with water, and the organic solution was dried (Na₂SO₄), filtered, and evaporated to give crude product. Crystallization from diethyl ether-hexane provided pure 4-[[2-(2-naphthalenyloxy)ethyl]oxy]-alpha-oxobenzeneacetic acid 2-(dimethylamino)ethyl ester as a colorless solid, mp 93-94 °C.

| | | | |
|---|---|---|---|
| Analysis Calculated for C₂₄H₂₅NO₅: | C, 70.75; | H, 6.18; | N, 3.44 |
| Found: | C, 70.91; | H, 6.23; | N, 3.45 |

### Example 107

### Preparation of 4-[[2-(2-anthracenyloxy)ethyl]oxy]-alpha-oxobenzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate of 2-(2-hydroxyethoxy)anthracene (1.26 g). The mixture was heated under argon at 60 °C overnight. The cooled mixture was treated with glacial acetic acid (2 drops) and the volatiles were removed under vacuum. The residue was mixed with water and the solids were filtered off, dissolved in dichloromethane, dried (Na₂SO₄), filtered, and evaporated to give crude product. Crystallization from dichloro-methane-diethyl ether provided 0.75 g of 4-[[2-(2-anthracenyloxy)ethyl]oxy]-alpha-oxobenzeneacetic acid methyl ester as a yellow solid, mp 188-189 °C.

| | | |
|---|---|---|
| Analysis Calculated for C₂₅H₂₀O₅: | C, 74.99; | H, 5.03 |
| Found: | C, 74.68; | H, 4.96 |

### Example 108

### Preparation of 4-[[2-(2-anthracenyloxy)ethyl]oxy]-alpha-oxobenzeneacetic acid

A mixture of 4-[[2-(2-anthracenyloxy)ethyl]oxy]-alpha-oxobenzeneacetic acid methyl ester (0.615 g) in hot tetrahydrofuran (100 ml) was treated with 1N sodium hydroxide (4 ml) and diluted with water. The resulting solids were recovered by filtration and stirred in a mixture of dichloromethane and excess 2N hydrochloric acid until the solids dissolved. The organic layer was separated, dried (Na₂SO₄), filtered, and evaporated to give crude product. Crystallization from acetone-hexane gave 0.45 g of 4-[[2-(2-anthracenyloxy) ethyl]oxy]-alpha-oxobenzene-acetic acid as a colorless solid, mp 213-214 °C.

| | | |
|---|---|---|
| Analysis Calculated for C₂₄H₁₈O₅: | C, 74.60; | H, 4.70 |
| Found: | C, 74.25; | H, 4.63 |

### Example 109

### Preparation of alpha-oxo-4-[[2-(9-phenanthrenyloxy)ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with 2-(9-phenanthrenyloxy)ethyl methanesulfonate (1.26 g). The mixture was heated under argon at 60 °C overnight. The cooled mixture was treated with glacial acetic acid (2 drops) and the volatiles were removed under vacuum. The residue was mixed with water and the solids were filtered off, dissolved in dichloromethane, dried (Na₂SO₄), filtered, and evaporated to give crude product. Crystallization from dichloro-methane-diethyl ether provided 0.8 g of alpha-oxo-4-[[2-(9-phenanthrenyloxy)ethyl]oxy]benzeneacetic acid methyl ester as a yellow solid, mp 147-148 °C.

| | | |
|---|---|---|
| Analysis Calculated for C₂₅H₂₀O₅: | C, 74.99; | H, 5.03 |
| Found: | C, 74.81; | H, 5.00 |

### Example 110

### Preparation of alpha-oxo-4-[[2-(9-phenanthrenyloxy)ethyl]oxy]benzeneacetic acid

A mixture of alpha-oxo-4-[[2-(9-phenanthrenyloxy)ethyl]oxy]benzeneacetic acid methyl ester (0.685 g) in hot tetrahydrofuran (100 ml) was treated with 1N sodium hydroxide (4 ml) and diluted with water. The organic solvent was removed under vacuum and the aqueous solution was acidified with excess hydrochloric acid and extracted with dichloromethane. The organic layer was dried (Na₂SO₄), filtered, and evaporated to give crude product. Crystallization from acetone-hexane provided 0.45 g of alpha-oxo-4-[[2-(9-phenanthrenyloxy)ethyl]oxy]benzeneacetic acid as a colorless solid, mp 179-180 °C.

| | | |
|---|---|---|
| Analysis Calculated for C₂₄H₁₈O₅: | C, 74.60; | H, 4.70 |
| Found: | C, 74.46; | H, 4.70 |

### Example 111

### Preparation of alpha-oxo-4-[[2-(5,6,7,8-tetrahydro-2-naphthalenyloxy)ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with 2-(5,6,7,8-tetrahydro-2-naphthalenyloxy)ethyl methanesulfonate (1.08 g). The mixture was heated under argon at 60 °C overnight. The cooled mixture was treated with glacial acetic acid (2 drops) and the volatiles were removed under vacuum. The residue was mixed with water and extracted with dichloromethane. The organic layers were dried (Na₂SO₄), filtered and evaporated to give crude product. The material was purified by HPLC (dichloromethane-hexane; 4:1) to provide, after crystallization from diethyl ether-hexane, 0.86 g of alpha-oxo-4-[[2-(5,6,7,8-tetrahydro-2-naphthalenyloxy)ethyl]oxy]benzeneacetic acid methyl ester as a colorless solid, mp 95-99 °C.

| | | |
|---|---|---|
| Analysis Calculated for C₂₁H₂₂O₅: | C, 71.17; | H, 6.26 |
| Found: | C, 71.15; | H, 6.28 |

### Example 112

### Preparation of alpha-oxo-4-[[2-(5,6,7,8-tetrahydro-2-naphthalenyloxy) ethyl]oxy]benzeneacetic acid

A mixture of alpha-oxo-4-[[2-(5,6,7,8-tetrahydro-2-naphthalenyloxy)ethyl] oxy]benzeneacetic acid methyl ester (0.75 g) in hot methanol (10 ml) plus enough tetrahydrofuran to dissolve all solids, was treated with 1N sodium hydroxide (4 ml) and diluted with water. The organic solvent was removed under vacuum and the residue was mixed with water, acidified with excess hydrochloric acid, and extracted with dichloromethane. The organic layer was dried (Na₂SO₄), filtered, and evaporated to give crude product. Crystallization from diethyl ether-hexane provided 0.56 g of alpha-oxo-4-[[2-(5,6,7,8-tetrahydro-2-naphthalenyloxy)ethyl]oxy]benzeneacetic acid as a colorless solid, mp 144-145 °C.

| | | |
|---|---|---|
| Analysis Calculated for C₂₀H₂₀O₅: | C, 70.58; | H, 5.92 |
| Found: | C, 70.42; | H, 5.92 |

### Example 113

### Preparation of rac.-alpha-oxo-4-[[2-(1,2,3,4-tetrahydro-2-naphthalenyloxy)ethyl]oxy]benzeneacetic acid methyl ester

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with rac.-2-(1,2,3,4-tetrahydro-2-naphthalenyloxy)ethyl methanesulfonate (1.08 g). The mixture was heated under argon at 60 °C overnight. The cooled mixture was treated with glacial acetic acid (2 drops) and the volatiles were removed under vacuum. The residue was mixed with water and extracted with dichloromethane. The organic layers were dried (Na₂SO₄), filtered and evaporated to give crude product. The material was purified by HPLC (dichloromethane-hexane; 1:1 plus 2% ethyl acetate) to provide, after crystallization from diethyl ether-hexane, 0.79 g of rac.-alpha-oxo-4-[[2-(1,2,3,4-tetrahydro-2-naphthalenyloxy)ethyl]oxy]benzeneacetic acid methyl ester as a colorless solid, mp 86-87 °C.

| | | |
|---|---|---|
| Analysis Calculated for C₂₁H₂₂O₅: | C, 71.17; | H, 6.26 |
| Found: | C, 71.11; | H, 6.21 |

### Example 114

### Preparation of rac.-alpha-oxo-4-[[2-(1,2,3,4-tetrahydro-2-naphthalenyloxy)ethyl] oxy]benzeneacetic acid

A mixture of rac.-alpha-oxo-4-[[2-(1,2,3,4-tetrahydro-2-naphthalenyloxy)ethyl]oxy] benzeneacetic acid methyl ester (0.69 g) in hot methanol (10 ml) plus enough tetrahydrofuran to dissolve the solids, was treated with 1N sodium hydroxide (4 ml) and diluted with water. The organic solvent was removed under vacuum and the residue was mixed with water, acidified with excess hydrochloric acid, and extracted with -dichloromethane. The organic layer was dried (Na₂SO₄), filtered, and evaporated to give crude product. Crystallization from diethyl ether-hexane provided 0.575 g of rac.-alpha-oxo-4-[[2-(1,2,3,4-tetrahydro-2-naphthalenyloxy)ethyl]oxy] benzeneacetic acid as a colorless solid, mp 119-120 °C.

| | | |
|---|---|---|
| Analysis Calculated for C₂₀H₂₀O₅: | C, 70.58; | H, 5.92 |
| Found: | C, 70.51; | H, 5.90 |

### Example 115

### Preparation of alpha-oxo-4-[[2-[3-(2-phenoxyethoxy)-2-naphthalenyloxy]ethyl]oxy] benzeneacetic acid methyl ester (2:1) hydrate

A mixture of 2,3-dihydroxynaphthalene (5.6 g) and sodium bicarbonate powder (2.9 g) in dimethylformamide (50 ml) was stirred and heated at 135 °C for 1 hour. When the gas evolution ceased, the mixture was cooled to about 60 °C and beta-bromophenetole (7 g) was added. The mixture was stirred at 60 °C overnight, cooled, diluted with water and filtered. The solids were crystallized from dichloromethane-diethyl ether to give 3.9 g of 3-(2-phenoxyethoxy)-2-naphthol as a colorless solid, mp 141-142 °C.

A stirred mixture of 3-(2-phenoxyethoxy)-2-naphthol (3.9 g), ethylene carbonate (1.4 g) and tetraethylammonium bromide (0.8 g) was heated at 155-160 °C for 2 hours. The cooled reaction was diluted with dichloromethane and washed with 1N sodium hydroxide and with water. The organic layer was dried (Na₂SO₄), filtered, and evaporated to give crude material which was purified by HPLC (dichloromethane-ethyl acetate; 20:1) and crystallized from dichloromethane-diethyl ether to give 3.3 g of 2-[[3-(2-phenoxyethoxy)-2-naphthalenyl]oxy]benzeneacetic as a colorless solid, mp 119-120°C.

A stirred mixture of 4-hydroxy-alpha-oxobenzeneacetic acid methyl ester (0.724 g) in dimethylformamide (10 ml) under argon was treated with 55% sodium hydride (0.175 g), stirred for 15 minutes and treated with the mesylate (1.6 g) prepared from 2-[[3-(2-phenoxyethoxy)-2-naphthalenyloxy]ethanol. The mixture was heated under argon at 60 °C overnight. The cooled mixture was treated with glacial acetic acid (2 drops) and the volatiles were removed under vacuum. The residue was mixed with water and extracted with dichloromethane. The organic layer was washed with water, dried (Na₂SO₄), filtered, and evaporated to give crude product. The material was purified by HPLC (dichloromethane-hexane; 4:1 plus 2% ethyl acetate) to provide, after crystallization from dichloromethane-diethyl ether, 0.525 g of alpha-oxo-4-[[2-[3-(2-phenoxyethoxy)-2-naphthalenyloxy]ethyl]oxy]benzeneacetic acid methyl ester (2:1) hydrate as a colorless solid, mp 146-147 °C.

| | | |
|---|---|---|
| Analysis Calculated for C₂₉H₂₆O₇.2:1 H₂O: | C, 70.29; | H, 5.49 |
| Found: | C, 70.45; | H, 5.24 |

### Example 116

### Preparation of alpha-oxo-4-[[2-[3-(2-phenoxyethoxy)-2-naphthalenyloxy]ethyl]oxy] benzeneacetic acid

A mixture of alpha-oxo-4-[[2-[3-(2-phenoxyethoxy)-2-naphthalenyloxy]ethyl]oxy] benzeneacetic acid methyl ester (0.50 g) in hot methanol (10 ml) plus enough tetrahydrofuran to dissolve the solids, was treated with 1N sodium hydroxide (2 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give crude product. Crystallization from acetone-hexane provided 0.45 g of alpha-oxo-4-[[2-[3-(2-phenoxyethoxy)-2-naphthalenyloxy]ethyl]oxy]benzeneacetic acid as a colorless solid, mp 176-177 °C.

| | | |
|---|---|---|
| Analysis Calculated for C₂₈H₂₄O₇: | C, 71.18; | H, 5.12 |
| Found: | C, 71.27; | H, 5.09 |

### Example 117

### Preparation of 4-[[2-[3-(2-hydroxyethoxy)-2-naphthalenyloxy]ethyl]oxy]-alpha-oxobenzeneacetic acid methyl ester

A stirred mixture of 2,3-dihydroxynaphthalene (4.8 g), ethylene carbonate (5.8 g) and tetraethylammonium bromide (2.1 g) was heated at 155-160 °C for 2 hours. The cooled reaction was diluted with dichloromethane, filtered, and the crude material was crystallized from toluene to give 4.3 g of 2,2-[2,3-naphthalenebis(oxy)]bis-ethanol as a colorless solid, mp 143-145°C.

A stirred mixture of 2,2-[2,3-naphthalenebis(oxy)]bis-ethanol (4.2 g) and pyridine (40 ml) was chilled in a dry ice/acetone bath to just above freezing and treated dropwise with methanesulfonyl chloride (1.3 ml). The mixture was stirred for 1 hour at 0 °C and then overnight at room temperature. The pyridine solution was diluted with ice and water and acidified with excess 2N hydrochloric acid. The mixture was extracted with dichloromethane, washed with water, dried (Na₂SO₄), filtered, and evaporated. The material was purified by HPLC (dichloromethane-ethyl acetate; 1:1) to provide, after crystallization from dichloromethane-diethyl ether, 2.6 g of 2-[3-(2-hydroxyethoxy)-2-naphthalenyloxy)]ethyl methanesulfonate as a colorless solid, mp 100-102 °C.

A stirred mixture of 4-hydroxy-alpha-oxobenzeneacetic acid methyl ester (1.27 g) in dimethylformamide (10 ml) under argon was treated with 55% sodium hydride (0.306 g), stirred for 15 minutes and treated with 2-[3-(2-hydroxyethoxy)-2-naphthalenyloxy)]ethyl methanesulfonate (2.3 g). The mixture was heated under argon at 60 °C overnight. The cooled mixture was treated with glacial acetic acid (2 drops) and the volatiles were removed under vacuum. The residue was mixed with water and extracted with dichloromethane. The organic layer was washed with water, dried (Na₂SO₄), filtered, and evaporated to give crude product. The material was purified by HPLC (dichloromethane-ethyl acetate; 9:1) to provide, after crystallization from dichloromethane-diethyl ether, 0.67 g of 4-[[2-[3-(2-hydroxyethoxy)2-naphthalenyloxy]ethyl]oxy]-alpha-oxobenzeneacetic acid methyl ester as a colorless solid, mp 130-135 °C which was used without further purification.

### Example 118

### Preparation of 4-[[2-[3-(2-hydroxyethoxy)-2-naphthalenyloxy]ethyl]oxy]-alpha-oxobenzeneacetic acid

A mixture of 4-[[2-[3-(2-hydroxyethoxy)-2-naphthalenyloxy]ethyl]oxy]-alpha-oxobenzeneacetic acid methyl ester (0.6 g) in hot methanol (10 ml) plus enough tetrahydrofuran to dissolve the solids, was treated with 1N sodium hydroxide (4 ml) and diluted with water. The organic solvent was removed under vacuum and the residue was mixed with water, acidified with excess 2 N hydrochloric acid, and extracted with dichloromethane-tetrahydrofuran. The organic layer was dried (Na₂SO₄), filtered, and evaporated to give crude product. Crystallization from acetone-hexane provided 0.4 g of 4-[[2-[3-(2-hydroxyethoxy)-2-naphthalenyloxy]ethyl]oxy]-alpha-oxobenzeneacetic acid as a colorless solid, mp 176-177 °C.

| | | |
|---|---|---|
| Analysis Calculated for C₂₂H₂₀O₇: | C, 66.66; | H, 5.09 |
| Found: | C, 66.38; | H, 4.99 |

### Example 119

### Preparation of alpha-oxo-4-[[2-[3-(phenylmethoxy)-2-naphthalenyloxy]ethyl]oxy] benzeneacetic acid methyl ester

A stirred mixture of 3-(phenylmethoxy)-2-naphthalenol (5 g), ethylene carbonate (1.9 g) and tetraethylammonium bromide (1.5 g) was heated at 155-160 °C for 2 hours. The cooled reaction was diluted with dichloromethane, washed with 1N sodium hydroxide, water, dried (Na₂SO₄), filtered, and evaporated. The material was purified by HPLC (dichloromethane-ethyl acetate; 20:1) to provide, after crystallization from dichloromethane-diethyl ether, 3.9 g of 2-[3-(phenylmethoxy)-2-naphthalenyloxy]ethanol as a colorless solid, mp 95-97 °C.

A stirred mixture of 4-hydroxy-alpha-oxobenzeneacetic acid methyl ester (2.29 g) in dimethylformamide (30 ml) under argon was treated with 55% sodium hydride (0.552 g), stirred for 15 minutes and treated with the mesylate (4.3 g) prepared from 2-[3-(phenylmethoxy)-2-naphthalenyloxy]ethanol. The mixture was heated under argon at 60 °C overnight. The cooled mixture was treated with glacial acetic acid (2 drops) and the volatiles were removed under vacuum. The residue was mixed with water and extracted with dichloromethane. The organic layer was washed with water, dried (Na₂SO₄), filtered, and evaporated to give crude product. The material was purified by HPLC (dichloromethane-hexane; 3:1 plus 2% ethyl acetate) to provide, after crystallization from dichloromethane-methanol, 3.0 g of alpha-oxo-4-[[2-[3-(phenylmethoxy)-2-naphthalenyloxy]ethyl]oxy]benzeneacetic acid methyl ester as a colorless solid, mp 115-117 °C.

| | | |
|---|---|---|
| Analysis Calculated for C₂₈H₂₄O₆: | C, 73.67; | H, 5.30 |
| Found: | C, 73.32; | H, 5.20 |

### Example 120

### Preparation of 4-[[2-(3-hydroxy-2-naphthalenyloxy)ethyl]oxy]-alpha-oxobenzeneacetic acid methyl ester

A mixture of alpha-oxo-4-[[2-[3-(phenylmethoxy)-2-naphthalenyloxy] ethyl]oxy]benzeneacetic acid methyl ester (0.456 g) in tetrahydrofuran (3 ml) was added to a flask containing 10% palladium on carbon catalyst and 5 ml of tetrahydrofuran in a hydrogen atmosphere. The mixture was stirred until one equivalent of hydrogen was absorbed and the reaction was filtered and the organic solvent was removed by evaporation. Chromatography on silica gel and elution with dichloromethane-hexane mixtures provided purified product. Crystallization from dichloromethane-diethyl ether gave 0.067 g of pure 4-[[2-(3-hydroxy-2-naphthalenyloxy)ethyl]oxy]-alpha-oxobenzeneacetic acid methyl ester as a colorless solid, mp 177-178 °C.

### Example 121

### Preparation of 4-[[2-(3-hydroxy-2-naphthalenyloxy)ethyl]oxy]-alpha-oxobenzeneacetic acid

A mixture of 4-[[3-(2-hydroxy-2-naphthalenyloxy)ethyl]oxy]-alpha-oxobenzeneacetic acid methyl ester (0.065 g) in hot methanol (2 ml) plus tetrahydrofuran (5 ml) was treated with 1N sodium hydroxide (0.5 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give crude product. Crystallization from dichloromethane-diethyl ether provided 0.055 g of 4-[[(3-hydroxy-2-naphthalenyloxy) ethyl]oxy]-alpha-oxobenzeneacetic acid as a colorless solid, mp 191-192 °C.

| | | |
|---|---|---|
| Analysis Calculated for C₂₀H₁₆O₆: | C, 68.18; | H, 4.58 |
| Found: | C, 67.78; | H, 4.51 |

### Example 122

### Preparation of alpha-oxo-4-[4-(3-pyridinyl)butoxy]benzeneacetic acid methyl ester (4:1) molar hydrate

A stirred mixture of 4-hydroxy-alpha-oxobenzeneacetic acid methyl ester (1.14 g), 3-pyridinebutanol (1.04 g), triphenylphosphine (2.07 g), and tetrahydrofuran (25 ml) was stirred at 0 °C while adding dropwise a solution of diethyl azodicarboxylate (1.37 g) in tetrahydrofuran (10 ml). The mixture was stirred for 2 hours at 0 °C and evaporated to dryness. The material was purified by HPLC (hexane-acetone; 2:1) to provide 1.4 g of alpha-oxo-4-[4-(3-pyridinyl)butoxy]benzeneacetic acid methyl ester (4:1) molar hydrate as a colorless oil.

| | | | |
|---|---|---|---|
| Analysis Calculated for C₁₈H₁₉NO₄.4:1H₂O: | C, 68.02; | H, 6.18; | N, 4.41 |
| Found: | C, 68.24; | H, 6.19; | N, 4.68 |

### Example 123

### Preparation of alpha-oxo-4-[4-(3-pyridinyl)butoxy]benzeneacetic acid

A mixture of alpha-oxo-4-[4-(3-pyridinyl)butoxy]benzeneacetic acid methyl ester (4:1) molar hydrate (1.1 g) in hot methanol (10 ml) was treated with 1N sodium hydroxide (5 ml) and diluted with water. The organic solvent was removed under vacuum and the residue in water was washed with diethyl ether. The aqueous layer was concentrated to about 25 ml and chilled in ice. Cold 2N hydrochloric acid (2.5 ml) was added dropwise and the product was allowed to crystallize and was filtered and air dried. Recrystallization from acetone provided 0.64 g of alpha-oxo-4-[4-(3-pyridinyl)butoxy]benzeneacetic acid as a colorless solid, mp 163-165 °C.

| | | | |
|---|---|---|---|
| Analysis Calculated for C₁₇H₁₇NO₄: | C, 68.22; | H, 5.72; | N, 4.68 |
| Found: | C, 67.83; | H, 5.73; | N, 5.02 |

### Example 124

### Preparation of alpha-oxo-4-[4-(4-pyridinyl)butoxy]benzeneacetic acid methyl ester

A stirred mixture of 4-hydroxy-alpha-oxobenzeneacetic acid methyl ester (1.086 g) in dimethylformamide (10 ml) under argon was treated with 55% sodium hydride (0.261 g), stirred for 15 minutes and treated with the mesylate prepared from 0.906 g of 4-pyridinebutanol. The mixture was heated under argon at 60 °C for 4 hours. The cooled mixture was treated with glacial acetic acid (2 drops) and the volatiles were removed under vacuum. The residue was mixed with dichloromethane and dilute cold sodium bicarbonate solution. The dichloromethane extracts were dried (Na₂SO₄), filtered, and evaporated to give crude product. The material was purified by HPLC (hexane-acetone; 2:1) to provide 0.58 g of alpha-oxo-4-[4-(4-pyridinyl)butoxy]benzeneacetic acid methyl ester as a colorless oil whose NMR was compatible with the desired product.

### Example 125

### Preparation of alpha-oxo-4-[4-(4-pyridinyl)butoxy]benzeneacetic acid

A mixture of alpha-oxo-4-[4-(4-pyridinyl)butoxy]benzeneacetic acid methyl ester (0.58 g) in hot methanol (10 ml) was treated with 1N sodium hydroxide (3.0 ml) and diluted with water. The organic solvent was removed under vacuum and the residue in water was washed with diethyl ether. The aqueous layer was concentrated to about 25 ml and chilled in ice. Cold 2N hydrochloric acid (1.5 ml) was added dropwise and the product was allowed to crystallize and was filtered and air dried. Recrystallization from water provided 0.55 g of alpha-oxo-4-[4-(4-pyridinyl)butoxy]benzeneacetic acid as a colorless solid, mp 198-199 °C.

| | | | |
|---|---|---|---|
| Analysis Calculated for C₁₇H₁₇NO₄:. | C, 68.22; | H, 5.72; | N, 4.68 |
| Found: | C, 67.95; | H, 5.68; | N, 4.56 |

### Example 126

### Preparation of alpha-oxo-4-[[2-(7-quinolyloxy)ethyl]oxy]benzeneacetic acid methyl ester

A stirred mixture of 4-hydroxy-alpha-oxobenzeneacetic acid methyl ester (1.267 g) in dimethylformamide (10 ml) under argon was treated with 55% sodium hydride (0.305 g), stirred for 15 minutes and treated with the mesylate prepared from 1.135 g of 2-(7-quinolyloxy)ethanol. The mixture was heated under argon at 60 °C for 5 hours. The cooled mixture was treated with glacial acetic acid (2 drops) and the volatiles were removed under vacuum. The residue was mixed with dichloromethane and dilute cold sodium bicarbonate solution. The dichloromethane extracts were washed with water, dried (Na₂SO₄), filtered, and evaporated to give crude product. The material was purified by HPLC (ethyl acetate) and crystallized from dichloromethane-hexane to provide 0.93 g of alpha-oxo-4-[[2-(7-quinolyloxy)ethyl]oxy]benzeneacetic acid methyl ester as a colorless solid, mp 92-94 °C.

| | | | |
|---|---|---|---|
| Analysis Calculated for C₂₀H₁₇NO₅: | C, 68.37; | H, 4.88; | N, 3.99 |
| Found: | C, 68.68; | H, 4.86; | N, 3.99 |

### Example 127

### Preparation of alpha-oxo-4-[[2-(7-quinolyloxy)ethyl]oxy]benzeneacetic acid monohydrate

A mixture of alpha-oxo-4-[[2-(7-quinolyloxy)ethyl]oxy]benzeneacetic acid methyl ester (0.5 g) in hot methanol (5 ml) and tetrahydrofuran (5 ml) was treated with 1N sodium hydroxide (2.0 ml) and diluted with water. The organic solvent was removed under vacuum and the residue was dissolved in hot water. Cold 2N hydrochloric acid (1.0 ml) was added dropwise and the product was allowed to crystallize and was filtered, washed with water and dried to give 0.45 g of alpha-oxo-4-[[2-(7-quinolyloxy)ethyl]oxy] benzeneacetic acid monohydrate as a colorless solid, mp 229-231 °C.

| | | | |
|---|---|---|---|
| Analysis Calculated for C₁₉H₁₅NO₅.H₂O: | C, 64.22; | H, 4.82; | N, 3.94 |
| Found: | C, 64.30; | H, 4.50; | N, 3.94 |

### Example 128

### Preparation of 4-[[2-(7-isoquinolyloxy)ethyl]oxy]alpha-oxobenzeneacetic acid methyl ester

A stirred mixture of 4-hydroxy-alpha-oxobenzeneacetic acid methyl ester (1.267 g) in dimethylformamide (10 ml) under argon was treated with 55% sodium hydride (0.305 g), stirred for 15 minutes and treated with the mesylate prepared from 1.135 g of 2-(7-isoquinolyloxy)ethanol. The mixture was heated under argon at 60 °C for 5 hours. The cooled mixture was treated with glacial acetic acid (2 drops) and the volatiles were removed under vacuum. The residue was partitioned between dichloromethane and dilute cold sodium bicarbonate solution. The dichloromethane layer was washed with water, dried (Na₂SO₄), filtered, and evaporated to give crude product. The material was purified by HPLC (ethyl acetate) and crystallized from dichloromethane-hexane to provide 0.83 g of 4-[[2-(7-isoquinolyloxy)ethyl]oxy]-alpha-oxobenzeneacetic acid methyl ester as a colorless solid, mp 144-145 °C.

| | | | |
|---|---|---|---|
| Analysis Calculated for C₂₀H₁₇NO₅: | C, 68.37; | H, 4.88; | N, 3.99 |
| Found: | C, 68.25; | H, 4.82; | N, 3.88 |

### Example 129

### Preparation of 4-[[2-(7-isoquinolyloxy)ethyl]oxy]-alpha-oxobenzeneacetic acid (5:2) molar hydrate

A mixture of 4-[[2-(7-isoquinolyloxy)ethyl]oxy]-alpha-oxobenzeneacetic acid methyl ester (0.5 g) in hot methanol (5 ml) and tetrahydrofuran (5 ml) was treated with 1N sodium hydroxide (2.0 ml) and diluted with water. The organic solvent was removed under vacuum and the residue was dissolved in hot water. Cold 2N hydrochloric acid (1.0 ml) was added dropwise and the product was allowed to crystallize and was filtered, washed with water and dried to give 0.45 g of 4-[[2-(7-isoquinolyloxy)ethyl]oxy]-alpha-oxobenzeneacetic acid as a colorless solid, mp 260 °C with decomposition.

| | | | | |
|---|---|---|---|---|
| Analysis Calculated for C₁₉H₁₅NO₅.5:2 H₂O: | C, 66.23; | H, 4.62; | N, 4.06; | H₂O, 2.09 |
| Found: | C, 66.04; | H, 4.49; | N, 4.03; | H₂O, 1.79 |

### Example 130

### Preparation of alpha-oxo-4-[[2-(4-quinolyloxy)ethyl]oxy]benzeneacetic acid methyl ester

A stirred mixture of 4-hydroxy-alpha-oxobenzeneacetic acid methyl ester (1.267 g) in dimethylformamide (10 ml) under argon was treated with 55% sodium hydride (0.305 g), stirred for 15 minutes and treated with the mesylate prepared from 1.135 g of 2-(4-quinolyloxy)ethanol. The mixture was heated under argon at 60 °C for 3 hours. The cooled mixture was treated with glacial acetic acid (2 drops) and the volatiles were removed under vacuum. The residue was mixed with dichloromethane and dilute cold sodium bicarbonate solution. The dichloromethane extracts were washed with water, dried (Na₂SO₄), filtered, and evaporated to give crude product. The material was purified by HPLC (ethyl acetate-methanol; 20:1) and crystallized from dichloromethane-hexane to provide 1.2 g of alpha-oxo-4-[[2-(4-quinolyloxy)ethyl]oxy]benzeneacetic acid methyl ester as a colorless solid, mp 135-136 °C.

| | | | |
|---|---|---|---|
| Analysis Calculated for C₂₀H₁₇NO₅: | C, 68.37; | H, 4.88; | N, 3.99 |
| Found: | C, 68.35; | H, 4.87; | N, 4.01 |

### Example 131

### Preparation of alpha-oxo-4-[[2-(4-quinolyloxy)ethyl]oxy]benzeneacetic acid

A mixture of alpha-oxo-4-[[2-(4-quinolyloxy)ethyl]oxy]benzeneacetic acid methyl ester (0.50 g) in hot methanol (10 ml) was treated with 1N sodium hydroxide (4.0 ml) and diluted with water. The organic solvent was removed under vacuum and the residue was dissolved in water and chilled in ice. Cold 2N hydrochloric acid (2.0 ml) was added dropwise and the product was allowed to crystallize and was filtered, washed with water and dried to give 0.477 g of alpha-oxo-4-[[2-(4-quinolyloxy)ethyl]oxy]benzeneacetic acid as a colorless solid, mp 271 °C with decomposition.

| | | | |
|---|---|---|---|
| Analysis Calculated for C₁₉H₁₅NO₅: | C, 67.65; | H, 4.48; | N, 4.15 |
| Found: | C, 67.25; | H, 4.80; | N, 3.90 |

### Example 132

### Preparation of alpha-oxo-4-[[(trifluoromethyl)sulfonyl]oxy]benzeneacetic acid methyl ester

A solution of alpha-oxo-4-hydroxybenzeneacetic acid methyl ester (2 g) and phenyl-bis-[(trifluoromethyl)sulfonyl]amine (4.2 g) in dichloromethane (30 ml) was cooled in an ice bath and triethylamine (1.65 ml) was added dropwise. The mixture was held at 0 °C for 1 hour and was allowed to warm to room temperature over 2 hours. It was diluted to 100 ml with diethyl ether and washed with successive 25 ml portions of water (twice), 0.5N sodium hydroxide, 0.5N hydrochloric acid, and saturated brine. Concentration of the dried (MgSO₄) extract gave a mixture of an oil and a solid from which the oil was decanted to give 2.77 g of alpha-oxo-4-[[(trifluoromethyl)sulfonyl]oxy]benzeneacetic acid methyl ester suitable for use in the next step. The analytical sample was obtained as a colorless oil by chromatography of a portion over silica gel, eluting with ethyl acetate-hexane (9:1).

| | | | | |
|---|---|---|---|---|
| Analysis Calculated for C₁₀H₇F₃O₆S: | C, 38.47; | H, 2.26; | S, 10.27; | F, 18.25 |
| Found: | C, 38.75; | H, 2.29; | S, 9.98; | F, 18.55 |

### Example 133

### Preparation of 4-[[2-[8-(2,2-dimethyl-1-oxobutoxy)-2-naphthalenyloxy]ethyl]oxy]-alpha-oxobenzeneacetic acid methyl ester

To a stirred suspension of sodium hydride (55% dispersion in mineral oil, 0.105 g) in dimethylformamide (5 ml) was added dropwise a solution of 4-hydroxy-alpha-oxobenzene acetic acid methyl ester (0.432 g) in dimethylformamide (5 ml) under argon. The mixture was then treated dropwise with a solution of 2-[8-(2,2-dimethyl-1-oxobutoxy)-2-naphthalenyloxy] ethyl methanesulfonate (0.76 g) in dimethylformamide (5 ml). The reaction was heated for 18 hours at 60 °C, diluted with brine, and extracted with ethyl acetate. After evaporation of volatiles, the residue was purified by chromatography over silica gel (hexane-ethyl acetate; 10:1) to yield 0.375 g of 4-[[2-[8-(2,2-dimethyl-1-oxobutoxy)-2-naphthalenyloxy]ethyl]oxy]-alpha-oxobenzeneacetic acid methyl ester as a white solid, mp 119-120 °C.

| | | |
|---|---|---|
| Analysis Calculated for C₂₇H₂₈O₇: | C, 69.80; | H, 6.07 |
| Found: | C, 68.48; | H, 5.90 |

### Example 134

### Preparation of 4-[[2-[8-(2,2-dimethyl-1-oxobutoxy)-2-naphthalenyloxy]ethyl]oxy]-alpha-oxobenzeneacetic acid

To a solution of 4-[[2-[8-(2,2-dimethyl-1-oxobutoxy)-2-naphthalenyloxy]ethyl]oxy]-alpha-oxobenzeneacetic acid methyl ester (0.3 g) in methanol (7 ml) was added aqueous 1N sodium hydroxide (1.35 ml). After 15 minutes, the reaction was acidified with 1N hydrochloric acid (2 ml) and extracted with dichloromethane. The organic layer was washed with brine and evaporated to provide 4-[[2-[8-(2,2-dimethyl-1-oxobutoxy)-2-naphthalenyloxy]ethyl]oxy]-alpha-oxobenzeneacetic acid as a white solid, mp 167-168 °C.

| | | |
|---|---|---|
| Analysis Calculated for C₂₆H₂₆O₇: | C, 69.32; | H, 5.82 |
| Found: | C, 69.17; | H, 5.89 |

### Example 135

### Preparation of 4-[[2-[2-[(2,2-dimethyl-1-oxobutoxy)methyl]-6-methylphenoxy] ethyl]oxy]-alpha-oxobenzeneacetic acid methyl ester

To a stirred suspension of sodium hydride (55% dispersion in mineral oil; 0.218 g) in dimethylformamide (5 ml) was added dropwise 4-hydroxy-alpha-oxobenzeneacetic acid methyl ester (0.9 g) in dimethylformamide (5 ml) under argon. The resulting mixture was then treated dropwise with a solution of 2-[2-[(2,2-dimethyl-1-oxobutoxy)methyl]-6-methyl-phenoxy]ethyl 4-methylphenylsulfonate (1.4 g) in dimethylformamide (5 ml). The reaction was heated for 18 hours at 60 °C, then was diluted with brine, and extracted with ethyl acetate. After evaporation of the extracts, the residue was purified by chromatography over silica gel (hexane-ethyl acetate; 10:1) to yield 0.606 g of 4-[[2-[2-[(2,2-dimethyl-1-oxobutoxy)methyl]-6-methylphenoxy]ethyl]oxy]-alpha-oxobenzeneacetic acid methyl ester as a clear oil.

| | | |
|---|---|---|
| Analysis Calculated for C₂₅H₃₀O₇: | C, 67.86; | H, 6.83 |
| Found: | C, 67.42; | H, 6.18 |

### Example 136

### Preparation of 4-[[2-[2-[(2,2-dimethyl-1-oxobuyloxy)methyl]-6-methylphenoxy] ethyl]oxy]-alpha-oxobenzeneacetic acid (2:1) hydrate

To a solution of 4-[[2-[2-[(2,2-dimethyl-1-oxobuyloxy)methyl]-6-methylphenoxy] ethyl]oxy]-alpha-oxobenzeneacetic acid methyl ester (0.554 g) in methanol (10 ml) was added aqueous 1N sodium hydroxide (2.5 ml). After 30 minutes at 50 °C, the reaction was acidified with 1N hydrochloric acid (2 ml) and extracted with dichloromethane. The organic phase was washed with brine and evaporated to provide 0.47 g of 4-[[2-[2-[(2,2-dimethyl-1-oxobuyloxy)methyl]-6-methylphenoxy]ethyl]oxy]-alpha-oxobenzeneacetic acid (2:1) hydrate as a clear oil.

| | | |
|---|---|---|
| Analysis Calculated for C₂₄H₂₈O₇.2:1 H₂O: | C, 65.83; | H, 6.40 |
| Found: | C, 65.64; | H, 6.53 |

### Example 137

### Preparation of 4-[[2-[2-(hydroxymethyl)-6-methylphenoxy]ethyl]oxy]-alpha-oxobenzeneacetic acid

To a solution of 4-[[2-[2-[(2,2-dimethyl-1-oxobuyloxy)methyl]-6-methylphenoxy] ethyl]oxy]-alpha-oxobenzeneacetic acid [2:1] hydrate (0.032 g) in methanol (10 ml) was added aqueous 1N sodium hydroxide (1.0 ml). After 18 hours at 40 °C, the reaction was acidified with 1N hydrochloric acid (2 ml), then concentrated in vacuo to ∼ 2ml and extracted with dichloromethane. The organic extract was evaporated to provide 0.014 g of 4-[[2-[2-(hydroxymethyl)-6-methylphenoxy]ethyl]oxy]-alpha-oxobenzeneacetic acid as a white solid.

| | |
|---|---|
| Analysis by (+)-FAB Calculated for [C₁₈H₁₈O₆.H]+: | 331.1182 |
| Found: | 331.1186 |
| Calculated for [C₁₈H₁₈O₆-H]+: | 329.1025 |
| Found: | 329.1002 |

### Example 138

### Preparation of 4-[[2-[6-(acetyloxy)-2-naphthalenyloxy]ethyl]oxy]-alpha-oxobenzeneacetic acid methyl ester

To a stirred suspension of sodium hydride (55% dispersion in mineral oil; 0.393 g) in dimethylformamide (20 ml) was added dropwise 4-hydroxy-alpha-oxobenzeneacetic acid methyl ester (1.6 g) in dimethylformamide (15 ml) under argon. The resulting mixture was then treated dropwise with a solution of 2-[6-(acetyloxy)-2-naphthalenyloxy]ethyl methanesulfonate (2.23 g) in dimethylformamide (15 ml). The reaction was heated for 18 hours at 65 °C, diluted with brine and extracted with ethyl acetate. After evaporation of the extracts, the residue was purified by chromatography over silica gel (hexane-diethyl ether; 1:1, increasing to 1:2) to yield 1.0 g of 4-[[2-[6-(acetyloxy)-2-naphthalenyloxy]ethyl]oxy]-alpha-oxobenzeneacetic acid methyl ester as a white solid, mp 120-122 °C.

| | | |
|---|---|---|
| Analysis Calculated for C₂₃H₂₀O₇: | C, 67.64; | H, 4.94 |
| Found: | C, 67.32; | H, 4.84 |

### Example 139

### Preparation of 4-[[3-(2-naphthoylamino)propyl]oxy]-alpha-oxobenzeneacetic acid methyl ester

A stirred mixture of 4-hydroxy-alpha-oxobenzeneacetic acid methyl ester (1.27 g) in dimethylformamide (10 ml) under argon was treated with 55% sodium hydride (0.306 g), stirred for 10 minutes and treated with N-(3-bromopropyl)-2-naphthylenecarboxamide (1.7 g). The mixture was heated under argon at 60 °C for 5 hours. The cooled mixture was treated with glacial acetic acid (2 drops) and the volatiles were removed under vacuum. The residue was mixed with water and extracted with dichloromethane. The organic extracts were washed with water, dried (Na₂SO₄), filtered, and evaporated to give crude product. The material was purified by HPLC (dichloromethane-ethyl acetate; 9:1) and crystallized from dichloromethane-ethyl ether to provide 0.9 g of 4-[[3-(2-naphthoylamino)propyl]oxy]-alpha-oxobenzeneacetic acid methyl ester as a colorless solid, mp 143-144 °C.

| | | | |
|---|---|---|---|
| Analysis Calculated for C₂₃H₂₁NO₅: | C, 70.58; | H, 5.41; | N, 3.58 |
| Found: | C, 70.24; | H, 5.55; | N, 3.49 |

### Example 140

### Preparation of 4-[[3-(2-naphthoylamino)propyl]oxy]-alpha-oxobenzeneacetic acid

A mixture of 4-[[3-(2-naphthoylamino)propyl]oxy]-alpha-oxobenzeneacetic acid methyl ester (0.76 g) in hot methanol (10 ml) and sufficient tetrahydrofuran to dissolve the solids was treated with 1N sodium hydroxide (4.0 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give crude product. Crystallization from acetone-hexane provided 0.43 g of 4-[[3-(2-naphthoylamino)propyl]oxy]-alpha-oxobenzeneacetic acid as a colorless solid, mp 169-170 °C.

| | | | |
|---|---|---|---|
| Analysis Calculated for C₂₂H₁₉NO₅: | C, 70.05; | H, 5.07; | N, 3.71 |
| Found: | C, 69.89; | H, 5.16; | N, 3.60 |

### Example 141

### Preparation of 4-[(2-benzofuranyl)methoxy]-alpha-oxobenzeneacetic acid

A stirred mixture of 4-hydroxy-alpha-oxobenzeneacetic acid methyl ester (1.52 g) in dimethylformamide (10 ml) under argon was treated with 55% sodium hydride (0.405 g), stirred for 10 minutes and treated with 2-chloromethylbenzofuran (1.4 g). The mixture was stirred at room temperature under argon for 3 hours, diluted with water, and extracted with ethyl acetate. Evaporation of the organic extracts provided crude methyl ester as a liquid. Treatment of a portion of this ester (1.02 g) with potassium hydroxide (0.18 g) in a methanol-water mixture, followed by acidification of the mixture with hydrochloric acid and filtration provided 4-[(2-benzofuranyl)methoxy]-alpha-oxobenzeneacetic acid as a white solid, mp 120 °C.

| | | |
|---|---|---|
| Analysis Calculated for C₁₇H₁₂O₅: | C, 68.92; | H, 4.05 |
| Found: | C, 68.64; | H, 4.26 |

### Example 142

### Preparation of 4-[3-(2-naphthalenyloxy)-1-propynyl]-alpha-oxobenzeneacetic acid

Argon was bubbled through a solution of 2-(2-propynyl)oxynaphthalene (0.36 g), alpha-oxo-4-[[(trifluoromethyl)sulfonyl]oxy]benzeneacetic acid methyl ester (0.51 g), methylamine (2.0 ml) and lithium chloride (0.2 g) in dimethylformamide (5 ml) for 15 minutes and then bis(triphenylphosphine)palladium dichloride (0.06 g) was added. The bath temperature was raised to 90 °C for 2 hours. The mixture was cooled, diluted with diethyl ether-ethyl acetate (1:1; 50 ml) and extracted with water(2 x 50 ml). Addition of saturated sodium chloride solution to the combined aqueous layers gave a precipitate which was collected, dissolved in tetrahydrofuran (25 ml) and acidified by the addition of excess 6N hydrochloric acid plus saturated brine. The layers were separated and the organic phase was dried (MgSO₄), evaporated and the residue crystallized from dichloromethane-hexane to afford 0.216 g of 4-[3-(2-naphthalenyloxy)-1-propynyl]-alpha-oxobenzeneacetic acid, mp 120-124 °C (dec).

| | | |
|---|---|---|
| Analysis Calculated for C₂₁H₁₄O₄: | C, 76.36; | H, 4.27 |
| Found: | C, 76.07; | H, 4.18 |

### Example 143

### Preparation of 4-[3-(2-naphthalenyloxy)propyl]-alpha-oxobenzeneacetic acid

A suspension of 4-[3-(2-naphthalenyloxy)-1-propynyl]-alpha-oxobenzeneacetic acid (0.13 g) in dichloromethane (19 ml) was hydrogenated over 10% palladium on carbon (0.016 g) at atmospheric pressure. The resulting mixture was filtered twice through a pad of celite to remove insoluble materials, then the filtrate was evaporated and the residue was crystallized from ethyl acetate-hexane to afford 0.083 g of 4-[3-(2-naphthalenyloxy) propyl]-alpha-oxobenzeneacetic acid, mp 151-154 °C.

| | | |
|---|---|---|
| Analysis Calculated for C₂₁H₁₈O₄: | C, 75.43; | H, 5.43 |
| Found: | C, 74.92; | H, 5.35 |

### Example 144

### Preparation of N-(2-propynyl)-2-naphthalenecarboxamide

Propargylamine (0.80 ml) and triethylamine (1.67 ml) were added simultaneously to a solution of 2-naphthoyl chloride (2.22 g) in dichloromethane (25 ml) cooled in an ice bath. The mixture was held at 0 °C for 1 hour and allowed to warm to room temperature over 3 hours. The solution was diluted with ethyl acetate, washed in turn with water, 1N hydrochloric acid, 1N sodium hydroxide, and saturated brine. Evaporation of the dried (K₂CO₃) organic layer gave a residue which was crystallized from dichloromethane-hexane to give 2.06 g of N-(2-propynyl)-2-naphthalenecarboxamide, mp 163-165 °C.

| | | | |
|---|---|---|---|
| Analysis Calculated for C₁₄H₁₁NO: | C, 80.36; | H, 5.30; | N, 6.69 |
| Found: | C, 80.09; | H, 5.27; | N, 6.65 |

### Example 145

### Preparation of 4-[3-[[(2-naphthalenyl)carbonyl]amino]-1-propynyl]-alpha-oxobenzeneacetic acid methyl ester

Argon was bubbled through a solution of N-(2-propynyl)-2-naphthalenecarboxamide (0.335 g), alpha-oxo-4-[[(trifluoromethyl)sulfonyl]oxy]benzeneacetic acid methyl ester (0.5g), triethylamine (0.44 ml) and lithium chloride (0.1 g) in dimethylformamide (4 ml) for 15 minutes and then bis(triphenylphosphine)palladium dichloride (0.07 g) was added. The mixture was allowed to stir overnight at room temperature, diluted with 50 ml of dichloro-methane and washed with water (2 x 50 ml) and brine (25 ml). The dried (MgSO₄) organic phase was concentrated to give a residue which was chromatographed over 50 g of silica gel (dichloromethane-ethyl acetate; 9:1) to afford 0.33 g of a yellow powder. Crystallization from ethyl acetate-hexane gave 0.25 g of 4-[3-[[(2-naphthalenyl) carbonyl]amino]-1-propynyl]-alpha-oxobenzeneacetic acid methyl ester, mp 113-114 °C.

| | | | |
|---|---|---|---|
| Analysis Calculated for C₂₃H₁₇NO₄: | C, 74.38; | H, 4.61; | N, 3.77 |
| Found: | C, 74.20; | H, 4.56; | N, 3.82 |

### Example 146

### Preparation of 4-[3-[[(2-naphthalenyl)carbonyl]amino]-1-propynyl]-alpha-oxobenzeneacetic acid

A solution of 4-[3-[[(2-naphthalenyl)carbonyl]amino]-1-propynyl]-alpha-oxobenzeneacetic acid methyl ester (0.225 g) in tetrahydrofuran (2 ml) and methanol (1 ml) was treated with 1N sodium hydroxide (1 ml). After five minutes, the reaction mixture was concentrated and the resulting white suspension partitioned between dichloro-methane (10 ml) and 1N hydrochloric acid (2 ml). The aqueous layer was diluted with saturated sodium chloride and was extracted with tetrahydrofuran (2 x 10 ml). The combined extracts were dried (MgSO₄) and concentrated to give 0.2 g of a white solid, mp 153-155 °C. Crystallization from dichloromethane-ethyl acetate-hexane gave 0.15 g of 4-[3-[[(2-naphthalenyl)carbonyl]amino]-1-propynyl]-alpha-oxobenzeneacetic acid, mp 156-158 °C.

| | | | |
|---|---|---|---|
| Analysis Calculated for C₂₂H₁₅NO₄: | C, 73.94; | H, 4.23; | N, 3.92 |
| Found: | C, 73.76; | H, 4.21; | N, 3.80 |

### Example 147

### Preparation of 4-[3-[[(2-naphthalenyl)carbonyl]amino]propyl]alpha-oxobenzeneacetic acid

A solution of 4-[3-[[(2-naphthalenyl)carbonyl]amino]-1-propynyl]-alpha-oxobenzeneacetic acid (0.1 g) in dichloromethane (5 ml) was hydrogenated over 10% palladium on carbon (0.021 g) at atmospheric pressure for 4 hours. The mixture was filtered through a pad of celite and concentrated to give 0.106 g of a solid. Crystallization from ethyl acetate-hexane gave 0.056 g of 4-[3-[[(2-naphthalenyl)carbonyl]amino]propyl]-alpha-oxobenzeneacetic acid, mp 126-128 °C.

| | | | |
|---|---|---|---|
| Analysis Calculated for C₂₂H₁₉NO₄: | C, 72.67; | H, 5.39; | N, 3.78 |
| Found: | C, 72.41; | H, 5.41; | N, 3.79 |

### Example 148

### Preparation of 4-[[2-(2-naphthalenyloxy)ethyl]thio]-alpha-oxobenzeneacetic acid methyl ester

A stirred mixture of 1-(4-mercaptophenyl)ethanone (1.2 g) in dimethylformamide (10 ml) under argon was treated with 55% sodium hydride (0.350 g), stirred for 15 minutes and treated with the mesylate of 2-(2-naphthyloxy)ethanol (2.2 g). The mixture was heated at 60°C for 2 hours and diluted with water and filtered. The solids were dissolve in dichloromethane, washed once with water, dried (Na₂SO₄), filtered and evaporated. Crystallization from dichloromethane-hexane provided 2.0 g of 1-[4-[[2-(2-naphthalenyloxy)ethyl]thio] phenyl]ethanone, mp 151-152 °C.

| | | | |
|---|---|---|---|
| Analysis Calculated for C₂₀H₁₈O₂S: | C, 74.51; | H, 5.63; | S, 9.94 |
| Found: | C, 74.73; | H, 5.64; | S, 9.70 |

A solution of 1-[4-[[2-(2-naphthalenyloxy)ethyl]thio]phenyl]ethanone (1.9 g) in pyridine (10 ml) was treated with selenium dioxide (0.732 g) and heated at 100 °C overnight. The solution was filtered, acidified with 6N hydrochloric acid and the solids collected by filtration. The solids were dissolved in dichloromethane, washed with water, dried (Na₂SO₄), filtered and evaporated. The crude 4-[[2-(2-naphthalenyloxy)ethyl]thio]-alpha-oxobenzeneacetic acid was purified by preparation of the methyl ester.

A solution of crude 4-[[2-(2-naphthalenyloxy)ethyl]thio]-alpha-oxobenzeneacetic acid (1.6 g) in dichloromethane (500 ml) and triethylamine (1 ml) was treated with methyl chloroformate (0.58 ml) and stirred at room temperature for 1 hour. The mixture was diluted with water, extracted with dichloromethane, dried (Na₂SO₄), filtered and evaporated. The material from dichloromethane extraction was purified by HPLC (dichloromethane-hexane; 2:1) and crystallized from dichloromethane-diethyl ether to provide 0.85 g of 4-[[2-(2-naphthalenyloxy)ethyl]thio]-alpha-oxobenzeneacetic acid methyl ester, mp 124-125 °C.

| | | | |
|---|---|---|---|
| Analysis Calculated for C₂₁H₁₈O₄S: | C, 68.83; | H, 4.95; | S, 8.75 |
| Found: | C, 69.02; | H, 5.08; | S, 8.50 |

### Example 149

### Preparation of 4-[[2-(2-naphthalenyloxy)ethyl]thio]-alpha-oxobenzeneacetic acid

A mixture of 4-[[2-(2-naphthalenyloxy)ethyl]thio]-alpha-oxobenzeneacetic acid methyl ester (0.72 g) in hot methanol (10 ml) and sufficient tetrahydrofuran to dissolve the solids was treated with 1N sodium hydroxide (4.0 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give crude product. Crystallization from acetone-hexane provided 0.6 g of 4-[[2-(2-naphthalenyloxy)ethyl]thio]-alpha-oxobenzeneacetic acid as a colorless solid, mp 161-162 °C.

| | | | |
|---|---|---|---|
| Analysis Calculated for C₂₀H₁₆O₄S: | C, 68.17; | H, 4.58; | S, 9.10 |
| Found: | C, 68.14; | H, 4.47; | S, 8.91 |

### Example 150

### Preparation of rac.-4-[[2-(2-naphthalenyloxy)ethyl]sulfinyl]-alpha-oxobenzeneacetic acid methyl ester

A solution of 4-[[2-(2-naphthalenyloxy)ethyl]thio]-alpha-oxobenzeneacetic acid methyl ester (2.56 g) in dichloromethane (75 ml) was chilled in ice and 85 % meta-chloroperbenzoic acid (1.42 g) was added. The mixture was stirred cold for 1 hour, diluted with saturated sodium bicarbonate solution, and extracted with dichloromethane. The dried (Na₂SO₄) was filtered, evaporated and the residue was crystallized from dichloromethane-hexane to give 2.2 g of rac.-4-[[2-(2-naphthalenyloxy)ethyl]sulfinyl]-alpha-oxobenzeneacetic acid methyl ester, mp 129-130 °C.

| | | | |
|---|---|---|---|
| Analysis Calculated for C₂₁H₁₈O₅S: | C, 65.95; | H, 4.74; | S, 8.38 |
| Found: | C, 65.90; | H, 4.75; | S, 8.28 |

### Example 151

### Preparation of rac.-4-[[2-(2-naphthalenyloxy)ethyl]sulfinyl]-alpha-oxobenzeneacetic acid

A mixture of rac.-4-[[2-(2-naphthalenyloxy)ethyl]sulfinyl]-alpha-oxobenzeneacetic acid methyl ester (0.5 g) in hot methanol (5 ml) and sufficient tetrahydrofuran to dissolve the solids was treated with 1N sodium hydroxide (2.0 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give crude product. Crystallization from acetone-hexane provided 0.35 g of 4-[[2-(2-naphthalenyloxy) ethyl]sulfinyl]-alpha-oxobenzeneacetic acid as a yellow solid, mp 157-158 °C.

| | | | |
|---|---|---|---|
| Analysis Calculated for C₂₀H₁₆O₅S: | C, 65.21; | H, 4.38; | S, 8.70 |
| Found: | C, 65.06; | H, 4.27; | S, 8.99 |

### Example 152

### Preparation of 4-[[2-(2-naphthalenyloxy)acetyl]amino]-alpha-oxobenzeneacetic acid methyl ester

A stirred mixture of para-aminobenzophenone (6.75 g) in dichloromethane (75 ml) and triethylamine (20 ml) was chilled to -78 °C and treated with the acid chloride prepared from 2-naphthoxyacetic acid (10.1 g). The mixture was stirred for 30 minutes at -78 °C and for 1 hour at room temperature. The mixure was diluted with water and sodium bicarbonate solution and the solids were recovered by filtration and washed with water and dichloromethane to give 13.3 g of N-(4-acetylphenyl)-2-naphthoxyacetamide, 208-210 °C.

A mixture of N-(4-acetylphenyl)-2-naphthoxyacetamide (3.0 g) in pyridine (50 ml) was treated with selenium dioxide (1.66 g) and heated at 90°C overnight under argon. The mixture was filtered, diluted with 6N hydrochloric acid and the solids were recovered by filtration to give 3.0 g of crude 4-[[2-(2-naphthalenyloxy)acetyl]amino]-alpha-oxobenzeneacetic acid.

A mixture of 4-[[2-(2-naphthalenyloxy)acetyl]amino]-alpha-oxobenzeneacetic acid (3.0 g) in dichloromethane (50 ml) and triethylamine (2.1 g) was stirred at 0°C and treated with methyl chloroformate (1.16 ml). The mixture was stirred for one hour and diluted with water, extracted with dichloromethane, dried (Na₂SO₄), filtered and evaporated. The residue was crystallized from dichloromethane-hexane to give 2.2 g of 4-[[2-(2-naphthalenyloxy)acetyl]amino]-alpha-oxobenzeneacetic acid methyl ester, mp 175-176 °C.

| | | | |
|---|---|---|---|
| Analysis Calculated for C₂₁H₁₇NO₅: | C, 69.41; | H, 4.72; | N, 3.85 |
| Found: | C, 69.10; | H, 4.71; | N, 3.85 |

### Example 153

### Preparation of 4-[[2-(2-naphthalenyloxy)acetyl]amino]-alpha-oxobenzeneacetic acid

A mixture of 4-[[2-(2-naphthalenyloxy)acetyl]amino]-alpha-oxobenzeneacetic acid methyl ester (0.7 g) in hot methanol (10 ml) and sufficient tetrahydrofuran to dissolve the solids was treated with 1N sodium hydroxide (2.5 ml) and diluted with water. The organic solvent was removed under vacuum and the residue was mixed with water, acidified with excess 2N hydrochloric acid, and extracted with dichloromethane. The organic layer was dried (Na₂SO₄), filtered, and evaporated to give the crude product. Crystallization from acetone-hexane provided 0.61 g of 4-[[2-(2-naphthalenyloxy)acetyl]amino]-alpha-oxobenzeneacetic acid as a colorless solid, mp 215-216 °C.

| | | | |
|---|---|---|---|
| Analysis Calculated for C₂₀H₁₅NO₅: | C, 68.76; | H, 4.33; | N, 4.01 |
| Found: | C, 68.68; | H, 4.35; | N, 3.91 |

### Example 154

### Preparation of 4-[(2-naphthalenyl)methoxy]-alpha-oxobenzeneacetic acid methyl ester

A solution of 4-hydroxy-alpha-oxobenzeneacetic acid methyl ester (0.50 g in 5 ml of dimethylformamide was treated with 60% sodium hydride (0.112 g) and after 15 minutes, 2-bromomethylnaphthalene (0.61 g) was added. The reaction mixture was stirred 45 minutes at room temperature, was quenched with 0.1 ml of acetic acid and was diluted with diethyl ether-ethyl acetate (10:1, 50 ml). The mixture was washed with water (2 x 10 ml) and brine (1 x 10 ml), dried (MgSO₄) and chromatographed over 100 g of silica gel eluting with dichloromethane-hexane (1:1) to give 0.61 g of 4-[(2-naphthalenyl)methoxy]-alpha-oxobenzeneacetic acid methyl ester, mp 101-102 °C.

| | | |
|---|---|---|
| Analysis Calculated for C₂₀H₁₆O₄: | C, 74.99; | H, 5.03 |
| Found: | C, 74.59; | H, 5.10 |

### Example 155

### Preparation of 4-[(2-naphthalenyl)methoxy]-alpha-oxobenzeneacetic acid

A solution of 4-[(2-naphthalenyl)methoxy]-alpha-oxobenzeneacetic acid methyl ester (0.58 g) in methanol (3 ml) and tetrahydrofuran (8 ml) was treated with 1N sodium hydroxide (2.0 ml) and a white precipitate formed immediately. After 5 minutes, the reaction mixture was concentrated, the residue was acidified with excess hydrochloric acid and partitioned between dichloromethane (100 ml) and water (10 ml). The organic layer was dried (MgSO₄) and concentrated. The resulting residue was crystallized from dichloromethane-hexane-tetrahydrofuran to give 0.315 g of 4-[(2-naphthalenyl)methoxy]-alpha-oxobenzeneacetic acid, mp 145-146 °C.

| | | |
|---|---|---|
| Analysis Calculated for C₁₉H₁₄O₄: | C, 74.50; | H, 4.61 |
| Found: | C, 74.31; | H, 4.50 |

### Example 156

### Preparation of 4-[2-(2-naphthalenyl)-2-oxoethoxy]-alpha-oxobenzeneacetic acid methyl ester

A solution of 4-hydroxy-alpha-oxobenzeneacetic acid methyl ester (0.50 g) in 5 ml of dimethylformamide was treated with 60% sodium hydride (0.112 g) and after 20 minutes, bromomethyl 2-naphthyl ketone (0.70 g) was added. The reaction mixture was stirred 3 hours at room temperature, was quenched with 0.1 ml of acetic acid and was diluted with ethyl acetate (75 ml). The mixture was washed with water (1 x 25 ml) and brine (1 x 25 ml), dried (MgSO₄) and chromatographed over 100 g of silica gel, eluting with dichoromethane-ethyl acetate (50:1) to give 0.62 g of 4-[2-(2-naphthalenyl)-2-oxoethoxy]-alpha-oxobenzeneacetic acid methyl ester, mp 120-121 °C.

| | | |
|---|---|---|
| Analysis Calculated for C₂₁H₁₆O₅·0.33 H₂O: | C, 71.19; | H, 4.73 |
| Found: | C, 71.18; | H, 4.65 |

### Example 157

### Preparation of 4-[2-(2-naphthalenyl)-2-oxoethoxy]-alpha-oxobenzeneacetic acid

A solution of 4-[2-(2-naphthalenyl)-2-oxoethoxy]-alpha-oxobenzeneacetic acid methyl ester (0.57 g) in methanol (3 ml) and tetrahydrofuran (8 ml) was treated with 1N sodium hydroxide (1.7 ml) and a white precipitate formed after 1 minutes. After 5 minutes, the reaction mixture was concentrated, the residue was acidified with excess hydrochloric acid and partitioned between dichloromethane (100 ml) and water (10 ml). The organic layer was dried (MgSO₄) and concentrated. The resulting residue was crystallized from ethyl acetate-hexane-tetrahydrofuran to give 0.343 of 4-[2-(2-naphthalenyl)-2-oxoethoxy]-alpha-oxobenzeneacetic acid, mp 167-170 °C.

| | | |
|---|---|---|
| Analysis Calculated for C₂₀H₁₄O₅: | C, 71.85; | H, 4.22 |
| Found: | C, 71.52; | H, 4.20 |

### Example 158

### Preparation of 4-[(2-quinolinyl)methoxy]-alpha-oxobenzeneacetic acid methyl ester

A solution of 4-hydroxy-alpha-oxobenzeneacetic acid methyl ester (0.50 g) and 2-chloromethylquinoline hydrochloride (0.60 g) were suspended in 6 ml of dimethylformamide and 60% sodium hydride (0.224 g) was added. When gas evolution ceased, the bath temperature was raised to 50 °C for 2 hours. The reaction mixture was quenched with 0.1 ml of acetic acid and was diluted with ethyl acetate (75 ml). The mixture was washed with water (2 x 25 ml) and brine (1 x 25 ml), dried (MgSO₄) and chromatographed over 100 g of silica gel, eluting with dichoromethane-ethylacetate (50:1) to give 0.39 g of 4-[(2-quinolinyl)methoxy]-alpha-oxobenzeneacetic acid methyl ester, mp 109-110 °C.

| | | | |
|---|---|---|---|
| Analysis Calculated for C₁₉H₁₅NO₄·0.05 CH₂Cl₂: | C, 70.27; | H, 4.67; | N, 4.30 |
| Found: | C, 70.24; | H, 4.57; | N, 4.32 |

### Example 159

### Preparation of 4-[(2-quinolinyl)methoxy]-alpha-oxo-benzeneacetic acid

A solution of 4-[(2-quinolinyl)methoxy]-alpha-oxobenzeneacetic acid methyl ester (0.34 g) in methanol (2 ml) and tetrahydrofuran (8 ml) was treated with 1N sodium hydroxide (1.1 ml) and a white precipitate formed. After 5 minutes, the reaction mixture was concentrated and the residue was acidified with excess hydrochloric acid. The residue was triturated with water (25 ml) and dichloromethane-tetrahydrofuran (150 ml, 9:1) and the solids were crystallized twice from dimethylsulfoxide-acetonitrile-water to give 0.142 g of 4-[(2-quinolinyl)methoxy]-alpha-oxo-benzeneacetic acid, mp 225-228 °C.

| | | | |
|---|---|---|---|
| Analysis Calculated for C₁₈H₁₃NO₄·0.1 H₂O: | C, 69.92; | H, 4.27; | N, 4.53 |
| Found: | C, 69.75; | H, 4.17; | N, 4.52 |

### Example 160

### Preparation of 4-[[2-(2-naphthalenyloxy)ethyl]oxy]-alpha-oxobenzeneacetamide

A solution of 4-[[2-(2-naphthalenyloxy)ethyl]oxy]-alpha-oxobenzeneacetic acid methyl ester (0.5 g) in methanol (40 ml) and tetrahydrofuran (40 ml) was chilled in an ice bath and saturated with ammonia gas. The cooling bath was removed and the mixture was stirred at room temperature for 4 hours and the solution was evaporated to dryness. Crystallization from tetrahydrofuran-ethyl alcohol provided 0.4 g of 4-[[2-(2-naphthalenyloxy)ethyl]oxy]-alpha-oxobenzeneacetamide as a colorless solid, mp 194-196 °C.

| | | | |
|---|---|---|---|
| Analysis Calculated for C₂₀H₁₇NO₄: | C, 71.63; | H, 5.11; | N, 4.18 |
| Found: | C, 71.60; | H, 5.14; | N, 4.14 |

### Example 161

### Preparation of N,N-dimethyl-4-[[2-(2-naphthalenyloxy)ethyl]oxy]-alpha-oxobenzeneacetamide

A solution of 4-[[2-(2-naphthalenyloxy)ethyl]oxy]-alpha-oxobenzeneacetic acid methyl ester (0.5 g) in methanol (40 ml) and tetrahydrofuran (40 ml) was chilled in an ice bath and excess anhydrous gaseous dimethylamine was bubbled into the solution. The cooling bath was removed and the mixture was stirred at room temperature for 6 hours and the mixture was evaporated to dryness. Crystallization from tetrahydrofuran-ethyl alcohol provided 0.5 g of N,N-dimethyl-4-[[2-(2-naphthalenyloxy)ethyl]oxy]-alpha-oxobenzeneacetamide as a colorless solid, mp 170-172 °C.

| | | | |
|---|---|---|---|
| Analysis Calculated for C₂₂H₂₁NO₄: | C, 72.71; | H, 5.82; | N, 3.85 |
| Found: | C, 72.67; | H, 5.88; | N, 3.78 |

### Example 162

### Preparation of N-(2-hydroxyethyl)-4-[[2-(2-naphthalenyloxy)ethyl]oxy]-alpha-oxobenzeneacetamide

A solution of 4-[[2-(2-naphthalenyloxy)ethyl]oxy]-alpha-oxobenzeneacetic acid methyl ester (0.5 g) in methanol (40 ml) and tetrahydrofuran (10 ml) was treated with ethanolamine (0.094 g) and the mixture was refluxed for 48 hours. The solvents were removed by evaporation and the residue was purified by HPLC (ethyl acetate-methanol-triethylamine; 95:5:2) followed by crystallization from tetrahydrofuran-ethyl alcohol to give 0.29 g of N-(2-hydroxyethyl)-4-[[2-(2-naphthalenyloxy)ethyl]oxy]-alpha-oxobenzeneacetamide as a colorless solid, mp 138-140 °C.

| | | | |
|---|---|---|---|
| Analysis Calculated for C₂₂H₂₁NO₅: | C, 69.65; | H, 5.58; | N, 3.69 |
| Found: | C, 69.36; | H, 5.52; | N, 3.54 |

### Example 163

### Preparation of N,N-bis(2-hydroxyethyl)-4-[[2-(2-naphthalenyloxy)ethyl]oxy]-alpha-oxobenzeneacetamide

A solution of 4-[[2-(2-naphthalenyloxy)ethyl]oxy]-alpha-oxobenzeneacetic acid methyl ester (0.5 g) in methanol (40 ml) and tetrahydrofuran (10 ml) was treated with diethanolamine (0.162 g) and the mixture was refluxed for 18 hours. The solvents were removed by evaporation and the residue was crystallized from acetone to give 0.185 g of N,N-bis(2-hydroxyethyl)-4-[[2-(2-naphthalenyloxy)ethyl]oxy]-alpha-oxobenzeneacetamide as a colorless solid, mp 128-133 °C.

| | | | |
|---|---|---|---|
| Analysis Calculated for C₂₄H₂₅NO₆: | C, 68.07; | H, 5.95; | N, 3.31 |
| Found: | C, 68.10; | H, 5.87; | N, 3.28 |

### Example 164

### Preparation of N-[2-(dimethylamino)ethyl]-4-[[2-(2-naphthalenyloxy)ethyl]oxy]-alpha-oxobenzeneacetamide

A solution of 4-[[2-(2-naphthalenyloxy)ethyl]oxy]-alpha-oxobenzeneacetic acid methyl ester (0.5 g) in methanol (75 ml) was treated with N,N-dimethylethylenediamine (0.2 ml) and the mixture was refluxed for 18 hours. The solvents were removed by evaporation and the residue was purified by HPLC (ethyl acetate-methanol-triethylamine; 95:5:2) followed by crystallization from ethyl acetate-hexane to give 0.28 g of N-[2-(dimethylamino)ethyl]-4-[[2-(2-naphthalenyloxy)ethyl]oxy]-alpha-oxobenzeneacetamide as a colorless solid, mp 102-104 °C.

| | | | |
|---|---|---|---|
| Analysis Calculated for C₂₄H₂₆N₂O₄: | C, 70.92; | H, 6.45; | N, 6.89 |
| Found: | C, 71.00; | H, 6.45; | N, 6.94 |

### Example 165

### Preparation of 4-[N-[2-(2-naphthalenyloxy)ethyl]formamido]-alpha-oxobenzeneacetic acid methyl ester

A stirred mixture of N-(4-acetylphenyl)formamide (4.9 g) in dimethylformamide (50 ml) under argon was treated with 55% sodium hydride (1.31 g), stirred for 15 minutes and treated with 2-(2-naphthalenyloxy)ethyl methanesulfonate (7.6 g). The mixture was heated at 60 °C overnight and worked up as in Example 20. The material from dichloromethane extraction was purified by HPLC (dichloromethane-ethyl acetate; 20:1) and crystallized from dichloromethane-hexane to provide 5.9 g of N-(4-acetylphenyl)-N-[2-(2-naphthalenyloxy)ethyl]formamide as a colorless solid, mp 115-116 °C.

| | | | |
|---|---|---|---|
| Analysis Calculated for C₂₁H₁₉NO₃: | C, 75.66; | H, 5.74; | N, 4.20 |
| Found: | C, 75.38; | H, 5.72; | N, 4.16 |

A solution of N-(4-acetylphenyl)-N-[2-(2-naphthalenyloxy)ethyl]formamide (1.2 g) in pyridine (10 ml) was treated with selenium dioxide (0.61 g) and heated at 100 °C for five hours. The solution was cooled, diluted with dichloromethane (40 ml), and filtered through celite. The filtrate was chilled in an ice bath and treated with methyl chloroformate (2 ml) and stirred for 15 minutes. The resulting mixure was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-ethyl acetate; 50:1) and crystallized from dichloromethane-hexane to provide 0.92 g of 4-[N-[2-(2-naphthalenyloxy)ethyl] formamido]-alpha-oxobenzeneacetic acid methyl ester as a colorless solid, mp 103-104 °C.

| | | | |
|---|---|---|---|
| Analysis Calculated for C₂₂H₁₉NO₅: | C, 70.02; | H, 5.07; | N, 3.71 |
| Found: | C, 69.81; | H, 4.89; | N, 3.50 |

### Example 166

### Preparation of 4-[N-[2-(2-naphthalenyloxy)ethyl]formamido]-alpha-oxobenzeneacetic acid

A solution of 4-[N-[2-(2-naphthalenyloxy)ethyl]formamido]-alpha-oxobenzeneacetic acid methyl ester (0.5 g) in warm methanol (5 ml) and tetrahydrofuran (5 ml) was treated with 1N sodium hydroxide (2 ml) and the mixture was concentrated to remove the organic solvents. The residue was acidified with excess hydrochloric acid and extracted with dichloromethane. The organic layer was washed with water, dried (Na₂SO₄), filtered and evaporated. The residue was crystallized from acetone-hexane to give 0.32 g of 4-[N-[2-(2-naphthalenyloxy)ethyl]formamido]-alpha-oxobenzeneacetic acid, mp 155-156 °C as a yellow solid.

| | | | |
|---|---|---|---|
| Analysis Calculated for C₂₁H₁₇NO₅: | C, 69.41; | H, 4.72; | N, 3.85 |
| Found: | C, 69.25; | H, 4.61; | N, 3.80 |

### Example 167

### Preparation of 4-[[2-(2-naphthalenyloxy)ethyl]amino]-alpha-oxobenzeneacetic acid methyl ester

A solution of 4-[N-[2-(2-naphthalenyloxy)ethyl]formamido]-alpha-oxobenzeneacetic acid methyl ester (1.13 g) in tetrahydrofuran (30 ml) and 1N hydrochloric acid (9 ml) was refluxed for four hours and cooled. The mixture was diluted with dichloromethane (100 ml), washed with saturated aqueous sodium bicarbonate solution and water, dried (Na₂SO₄), filtered and evaporated to give 1 g of crude product. Crystallization from acetone provided 0.8 g of purified 4-[N-[2-(2-naphthalenyloxy)ethyl]formamido]-alpha-oxobenzeneacetic acid methyl ester, mp 177-179 °C, as a yellow solid.

| | | | |
|---|---|---|---|
| Analysis Calculated for C₂₁H₁₉NO₄: | C, 72.19; | H, 5.48; | N, 4.01 |
| Found: | C, 71.95; | H, 5.32; | N, 3.90 |

### Example 168

### Preparation of 4-[[2-(2-naphthalenyloxy)ethyl]amino]-alpha-oxobenzeneacetic acid

A solution of 4-[N-[2-(2-naphthalenyloxy)ethyl]formamido]-alpha-oxobenzeneacetic acid methyl ester (0.5 g) in hot methanol (5 ml) and tetrahydrofuran (10 ml) was treated with 1N sodium hydroxide (3.0 ml) and the mixture was concentrated to remove the organic solvents. The residue, in water, was treated with 2N hydrochloric acid and the mixture was chilled, filtered, and washed with water. The solids were dissolved in a mixture of tetrahydrofuran and dichloromethane, dried (Na₂SO₄), filtered, and evaporated to give crude product. Crystallization from acetone-hexane provided 0.4 g of purified 4-[N-[2-(2-naphthalenyloxy)ethyl]formamido]-alpha-oxobenzeneacetic acid as a yellow solid, mp 172 °C with decomposition.

| | | | |
|---|---|---|---|
| Analysis Calculated for C₂₀H₁₇NO₄: | C, 71.63; | H, 5.11; | N, 4.18 |
| Found: | C, 71.50; | H, 4.98; | N, 4.17 |

### Example 169

### Preparation of rac.-4-[[2-(2-naphthalenyloxy)ethyl]oxy]-alpha-oxobenzeneacetic acid (2,2-dimethyl-1,3-dioxolan-4-yl)methyl ester

Oxalyl chloride (1 ml) was added dropwise with stirring to a chilled (0 °C) solution of 2-[[2-(2-naphthalenyloxy)ethyl]oxy]-alpha-oxobenzeneacetic acid (1 g) in dichloromethane (20 ml) containing a catalytic amount of dimethylformamide. After the addition was complete, the cooling bath was removed and the mixture was stirred at room temperature for 1 hour. The solvent was then removed in vacuo to give the crude acid chloride.

A solution of the above acid chloride in dichloromethane (15 ml) was added dropwise with stirring to a chilled (0 °C) solution of solketal (0.529 g) and triethylamine (0.7 ml) in dichloromethane (20 ml) and the reaction was stirred cold for one hour. The mixture was washed with water, dried (Na₂SO₄), filtered and evaporated to give crude product. The material was purified by HPLC (dichloromethane-hexane-ethyl acetate; 10:10:1) and the resulting solid was crystallized from dichloromethane-hexane to yield 1 g of colorless rac.-4-[[2-(2-naphthalenyloxy)ethyl]oxy]-alpha-oxobenzeneacetic acid (2,2-dimethyl-1,3-dioxolan-4-yl)methyl ester, mp 128-129 °C.

| | | |
|---|---|---|
| Analysis Calculated for C₂₆H₂₆O₇: | C, 69.32; | H, 5.82 |
| Found: | C, 69.03; | H, 5.73 |

### Example 170

### Preparation of 4-[[2-(2-naphthalenyloxy)ethyl]sulfonyl]-alpha-oxobenzeneacetic acid methyl ester

A solution of rac.-4-[[2-(2-naphthalenyloxy)ethyl]sulfinyl]-alpha-oxobenzeneacetic acid methyl ester (1 g) in dichloromethane (50 ml) was treated with 85 % meta-chloroperbenzoic acid (0.81 g) and stirred at room temperature for one hour. The mixture was washed with saturated sodium bicarbonate solution, dried (Na₂SO₄), filtered, passed through silica gel (5 g) and the product eluted with dichloromethane. Crysallization from dichloromethane-hexane gave 0.61 g of 4-[[2-(2-naphthalenyloxy)ethyl]sulfonyl]-alpha-oxobenzeneacetic acid methyl ester, mp 110-111 °C.

| | | | |
|---|---|---|---|
| Analysis Calculated for C₂₁H₁₈O₆S: | C, 63.31; | H, 4.55; | S, 8.05 |
| Found: | C, 63.10; | H, 4.44; | S, 8.11 |

### Example 171

### Preparation of 4-[[2-(2-naphthalenyloxy)ethyl]sulfonyl]-alpha-oxobenzeneacetic acid.

A solution of 4-[[2-(2-naphthalenyloxy)ethyl]sulfonyl]-alpha-oxobenzeneacetic acid methyl ester (0.76 g) in warm methanol (5 ml) and tetrahydrofuran (5 ml) was treated with 1N sodium hydroxide (2.1 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from diethyl ether-hexane provided 0.39 g of purified 4-[[2-(2-naphthalenyloxy)ethyl]sulfonyl]-alpha-oxobenzeneacetic acid as a colorless solid, mp 135-136 °C.

| | | | |
|---|---|---|---|
| Analysis Calculated for C₂₀H₁₆O₆S: | C, 62.49; | H, 4.20; | S, 8.34 |
| Found: | C, 62.18; | H, 4.25; | S, 8.26 |

### Example 172

### Preparation of 4-[[2-(cyclooctyloxy)ethyl]thio]-alpha-oxobenzeneacetic acid methyl ester.

A stirred mixture of 1-(4-mercaptophenyl)ethanone (0.76 g) in dimethylformamide (10 ml) under argon was treated with 55% sodium hydride (0.218 g), stirred for 20 minutes and treated with 2-(cyclooctyloxy)ethyl methanesulfonate (1.25 g). The mixture was heated at 60 °C overnight, cooled, diluted with water, extracted twice with dichloromethane, and the organic layers were washed with water. The combined organic layers were dried (Na₂SO₄), filtered, and evaporated to give crude material which was purified by HPLC (dichloromethane-hexane-ethyl acetate; 48:48:4) to provide 1.08 g of 1-[4-[[2-(cyclooctyloxy)ethyl]thio]phenyl]ethanone as a colorless oil.
A mixture of 1-[4-[[2-(cyclooctyloxy)ethyl]thio]phenyl]ethanone (1.08 g) in pyridine (10 ml) was treated with selenium dioxide (0.78 g) and heated at 100 °C overnight under argon. The mixture was cooled, diluted with dichloromethane (50 ml), and filtered through celite. The filtrate was chilled in an ice bath and treated with methyl chloroformate (3 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane) to provide 1.04 g of 4-[[2-(cyclooctyloxy)ethyl]thio]-alpha-oxobenzeneacetic acid methyl ester as a pale yellow oil.

| | | | |
|---|---|---|---|
| Analysis Calculated for C₁₉H₂₆O₄S: | C, 65.11; | H, 7.48; | S, 9.15 |
| Found: | C, 65.01; | H, 7.48; | S, 8.91 |

### Example 173

### Preparation of 4-[[2-(cyclooctyloxy)ethyl]thio]-alpha-oxobenzeneacetic acid.

A solution of 4-[[2-(cyclooctyloxy)ethyl]thio]-alpha-oxobenzeneacetic acid methyl ester (0.96 g) in warm methanol (10 ml) and tetrahydrofuran (10 ml) was treated with 1N sodium hydroxide (3.5 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from diethyl ether-hexane provided 0.86 g of purified 4-[[2-(cyclooctyloxy)ethyl]thio]-alpha-oxobenzeneacetic acid as a yellow solid, mp 84-85 °C.

| | | | |
|---|---|---|---|
| Analysis Calculated for C₁₈H₂₄O₄S: | C, 64.26; | H, 7.19; | S, 9.53 |
| Found: | C, 64.30; | H, 7.23; | S, 9.41 |

### Example 174

### Preparation of 4-[[2-[2-[(2,2-dimethyl-1-oxobutoxy)methyl]-6-methylphenoxy]ethyl]thio]-alpha-oxobenzeneacetic acid methyl ester.

A stirred mixture of 1-(4-mercaptophenyl)ethanone (0.9 g) in dimethylformamide (10 ml) under argon was treated with 55% sodium hydride (0.262 g), stirred for 10 minutes and treated with 2-[[6-methyl-2-(2,2-dimethyl-1-oxobutoxy)methyl] phenoxy]ethyl methanesulfonate (2.15 g). The mixture was heated at 60 °C for one hour, cooled, acidified with 3 drops of glacial acetic acid and the volatiles removed by evaporation. The residue was diluted with water, extracted twice with dichloromethane, and the organic layers were washed with water. The combined organic layers were dried (Na₂SO₄), filtered, and evaporated to give crude material which was purified by HPLC (dichloromethane-hexane-diethyl ether); 48:48:4) to provide 1.97 g of 2,2-dimethylbutanoic acid [2-[2-[(4-acetylphenyl)thio]ethoxy]-3-methylphenyl] methyl ester as a yellow oil.

| | | | |
|---|---|---|---|
| Analysis Calculated for C₂₄H₃₀O₄S: | C, 69.54; | H, 7.29; | S, 7.73 |
| Found: | C, 69.42; | H, 7.36; | S, 7.86 |

A mixture of 2,2-dimethylbutanoic acid [2-[2-[(4-acetylphenyl)thio]ethoxy]-3-methylphenyl]methyl ester (1.97 g) in pyridine (10 ml) was treated with selenium dioxide (1.05 g) and heated at 100 °C overnight under argon. The mixture was cooled, diluted with dichloromethane (50 ml), and filtered through celite. The filtrate was chilled in an ice bath and treated with methyl chloroformate (3 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane-diethyl ether; 49:49:2) to provide 2.07 g of 4-[[2-[2-[(2,2-dimethyl-1-oxobutoxy)methyl]-6-methylphenoxy]ethyl]thio]-alpha-oxobenzeneacetic acid methyl ester as a pale yellow oil.

| | | | |
|---|---|---|---|
| Analysis Calculated for C₂₅H₃₀O₆S: | C, 65.48; | H, 6.59; | S, 6.99 |
| Found: | C, 65.75; | H, 6.68; | S, 7.13 |

### Example 175

### Preparation of 4-[[2-[2-[(2,2-dimethyl-1-oxobutoxy)methyl]-6-methylphenoxy]ethyl]thio]-alpha-oxobenzeneacetic acid.

A solution of 4-[[2-[2-[(2,2-dimethyl-1-oxobutoxy)methyl]-6-methylphenoxy] ethyl]thio]-alpha-oxobenzeneacetic acid methyl ester (1.9 g) in warm methanol (10 ml) and tetrahydrofuran (5 ml) was treated with 1N sodium hydroxide (5 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Purification was accomplished by chromatography on 60 g of silica gel using dichloromethane-diethyl ether-formic acid (50:50:1) to elute impurities followed by elution with diethyl ether-formic acid (99:1) to recover 1.1 g of pure 4-[[2-[2-[(2,2-dimethyl-1-oxobutoxy) methyl]-6-methylphenoxy]ethyl]thio]-alpha-oxobenzeneacetic acid as a yellow oil.

| | | | |
|---|---|---|---|
| Analysis Calculated for C₂₄H₂₈O₆S: | C, 64.84; | H, 6.35; | S, 7.21 |
| Found: | C, 64.83; | H, 6.34; | S, 7.28 |

### Example 176

### Preparation of 4-[[[2-(2-naphthalenyloxy)ethyl]amino]carbonyl]-alpha-oxobenzeneacetic acid methyl ester.

A stirred mixture of 2-(2-naphthalenyloxy)ethanamine (4.9 g) in dichloromethane (25 ml) and triethylamine (5 ml) was chilled to 0 °C and treated with the acid chloride prepared from 4-acetyl benzoic acid (4.3 g) and then the mixture was stirred at room temperature for 10 minutes. The mixture was diluted with water and acidified with excess 2N hydrochloric acid, extracted with dichloromethane and the organic layer was washed in turn with sodium bicarbonate solution and saturated sodium chloride solution. The organic layer was dried (Na₂SO₄), filtered, and evaporated to give crude material which was purified by HPLC (ethyl acetate-dichloromethane; 1:9) and crystallized from ethyl acetate to provide 4.7 g of 4-acetyl-N-[2-(2-naphthalenyloxy)ethyl]benzamide as a colorless solid, mp 131-132 °C.

| | | | |
|---|---|---|---|
| Analysis Calculated for C₂₁H₁₉NO₃: | C, 75.66; | H, 5.74; | N, 4.20 |
| Found: | C, 75.41; | H, 5.75; | N, 4.09 |

A mixture of 4-acetyl-N-[2-(2-naphthalenyloxy)ethyl]benzamide (1.2 g) in pyridine (10 ml) was treated with selenium dioxide (0.61 g) and heated at 100 °C for 5 hours under argon. The mixture was cooled, diluted with dichloromethane (30 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-ethyl acetate; 20:1) and crystallized from dichloromethane-hexane to provide 0.815 g of 4-[[[2-(2-naphthalenyloxy)ethyl]amino]carbonyl]-alpha-oxobenzeneacetic acid methyl ester as a colorless solid, mp 114-115 °C.

| | | | |
|---|---|---|---|
| Analysis Calculated for C₂₂H₁₉NO₅: | C, 70.02; | H, 5.07; | N, 3.71 |
| Found: | C, 70.09; | H, 4.98; | N, 3.51 |

### Example 177

### Preparation of 4-[[[2-(2-naphthalenyloxy)ethyl]amino]carbonyl]-alpha-oxobenzeneacetic acid.

A solution of 4-[[[2-(2-naphthalenyloxy)ethyl]amino]carbonyl]-alpha-oxobenzeneacetic acid methyl ester (0.35 g) in methanol (5 ml) and tetrahydrofuran (5 ml) was treated with 1N sodium hydroxide (1 ml) and after 5 minutes the mixture was concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.275 g of purified 4-[[[2-(2-naphthalenyloxy)ethyl]amino]carbonyl]-alpha-oxobenzeneacetic acid as a colorless solid, mp 173-175 °C.

| | | | |
|---|---|---|---|
| Analysis Calculated for C₂₁H₁₇NO₅: | C, 69.41; | H, 4.72; | N, 3.85 |
| Found: | C, 69.35; | H, 4.56; | N, 3.64 |

### Example 178

### Preparation of 4-[2-(2-naphthalenyloxy)ethoxy]-3-nitro-alpha-oxobenzeneacetic acid methyl ester.

A stirred mixture of 4-hydroxy-3-nitroacetophonone (2 g) in dimethylformamide (30 ml) under argon was treated with 55% sodium hydride (0.48 g), stirred for 30 minutes and treated with 2-(2-naphthalenyloxy)ethyl methanesulfonate (2.75 g). The mixture was heated at 60 °C for 72 hours and the dimethylformamide evaporated. The residue was mixed with water and excess sodium hydroxide solution, the product was extracted twice with dichloromethane, and the organic layers were washed with water. The combined organic layers were dried (Na₂SO₄), filtered, and evaporated to give crude material which was purified by HPLC (dichloromethane-hexane; 9:1) and crystallized from dichloromethane-diethyl ether to provide 0.9 g of 1-[4-[2-(2-naphthalenyloxy)ethoxy]-3-nitrophenyl]ethanone as a yellow solid, mp 158-160 °C.

| | | | |
|---|---|---|---|
| Analysis Calculated for C₂₀H₁₇NO₅: | C, 68.37; | H, 4.88; | N, 3.99 |
| Found: | C, 68.40; | H, 4.90; | N, 4.21 |

A mixture of 1-[4-[2-(2-naphthalenyloxy)ethoxy]-3-nitrophenyl]ethanone (0.875 g) in pyridine (20 ml) was treated with selenium dioxide (0.416 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium chloride solution. The organic extracts were dried (Na₂SO₄), filtered and evaporated. The crude material was purified by HPLC (dichloromethane-hexane; 9:1) and crystallized from dichloromethane-hexane to provide 0.65 g of 4-[2-(2-naphthalenyloxy)ethoxy]-3-nitro-alpha-oxobenzeneacetic acid methyl ester as a yellow solid, mp 127-129 °C.

| | | | |
|---|---|---|---|
| Analysis Calculated for C₂₁H₁₇NO₇: | C, 63.80; | H, 4.33; | N, 3.54 |
| Found: | C, 63.53; | H, 4.30; | N, 3.43 |

### Example 179

### Preparation of 4-[2-(2-naphthalenyloxy)ethoxy]-3-nitro-alpha-oxobenzeneacetic acid.

A solution of 4-[2-(2-naphthalenyloxy)ethoxy]-3-nitro-alpha-oxobenzeneacetic acid methyl ester (0.55 g) in warm methanol (10 ml) and tetrahydrofuran (10 ml) was treated with 1N sodium hydroxide (2 ml) and after 5 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.42 g of purified 4-[2-(2-naphthalenyloxy)ethoxy]-3-nitro-alpha-oxobenzeneacetic acid as a yellow solid, mp 164 °C with decomposition.

| | | | |
|---|---|---|---|
| Analysis Calculated for C₂₀H₁₅NO₇: | C, 62.99; | H, 3.96; | N, 3.67 |
| Found: | C, 62.94; | H, 4.06; | N, 3.46 |

### Example 180

### Preparation of 3-methyl-4-[2-(2-naphthalenyloxy)ethoxy]-alpha-oxobenzeneacetic acid methyl ester.

A stirred mixture of 4-hydroxy-3-methylacetophonone (2 g) in dimethylformamide (30 ml) under argon was treated with 55% sodium hydride (0.58 g), stirred for 30 minutes and treated with 2-(2-naphthalenyloxy)ethyl methanesulfonate (3.65 g). The mixture was heated at 60 °C for 2 hours, cooled, diluted with water and filtered. The damp solids were mixed with water and excess sodium hydroxide solution, the product was extracted twice with dichloromethane, and the organic layers were washed with water. The combined organic layers were dried (Na₂SO₄), filtered, and evaporated to give crude material which was purified by HPLC (dichloromethane-hexane; 9:1) and crystallized from dichloromethane-diethyl ether to provide 3.2 g of 1-[3-methyl-4-[2-(2-naphthalenyloxy)ethoxy]phenyl]ethanone as a colorless solid, mp 129-131 °C.

| | | |
|---|---|---|
| Analysis Calculated for C₂₁H₂₀O₃: | C, 78.73; | H, 6.29 |
| Found: | C, 78.91; | H, 6.16 |

A mixture of 1-[3-methyl-4-[2-(2-naphthalenyloxy)ethoxy]phenyl]ethanone (1 g) in pyridine (20 ml) was treated with selenium dioxide (0.522 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from dichloromethane-hexane to provide 0.8 g of 3-methyl-4-[2-(2-naphthalenyloxy)ethoxy]-alpha-oxobenzeneacetic acid methyl ester as a colorless solid, mp 118-119 °C.

| | | |
|---|---|---|
| Analysis Calculated for C₂₂H₂₀O₅: | C, 72.51; | H, 5.53 |
| Found: | C, 72.56; | H, 5.44 |

### Example 181

### Preparation of 3-methyl-4-[2-(2-naphthalenyloxy)ethoxy]-alpha-oxobenzeneacetic acid.

A solution of 3-methyl-4-[2-(2-naphthalenyloxy)ethoxy]-alpha-oxobenzeneacetic acid methyl ester (0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.5 g of purified 3-methyl-4-[2-(2-naphthalenyloxy)ethoxy]-alpha-oxobenzeneacetic acid as a colorless solid, mp 167-169 °C.

| | | |
|---|---|---|
| Analysis Calculated for C₂₁H₁₈O₅: | C, 71.99; | H, 5.18 |
| Found: | C, 72.04; | H, 5.21 |

### Example 182

### Preparation of 2-methyl-4-[2-(2-naphthalenyloxy)ethoxy]-alpha-oxobenzeneacetic acid methyl ester.

A stirred mixture of 4-hydroxy-2-methylacetophonone (2 g) in dimethylformamide (30 ml) under argon was treated with 55% sodium hydride (0.58 g), stirred for 30 minutes and treated with 2-(2-naphthalenyloxy)ethyl methanesulfonate (3.65 g). The mixture was heated at 60 °C for 2 hours, cooled, diluted with water and filtered. The damp solids were mixed with water and excess sodium hydroxide solution, the product was extracted twice with dichloromethane, and the organic layers were washed with water. The combined organic layers were dried (Na₂SO₄), filtered, and evaporated to give crude material which was crystallized from dichloromethane-diethyl ether to provide 3.5 g of 1-[2-methyl-4-[2-(2-naphthalenyloxy)ethoxy]phenyl]ethanone as a colorless solid, mp 128-130 °C.

| | | |
|---|---|---|
| Analysis Calculated for C₂₁H₂₀O₃: | C, 78.73; | H, 6.29 |
| Found: | C, 78.81; | H, 6.25 |

A mixture of 1-[2-methyl-4-[2-(2-naphthalenyloxy)ethoxy]phenyl]ethanone (1 g) in pyridine (20 ml) was treated with selenium dioxide (0.522 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from dichloromethane-hexane to provide 0.8 g of 2-methyl-4-[2-(2-naphthalenyloxy)ethoxy]-alpha-oxobenzeneacetic acid methyl ester as a colorless solid, mp 118-120 °C.

| | | |
|---|---|---|
| Analysis Calculated for C₂₂H₂₀O₅: | C, 72.51; | H, 5.53 |
| Found: | C, 72.72; | H, 5.57 |

### Example 183

### Preparation of 2-methyl-4-[2-(2-naphthalenyloxy)ethoxy]-alpha-oxobenzeneacetic acid (4:1) hydrate.

A solution of 2-methyl-4-[2-(2-naphthalenyloxy)ethoxy]-alpha-oxobenzeneacetic acid methyl ester (0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.52 g of purified 2-methyl-4-[2-(2-naphthalenyloxy)ethoxy]-alpha oxobenzeneacetic acid (4:1) hydrate as a colorless solid, mp 129-131°C.

| | | | |
|---|---|---|---|
| Analysis Calculated for C₂₁H₁₈O₅.4:1 H₂O: | C, 71.08; | H, 5.25; | H₂O, 1.27 |
| Found: | C, 71,17; | H, 5.21; | H₂O, 1.15 |

### Example 184

### Preparation of 3-chloro-4-[2-(2-naphthalenyloxy)ethoxy]-alpha-oxobenzeneacetic acid methyl ester.

A stirred mixture of 3-chloro-4-hydroxyacetophonone (2 g) in dimethylformamide (30 ml) under argon was treated with 55% sodium hydride (0.524 g), stirred for 30 minutes and treated with 2-(2-naphthalenyloxy)ethyl methanesulfonate (3 g). The mixture was heated at 60 °C overnight, cooled, diluted with water and filtered. The damp solids were mixed with water and excess sodium hydroxide solution, the product was extracted twice with dichloromethane, and the organic layers were washed with water. The combined organic layers were dried (Na₂SO₄), filtered, and evaporated to give crude material which was crystallized from dichloromethane-diethyl ether to provide 2.9 g of 1-[3-chloro-4-[2-(2-naphthalenyloxy)ethoxy]phenyl]ethanone as a colorless solid, mp 164-166 °C.

| | | | |
|---|---|---|---|
| Analysis Calculated for C₂₀H₁₇ClO₃: | C, 70.49; | H, 5.03; | Cl, 10.40 |
| Found: | C, 70.43; | H, 5.04; | Cl, 10.64 |

A mixture of 1-[3-chloro-4-[2-(2-naphthalenyloxy)ethoxy]phenyl]ethanone (1 g) in pyridine (20 ml) was treated with selenium dioxide (0.488 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from dichloromethane-hexane to provide 0.75 g of 3-chloro-4-[2-(2-naphthalenyloxy)ethoxy]-alpha-oxobenzeneacetic acid methyl ester as a colorless solid, mp 133-134 °C.

| | | | |
|---|---|---|---|
| Analysis Calculated for C₂₁H₁₇ClO₅: | C, 65.55; | H, 4.45; | Cl, 9.21 |
| Found: | C, 65.42; | H, 4.39; | Cl, 9.05 |

### Example 185

### Preparation of 3-chloro-4-[2-(2-naphthalenyloxy)ethoxy]-alpha-oxobenzeneacetic acid.

A solution of 3-chloro-4-[2-(2-naphthalenyloxy)ethoxy]-alpha-oxobenzeneacetic acid methyl ester (0.7 g) in warm methanol (10 ml) and tetrahydrofuran (10 ml) was treated with 1N sodium hydroxide (2.5 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.63 g of purified 3-chloro-4-[2-(2-naphthalenyloxy)ethoxy]-alpha-oxobenzeneacetic acid as a colorless solid, mp 167-168 °C.

| | | | |
|---|---|---|---|
| Analysis Calculated for C₂₀H₁₅ClO₅: | C, 64.79; | H, 4.08; | Cl, 9.56 |
| Found: | C, 64.78; | H, 3.98; | Cl, 9.79 |

### Example 186

### Preparation of 2-chloro-4-[2-(2-naphthalenyloxy)ethoxy]-alpha-oxobenzeneacetic acid methyl ester.

A stirred mixture of 2-chloro-4-hydroxyacetophonone (2 g) in dimethylformamide (30 ml) under argon was treated with 55% sodium hydride (0.524 g), stirred for 30 minutes and treated with 2-(2-naphthalenyloxy)ethyl methanesulfonate (3 g). The mixture was heated at 60 °C overnight, cooled, diluted with water and filtered. The damp solids were mixed with water and excess sodium hydroxide solution, the product was extracted twice with dichloromethane, and the organic layers were washed with water. The combined organic layers were dried (Na₂SO₄), filtered, and evaporated to give crude material which was crystallized from dichloromethane-diethyl ether to provide 3.2 g of 1-[2-chloro-4-[2-(2-naphthalenyloxy)ethoxy]phenyl]ethanone as a colorless solid, mp 119-120 °C.

| | | | |
|---|---|---|---|
| Analysis Calculated for C₂₀H₁₇ClO₃: | C, 70.49; | H, 5.03; | Cl, 10.40 |
| Found: | C, 70.28; | H, 5.02; | Cl, 10.45 |

A mixture of 1-[2-chloro-4-[2-(2-naphthalenyloxy)ethoxy]phenyl]ethanone (1 g) in pyridine (20 ml) was treated with selenium dioxide (0.482 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from dichloromethane-hexane to provide 0.7 g of 2-chloro-4-[2-(2-naphthalenyloxy)ethoxy]-alpha-oxobenzeneacetic acid methyl ester as a colorless solid, mp 123-125 °C.

| | | | |
|---|---|---|---|
| Analysis Calculated for C₂₁H₁₇ClO₅: | C, 65.55; | H, 4.45; | Cl, 9.21 |
| Found: | C, 65.48; | H, 4.45; | Cl, 9.51 |

### Example 187

### Preparation of 2-chloro-4-[2-(2-naphthalenyloxy)ethoxy]-alpha-oxobenzeneacetic acid.

A solution of 2-chloro-4-[2-(2-naphthalenyloxy)ethoxy]-alpha-oxobenzeneacetic acid methyl ester (0.56 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.435 g of purified 2-chloro-4-[2-(2-naphthalenyloxy)ethoxy]-alpha-oxobenzeneacetic acid as a colorless solid, mp 152-153 °C.

| | | | |
|---|---|---|---|
| Analysis Calculated for C₂₀H₁₅ClO₅: | C, 64.79; | H, 4.08; | Cl, 9.56 |
| Found: | C, 64.53; | H, 4.04; | Cl, 9.86 |

### Example 188

### Preparation of 3,5-dichloro-4-[2-(2-naphthalenyloxy)ethoxy]-alpha-oxobenzeneacetic acid methyl ester.

A stirred mixture of 3,5-dichloro-4-hydroxyacetophonone (1.7 g) in dimethylformamide (30 ml) under argon was treated with 55% sodium hydride (0.362 g), stirred for 30 minutes and treated with 2-(2-naphthalenyloxy)ethyl methanesulfonate (2 g). The mixture was heated at 75 °C for 48 hours and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated to give crude material which was purified by HPLC (dichloromethane-hexane; 3:1) and crystallized from dichloromethane-diethyl ether to provide 2.4 g of 1-[3,5-dichloro-4-[2-(2-naphthalenyloxy)ethoxy]phenyl]ethanone as a colorless solid, mp 110-111 °C.

| | | | |
|---|---|---|---|
| Analysis Calculated for C₂₀H₁₆Cl₂O₃: | C, 64.02; | H, 4.30; | Cl, 18.90 |
| Found: | C, 63.77; | H, 4.32; | Cl, 18.63 |

A mixture of 1-[3,5-dichloro-4-[2-(2-naphthalenyloxy)ethoxy]phenyl]ethanone (1.5 g) in pyridine (25 ml) was treated with selenium dioxide (0.665 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 3:1) and crystallized from dichloromethane-hexane to provide 0.9 g of 3,5-dichloro-4-[2-(2-naphthalenyloxy)ethoxy]-alpha-oxobenzeneacetic acid methyl ester as a colorless solid, mp 120-121 °C.

| | | | |
|---|---|---|---|
| Analysis Calculated for C₂₁H₁₆Cl₂O₅: | C, 60.16; | H, 3.85; | Cl, 16.91 |
| Found: | C, 60.45; | H, 3.81; | Cl, 17.18 |

### Example 189

### Preparation of 3,5-dichloro-4-[2-(2-naphthalenyloxy)ethoxy]-alpha-oxobenzeneacetic acid (6:1) molar benzene solvate.

A solution of 3,5-dichloro-4-[2-(2-naphthalenyloxy)ethoxy]-alpha-oxobenzeneacetic acid methyl ester (0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from benzene-hexane provided 0.575 g of purified 3,5-dichloro-4-[2-(2-naphthalenyloxy)ethoxy]-alpha-oxobenzeneacetic acid (6:1) molar benzene solvate as a colorless solid, mp 108-110 °C.

| | | | |
|---|---|---|---|
| Analysis Calculated for C₂₀H₁₄Cl₂O₅.6:1 C₆H₆: | C, 60.31; | H, 3.61; | Cl, 16.95 |
| Found: | C, 60.62; | H, 3.49; | Cl, 17.25 |

### Example 190

### Preparation of 2,6-dichloro-4-[2-(2-naphthalenyloxy)ethoxy]-alpha-oxobenzeneacetic acid methyl ester.

A stirred mixture of 4-bromo-3,5-dichlorophenol (1.9 g) in dimethylformamide (20 ml) under argon was treated with 55% sodium hydride (0.345 g), stirred for 30 minutes and treated with 2-(2-naphthalenyloxy)ethyl methanesulfonate (2.1 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, evaporated and crystallized from dichloromethane-diethyl ether to provide 3.3 g of 2-[2-(4-bromo-3,5-dichlorophenoxy)ethoxy]naphthalene as a colorless solid, mp 140-142 °C.

| | | | | |
|---|---|---|---|---|
| Analysis Calculated for C₁₈H₁₃BrCl₂O₂: | C, 52.46; | H, 3.18; | Br, 19.39; | Cl, 17.21 |
| Found: | C, 52.40; | H, 3.08; | Br, 19.09; | Cl, 16.95 |

A mixture of 2-[2-(4-bromo-3,5-dichlorophenoxy)ethoxy]naphthalene (3.28 g) in dimethylformamide (10 ml) and cuprous cyanide (0.833 g) was heated under argon at 155 °C for four hours. The cooled mixture was diluted with aqueous 10% sodium cyanide solution (100 ml) and extracted twice with dichloromethane. The organic solutions were washed in turn with aqueous 10% sodium cyanide solution and water, dried (Na₂SO₄), filtered and evaporated to give 2.2 g of crude 2,6-dichloro-4-[2-(2-naphthalenyloxy)ethoxy]benzonitrile. A portion crystallized from dichloromethane-diethyl ether provide pure colorless material, mp 156-158 °C.

| | | | | |
|---|---|---|---|---|
| Analysis Calculated for C₁₉H₁₃Cl₂NO₂: | C, 63.71; | H, 3.66; | N, 3.91; | Cl, 19.79 |
| Found: | C, 63.46; | H, 3.71; | N, 3.88; | Cl, 19.61 |

Methyl magnesium bromide was prepared in the usual way from magnesium metal (0.634g) and methyl iodide (1.6 ml) in diethyl ether. Most of the diethyl ether was removed by distillation prior to the addition of a solution of 2,6-dichloro-4-[2-(2-naphthalenyloxy)ethoxy]benzonitrile (3.1 g) in benzene (25 ml). Distillation was continued to remove all of the diethyl ether and the resulting mixture was refluxed at 80 °C for 5 hours and allowed to stand overnight at room temperature. An aqueous solution of 2N hydrochloric acid (20 ml) was added and the benzene was removed by distillation and the aqueous mixture was boiled at 100 °C for 30 minutes, cooled and diluted with water. The crude product was extracted with dichloromethane (twice), washed in turn with dilute hydrochloric acid, dilute aqueous sodium bicarbonate solution, dried (Na₂SO₄), filtered and evaporated. The crude material was purified by HPLC (dichloromethane-diethyl ether; 20:1) and crystallized from dichloromethane-diethyl ether to give 1.85 g of 1-[2,6-dichloro-4-[2-(2-naphthalenyloxy) ethoxy] phenyl]ethanone as a colorless solid, mp 127-129 °C.

| | | | |
|---|---|---|---|
| Analysis Calculated for C₂₀H₁₆Cl₂O₃: | C, 64.02; | H, 4.30; | Cl, 18.90 |
| Found: | C, 63.72; | H, 4.38; | Cl, 18.82 |

A mixture of 1-[2,6-dichloro-4-[2-(2-naphthalenyloxy)ethoxy]phenyl]ethanone (1.5 g) in pyridine (25 ml) was treated with selenium dioxide (0.665 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 3:1) and crystallized from dichloromethane-hexane to provide 1 g of 2,6-dichloro-4-[2-(2-naphthalenyloxy)ethoxy]-alpha-oxobenzeneacetic acid methyl ester as a colorless solid, mp 142-144 °C.

| | | | |
|---|---|---|---|
| Analysis Calculated for C₂₁H₁₆Cl₂O₅: | C, 60.16; | H, 3.85; | Cl, 16.91 |
| Found: | C, 59.99; | H, 3.81; | Cl, 16.73 |

### Example 191

### Preparation of 2,6-dichloro-4-[2-(2-naphthalenyloxy)ethoxy]-alpha-oxobenzeneacetic acid.

A solution of 2,6-dichloro-4-[2-(2-naphthalenyloxy)ethoxy]-alpha-oxobenzeneacetic acid methyl ester(0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.53 g of purified 2,6-dichloro-4-[2-(2-naphthalenyloxy)ethoxy]-alpha-oxobenzeneacetic acid as a colorless solid, mp 155-156 °C.

| | | | |
|---|---|---|---|
| Analysis Calculated for C₂₀H₁₄Cl₂O₅: | C, 59.28; | H, 3.48; | Cl, 17.50 |
| Found: | C, 59.94; | H, 3.41; | Cl, 17.22 |

### Example 192

### Preparation of 3,5-dimethoxy-4-[2-(2-naphthalenyloxy)ethoxy]-alpha-oxobenzeneacetic acid methyl ester.

A stirred mixture of 3,5-dimethoxy-4-hydroxyacetophonone (2 g) in dimethylformamide (30 ml) under argon was treated with 55% sodium hydride (0.445 g), stirred for 30 minutes and treated with 2-(2-naphthalenyloxy)ethyl methanesulfonate (2.7 g). The mixture was heated at 80 °C for 48 hours and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated to give crude material which was purified by HPLC (dichloromethane-diethyl ether; 49:1) and crystallized from dichloromethane-diethyl ether to provide 2.1 g of 1-[3,5-dimethoxy-4-[2-(2-naphthalenyloxy)ethoxy]phenyl]ethanone as a colorless solid, mp 76-77 °C.

| | | |
|---|---|---|
| Analysis Calculated for C₂₂H₂₂O₅: | C, 72.12; | H, 6.05 |
| Found: | C, 72.09; | H, 6.10 |

A mixture of 1-[3,5-dimethoxy-4-[2-(2-naphthalenyloxy)ethoxy]phenyl]ethanone (1.5 g) in pyridine (25 ml) was treated with selenium dioxide (0.682 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane-diethyl ether; 75:25:2) and crystallized from diethyl ether to provide 1 g of 3,5-dimethoxy-4-[2-(2-naphthalenyloxy)ethoxy]-alpha-oxobenzeneacetic acid methyl ester as a pale yellow solid, mp 69-71 °C.

| | | |
|---|---|---|
| Analysis Calculated for C₂₃H₂₂O₇: | C, 67.31; | H, 5.40 |
| Found: | C, 67.17; | H, 5.45 |

### Example 193

### Preparation of 3,5-dimethoxy-4-[2-(2-naphthalenyloxy)ethoxy]-alpha-oxobenzeneacetic acid.

A solution of 3,5-dimethoxy-4-[2-(2-naphthalenyloxy)ethoxy]-alpha-oxobenzeneacetic acid methyl ester (0.5 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from diethyl ether-hexane provided 0.45 g of purified 3,5-dimethoxy-4-[2-(2-naphthalenyloxy)ethoxy]-alpha-oxobenzeneacetic acid as a yellow solid, mp 114-116 °C.

| | | |
|---|---|---|
| Analysis Calculated for C₂₂H₂₀O₇: | C, 66.66; | H, 5.09 |
| Found: | C, 66.42; | H, 5.09 |

### Example 194

### Preparation of 2,6-dimethoxy-4-[2-(2-naphthalenyloxy)ethoxy]-alpha-oxobenzeneacetic acid methyl ester.

A stirred mixture of impure 2,6-dimethoxy-4-hydroxyacetophonone (3.6 g) in dimethylformamide (30 ml) under argon was treated with 55% sodium hydride (0.803 g), stirred for 30 minutes and treated with 2-(2-naphthalenyloxy)ethyl methanesulfonate (4.9 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated to give crude material which was purified by HPLC (dichloromethane-hexane-diethyl ether; 50:50:3) and crystallized from dichloromethane-diethyl ether to provide 1.8 g of 1-[2,6-dimethoxy-4-[2-(2-naphthalenyloxy)ethoxy]phenyl]ethanone as a colorless solid, mp 97-98 °C.

| | | |
|---|---|---|
| Analysis Calculated for C₂₂H₂₂O₅: | C, 72.12; | H, 6.05 |
| Found: | C, 72.08; | H, 6.04 |

A mixture of 1-[2,6-dimethoxy-4-[2-(2-naphthalenyloxy)ethoxy]phenyl]ethanone (1.5 g) in pyridine (25 ml) was treated with selenium dioxide (0.682 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-diethyl ether; 49:1) and crystallized from dichloromethane-diethyl ether to provide 1.1 g of 2,6-dimethoxy-4-[2-(2-naphthalenyloxy)ethoxy]-alpha-oxobenzeneacetic acid methyl ester as a pale yellow solid, mp 161-162 °C.

| | | |
|---|---|---|
| Analysis Calculated for C₂₃H₂₂O₇: | C, 67.31; | H, 5.40 |
| Found: | C, 67.00; | H, 5.53 |

### Example 195

### Preparation of 2,6-dimethoxy-4-[2-(2-naphthalenyloxy)ethoxy]-alpha-oxobenzeneacetic acid.

A solution of 2,6-dimethoxy-4-[2-(2-naphthalenyloxy)ethoxy]-alpha-oxobenzeneacetic acid methyl ester (0.5 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.45 g of purified 2,6-dimethoxy-4-[2-(2-naphthalenyloxy)ethoxy]-alpha-oxobenzeneacetic acid as a yellow solid, mp 154 °C with decomposition.

| | | |
|---|---|---|
| Analysis Calculated for C₂₂H₂₀O₇: | C, 66.66; | H, 5.09 |
| Found: | C, 66.39; | H, 5.17 |

### Example 196

### Preparation of 2-fluoro-4-[2-(2-naphthalenyloxy)ethoxy]-alpha-oxobenzeneacetic acid methyl ester.

A stirred mixture of impure 2-fluoro-4-hydroxyacetophonone (1.85 g) in dimethylformamide (30 ml) under argon was treated with 55% sodium hydride (0.524 g), stirred for 30 minutes and treated with 2-(2-naphthalenyloxy)ethyl methanesulfonate (2.9 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated to give crude material which was crystallized from dichloromethane-diethyl ether to provide 3.0 g of 1-[2-fluoro-4-[2-(2-naphthalenyloxy)ethoxy]phenyl]ethanone as a colorless solid, mp 144-146 °C.

| | | | |
|---|---|---|---|
| Analysis Calculated for C₂₀H₁₇FO₃: | C, 74.06; | H, 5.28; | F, 5.86 |
| Found: | C, 74.11; | H, 5.34; | F, 6.03 |

A mixture of 1-[2-fluoro-4-[2-(2-naphthalenyloxy)ethoxy]phenyl]ethanone (1 g) in pyridine (20 ml) was treated with selenium dioxide (0.5 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from dichloromethane-hexane to provide 0.55 g of 2-fluoro-4-[2-(2-naphthalenyloxy)ethoxy]-alpha-oxobenzeneacetic acid methyl ester as a colorless solid, mp 135-136 °C.

| | | | |
|---|---|---|---|
| Analysis Calculated for C₂₁H₁₇FO₅: | C, 68.47; | H, 4.65; | F, 5.16 |
| Found: | C, 68.39; | H, 4.63; | F, 5.06 |

### Example 197

### Preparation of 2-fluoro-4-[2-(2-naphthalenyloxy)ethoxy]-alpha-oxobenzeneacetic acid.

A solution of 2-fluoro-4-[2-(2-naphthalenyloxy)ethoxy]-alpha-oxobenzeneacetic acid methyl ester (0.5 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.425 g of purified 2-fluoro-4-[2-(2-naphthalenyloxy)ethoxy]-alpha-oxobenzeneacetic acid as a yellow solid, mp 155-156 °C.

| | | | |
|---|---|---|---|
| Analysis Calculated for C₂₀H₁₅FO₅: | C, 67.79; | H, 4.27; | F, 5.36 |
| Found: | C, 67.52; | H, 4.41; | F, 5.20 |

### Example 198

### Preparation of 3-fluoro-4-[2-(2-naphthalenyloxy)ethoxy]-alpha-oxobenzeneacetic acid methyl ester.

A stirred mixture of impure 2-fluoro-4-hydroxyacetophonone (1.85 g) in dimethylformamide (30 ml) under argon was treated with 55% sodium hydride (0.524 g), stirred for 30 minutes and treated with 2-(2-naphthalenyloxy)ethyl methanesulfonate (2.9 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated to give crude material which was crystallized from dichloromethane-diethyl ether to provide 2.8 g of 1-[3-fluoro-4-[2-(2-naphthalenyloxy)ethoxy]phenyl]ethanone as a colorless solid, mp 171-173 °C.

| | | | |
|---|---|---|---|
| Analysis Calculated for C₂₀H₁₇FO₃: | C, 74.06; | H, 5.28; | F, 5.86 |
| Found: | C, 74.01; | H, 5.28; | F, 5.58 |

A mixture of 1-[3-fluoro-4-[2-(2-naphthalenyloxy)ethoxy]phenyl]ethanone (1 g) in pyridine (20 ml) was treated with selenium dioxide (0.5 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution. After the organic extracts were dried (Na₂SO₄), filtered and evaporated, the crude material was purified by HPLC (dichloromethane-hexane; 9:1) and crystallized from dichloromethane-hexane to provide 0.75 g of 3-fluoro-4-[2-(2-naphthalenyloxy)ethoxy]-alpha-oxobenzeneacetic acid methyl ester as a colorless solid, mp 136-138 °C.

| | | | |
|---|---|---|---|
| Analysis Calculated for C₂₁H₁₇FO₅: | C, 68.47; | H, 4.65; | F, 5.16 |
| Found: | C, 68.44; | H, 4.60; | F, 4.94 |

### Example 199

### Preparation of 3-fluoro-4-[2-(2-naphthalenyloxy)ethoxy]-alpha-oxobenzeneacetic acid.

A solution of 3-fluoro-4-[2-(2-naphthalenyloxy)ethoxy]-alpha-oxobenzeneacetic acid methyl ester (0.5 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from acetone-hexane provided 0.42 g of purified 3-fluoro-4-[2-(2-naphthalenyloxy)ethoxy]-alpha-oxobenzeneacetic acid as a yellow solid, mp 168-169 °C.

| | | | |
|---|---|---|---|
| Analysis Calculated for C₂₀H₁₅FO₅: | C, 67.79; | H, 4.27; | F, 5.36 |
| Found: | C, 67.86; | H, 4.29; | F, 5.44 |

### Example 200

### Preparation of 2,6-difluoro-4-[2-(2-naphthalenyloxy)ethoxy]-alpha-oxobenzeneacetic acid methyl ester.

A stirred mixture of 2,6-difluoro-4-hydroxybenzonitrile (3 g) in dimethylformamide (30 ml) under argon was treated with 55% sodium hydride (0.842 g), stirred for 30 minutes and treated with 2-(2-naphthalenyloxy)ethyl methanesulfonate (5.1 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated. The crude material was purified by HPLC (dichloromethane-hexane 2:1) and crystallized from dichloromethane-hexane to provide 4.3 g of 2,6-difluoro-4-[2-(2-naphthalenyloxy) ethoxy]benzonitrile as a colorless solid, mp 148-149 °C.

| | | | | |
|---|---|---|---|---|
| Analysis Calculated for C₁₉H₁₃F₂NO₂: | C, 70.15; | H, 4.03; | N, 4.31; | F, 11.68 |
| Found: | C, 69.99; | H, 3.92; | N, 4.19; | F, 11.56 |

Methyl magnesium bromide was prepared in the usual way from magnesium metal (0.94 g) and methyl iodide (2.4 ml) in diethyl ether. Most of the diethyl ether was removed by distillation prior to the addition of a solution of 2,6-difluoro-4-[2-(2-naphthalenyloxy)ethoxy]benzonitrile (4.2 g) in tetrahydrofuran (35 ml). Distillation was continued to remove all of the diethyl ether and the resulting mixture was refluxed at 60 °C for 2 hours and chilled in ice. An aqueous solution of 2N hydrochloric acid (30 ml) was added and the tetrahydrofuran was removed by distillation and the aqueous mixture was boiled at 100 °C for 45 minutes, cooled and diluted with water. The crude product was extracted with dichloromethane (twice), washed in turn with dilute hydrochloric acid, dilute aqueous sodium bicarbonate solution, dried (Na₂SO₄), filtered and evaporated. The residue was purified by HPLC (dichloromethane-hexane; 3:1) and crystallized from dichloromethane-diethyl ether to give 2.5 g of 1-[2,6-difluoro-4-[2-(2-naphthalenyloxy)ethoxy]phenyl]ethanone as a colorless solid, mp 119-120 °C.

| | | | |
|---|---|---|---|
| Analysis Calculated for C₂₀H₁₆F₂O₃: | C, 70.17; | H, 4.71; | F, 11.10 |
| Found: | C, 70.06; | H, 4.71; | F, 11.43 |

A mixture of 1-[2,6-difluoro-4-[2-(2-naphthalenyloxy)ethoxy]phenyl]ethanone (2.5 g) in pyridine (30 ml) was treated with selenium dioxide (1.2 g) and heated at 100 °C overnight under argon. The mixture was cooled, diluted with dichloromethane (75 ml), and filtered through celite. The filtrate was chilled in an ice bath and treated with methyl chloroformate (2.5 ml) and stirred for 10 minutes. The resulting mixture was washed with successive portions of 1N hydrochloric acid (twice) and saturated aqueous sodium bicarbonate solution. After the organic extracts were dried (Na₂SO₄), filtered and evaporated, the crude residue was purified by HPLC (dichloromethane-hexane; 9:1) and crystallized from dichloromethane-methanol to provide 1.4 g of 2,6-difluoro-4-[2-(2-naphthalenyloxy)ethoxy]-alpha-oxobenzeneacetic acid methyl ester as a colorless solid, mp 130-131 °C.

| | | | |
|---|---|---|---|
| Analysis Calculated for C₂₁H₁₆F₂O₅: | C, 65.29; | H, 4.17; | F, 9.83 |
| Found: | C, 65.39; | H, 3.92; | F, 9.53 |

### Example 201

### Preparation of 2,6-difluoro-4-[2-(2-naphthalenyloxy)ethoxy]-alpha-oxobenzeneacetic acid.

A solution of 2,6-difluoro-4-[2-(2-naphthalenyloxy)ethoxy]-alpha-oxobenzeneacetic acid methyl ester (0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.51 g of 2,6-difluoro-4-[2-(2-naphthalenyloxy)ethoxy]-alpha-oxobenzeneacetic acid as a yellow solid, mp 157-158 °C.

| | | | |
|---|---|---|---|
| Analysis Calculated for C₂₀H₁₄F₂O₅: | C, 64.52; | H, 3.79; | F, 10.21 |
| Found: | C, 64.42; | H, 3.70; | F, 10.44 |

### Example 202

### Preparation of 3,5-difluoro-4-[2-(2-naphthalenyloxy)ethoxy]-alpha-oxobenzeneacetic acid methyl ester.

A stirred mixture of 3,5-difluoro-4-hydroxybenzonitrile (2.07 g) in dimethylformamide (30 ml) under argon was treated with 55% sodium hydride (0.524 g), stirred for 30 minutes and treated with 2-(2-naphthalenyloxy)ethyl methanesulfonate (2.9 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated. The crude material was first purified by HPLC (dichloromethane-hexane 3:1) and then crystallized from dichloromethane-hexane to provide 2.8 g of 1-[3,5-difluoro-4-[2-(2-naphthalenyloxy)ethoxy]phenyl]ethanone as a colorless solid, mp 108-110 °C.

| | | | |
|---|---|---|---|
| Analysis Calculated for C₂₀H₁₆F₂O₃: | C, 70.17; | H, 4.71; | F, 11.10 |
| Found: | C, 70.20; | H, 4.53; | F, 11.28 |

A mixture of 1-[3,5-difluoro-4-[2-(2-naphthalenyloxy)ethoxy]phenyl]ethanone (1.5 g) in pyridine (25 ml) was treated with selenium dioxide (0.732 g) and heated at 100 °C overnight under argon. The mixture was cooled, diluted with dichloromethane (50 ml), and filtered through celite. The filtrate was chilled in an ice bath and treated with methyl chloroformate (2 ml) and stirred for 10 minutes. The resulting mixture was washed with successive portions of 1N hydrochloric acid (twice) and saturated aqueous sodium bicarbonate solution, then the organic layer was dried (Na₂SO₄), filtered and evaporated. The isolated material was purified by HPLC (dichloromethane-hexane; 3:1) and crystallized from dichloromethane-hexane to provide 1.1 g of 3,5-difluoro-4-[2-(2-naphthalenyloxy)ethoxy]-alpha-oxobenzeneacetic acid methyl ester as a colorless solid, mp 114-115 °C.

| | | | |
|---|---|---|---|
| Analysis Calculated for C₂₁H₁₆F₂O₅: | C, 65.29; | H, 4.17; | F, 9.83 |
| Found: | C, 65.51; | H, 3.90; | F, 9.71 |

### Example 203

### Preparation of 3,5-difluoro-4-[2-(2-naphthalenyloxy)ethoxy]-alpha-oxobenzeneacetic acid.

A solution of 3,5-difluoro-4-[2-(2-naphthalenyloxy)ethoxy]-alpha-oxobenzeneacetic acid methyl ester (0.6 g) in warm methanol (10 ml) and tetrahydrofuran (10 ml) was treated with 1N sodium hydroxide (2 ml) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated. Crystallization of the residue from dichloromethane-hexane provided 0.525 g of purified 3,5-difluoro-4-[2-(2-naphthalenyloxy)ethoxy]-alpha-oxobenzeneacetic acid as a yellow solid, mp 129-130 °C.

| | | | |
|---|---|---|---|
| Analysis Calculated for C₂₀H₁₄F₂O₅: | C, 64.52; | H, 3.79; | F, 10.21 |
| Found: | C, 64.19; | H, 3.82; | F, 10.23 |

### Example 204

### Preparation of 2,3,5,6-tetrafluoro-4-[2-(2-naphthalenyloxy)ethoxy]-alpha-oxobenzeneacetic acid methyl ester.

A stirred chilled mixture of aluminum chloride (40 g) and carbon disulfide (40 ml) was maintained below 5 °C during the sequential dropwise addition of a solution composed of 2,3,5,6-tetrafluorophenol (24.9 g) in carbon disulfide (15 ml) followed by acetyl chloride (21.3 ml). The resulting mixture was refluxed for 4 days and poured onto a mixture of ice and hydrochloric acid. The organic material was extracted with diethyl ether (3 times), washed in turn with water, combined and evaporated. The wet residue was mixed with a little methanol and 4N aqueous sodium hydroxide (100 ml), heated at 100 °C for 30 minutes, diluted with water, cooled and acidified with hydrochloric acid. The mixture was extracted with diethyl ether (3 times), washed with water, dried (Na₂SO₄), filtered and evaporated. Crystallization of the isolated material from diethyl ether-hexane provided 3.4 g of 1-[(4-hydroxy-2,3,5,6-tetrafluoro)phenyl]ethanone, mp 109-111 °C.
A stirred mixture of 1-[(4-hydroxy-2,3,5,6-tetrafluoro)phenyl]ethanone (2.1 g) in dimethylformamide (20 ml) under argon was treated with 55% sodium hydride (0.436 g), stirred for 30 minutes and treated with 2-(2-naphthalenyloxy)ethyl methanesulfonate (2.7 g). The mixture was heated at 60 °C overnight and evaporated to dryness. The residue was mixed with water and excess sodium hydroxide solution, the product was extracted twice with dichloromethane, and the organic layers were washed with water. The combined organic layers were dried (Na₂SO₄), filtered, and evaporated. The crude material was purified by HPLC (dichloromethane-hexane 2:1) and crystallized from dichloromethane-diethyl ether to provide 2.25 g of 1-[2,3,5,6-tetrafluoro-4-[2-(2-naphthalenyloxy)ethoxy]phenyl]ethanone as a colorless solid, mp 114-115 °C.

| | | | |
|---|---|---|---|
| Analysis Calculated for C₂₀H₁₄F₄O₃: | C, 63.50; | H, 3.73; | F, 20.09 |
| Found: | C, 63.70; | H, 3.69; | F, 20.27 |

A mixture of 1-[2,3,5,6-tetrafluoro-4-[2-(2-naphthalenyloxy)ethoxy]phenyl]ethanone (2.1 g) in pyridine (25 mL) was treated with selenium dioxide (0.932 g) and heated at 100 °C overnight under argon. The mixture was cooled, diluted with dichloromethane (50 mL), and filtered through celite. The filtrate was chilled in an ice bath and treated with methyl chloroformate (2 mL) and stirred for 10 minutes. The resulting mixture was washed with successive portions of 1N hydrochloric acid (twice) and saturated aqueous sodium bicarbonate solution. The organic extracts were dried (Na₂SO₄), filtered and evaporated. The crude material was purified by HPLC (dichloromethane-hexane; 9:1) and crystallized from diethyl ether-hexane to provide 0.95 g of 2,3,5,6-tetrafluoro-4-[2-(2-naphthalenyloxy)ethoxy]-alpha-oxobenzeneacetic acid methyl ester as a colorless solid, mp 69-71 °C.

| | | | |
|---|---|---|---|
| Analysis Calculated for C₂₁H₁₄F₄O₅: | C, 59.72; | H, 3.34; | F, 17.99 |
| Found: | C, 59.75; | H, 3.25; | F, 18.27 |

### Example 205

### Preparation of 2,3,5,6-tetrafluoro-4-[2-(2-naphthalenyloxy)ethoxy]-alpha-oxobenzeneacetic acid.

A solution of 2,3,5,6-tetrafluoro-4-[2-(2-naphthalenyloxy)ethoxy]-alpha-oxobenzeneacetic acid methyl ester (0.5 g) in warm methanol (10 mL) and tetrahydrofuran (10 mL) was treated with 1N sodium hydroxide (2 mL) and after 10 minutes the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.185 g of purified 2,3,5,6-tetrafluoro-4-[2-(2-naphthalenyloxy)ethoxy]-alpha-oxobenzeneacetic acid as a yellow solid, mp 146-148 °C.

| | | | |
|---|---|---|---|
| Analysis Calculated for C₂₀H₁₂F₄O₅: | C, 58.83; | H, 2.96; | F, 18.61 |
| Found: | C, 58.87; | H, 2.97; | F, 18.82 |

### Example 206

### Preparation of 3-methyl-2-(trifluoromethylsulfonyloxy)benzoic acid methyl ester.

In an inert atmosphere, a stirred solution of 3-methylsalicylic acid methyl ester (9.96g) in dichloromethane (150 ml) cooled in an ice bath, was treated portionwise with 55% sodium hydride dispersion in mineral oil (3.0 g). The mixture was stirred at 0 °C for 15 minutes, then triflic anhydride (18.65 ml) was added dropwise over 20 minutes. The cooling bath was removed and the reaction was allowed to proceed for an additional hour. Water (75 ml) was added and the phases were separated. The organic layer was washed in turn with 10% potassium carbonate (75 ml) and with brine. The dried (Na₂SO₄) organic layer was evaporated and the resulting amber oil was purified by HPLC (diethyl ether-hexane; 1:4) to yield 14.3 g of 3-methyl-2-(trifluoromethylsulfonyloxy)benzoic acid methyl ester as an oil.

| | | | | |
|---|---|---|---|---|
| Analysis Calculated for C₁₀H₉F₃O₅S: | C, 40.27; | H, 3.04; | F, 19.11; | S, 10.75 |
| Found: | C, 40.48; | H, 3.20; | F, 19.35; | S, 11.10 |

### Example 207

### Preparation of rac-3-methyl-2-[3-(2-tetrahydropyranyloxy)-1-propynyl]benzoic acid methyl ester.

Argon was bubbled for 30 minutes through a solution of 3-methyl-2-(trifluoromethylsulfonyloxy)benzoic acid methyl ester (18.7 g), rac-tetrahydro-2-(2-propynyloxy)-2H-pyran (13.2 g) and triethylamine (60 ml) in dry dimethylformamide (200 ml), then bis(triphenylphosphine)palladium (II) chloride (1.32g) was added and the mixture was stirred at 85-90 °C for 3 hours. The reaction was cooled and the solvents were removed in vacuo to give a dark oil which was purified by HPLC to give 3-methylsalicylic acid methyl ester (3.04g) as a non-polar impurity as well as 8.15 g of the desired rac-3-methyl-2-[3-(2-tetrahydropyranyloxy)-1-propynyl]benzoic acid methyl ester as a straw colored oil.

| | | |
|---|---|---|
| Analysis Calculated for C₁₇H₂₀O₄: | C, 70.81; | H, 6.99 |
| Found: | C, 70.81; | H, 7.01 |

### Example 208

### Preparation of rac-3-methyl-2-[3-[2-(tetrahydropyranyloxy)]-1-propynyl]benzenemethanol.

A solution of diisobutylaluminum hydride (1.5 M in hexane; 39.3 ml) was added over 15 minutes to a solution of rac-3-methyl-2-[3-(2-tetrahydropyranyloxy)-1-propynyl] benzoic acid methyl ester (7.52 g) in toluene (200 ml) maintained at -40 °C by using a dry ice-acetonitrile bath. After 20 minutes, water (25 ml) was added and the cooling bath was removed. When the mixture had warmed to 15 °C, it was diluted with a saturated sodium potassium tartrate solution (150 ml) and toluene (100 ml). After the phases were separated, the aqueous layer was washed with toluene (2 x 100 ml), then the organic extracts were washed in turn with sodium potassium tartrate solution (100 ml) and brine, dried (Na₂SO₄), filtered and evaporated to give 6.75 g of rac-3-methyl-2-[3-[2-(tetrahydropyranyloxy)]-1-propynyl]benzenemethanol as a colorless oil. A small portion (0.1 g) was purified for analysis by chromatography over silica gel (2 g; diethyl ether-hexane).

| | | |
|---|---|---|
| Analysis Calculated for C₁₆H₂₀O₃: | C, 73.82; | H, 7.74 |
| Found: | C, 74.19; | H, 8.12 |

### Example 209

### Preparation of 2,2-dimethylbutanoic acid [3-methyl-2-[3-(2-tetrahydropyranyloxy)-1-propynyl]phenyl]methyl ester.

2,2-Dimethylbutyryl chloride (4.2 g) was added dropwise to a solution of rac-3-methyl-2-[3-[2-(tetrahydropyranyloxy)-1-propynyl]benzenemethanol (6.65 g), triethylamine (9 ml) and N,N-dimethylaminopyridine (0.002 g) in dichloromethane (100 ml). After stirring for 22 hours at room temperature, the reaction mixture was diluted with dichloromethane (50 ml) and then was washed in turn with sodium bicarbonate solution and with brine. Evaporation of the dried (Na₂SO₄) organic layer furnished 9 g of an oil which was purified by HPLC (diethyl ether-hexane; 1:9) to provide 7.6 g of rac-2,2-dimethylbutanoic acid [3-methyl-2-[3-(2-tetrahydropyranyloxy)-1-propynyl]phenyl]methyl ester as a colorless oil.

| | | |
|---|---|---|
| Analysis Calculated for C₂₂H₃₀O₄: | C, 73.71; | H, 8.44 |
| Found: | C, 73.91; | H, 8.56 |

### Example 210

### Preparation of 2,2-dimethylbutanoic acid [2-(3-hydroxy-1-propynyl)-3-methylphenyl]methyl ester.

A solution of rac-2,2-dimethylbutanoic acid [3-methyl-2-[3-(2-tetrahydropyranyloxy)-1-propynyl]phenyl]methyl ester (2 g) and pyridinium p-toluenesulfonate in ethanol (30 ml) was stirred and heated in an oil bath maintained at 55°C for 3.5 hours then the solvent was evaporated and the residue triturated with diethyl ether. After a small amount of pyridinium p-toluenesulfonate was filtered off, the filtrate was evaporated and the residual oil was purified by HPLC (diethyl ether-hexane; 1:3) to furnish 1.38 g of 2,2-dimethylbutanoic acid [2-(3-hydroxy-1-propynyl)-3-methylphenyl]methyl ester as a colorless oil.

| | | |
|---|---|---|
| Analysis Calculated for C₁₇H₂₂O₃: | C, 74.42; | H, 8.08 |
| Found: | C, 74.16; | H, 8.23 |

### Example 211

### Preparation of 4-[3-[2-[(2,2-dimethyl-1-oxobutoxy)methyl]-6-methylphenyl]-2-propynyloxy]-alpha-oxobenzeneacetic acid methyl ester.

A stirred solution of 2,2-dimethylbutanoic acid [2-(3-hydroxy-1-propynyl)-3-methylphenyl]methyl ester (1.05 g), 4-hydroxyphenylglyoxylic acid methyl ester (0.693 g) and triphenylphosphine (1.26 g) in tetrahydrofuran (30 ml) was cooled to 0 °C, and a solution of diethyl azodicarboxylate (0.76 ml) in tetrahydrofuran (5 ml) was added over 5 minutes. The reaction mixture was stirred at 0 °C for 1 hour then at room temperature overnight. The solvent was evaporated and the residue triturated with diethyl ether to remove most of the triphenylphosphine oxide. The filtrate was concentrated in vacuo and the resulting oil was chromatographed over silica gel (10 g; diethyl ether-hexane; 1:19) to furnish 1.4 g of 4-[3-[2-[(2,2-dimethyl-1-oxobutoxy) methyl]-6-methylphenyl]-2-propynyloxy]-alpha-oxobenzeneacetic acid methyl ester as a colorless oil.

| | | |
|---|---|---|
| Analysis Calculated for C₂₆H₂₈O₆: | C, 71.54; | H, 6.47 |
| Found: | C, 71.53; | H, 6.43 |

### Example 212

### Preparation of 4-[3-[2-[(2,2-dimethyl-1-oxobutoxy)methyl]-6-methylphenyl]-2-propynyloxy]-alpha-oxobenzeneacetic acid (1:1) morpholine salt.

A solution of 4-[3-[2-[(2,2-dimethyl-1-oxobutoxy)methyl]-6-methylphenyl]-2-propynyloxy]-alpha-oxobenzeneacetic acid methyl ester (1.25 g) in methanol (5 ml) was treated with 3 N sodium hydroxide solution (1.25 ml) and the mixture was stirred for 5 minutes at room temperature. Most of the methanol was removed under reduced pressure, and the concentrate was partitioned between diethyl ether (25 ml) and water (25 ml). The separated aqueous layer was acidified with 1N hydrochloric acid (5 ml) and extracted with diethyl ether (2 x 50 ml). The combined organic phases were washed with brine, dried (Na₂SO₄) and evaporated in vacuo to give 1.13 g of 4-[3-[2-[(2,2-dimethyl-1-oxobutoxy)methyl]-6-methylphenyl]-2-propynyloxy]-alpha-oxobenzeneacetic acid as a colorless oil.
Morpholine (0.2 ml) was added to a solution of the crude acid (0.97 g) in diethyl ether (25 ml) and the mixture was stirred at room temperature until a crystalline solid began to form, then was chilled at 0 °C for 1 hour. The solids were filtered off and recrystallized from ethyl acetate to provide 0.89 g of 4-[3-[2-(2,2-dimethyl-1-oxobutoxy)methyl]-6-methylphenyl]-2-propynyloxy]-alpha-oxobenzeneacetic acid (1:1) morpholine salt, mp 111-112 °C.

| | | | |
|---|---|---|---|
| Analysis Calculated for C₂₅H₂₆O₆.(1:1)C₄H₉NO: | C, 68.35; | H, 6.92; | N 2.75 |
| Found: | C, 68.45; | H, 6.75; | N 2.79 |

### Example 213

### Preparation of (Z)-2,2-dimethylbutanoic acid [2-(3-hydroxy-1-propenyl)-3-methylphenyl] methyl ester.

A solution of rac-dimethylbutanoic acid [3-methyl-2-[3-(2-tetrahydropyranyloxy)-1-propynyl]phenyl]methyl ester (2 g) in methanol (50 ml) was hydrogenated over 10% palladium on carbon (0.2 g) at room temperature and ambient pressure. The reaction essentially stopped within 1 hour with the absorption of 145 ml of hydrogen. After the catalyst was removed by filtration through Celite, the methanol was removed in vacuo to give 1.6 g of an oil. Examination of the product by NMR indicated that the THP protecting group had been unexpectedly hydrolyzed, and that the oil was predominantly the cis-propenol, (Z)-2,2-dimethylbutanoic acid 2-(3-hydroxy-1-propenyl)-3-methylphenyl]methyl ester. A small portion was chromatographed over silica gel to obtain the analytically pure material.

| | | |
|---|---|---|
| Analysis Calculated for C₁₇H₂₄O₃: | C, 73.88; | H, 8.75 |
| Found: | C, 73.63; | H, 8.87 |

### Example 214

### Preparation of (Z)-4-[3-[2-[(2,2-dimethyl-1-oxobutoxy)methyl]-6-methylphenyl]-2-propenyloxy]-alpha-oxobenzeneacetic acid methyl ester.

As in Example 15, (Z)-2,2-dimethylbutanoic acid [2-(3-hydroxy-1-propenyl)-3-methylphenyl]methyl ester (0.649 g) was reacted with 4-hydroxyphenylglyoxylic acid methyl ester (0.430 g) in the presence of diethyl azodicarboxylate (0.47 g) and triphenylphosphine (0.776 g) in tetrahydrofuran (20 ml). After the usual work up, the crude product was purified by chromatography over silica gel (10 g; diethyl ether-hexane; 1:9) to furnish 0.823 g of (Z)-4-[3-[2-[(2,2-dimethyl-1-oxobutoxy)methyl]-6-methylphenyl]-2-propenyloxy]-alpha-oxobenzeneacetic acid methyl ester as an oil.

| | | |
|---|---|---|
| Analysis Calculated for C₂₆H₃₀O₆: | C, 71.21; | H, 6.90 |
| Found: | C, 70.91; | H, 7.05 |

### Example 215

### Preparation of 4-[3-[2-[(2,2-dimethyl-1-oxobutoxy)methyl]-6-methylphenyl]-2-propenyloxy]-alpha-oxobenzeneacetic acid (1:1) morpholine salt.

As in Example 19, (Z)-4-[3-[2-[(2,2-dimethyl-1-oxobutoxy)methyl]-6-methylphenyl]-2-propenyloxy]-alpha-oxobenzeneacetic acid methyl ester (0.718 g) in methanol (5 ml) was treated with 1N sodium hydroxide (1.8 ml). The usual work up gave 0.695 g 4-[3-[2-[(2,2-dimethyl-1-oxobutoxy)methyl]-6-methylphenyl]-2-propenyloxy]-alpha-oxobenzeneacetic acid as an oil.
As described before in Example 212, morpholine (0.145 ml) was added to a solution of the acid (0.655 g) in diethyl ether (25 ml). The mixture was stirred chilled in a ice bath to initiate crystallization, then resulting solids (0.775 g) were recovered by filtration and recrystallized from diethyl ether to yield 0.65 g of (Z)-4-[3-[2-(2,2-dimethyl-1-oxobutoxy)methyl]-6-methylphenyl]-2-propenyloxy]-alpha-oxobenzeneacetic acid (1:1) morpholine salt, mp 77-79 °C.

| | | | |
|---|---|---|---|
| Analysis Calculated for C₂₅H₂₈O₆.(1:1)C₄H₉NO: | C, 68.08; | H, 7.29; | N 2.74 |
| Found: | C, 68.18; | H, 7.34; | N 2.59 |

### Example 216

### Preparation of (Z)-2,2-dimethylbutanoic acid [2-(2-formylethenyl)-3-methylphenyl]methyl ester

A mixture of (Z)-2,2-dimethylbutanoic acid [2-(3-hydroxy-1-propenyl)-3-methylphenyl]methyl ester (0.81 g) and activated manganese dioxide in dichloromethane (50 ml) was stirred vigorously for 1 hour. The solids were filtered off, washed with water and the combined filtrates were evaporated to yield 0.775 g of (Z)-2,2-dimethylbutanoic acid [2-(2-formylethenyl)-3-methylphenyl]methyl ester as a colorless oil.

| | | |
|---|---|---|
| Analysis Calculated for C₁₇H₂₂O₃: | C, 74.42; | H, 8.08 |
| Found: | C, 74.94; | H, 8.46 |

### Example 217

### Preparation of (E)-2,2-dimethylbutanoic acid [2-(2-formylethenyl)-3-methylphenyl]methyl ester.

A solution of (Z)-2,2-dimethylbutanoic acid [2-(2-formylethenyl)-3-methylphenyl]methyl ester (0.77 g) in chloroform (40 ml) in a 200 ml round bottomed pyrex flask was placed in direct sunlight for 50 hours. The photoisomerization was followed by NMR and the reaction attained equilibrium within 6 hours with an E/Z ratio of 2.7:1. The solvent was evaporated and the isomers were separated by flash chromatography over 75 g of silica gel (diethyl ether-hexane; 1:9) to furnish 0.22 g of the starting (Z)-2,2-dimethylbutanoic acid [2-(2-formylethenyl)-3-methylphenyl]methyl ester and 0.395 g of the isomeric (E)-2,2-dimethylbutanoic acid [2-(2-formylethenyl)-3-methylphenyl]methyl ester as a colorless oil.

| | | |
|---|---|---|
| Analysis Calculated for C₁₇H₂₂O₃: | C, 74.42; | H, 8.08 |
| Found: | C, 74.33; | H, 8.12 |

### Example 218

### Preparation of (E)-2,2-dimethylbutanoic acid [2-(3-hydroxy-1-propenyl)-3-methylphenyl]methyl ester.

A solution of (E)-2,2-dimethylbutanoic acid [2-(2-formylethenyl)-3-methylphenyl]methyl ester (0.271 g) in methanol (5 ml) was treated with sodium borohydride (0.06 g) and the mixture was stirred at room temperature for 10 minutes. After the solvent was removed under reduced pressure, the residual material was partitioned between dichloromethane and water, then the dried organic phase (MgSO₄) was evaporated to afford 0.24 g of (E)-2,2-dimethylbutanoic acid [2-(3-hydroxy-1-propenyl)-3-methylphenyl]methyl ester as a colorless oil.

| | | |
|---|---|---|
| Analysis Calculated for C₁₇H₂₄O₃: | C, 73.88; | H, 8.75 |
| Found: | C, 74.01; | H, 8.78 |

### Example 219

### Preparation of (E)-4-[3-[2-[(2,2-dimethyl-1-oxobutoxy)methyl]-6-methylphenyl]-2-propenyloxy]-alpha-oxobenzeneacetic acid methyl ester.

As described in Example 15, (E)-2,2-dimethylbutanoic acid [2-(3-hydroxy-1-propenyl)-3-methylphenyl]methyl ester (0.24 g) was reacted with 4-hydroxyphenylglyoxylic acid methyl ester (0.16 g) in the presence of diethyl azodicarboxylate (0.192 g) and triphenylphosphine (0.287 g) in tetrahydrofuran (15 ml). After the previously described work up, the crude ester was purified by chromatography over silica gel (5 g; diethyl ether-hexane; 1:9) to afford 0.241 g of (E)-4-[3-[2-[(2,2-dimethyl-1-oxobutoxy)methyl]-6-methylphenyl]-2-propenyloxy]-alpha-oxobenzeneacetic acid methyl ester as an oil.

| | | |
|---|---|---|
| Analysis Calculated for C₂₆H₃₀O₆: | C, 71.21; | H, 6.90 |
| Found: | C, 71.05; | H, 6.83 |

### Example 220

### Preparation of (E)-4-[3-[2-[(2,2-dimethyl-1-oxobutoxy)methyl]-6-methylphenyl]-2-propenyloxy]-alpha-oxobenzeneacetic acid (1:1) morpholine salt .

As in Example 19, (E)-4-[3-[2-[(2,2-dimethyl-1-oxobutoxy)methyl]-6-methylphenyl]-2-propenyloxy]-alpha-oxobenzeneacetic acid methyl ester (0.201 g) in methanol (1 ml) was treated with 1N sodium hydroxide (0.5 ml). The usual work up gave 0.187 g of (E)-4-[3-[2-[(2,2-dimethyl-1-oxobutoxy)methyl]-6-methylphenyl]-2-propenyloxy]-alpha-oxobenzeneacetic acid as an oil.
As in Example 212, morpholine (0.04 ml) was added to a solution of the acid (0.187 g) in diethyl ether (5 ml). The mixture was stirred chilled in a ice bath to initiate crystallization, then the resulting solids were recovered by filtration and recrystallized from diethyl ether to yield 0.205 g of (E)-4-[3-[2-(2,2-dimethyl-1-oxobutoxy)methyl]-6-methylphenyl]-2-propenyloxy]-alpha-oxobenzeneacetic acid (1:1) morpholine salt, mp 71-73 °C.

| | | | |
|---|---|---|---|
| Analysis Calculated for C₂₅H₂₈O₆.(1:1)C₄H₉NO: | C, 68.08; | H, 7.29; | N 2.74 |
| Found: | C, 68.01; | H, 7.41; | N 2.73 |

### Example 221

### Preparation of 2,2-dimethylbutanoic acid [2-(3-hydroxypropyl)-3-methylphenyl]methyl ester.

A solution of rac-2,2-dimethylbutanoic acid[3-methyl-2-[3-(2-tetrahydropyranyloxy)-1-propynyl]phenyl]methyl ester (1.2 g) in methanol (50 ml) was hydrogenated over 10% palladium on carbon (0.2 g) at room temperature and ambient pressure for 90 minutes. After the catalyst was removed by filtration through Celite, the methanol was removed in vacuo to give 1.2 g of rac-2,2-dimethylbutanoic acid [2-[3-(2-tetrahydropyranyloxy)-1-propyl]-3-methylphenyl]methyl ester as an oil. A solution of the oil (1.2 g) and pyridinium p-toluenesulfonate (0.1 g) in ethanol (25 ml) was stirred and heated in an oil bath maintained at 55°C. After 6 hours, the solvent was evaporated and the residue was partitioned between diethyl ether and water. The aqueous phase was extracted with diethyl ether, then the combined organic extracts were dried (Na₂SO₄) and evaporated to furnish 1 g of an oil. The material was purified by HPLC (diethyl ether-hexane; 1:9) to give 0.72 g of 2,2-dimethylbutanoic acid [2-(3-hydroxy-1-propyl)-3-methylphenyl]methyl ester.

| | | |
|---|---|---|
| Analysis Calculated for C₁₇H₂₆O₃: | C, 73.35; | H, 9.41 |
| Found: | C, 72.90; | H, 9.38 |

### Example 222

### Preparation of 4-[3-[2-[(2,2-dimethyl-1-oxobutoxy)methyl]-6-methylphenyl]propoxy]-alpha-oxobenzeneacetic acid methyl ester.

As in Example 15, 2,2-dimethylbutanoic acid 2-(3-hydroxy-1-propyl)-3-methylphenyl]methyl ester (0.659 g) was reacted with 4-hydroxyphenylglyoxylic acid methyl ester (0.43 g) in the presence of diethyl azodicarboxylate (0.47 g) and triphenylphosphine (0.776 g) in tetrahydrofuran (20 ml). After the previously described work up, the crude ester was purified by chromatography over silica gel (10 g; diethyl ether-hexane; 1:9) to furnish 0.95 g of 4-[3-[2-[(2,2-dimethyl-1-oxobutoxy)methyl]-6-methylphenyl]propoxy]-alpha-oxobenzeneacetic acid methyl ester as an oil.

| | | |
|---|---|---|
| Analysis Calculated for C₂₆H₃₂O₆: | C, 70.89; | H, 7.35 |
| Found: | C, 71.35; | H, 7.52 |

### Example 223

### Preparation of 4-[3-[2-[(2,2-dimethyl-1-oxobutoxy)methyl]-6-methylphenyl]-propoxy]-alpha-oxobenzeneacetic acid (1:1) morpholine salt .

As in Example 19, 4-[3-[2-[(2,2-dimethyl-1-oxobutoxy)methyl]-6-methylphenyl]propoxy]-alpha-oxobenzeneacetic acid methyl ester (0.854 g) in methanol (4 ml) was treated with 1N sodium hydroxide (2.2 ml). The usual work up gave 0.685 g of 4-[3-[2-[(2,2-dimethyl-1-oxobutoxy)methyl]-6-methylphenyl]propoxy]-alpha-oxobenzeneacetic acid as an oil.
As described before in Example 212, morpholine (0.134 ml) was added to a solution of the acid (0.655 g) in diethyl ether (20 ml). The mixture was stirred chilled in a dry ice-acetone bath to induce crystallization, then the bath was removed and the mixture was stirred at room temperature for 1 hour. The solids were filtered off and recrystallized from diethyl ether to yield 0.415 g of 4-[3-[2-[(2,2-dimethyl-1-oxobutoxy)methyl]-6-methylphenyl]propoxy]-alpha-oxobenzeneacetic acid (1:1) morpholine salt, mp 78-80 °C.

| | | | |
|---|---|---|---|
| Analysis Calculated for C₂₅H₂₈O₆.(1:1)C₄H₉NO: | C, 68.08; | H, 7.29; | N 2.74 |
| Found: | C, 68.18; | H, 7.34; | N 2.59 |

### Example 224

### Preparation of 2-[4'-fluoro-3-(1-methylethyl)[1,1'-biphenyl]-2-yl]-4,5-dihydro-3,4,4-trimethyloxazolinium iodide (4:1) molar hydrate.

A solution of 4-fluorobromobenzene (9.7 g) in dry tetrahydrofuran (20 ml) was added dropwise to a stirred mixture of magnesium (1.38 g) in dry tetrahydrofuran (5 ml) and after the addition was completed, the reaction was stirred at 70 °C for 2 hours. The resulting solution was cooled and then was added over 10 minutes to a solution of 2-(2,6-dimethoxyphenyl)-4,5-dihydro-4,4-dimethyloxazoline (10.92 g) in dry tetrahydrofuran (50 ml). The mixture was stirred at 70 °C for 40 hours, then after the reaction mixture temperature was lowered to 45 °C, a solution of isopropyl magnesium bromide in diethyl ether (2M; 34.8 ml) was added and the stirred reaction was maintained at 45 °C for another 16 hours. The mixture was cooled in an ice bath and was treated with a saturated solution of ammonium chloride (100 ml). The separated aqueous layer was extracted with ethyl acetate ( 3 x 100 ml), then the organic phase and extracts were combined and washed in turn with water, 1N hydrochloric acid, water and brine. The dried (Na₂SO₄) organic layer was evaporated, and the residual oil (11.93 g) was purified by HPLC (ethyl acetate -hexane; 1:13) to provide 5.2 g of 2-[4'-fluoro-3-(1-methylethyl)[1,1'-biphenyl]-2-yl]-4,5-dihydro-4,4-dimethyloxazoline as an oil.
A solution of the above material (5.17 g) and methyl iodide (9.6 ml) in nitromethane (200 ml) was heated at 70 °C for 90 minutes, then the solvent was removed under reduced pressure. The residue was stirred with diethyl ether and the resulting orange solid was filtered off, washed with diethyl ether and dried to yield 6.5 g of 2-[4'-fluor-3-(1-methylethyl)[1,1'-biphenyl]-2-yl]-4,5-dihydro-3,4,4-trimethyloxazolinium iodide as its (4:1) molar hydrate, mp 188-189 °C. Crystallization from propanol furnished the analytical sample, mp 194-196 °C.

| | | | | | |
|---|---|---|---|---|---|
| Analysis Calculated for C₂₁H₂₅FINO.(4.1)H₂O: | C, 55.09; | H, 5.61; | F, 4.15; | N, 3.06; | H₂O, 1.02 |
| Found: | C, 54.88; | H, 5.96; | F, 3.77; | N, 2.94; | H₂O, 1.05 |

### Example 225

### Preparation of 4'-fluoro-3-(1-methylethyl)[1,1'-biphenyl]-2-carboxaldehyde.

Sodium borohydride (0.76 g) was added portionwise to a stirred solution of 2-[4'-fluoro-3-(1-methylethyl)[1,1'-biphenyl]-2-yl]-4,5-dihydro-3,4,4-trimethyloxazolinium iodide (4:1) molar hydrate (6.4 g) in tetrahydrofuran (110 ml) containing ethanol (50 ml) cooled in an ice bath. After the mixture was stirred at 0 °C for 30 minutes and then at room temperature for 3 hours, it was rechilled in an ice bath and 3N hydrochloric acid (60 ml) was added dropwise. The reaction was heated for 16 hours at 70 °C, then was cooled and concentrated to about half volume in vacuo. After water (100 ml) and dichloromethane (100 ml) were added, the phases were separated and the aqueous layer was extracted with dichloromethane (2 x 100 ml). The combined organic layers, washed in turn with brine, saturated sodium bisulfite solution, and brine, dried (MgSO₄) and evaporated to give 4.4 g of the crude aldehyde as an oil. The material was purified by bulb to bulb distillation (130-140 °C; 0.05 mm) to give 3.35 g of 4'-fluoro-3-(1-methylethyl)[1,1'-biphenyl]-2-carboxaldehyde.

| | | | |
|---|---|---|---|
| Analysis Calculated for C₁₆H₁₅FO: | C, 79.31; | H, 6.24; | F, 7.84 |
| Found: | C, 79.28; | H, 6.45: | F, 7.59 |

### Example 226

### Preparation of (E)-3-[4'-fluoro-3-(1-methylethyl)[1,1'-biphenyl]-2-yl]-2-propenoic acid methyl ester.

A solution of (carbomethoxymethylene)triphenylphosphorane (3.18 g) and 4'-fluoro-3-(1-methylethyl)[1,1'-biphenyl]-2-carboxaldehyde (2.27 g) in methanol (18 ml) was stirred at room temperature for 2 hours then the solvent was removed in vacuo. The residue was taken up in diethyl ether (50 ml) and after the mixture was stirred at ambient temperature for 20 minutes, the precipitated triphenylphosphine oxide was filtered off. Evaporation of the filtrate afforded 3.9 g of crude ester which, after purification by HPLC (ethyl acetate-hexane; 1:19) was crystallized from ethanol to provide 1.1 g of (E)-3-[4'-fluoro-3-(1-methylethyl)[1,1'-biphenyl]-2-yl]-2-propenoic acid methyl ester as a colorless solid, mp 87-89 °C

| | | | |
|---|---|---|---|
| Analysis Calculated for C₁₉H₁₉FO₂: | C, 76.49; | H. 6.42; | F, 6.37 |
| Found: | C, 76.85; | H, 6.51; | F, 6.36 |

### Example 227

### Preparation of (E)-3-[4'-fluoro-3-(1-methylethyl)[1,1'-biphenyl]-2-yl]-2-propen-1-ol.

A solution of (E)-3-[4'-fluoro-3-(1-methylethyl)[1,1'-biphenyl]-2-yl]-2-propenoic acid methyl ester (1.37 g) in dry dichloromethane (30 ml) was chilled to -60 °C and a solution of diisobutylaluminum hydride in toluene (1.5M; 7.3 ml) was added dropwise with stirring over 5 minutes. The reaction mixture was maintained at -60 °C for 3 hours, then a saturated sodium sulfate solution ( 20 ml) was slowly added over several minutes. The formed solids were removed by filtration and washed with dichloromethane. The organic phase of the filtrate was separated, then washed in turn with water and brine, dried (MgSO₄) and evaporated. The crude product was purified by flash chromatography over silica gel (ethyl acetate-hexane; 3:7) to give an oil (0.86 g) that was crystallized from hexane to yield 0.62 g of (E)-3-[4'-fluoro-3-(1-methylethyl)[1,1'-biphenyl]-2-yl]-2-propen-1-ol as a colorless solid, mp 71-72 °C.

| | | | |
|---|---|---|---|
| Analysis Calculated for C₁₈H₁₉FO: | C, 79.97; | H, 7.08; | F, 7.03 |
| Found: | C, 80.03; | H, 7.11; | F, 6.95 |

### Example 228

### Preparation of (E)-4-[3-[4'-fluoro-3-(1-methylethyl)[1,1'-biphenyl]-2-yl]-2-propenyloxy]-alpha-oxobenzeneacetic acid methyl ester.

As in Example 15, (E)-3-[4'-fluoro-3-(1-methylethyl)[1,1'-biphenyl]-2-yl]-2-propen-1-ol (0.58 g) was reacted with 4-hydroxyphenylglyoxylic acid methyl ester (0.387 g) in the presence of diethyl azodicarboxylate (0.448 g) and triphenylphosphine (0.68 g) in tetrahydrofuran (25 ml). The usual work up afforded 1.3 g of a crude mixture that was purified by flash chromatography over silica gel (130 g; ethyl acetate-hexane; 3:7) to furnish 0.55 g of (E)-4-[3-[4'-fluoro-3-(1-methylethyl)[1,1'-biphenyl]-2-yl]-2-propenyloxy]-alpha-oxobenzeneacetic acid methyl ester as an oil.

### Example 229

### Preparation of (E)-4-[3-[4'-fluoro-3-(1-methylethyl)[1,1'-biphenyl]-2-yl]-2-propenyloxy]- alpha-oxobenzeneacetic acid.

As in Example 19, (E)-4-[3-[4'-fluoro-3-(1-methylethyl)[1,1'-biphenyl]-2-yl]-2-propenyloxy]- alpha-oxobenzeneacetic acid methyl ester (0.5 g) in a mixture of methanol (4 ml) and tetrahydrofuran (2 ml) was treated with 1N potassium hydroxide (1.4 ml). The usual work up gave 0.4 g of a solid, which after crystallization from diethyl ether-hexane provided 0.3 g of (E)-4-[3-[4'-fluoro-3-(1-methylethyl)[1,1'-biphenyl]-2-yl]-2-propenyloxy]-alpha-oxobenzeneacetic acid as a colorless solid, mp 142-143 °C.

| | | | |
|---|---|---|---|
| Analysis Calculated for C₂₆H₂₃FO₄: | C, 74.63; | H, 5.54; | F, 4.54 |
| Found: | C, 74.63; | H, 5.50; | F, 4.63 |

### Example 230

### Preparation of 4'-fluoro-3-(1-methylethyl)[1,1'-biphenyl]-2-ol formate.

3-Chloroperbenzoic acid (4.73 g) was added to a solution of 4'-fluoro-3-(1-methylethyl)[1,1'-biphenyl]-2-carboxaldehyde (3.55 g) in dichloromethane (100 ml) and the reaction mixture was stirred at room temperature overnight. The precipitated solids were removed by filtration and the filtrate was washed in turn with water, saturated sodium bisulfite solution, sodium bicarbonate solution and brine. After the dried (MgSO₄) organic layer was evaporated, the crude product was purified by HPLC (ethyl acetate-hexane; 1:49) to afford 2.5 g of 4'-fluoro-3-(1-methylethyl)[1,1'-biphenyl]-2-ol formate as an oil.

| | | | |
|---|---|---|---|
| Analysis Calculated for C₁₆H₁₅FO₂: | C, 74.40; | H, 5.85; | F, 7.36 |
| Found: | C, 74.36; | H, 5.83; | F, 7.38 |

### Example 231

### Preparation of 4'-fluoro-3-(1-methylethyl)[1,1'-biphenyl]-2-ol.

To a solution of 4'-fluoro-3-(1-methylethyl)[1,1'-biphenyl]-2-ol formate (2 g) in ethanol (5 ml) was added a 10% solution of potassium hydroxide in ethanol (10 ml) and the reaction mixture was stirred at room temperature for 1 hour. The reaction was concentrated in vacuo, and the residue was partitioned between dichloromethane (20 ml) and 1N hydrochloric acid (20 ml). The separated aqueous layer was extracted with dichloromethane (2 x 20 ml), then the combined organic layers were washed with water, dried (MgSO₄) and evaporated to furnish 1.9 g of 4'-fluoro-3-(1-methylethyl) [1,1'-biphenyl]-2-ol as an oil which solidified on standing. A small sample was sublimed to give the analytical specimen, mp 41-43 °C.

| | | | |
|---|---|---|---|
| Analysis Calculated for C₁₅H₁₅FO: | C, 78.24; | H, 6.57; | F, 8.25 |
| Found: | C, 78.11; | H, 6.56; | F, 8.17 |

### Example 232

### Preparation of 2-[4'-fluoro-3-(1-methylethyl)[1,1'-biphenyl]-2-yloxy]ethanol.

A mixture of 4'-fluoro-3-(1-methylethyl)[1,1'-biphenyl]-2-ol (1.84g), ethylene carbonate (0.78 g) and tetraethylammonium iodide (0.68 g) was heated in an oil bath at 155 °C for 2 hours, then was cooled and taken up in dichloromethane. The solution was washed in turn with water, 1N sodium hydroxide, 1N hydrochloric acid and brine, then was dried (MgSO₄) and evaporated. The residual material was chromatographed over 70 g of silica gel (ethyl acetate-hexane; 3:7) to provide 0.57 g of 2-[4'-fluoro-3-(1-methylethyl)[1,1'-biphenyl]-2-yloxy]ethanol. The compound was crystallized from hexane to give the analytical sample, mp 77-78°C.

| | | | |
|---|---|---|---|
| Analysis Calculated for C₁₇H₁₉FO₂: | C, 74.43; | H, 6.98; | F, 6.98 |
| Found: | C, 74.49; | H, 6.94; | F, 6.94 |

### Example 233

### Preparation of 4-[2-[4'-fluoro-3-(1-methylethyl)[1,1'-biphenyl]-2-yloxy]ethoxy]-alpha-oxobenzeneacetic acid methyl ester.

As in Example 15, (2-[4'-fluoro-3-(1-methylethyl)[1,1'-biphenyl]-2-yloxy]ethanol (0.393 g) was treated with 4-hydroxyphenylglyoxylic acid methyl ester (0.258 g) in the presence of diethyl azodicarboxylate (0.298 g) and triphenylphosphine (0.449 g) in tetrahydrofuran (25 ml). The usual work up afforded 0.6 g of crude product which was purified by flash chromatography over silica gel (60 g; ethyl acetate-hexane; 3:7) to furnish 0.3 g of 4-[2-[4'-fluoro-3-(1-methylethyl)[1,1'-biphenyl]-2-yloxy]ethoxy]-alpha-oxobenzeneacetic acid methyl ester. Crystallization of the ester from ethyl acetate-hexane yielded the analytical specimen, mp 77-79 °C.

| | | | |
|---|---|---|---|
| Analysis Calculated for C₂₆H₂₅FO₅: | C, 71.55; | H, 5.77; | F, 4.35 |
| Found: | C, 71.51; | H, 5.74; | F, 4.53 |

### Example 234

### Preparation of 4-[2-[4'-fluoro-3-(1-methylethyl)[1,1'-biphenyl]-2-yloxy]ethoxy]-alpha-oxobenzeneacetic acid.

As in Example 19, 4-[2-[4'-fluoro-3-(1-methylethyl)[1,1'-biphenyl]-2-yloxy]ethoxy]-alpha-oxobenzeneacetic acid methyl ester (0.186 g) in a mixture of methanol (2 ml) and tetrahydrofuran (1 ml) was treated with 1N potassium hydroxide (0.52 ml). The usual work up gave 0.17 g of a solid, which after crystallization from diethyl ether-hexane afforded 0.12 g of 4-[2-[4'-fluoro-3-(1-methylethyl)[1,1'-biphenyl]-2-yloxy]ethoxy]-alpha-oxobenzeneacetic acid, mp 123-125 °C.

| | | |
|---|---|---|
| Analysis Calculated for C₂₅H₂₃FO₅: | C, 71.08; | H, 5.49 |
| Found: | C, 71.06; | H, 5.56 |

### Example 235

### Preparation of (E)-4-[3-[4-(4-fluorophenyl)-2-(1-methylethyl)-3-quinolinyl]-2-propenyloxy]-alpha-oxobenzeneacetic acid methyl ester.

As in Example 15, (E)-3-[4-(4-fluorophenyl)-2-(1-methylethyl)-3-quinolinyl]-2-propen-1-ol (1.0 g) was coupled with 4-hydroxyphenylglyoxylic acid methyl ester (0.565 g) in the presence of diethyl azodicarboxylate (0.683 g) and triphenylphosphine (1.02 g) in tetrahydrofuran (30 ml). The usual work up, followed by purification of the crude product by flash chromatography over silica gel (50 g; diethyl ether-hexane; 1:4) and crystallization from diethyl ether to provide 0.94 g of (E)-4-[3-[4-(4-fluorophenyl)-2-(1-methylethyl)-3-quinolinyl]-2-propenyloxy]-alpha-oxobenzeneacetic acid methyl ester, mp 109-111 °C.

| | | | | |
|---|---|---|---|---|
| Analysis Calculated for C₃₀H₂₆FNO₄: | C, 74.52; | H, 5.42; | F, 3.93; | N, 2.90 |
| Found: | C, 74.33; | H, 5.35; | F, 3.75; | N, 2.82 |

### Example 236

### Preparation of (E)-4-[3-[4-(4-fluorophenyl)-2-(1-methylethyl)-3-quinolinyl]-2-propenyloxy]-alpha-oxobenzeneacetic acid.

As in Example 19, (E)-4-[3-[4-(4-fluorophenyl)-2-(1-methylethyl)-3-quinolinyl]-2-propenyloxy]-alpha-oxobenzeneacetic acid methyl ester (0.84 g) in a mixture of methanol (10 ml) and tetrahydrofuran (1.5 ml) was treated with 1N sodium hydroxide (1.9 ml). The usual work up furnished 0.82 g of a solid, which was crystallized from ethyl acetate to yield 0.63 g of (E)-4-[3-[4-(4-fluorophenyl)-2-(1-methylethyl)-3-quinolinyl]-2-propenyloxy]-alpha-oxobenzeneacetic acid, mp 232-234 °C.

| | | | | |
|---|---|---|---|---|
| Analysis Calculated for C₂₉H₂₄FNO₄: | C, 74.19; | H, 5.15; | F, 4.05; | N, 2.98 |
| Found: | C, 73.87; | H, 5.21: | F, 3.87; | N, 2.95 |

### Example 237

### Preparation of 3-(4-fluorophenyl)-1-(1-methylethyl)-1H-indole-2-carboxaldehyde.

Phosphoryl oxychloride (6.7 ml) was added dropwise to dimethylformamide (20 ml) at such a rate that the temperature did not exceed 10 °C, then the mixture was heated to 80 °C as a solution of 3-(4-fluorophenyl)-1-(1-methylethyl)-1H-indole (15.6 g) in dimethylformamide (20 ml) was added, and then was stirred at that temperature for 21 hours. The reaction mixture was cooled (10 °C) during the addition of 4N sodium hydroxide (85 ml), it was stirred at 40 °C for 30 minutes then was allowed to equilibrate to room temperature. The resulting gummy solid was filtered off, dissolved in dichloromethane (150 ml), then washed with water, dried (MgSO₄) and evaporated. The residual solid was chromatographed over a short column of silica gel. The initial fractions, eluted with diether-hexane (1:9), gave 3.0 g of a mixture of starting indole and the desired aldehyde (3:1). Crystallization of this material from aqueous ethanol afforded 1.55 g of recovered starting material and the mother liquor was combined with later fractions that had been eluted from the column with diethyl ether-hexane (1:4) to give a total of 11.3 g of impure 3-(4-fluorophenyl)-1-(1-methylethyl)-1H-indole-2-carboxaldehyde contaminated with the starting indole (4:1). The bulk of this material was used in subsequent reactions without further purification, but a portion was flash chromatographed over silica gel, and then crystallized from ethanol to provide the analytically pure aldehyde, mp 95-96 °C.

| | | | | |
|---|---|---|---|---|
| Analysis Calculated for C₁₈H₁₆FNO: | C, 76.85; | H, 5.73; | F, 6.75; | N, 4.98 |
| Found: | C, 76.68; | H, 5.76; | F, 6.69; | N, 4.74 |

### Example 238

### Preparation of (E)-3-[3-(4-fluorophenyl)-1-(1-methylethyl)-1H-indol-2-yl]-2-propenoic acid methyl ester.

As in Example 226, the impure 3-(4-fluorophenyl)-1-(1-methylethyl)-1H-indole-2-carboxaldehyde (5.56 g) from the previous example was reacted in methanol (75 ml) with (carbomethoxymethylene)triphenylphosphorane (6.2 g) under stirring at room temperature for 1 hour. The pale yellow solids that formed were filtered and washed with cold methanol to furnish 4.58 g of (E)-3-[3-(4-fluorophenyl)-1-(1-methylethyl)-1H-indol-2-yl]-2-propenoic acid methyl ester. A sample was crystallized from dichloromethane-hexane to yield the analytical specimen, mp 155-157 °C.

| | | | | |
|---|---|---|---|---|
| Analysis Calculated for C₂₁H₂₀FNO₂: | C, 74.76; | H, 5.98; | F, 5.63; | N, 4.15 |
| Found: | C, 74.47; | H, 6.01; | F, 5.82; | N, 4.82 |

### Example 239

### Preparation of (E)-3-[3-(4-fluorophenyl)-1-(1-methylethyl)-1H-indol-2-yl]-2-propen-1-ol.

As in Example 227, (E)-3-[3-(4-fluorophenyl)-1-(1-methylethyl)-1H-indol-2-yl]-2 propenoic acid methyl ester (4.43 g) in dry dichloromethane (60 ml) was treated with diisobutylaluminum hydride in toluene (1.5M; 30 ml) at -65 °C for 1 hour, then was worked up as previously described to give 4.15 g of (E)-3-[3-(4-fluorophenyl)-1-(1-methylethyl)-1H-indol-2-yl]-2-propen-1-ol as a yellow oil. A portion (0.2 g) was purified by chromatography over silica gel (diethyl ether-hexane; 1:19) to furnish 0.175 g of the analytically pure alcohol as an oil.

| | | | | |
|---|---|---|---|---|
| Analysis Calculated for C₂₀H₂₀FNO: | C, 77.64; | H, 6.52; | F, 6.14; | N, 4.53 |
| Found: | C, 77.42; | H, 6.69; | F, 6.42; | N, 4.35 |

### Example 240

### Preparation of (E)-4-[3-[3-(4-fluorophenyl)-1-(1-methylethyl)-1H-indol-2-yl]-2-propenyloxy]-alpha-oxobenzeneacetic acid methyl ester.

As in Example 15, (E)-3-[3-(4-fluorophenyl)-1-(1-methylethyl)-1H-indol-2-yl]-2-propen-1-ol (1.0 g) was reacted with 4-hydroxyphenylglyoxylic acid methyl ester (0.585 g) in the presence of diethyl azodicarboxylate (0.71 g) and triphenylphosphine (1.06 g) in tetrahydrofuran (30 ml). The crude reaction product, isolated in the usual manner, was purified by flash chromatography over silica gel (130 g; ethyl acetate-hexane; 1:4) to furnish 0.61 g of (E)-4-[3-[3-(4-fluorophenyl)-1-(1-methylethyl)-1H-indol-2-yl]-2-propenyloxy]-alpha-oxobenzeneacetic acid methyl ester as a yellow oil. A portion (0.2 g) was rechromatographed over silica gel (diethyl ether-hexane; 1:19) to furnish 0.225 g of the analytically pure ester as an oil.

| | | | | |
|---|---|---|---|---|
| Analysis Calculated for C₂₉H₂₆FNO₄: | C, 73.87; | H, 5.56; | F, 4.03; | N, 2.97 |
| Found: | C, 73.49; | H, 5.93; | F, 3.78; | N, 2.88 |

### Example 241

### Preparation of (E)-4-[3-[3-(4-fluorophenyl)-1-(1-methylethyl)-1H-indol-2-yl]-2-propenyloxy)-alpha- oxobenzeneacetic acid (1:1) morpholine salt.

As in Example 19, (E)-4-[3-[3-(4-fluorophenyl)-1-(1-methylethyl)-1H-indol-2-yl]-2-propenyloxy]-alpha-oxobenzeneacetic acid methyl ester (0.354 g) in methanol (5 ml) was treated with 1N sodium hydroxide (0.9 ml). The usual work up gave 0.4 g of an oil, which was dissolved in diethyl ether and morpholine (0.07 g) was added. Crystallization was induced and the recovered solid was recrystallized from dichloro-methane-ethyl acetate to provide 0.26 g of (E)-4-[3-[3-(4-fluorophenyl)-1-(1-methylethyl)-1H-indol-2-yl]-2-propenyloxy]-alpha-oxobenzeneacetic acid (1:1) morpholine salt as a colorless solid, mp 151-152 °C.

| | | | | |
|---|---|---|---|---|
| Analysis Calculated for C₂₈H₂₄FNO₄.C₄H₉NO: | C, 70.57; | H, 6.11; | F, 3.49; | N, 5.14 |
| Found: | C, 69.88; | H, 6.22; | F, 3.79; | N, 4.90 |

### Example 242

### Preparation of (E)-4-[3-[1-(4-fluorophenyl)-4-(1-methylethyl)-2-phenyl-1H-imidazol-5-yl]-2- propenyloxy]-alpha-oxobenzeneacetic acid methyl ester.

As in Example 15, (E)-3-[1-(4-fluorophenyl)-4-(1-methylethyl)-2-phenyl-1H-imidazol-5-yl]-2-propen-1-ol (0.773 g) was reacted with 4-hydroxyphenylglyoxylic acid methyl ester (0.378 g) in the presence of diethyl azodicarboxylate (0.418 g) and triphenylphosphine (0.63 g) in tetrahydrofuran (20 ml). The crude reaction product, isolated in the usual manner, was purified by flash chromatography over silica gel (90 g; ethyl acetate-hexane; 1:3) to give 0.65 g of (E)-4-[3-[3-(4-fluorophenyl)-1-(1-methylethyl)-1H-indol-2-yl]-2-propenyloxy]-alpha-oxobenzeneacetic acid methyl ester as a pale yellow solid. A portion was crystallized from diethyl ether-hexane to provide the analytical specimen, mp 132-133 °C

| | | | | |
|---|---|---|---|---|
| Analysis Calculated for C₃₀H₂₇FN₂O₄: | C, 72.28; | H, 5.46; | F, 3.81; | N, 5.62 |
| Found: | C, 71.97; | H, 5.44; | F, 3.75; | N, 5.36 |

### Example 243

### Preparation of (E)-4-[3-[1-(4-fluorophenyl)-4-(1-methylethyl)-2-phenyl-1H-imidazol-5-yl]-2- propenyloxy]-alpha-oxobenzeneacetic acid methyl ester monohydrate and its (1:1) morpholine salt.

As in Example 19, (E)-4-[3-[1-(4-fluorophenyl)-4-(1-methylethyl)-2-phenyl-1H-imidazol-5-yl]-2-propenyloxy]-alpha-oxobenzeneacetic acid methyl ester (0.5 g) in methanol (5 ml) was treated with 1N sodium hydroxide (1.1 ml). The usual work up provided 0.5 g of a solid that was crystallized from acetone-hexane to furnish 0.42 g of (E)-4-[3-[1-(4-fluorophenyl)-4-(1-methylethyl)-2-phenyl-1H-imidazol-5-yl]-2-propenyloxy]-alpha-oxobenzeneacetic acid mono hydrate as a colorless solid, mp 183-185 °C.

| | | | | |
|---|---|---|---|---|
| Analysis Calculated for C₂₉H₂₅FN₂O₄.H₂0: | C, 69.31; | H, 5.42; | F, 3.78; | N, 5.57 |
| Found: | C, 69.63; | H, 5.10; | F, 3.36; | N, 5.36 |

A portion was converted as in Example 212 to its morpholine salt, which was crystallized from ethyl acetate-cyclohexane to give the analytical sample, mp 122-124 °C.

| | | | | |
|---|---|---|---|---|
| Analysis Calculated for C₂₉H₂₅FN₂O₄.C₄H₉NO: | C, 69.34; | H, 6.00; | F, 3.32; | N, 7.35 |
| Found: | C, 69.52; | H, 6.31; | F, 3.13; | N, 6.86 |

### Example 244

### Preparation of alpha-oxo-4-[2-oxo-2-(4-phenyl-1-piperidinyl)ethoxy]benzeneacetic acid methyl ester.

A mixture of 4-hydroxy-alpha-oxobenzeneacetic acid methyl ester (0.611 g) in dimethylformamide (6 ml) under argon was treated with 55% sodium hydride (0.148 g), stirred for 30 minutes in a ice-water bath and then 1-(bromoacetyl)-4-phenyl-piperidine (0.8 g) in dimethylformamide (5 ml) was added. The solution was stirred at room temperature for 18 hours and worked up as in Example 20. The crude product was purified by flash chromatography (ethyl acetate-hexane; 3:7 changing to 2:5) to provide 0.9 g of 4-[2-oxo-2-(4-phenyl-1-piperidinyl)ethoxy]-alpha-oxobenzeneacetic acid methyl ester as an oil.

| | | | |
|---|---|---|---|
| Analysis Calculated for C₂₂H₂₃NO₅: | C, 69.28; | H, 6.08; | N, 3.67 |
| Found: | C, 68.75; | H, 5.99; | N, 3.75 |

### Example 245

### Preparation of alpha-oxo-4-[2-oxo-2-(4-phenyl-1-piperidinyl)ethoxy]benzeneacetic acid.

4-[2-Oxo-2-(4-phenyl-1-piperidinyl)ethoxy]-alpha-oxobenzeneacetic acid ethyl ester (0.9 g) in methanol (80 ml) was treated with 1N sodium hydroxide solution (2.6 ml), and the mixture was stirred at room temperature for 25 minutes. After the methanol was evaporated under reduced pressure, water (25 ml) was added and the solution was acidified with 3N hydrochloric acid, then the aqueous layer was extracted with a dichloromethane-tetrahydrofuran mixture (35 ml; 5:2). The organic layer was dried (MgSO₄), evaporated and the resulting solid was crystallized from dichloromethane-tetrahydrofuran-hexane and then from dichloromethane-tetrahydrofuran to furnish 0.25 g of 4-[2-oxo-2-(4-phenyl-1-piperidinyl)ethoxy]-alpha-oxobenzeneacetic acid, mp 209-210 °C. An additional 0.16 g of the acid was recovered from the mother liquor.

| | | | |
|---|---|---|---|
| Analysis Calculated for C₂₁H₂₁NO₅: | C, 68.65; | H, 5.76; | N, 3.81 |
| Found: | C, 68.26; | H, 5.69; | N, 3.70 |

### Example 246

### Preparation of 4-[2-(cyclooctylamino)-2-oxoethoxy]-alpha-oxobenzeneacetic acid methyl ester.

Bromoacetyl chloride (0.25 ml) was added slowly to a chilled (ice-water-bath) solution of cyclooctylamine (0.763g) in dichloromethane (15 ml) and the mixture was stirred with cooling for 45 minutes, then at room temperature overnight. After water (20 ml) was added, the layers were separated and the dried organic layer (MgSO₄) was evaporated to provide 0.7 g of N-cycloocyl-bromoacetamide.
A solution of 4-hydroxy-alpha-oxobenzeneacetic acid methyl ester (0.605 g) in dimethylformamide (8 ml) under argon was treated with 55% sodium hydride (0.147 g), stirred for 30 minutes and then N-cyclooctyl-bromoacetamide (0.7 g) in dimethylformamide (5 ml) was added. The solution was stirred at room temperature for 18 hours and worked up as in Example 20. The crude material was purified by flash chromatography (ethyl acetate-hexane; 3:7) to yield 0.4 g of 4-[2-(cyclooctylamino)-2-oxoethoxy]-alpha-oxobenzeneacetic acid methyl ester as an oil.

| | | | |
|---|---|---|---|
| Analysis Calculated for C₁₉H₂₅NO₅: | C, 65.69; | H, 7.25; | N, 4.03 |
| Found: | C, 65.54; | H, 7.29; | N, 3.98 |

### Example 247

### Preparation of 4-[2-(cyclooctylamino)-2-oxoethoxy]-alpha-oxobenzeneacetic acid.

A solution of 4-[2-(cyclooctylamino)-2-oxoethoxy]-alpha-oxobenzeneacetic acid methyl ester (0.4 g) in methanol (40 ml) was treated with 1N sodium hydroxide solution (1.3 ml), and the mixture was stirred at room temperature for 15 minutes. After the solvents were evaporated under reduced pressure, water (20 ml) was added and the solution was acidified with 3N hydrochloric acid, then the aqueous layer was extracted with a mixture of dichloromethane-tetrahydrofuran (30 ml; 2:1). The organic layer was dried (MgSO₄), evaporated and the resulting oil was crystallized from diethyl ether-hexane to yield 0.084 g of 4-[2-(cyclooctylamino)-2-oxoethoxy]-alpha-oxobenzeneacetic acid, mp 139-140 °C.

| | | | |
|---|---|---|---|
| Analysis Calculated for C₁₈H₂₃NO₅: | C, 64.85; | H, 6.95; | N, 4.20 |
| Found: | C, 64.63; | H, 6.84; | N, 4.02 |

### Example 248

### Preparation of 4-[2-oxo-2-(4-phenyl-1-piperazinyl)ethoxy]-alpha-oxobenzeneacetic acid ethyl ester.

A mixture of 4-hydroxy-alpha-oxobenzeneacetic acid ethyl ester (0.582 g) in dimethylformamide (8 ml) under argon was treated with 55% sodium hydride (0.131 g), stirred for 30 minutes in a ice-water bath and then 1-(bromoacetyl)-4-phenyl-piperazine (1.0 g) in dimethylformamide (5 ml) was added. The solution was stirred at room temperature for 18 hours and worked up as in Example 20. The crude product was purified by HPLC (ethyl acetate-hexane; 1:1) to yield 0.8 g of 4-[2-oxo-2-(4-phenyl-1-piperazinyl)ethoxy]-alpha-oxobenzeneacetic acid ethyl ester as an oil.

| | | | |
|---|---|---|---|
| Analysis Calculated for C₂₂H₂₄N₂O₅: | C, 66.65; | H, 6.10; | N, 7.07 |
| Found: | C, 66.71; | H, 6.34; | N, 7.09 |

### Example 249

### Preparation of 4-[2-oxo-2-(4-phenyl-1-piperazinyl)ethoxy]-alpha-oxobenzeneacetic acid.

4-[2-Oxo-2-(4-phenyl-1-piperazinyl)ethoxy]-alpha-oxobenzeneacetic acid ethyl ester (0.7 g) in methanol (40 ml) was treated with sodium carbonate (0.24 g) in water (6 ml), and the mixture was stirred at room temperature for 1.25 hours. After the methanol was evaporated under reduced pressure, water was added and the solution was acidified with 3N hydrochloric acid, then the aqueous layer was extracted with a dichloromethane-tetrahydrofuran mixture (1:1). The organic layer was dried (MgSO₄), evaporated, and the residue was crystallized from acetone to furnish 0.3 g of 4-[2-oxo-2-(4-phenyl-1-piperazinyl)ethoxy]-alpha-oxobenzene acetic acid, mp 188-192 °C.

| | | | |
|---|---|---|---|
| Analysis Calculated for C₂₀H₂₀N₂O₅: | C, 65.21; | H, 5.47; | N, 7.60 |
| Found: | C, 65.08; | H, 5.39; | N, 7.41 |

### Example 250

### Preparation of 4-[2-oxo-2-(1,2,3,4-tetrahydro-2-isoquinolinyl)ethoxy]-alpha-oxobenzeneacetic acid ethyl ester.

Bromoacetyl chloride (0.25 ml) was added slowly to a cold (5 °C) solution of 1,2,3,4-tetrahydroisoquinoline (0.8 g) in dichloromethane (25 ml) and the mixture was stirred in an ice bath for 45 minutes, then at room temperature overnight. Water (20 ml) was added, the layers were separated and the organic layer was washed in turn with 0.5N hydrochloric acid and sodium bicarbonate solution. The dried extract (MgSO₄) was evaporated to provide 0.762 g of 2-(2-bromo-1-oxoethyl)-1,2,3,4-tetra-hydroisoquinoline.
A mixture of 4-hydroxy-alpha-oxobenzeneacetic acid ethyl ester (0.582 g) in dimethylformamide (8 ml) under argon was treated with 55% sodium hydride (0.141 g), stirred for 30 minutes and then 2-(2-bromo-1-oxoethyl)-1,2,3,4-tetra-hydroisoquinoline (0.762 g) in dimethylformamide (6 ml) was added. The solution was stirred at room temperature for 20 hours and worked up as in Example 20. The crude product was purified by HPLC (ethyl acetate-hexane; 2:3) to yield 0.3 g of 4-[2-oxo-2-(1,2,3,4-tetrahydro-2-isoquinolinyl)ethoxy]-alpha-oxobenzeneacetic acid ethyl ester as an oil.

| | | | |
|---|---|---|---|
| Analysis Calculated for C₂₁H₂₁NO₅: | C, 68.65; | H, 5.76; | N, 3.81 |
| Found: | C, 67.93; | H, 5.70; | N, 4.03 |

### Example 251

### Preparation of 4-[2-oxo-2-(1,2,3,4-tetrahydro-2-isoquinolinyl)ethoxy]-alpha-oxobenzeneacetic acid.

4-[2-Oxo-2-(1,2,3,4-tetrahydro-2-isoquinolinyl)ethoxy]-alpha-oxobenzeneacetic acid ethyl ester (0.3 g) in methanol (10 ml) was treated with sodium carbonate (0.113 g) in water (3 ml), and the mixture was stirred at room temperature for 20 minutes. After the solvents were removed in vacuo, water (10 ml) was added and the solution acidified with 3N hydrochloric acid, then the aqueous layer was extracted with dichloromethane-tetrahydrofuran (1:1). The organic layer was dried (MgSO₄), evaporated and the residual solid was crystallized from acetone-tetrahydrofuran-hexane to yield 0.078 g of 4-[2-oxo-2-(1,2,3,4-tetrahydro-2-isoquinolinyl)ethoxy]-alpha-oxobenzeneacetic acid, mp 218-220 °C.

| | | | |
|---|---|---|---|
| Analysis Calculated for C₁₉H₁₇NO₅: | C, 67.25; | H, 5.05; | N, 4.13 |
| Found: | C, 67.45; | H, 4.92; | N, 4.02 |

### Example 252

### Preparation of alpha-oxo-4-[2-oxo-2-[4-[2-[2-(trifluoromethyl)phenyl]ethyl]-1-piperazinyl]ethoxy]-benzeneacetic acid ethyl ester.

Bromoacetyl chloride (0.125 ml) was added slowly to a chilled (5 °C) solution of 1-[2-[2-(trifluoromethyl)phenyl]ethyl]piperazine (0.774 g) in dichloromethane (20 ml) and the mixture was stirred in an ice bath for 45 minutes, then at room temperature for 18 hours. After the addition of water (20 ml), the organic layer was separated and washed in turn with 0 .5N hydrochloric acid and sodium bicarbonate solution. The dried (MgSO₄) extract was evaporated to give 0.5 g of 1-(2-bromo-1-oxoethyl)-4-[2-[2-(trifluoromethyl)phenyl]ethyl]piperazine.
A mixture of 4-hydroxy-alpha-oxobenzeneacetic acid ethyl ester (0.256 g) in dimethylformamide (4 ml) under argon was treated with 55% sodium hydride (0.06 g), stirred for 15 minutes and then the above 1-(2-bromo-1-oxoethyl)-4-[2-[2-(trifluoromethyl)phenyl]ethyl]piperazine (0.5 g) in dimethylformamide (2 ml) was added. The mixture was stirred at room temperature for 23 hours and worked up as in Example 20. The crude product was purified by flash chromatography over silica gel (ethyl acetate-toluene; 1:1 increasing to 4:1) to afford 0.21 g of alpha-oxo-4-[2-oxo-2-[4-[2-[2-(trifluoromethyl)phenyl]ethyl]-1-piperazinyl]ethoxy]-benzeneacetic acid ethyl ester as an oil.

### Example 253

### Preparation of alpha-oxo-4-[2-oxo-2-[4-[2-[2-(trifluoromethyl)phenyl]ethyl]-1-piperazinyl]ethoxy]benzeneacetic acid (1:1) hydrochloride.

A solution of alpha-oxo-4-[2-oxo-2-[4-[2-[2-(trifluoromethyl)phenyl]ethyl]-1-piperazinyl]ethoxy]benzeneacetic acid ethyl ester (0.21 g) in methanol (10 ml) was treated with sodium carbonate (0.059 g) in water (3 ml), and the mixture was stirred at room temperature for 20 minutes. After the solvents were removed in vacuo, water (10 ml) was added and the solution was acidified with 3N hydrochloric acid. The resulting solids was filtered off, dried and crystallized from tetrahydrofuran-dichloromethane-hexane to yield 0.1 g of alpha-oxo-4-[2-oxo-2-[4-[2-[2-(trifluoro-methyl)phenyl]ethyl]-1-piperazinyl]ethoxy]benzeneacetic acid hydrochloride salt, mp 196-199 °C.

| | | | | |
|---|---|---|---|---|
| Analysis Calculated for C₂₃H₂₃F₃N₂O₅.HCl: | C, 55.14; | H, 4.83; | F, 11.38; | N, 5.59 |
| Found: | C, 55.76; | H, 4.83; | F, 12.00; | N, 5.60 |

### Example 254

### Preparation of 4-[2-(4-morpholinyl)-2-oxoethoxy]-alpha-oxobenzeneacetic acid ethyl ester.

A mixture of 4-hydroxy-alpha-oxobenzeneacetic acid ethyl ester (0.71 g) in dimethylformamide (7 ml) under argon was treated with 55% sodium hydride (0.16 g), stirred for 15 minutes and then 4-(bromoacetyl)morpholine (0.76 g) in dimethylformamide (5 ml) was added. The mixture was stirred at room temperature for 18 hours and worked up as in Example 20. The crude product was purified by HPLC (toluene-hexane; 3:1) and then crystallized twice from ethyl acetate-hexane to provide 0.76 g of 4-[2-(4-morpholinyl)-2-oxoethoxy]-alpha-oxobenzeneacetic acid ethyl ester, mp 103-106 °C.

| | | | |
|---|---|---|---|
| Analysis Calculated for C₁₆H₁₉NO₆: | C, 59.81; | H, 5.96; | N, 4.36 |
| Found: | C, 59.59; | H, 6.00; | N, 4.23 |

### Example 255

### Preparation of 4-[2-(4-morpholinyl)-2-oxoethoxy]-alpha-oxobenzeneacetic acid.

4-[2-(4-Morpholinyl)-2-oxoethoxy]-alpha-oxobenzeneacetic acid ethyl ester (0.4 g) in methanol (70 ml) was treated with sodium carbonate (0.158 g) in water (2 ml), and the mixture was stirred at room temperature for 1.5 hours. After the solvents were removed in vacuo, dichloromethane (40 ml) and water (40 ml) were added and the mixture was acidified with 3N hydrochloric acid. Tetrahydrofuran was added to dissolve the solids, then the separated aqueous layer was extracted twice with dichloromethane-tetrahydrofuran (1:1). The combined organic layers were dried (MgSO₄), evaporated, and the residual solid was crystallized from dichloromethane-tetrahydrofuran-hexane to yield 0.36 g of 4-[2-(4-morpholinyl)-2-oxoethoxy]-alpha-oxobenzeneacetic acid, mp 182-184 °C.

| | | | |
|---|---|---|---|
| Analysis Calculated for C₁₄H₁₅NO₆: | C, 57.34; | H, 5.16; | N, 4.78 |
| Found: | C, 56.96; | H, 5.06; | N, 4.63 |

### Example 256

### Preparation of 4-[3-(4-acetyl-3-hydroxy-2-propylphenoxy)propoxy]-alpha-oxobenzeneacetic acid ethyl ester.

A mixture of 4-hydroxy-alpha-oxobenzeneacetic acid ethyl ester (0.776 g) in dimethylformamide (10 ml) under argon was treated with 55% sodium hydride (0.175 g), stirred for 15 minutes and then 1-[4-(3-bromopropoxy)-2-hydroxy-3-propylphenyl] ethanone (1.26 g) was added and rinsed in with a little dimethylformamide. The mixture was heated at 50 °C for 6 hours and worked up as in Example 20. The crude material was purified by HPLC (ethyl acetate-hexane; 1:4) to furnish 0.7 g of 4-[3-(4-acetyl-3-hydroxy-2-propylphenoxy)propoxy]-alpha-oxobenzeneacetic acid ethyl ester as an oil.

| | | |
|---|---|---|
| Analysis Calculated for C₂₄H₂₈O₇: | C, 67.28; | H, 6.59 |
| Found: | C, 66.95; | H, 6.57 |

### Example 257

### Preparation of 4-[3-(4-acetyl-3-hydroxy-2-propylphenoxy)propoxy]-alpha-oxobenzeneacetic acid.

A solution of 4-[3-(4-acetyl-3-hydroxy-2-propylphenoxy)propoxy]-alpha-oxobenzeneacetic acid ethyl ester (0.7 g) in methanol (50 ml) was treated with sodium carbonate (0.38 g) in water (2.5 ml), and the reaction was stirred at room temperature for 2 hours. After the solvents were removed in vacuo, dichloromethane (40 ml) and water (40 ml) were added and the mixture was acidified with 3N hydrochloric acid. The dried (Na₂SO₄) organic layer was evaporated, and the residue was crystallized from dichloromethane-hexane to yield in two crops, 0.435 g of 4-[3-(4-acetyl-3-hydroxy-2-propylphenoxy)propoxy]-alpha-oxobenzeneacetic acid, mp 129-130 °C.

| | | |
|---|---|---|
| Analysis Calculated for C₂₂H₂₄O₇: | C, 65.99; | H, 6.04 |
| Found: | C, 66.14; | H, 6.10 |

### Example 258

### Preparation of 4-[6-[2,3-bis-(phenylmethoxy)phenyl]hexyloxy]-alpha-oxobenzeneacetic acid ethyl ester.

A solution of 4-hydroxy-alpha-oxobenzeneacetic acid ethyl ester (0.97 g) in dimethylformamide (12 ml) was treated with 55% sodium hydride (0.22 g), stirred for 15 minutes and then 1,2-bis-(phenylmethoxy)-3-(6-bromohexyl)benzene (2.27 g) was added and rinsed in with dimethylformamide (3 ml). The mixture was stirred overnight at room temperature and worked up as in Example 20. The residual oil was purified by HPLC (ethyl acetate-hexane; 1:9) and crystallized from diethyl ether-hexane to provide 1.7 g of 4-[6-[2,3-bis-(phenylmethoxy)phenyl]hexyloxy]-alpha-oxobenzeneacetic acid ethyl ester, mp 39-42 °C.

| | | |
|---|---|---|
| Analysis Calculated for C₃₆H₃₈O₆: | C, 76.30; | H, 6.76 |
| Found: | C, 76.53; | H, 6.72 |

### Example 259

### Preparation of 4-[6-[2,3-bis-(phenylmethoxy)phenyl]hexyloxy]-alpha-oxobenzeneacetic acid.

4-[6-[2,3-bis-(Phenylmethoxy)phenyl]hexyloxy]-alpha-oxobenzeneacetic acid ethyl ester (0.5 g) in methanol (50 ml) was treated with sodium carbonate (0.121 g) in water (2 ml), and the reaction was stirred at room temperature for 3 hours. After the solvents were removed in vacuo, dichloromethane and water were added and the mixture was acidified with 3N hydrochloric acid. The dried (Na₂SO₄) organic layer was evaporated to yield 0.4 g of 4-[6-[2,3-bis-(phenylmethoxy)phenyl]hexyloxy]-alpha-oxobenzeneacetic acid as an oil.

| | | |
|---|---|---|
| Analysis Calculated for C₃₄H₃₄O₆: | C, 75.82; | H, 6.36 |
| Found: | C, 75.30; | H, 6.47 |

### Example 260

### Preparation of 4-[6-(2,3-dihydroxyphenyl)hexyloxy]-alpha-oxobenzeneacetic acid.

A mixture of 4-[6-[2,3-bis-(phenylmethoxy)phenyl]hexyloxy]-alpha-oxobenzeneacetic acid (0.3 g) in acetic acid (20 ml) containing concentrated hydrochloric acid (5 ml) was stirred at 80 °C for 2.5 hours. The cooled solution was diluted with water (100 ml) and extracted with three portions of ethyl acetate, then the combined extracts were dried (Na₂SO₄), and evaporated in vacuo. The residual oil was triturated in turn with cyclohexane and carbon tetrachloride to remove non polar impurities, then was crystallized from dichloromethane and recrystallized from dichloromethane-carbon tetrachloride to give 4-[6-(2,3-dihydroxyphenyl)hexyloxy]-alpha-oxobenzeneacetic acid as a colorless solid, mp 127-130 °C.

| | | |
|---|---|---|
| Analysis Calculated for C₂₀H₂₂O₇: | C, 67.03; | H, 6.19 |
| Found: | C, 66.38; | H, 6.27 |

### Example 261

### Preparation of 4-[3-(5,6-dihydro-6-oxo-5-phenanthridinyl)propoxy]-alpha-oxobenzeneacetic acid ethyl ester.

A solution of 4-hydroxy-alpha-oxobenzeneacetic acid ethyl ester (0.376 g) in dimethylformamide (5 ml) was treated with 55% sodium hydride (0.085 g), stirred for 15 minutes and then 5-(3-bromopropyl)-5,6-dihydro-6-oxophenanthridine (0.613 g) in dimethylformamide (5 ml) was added. The mixture was stirred at room temperature for 22 hours and worked up as in Example 20. The residual oil was purified by HPLC (ethyl acetate-hexane; 7:13), and crystallization of the resulting solid from ethyl acetate-tetrahydrofuran gave 0.4 g of 4-[3-(5,6-dihydro-6-oxo-5-phenanthridinyl)propoxy]-alpha-oxobenzeneacetic acid ethyl ester, mp 95-97 °C. A second crop of the title compound (0.16 g, mp 95-97 °C) was recovered from the mother liquor.

| | | | |
|---|---|---|---|
| Analysis Calculated for C₂₆H₂₃NO₅: | C, 72.71; | H, 5.40; | N, 3.26 |
| Found: | C, 72.37; | H, 5.41; | N, 3.13 |

### Example 262

### Preparation of 4-[3-(5,6-dihydro-6-oxo-5-phenanthridinyl)propoxy]-alpha-oxobenzeneacetic acid.

A solution of 4-[3-(5,6-dihydro-6-oxo-5-phenanthridinyl)propoxy]-alpha-oxobenzeneacetic acid ethyl ester (0.4 g) in hot methanol (80 ml) was treated with sodium carbonate (0.118 g) in water (1.5 ml), and the reaction was stirred at room temperature for 4 hours. After the methanol was removed in vacuo, dichloromethane and water were added and the mixture was acidified with 3N hydrochloric acid. The resulting solid was filtered off, dried and crystallized from ethyl acetate-tetrahydrofuran to yield 0.321 g of 4-[3-(5,6-dihydro-6-oxo-5-phenanthridinyl)propoxy]-alpha-oxobenzeneacetic acid in two crops, mp 202-204 °C.

| | | | |
|---|---|---|---|
| Analysis Calculated for C₂₄H₁₉NO₅: | C, 71.81; | H, 4.77; | N, 3.49 |
| Found: | C, 71.36; | H, 4.64; | N, 3.25 |

### Example 263

### Preparation of 4-[3-(1,2-dihydro-2-oxo-1-quinolinyl)propoxy]-alpha-oxobenzeneacetic acid ethyl ester.

A solution of 4-hydroxy-alpha-oxobenzeneacetic acid ethyl ester (0.73 g) in dimethylformamide (10 ml) was treated with 55% sodium hydride (0.164 g), stirred for 15 minutes and then 1-(3-bromopropyl)-1,2-dihydro-2-oxoquinoline (1 g) was added and rinsed in with dimethylformamide (4 ml). The mixture was stirred at room temperature for 22 hours and worked up as in Example 20. The residual solid was purified by flash chromatography over silica gel (ethyl acetate-hexane; 1:4). Crystallization of the resulting solid furnished 1.0.g of 4-[3-(1,2-dihydro-2-oxo-1-quinolinyl)propoxy]-alpha-oxobenzeneacetic acid ethyl ester, mp 108-109 °C.

| | | | |
|---|---|---|---|
| Analysis Calculated for C₂₂H₂₁NO₅: | C, 69.69; | H, 5.58; | N, 3.69 |
| Found: | C, 69.78; | H, 5.54; | N, 3.63 |

### Example 264

### Preparation of 4-[3-(1,2-dihydro-2-oxo-1-quinolinyl)propoxy]-alpha-oxobenzeneacetic acid.

A solution of 4-[3-(1,2-dihydro-2-oxo-1-quinolinyl)propoxy]-alpha-oxobenzeneacetic acid ethyl ester (0.5 g) in hot methanol (50 ml) was treated with sodium carbonate (0.14 g) in water (1.5 ml), and the reaction was stirred at room temperature for 5 hours. After the methanol was removed in vacuo, dichloromethane and water were added and the mixture was acidified with 3N hydrochloric acid. The precipitated solid was filtered off, dried and then crystallized from ethyl acetate-tetrahydrofuran to afford 0.337 g of 4-[3-(1,2-dihydro-2-oxo-1-quinolinyl)propoxy]-alpha-oxobenzeneacetic acid, mp 183-186 °C.

| | | | |
|---|---|---|---|
| Analysis Calculated for C₂₀H₁₇NO₅: | C, 68.37; | H, 4.88; | N, 3.39 |
| Found: | C, 68.11; | H, 4.88; | N, 3.74 |

### Example 265

### Preparation of 4-[5-[3,5-bis(1,1-dimethylethyl)-4-hydroxyphenyl]-5-oxopentyloxy]-alpha-oxobenzeneacetic acid ethyl ester.

4-Hydroxy-alpha-oxobenzeneacetic acid ethyl ester (1.44 g) and 55% sodium hydride (0.36g) in dimethylformamide (30 ml) was stirred for 20 minutes and then treated with 5-bromo-1-[3,5-bis(1,1-dimethylethyl)-4-[(2-methoxyethoxy)methoxy] phenyl]pentanone (3.4 g) in dimethylformamide (6 ml). The mixture was stirred at room temperature for 20 hours and then after an additional 4 hours at 50 °C, it was worked up as in Example 20. The residual oil was purified by HPLC (ethyl acetate-toluene; 1:17) to give 2.7 g of 4-[5-[3,5-bis(1,1-dimethylethyl)-4-[(2-methoxyethoxy) methoxy]phenyl]-5-oxopentyloxy]-alpha-oxobenzeneacetic acid ethyl ester as an oil. A solution of the above 4-[5-[3,5-bis(1,1-dimethylethyl)-4-[(2-methoxyethoxy) methoxy]phenyl]-5-oxopentyloxy]-alpha-oxobenzeneacetic acid ethyl ester (2.1 g) was dissolved in dichloromethane (20 ml) containing trifluoroacetic acid (1.1 ml) and was stirred at room temperature for 31 hours, then saturated sodium bicarbonate solution was added (25 ml). The separated organic layer was dried (MgSO₄) and evaporated and the crude product was purified by HPLC (ethyl acetate-hexane; 4:21). Trituration of the resulting material with hexane afforded 1.4 g of 4-[5-[3,5-bis(1,1-dimethylethyl) -4-hydroxyphenyl]-5-oxopentyloxy]-alpha-oxobenzeneacetic acid ethyl ester, mp 95-96 °C.

| | | |
|---|---|---|
| Analysis Calculated for C₂₉H₃₈O₆: | C, 72.17; | H, 7.94 |
| Found: | C, 72.01; | H, 8.05 |

### Example 266

### Preparation of 4-[5-[3,5-bis(1,1-dimethylethyl)-4-hydroxyphenyl]-5-oxopentyloxy]-alpha-oxobenzeneacetic acid.

As in example 10, a solution of 4-[5-[3,5-bis(1,1-dimethylethyl)-4-hydroxyphenyl]-5-oxopentyloxy]-alpha-oxobenzeneacetic acid ethyl ester (0.8 g) in hot methanol (80 ml) was treated with 2N sodium hydroxide (1.75 ml) then stirred at room temperature for 75 minutes. The methanol was removed in vacuo, then the mixture was acidified with 3N hydrochloric acid and extracted with dichloromethane-tetrahydrofuran (2:1). The dried (MgSO₄) extracts were evaporated and the solid residue was triturated from hexane, and then crystallized from acetone-hexane to provide 0.7 g of 4-[5-[3,5-bis(1,1-dimethylethyl)-4-hydroxyphenyl]-5-oxopentyloxy]-alpha-oxobenzeneacetic acid, mp 144-145 °C.

| | | |
|---|---|---|
| Analysis Calculated for C₂₇H₃₄O₆: | C, 71.34; | H, 7.54 |
| Found: | C, 71.56; | H, 7.61 |

### Example 267

### Preparation of 4-[3-[3-hydroxy-4-(methoxycarbonyl)-2-(propylphenoxy)]propoxy]-alpha-oxobenzeneacetic acid ethyl ester.

A mixture of 4-hydroxy-alpha-oxobenzeneacetic acid ethyl ester (0.47 g) and 55% sodium hydride (0.116 g) in dimethylformamide (10 ml) was stirred for 15 minutes and then treated with 4-(3-bromopropoxy)-2-hydroxy-3-propylbenzoic acid methyl ester (0.8 g) in dimethylformamide (5 ml). The mixture was heated at 50 °C for 20 hours and worked up as in Example 20. The residual oil was purified by flash chromatography over silica gel (ethyl acetate-hexane; 3:17) and then was crystallized from hexane to give 0.6 g of 4-[3-[3-hydroxy-4-(methoxycarbonyl)-2-(propylphenoxy)]propoxy]-alpha-oxobenzeneacetic acid ethyl ester, mp 62-64 °C.

| | | |
|---|---|---|
| Analysis Calculated for C₂₄H₂₈O₈: | C, 64.85; | H, 6.35 |
| Found: | C, 64.90; | H, 6.10 |

### Example 268

### Preparation of 4-[3-[3-hydroxy-4-(methoxycarbonyl)-2-(propylphenoxy)]propoxy]-alpha-oxobenzeneacetic acid.

As in Example 19, a solution of 4-[3-[3-hydroxy-4-(methoxycarbonyl)-2-(propylphenoxy)]propoxy]-alpha-oxobenzeneacetic acid ethyl ester (0.3 g) in methanol (30 ml) was treated with 4N sodium hydroxide (0.53 ml) and then was stirred at room temperature for 1 hour. Most of the solvents were removed in vacuo, then the mixture was acidified with 3N hydrochloric acid and extracted with ethyl acetate. The dried (MgSO₄) extracts were evaporated and the crude acid was crystallized from a carbon tetrachloride-hexane mixture containing a small amount of dichloromethane to yield 0.164 g of 4-[3-[3-hydroxy-4-(methoxycarbonyl)-2-(propylphenoxy)]propoxy]-alpha-oxobenzeneacetic acid, as a colorless solid, mp 86-87 °C.

| | | |
|---|---|---|
| Analysis Calculated for C₂₂H₂₄O₈: | C, 63.45; | H, 5.81 |
| Found: | C, 63.45; | H, 5.68 |

### Example 269

### Preparation of 4-[3-[4-carboxy-3-hydroxy-2-(propylphenoxy)]propoxy]-alpha-oxobenzeneacetic acid.

A solution of 4-[3-[3-hydroxy-4-(methoxycarbonyl)-2-(propylphenoxy)]propoxy]-alpha-oxobenzeneacetic acid (0.3 g) in methanol (30 ml) was treated with 4N sodium hydroxide (0.58 ml) and then stirred at reflux for 46 hours. The methanol was removed in vacuo, then the mixture was acidified with 3N hydrochloric acid and extracted with dichloromethane-tetrahydrofuran (1:1). The dried (MgSO₄) extracts were evaporated and the solid residue was crystallized from dichloromethane-hexane, and then from acetone-hexane to afford 4-[3-[4-carboxy-3-hydroxy-2-(propylphenoxy)]propoxy]-alpha-oxobenzeneacetic acid, mp 182-185 °C.

| | | |
|---|---|---|
| Analysis Calculated for C₂₁H₂₂O₈: | C, 62.68; | H, 5.51 |
| Found: | C, 62.46; | H, 5.27 |

### Example 270

### Preparation of 4-[3-[4-acetyl-3-methoxy-2-(propylphenoxy)]propoxy]-alpha-oxobenzeneacetic acid ethyl ester.

A solution of 4-hydroxy-alpha-oxobenzeneacetic acid ethyl ester (0.471 g) in dimethylformamide (10 ml) was treated with 55% sodium hydride (0.117 g), stirred for 15 minutes and then a solution of with 1-[4-(3-bromopropoxy)-2-methoxy-3-propylphenyl]ethanone (0.8 g) in dimethylformamide (5 ml) was added. The mixture was heated at 50 °C for 17 hours and worked up as in Example 20. The residual oil was purified by HPLC (ethyl acetate-hexane; 1:4), followed by flash chromatography over silica gel (ethyl acetate-toluene; 1:19) to yield 0.4 g of 4-[3-[4-acetyl-3-methoxy-2-(propylphenoxy)]propoxy]-alpha-oxobenzeneacetic acid ethyl ester as an oil.

| | | |
|---|---|---|
| Analysis Calculated for C₂₅H₃₀O₇: | C, 67.86; | H, 6.83 |
| Found: | C, 67.81; | H, 7.00 |

### Example 271

### Preparation of 4-[3-[4-acetyl-3-methoxy-2-(propylphenoxy)]propoxy]-alpha-oxobenzeneacetic acid.

As in example 10, a solution of 4-[3-[4-acetyl-3-methoxy-2-(propylphenoxy)]propoxy]-alpha-oxobenzeneacetic acid ethyl ester (0.4 g) in methanol (30 ml) was treated with 2N sodium hydroxide (0.25 ml) then stirred at room temperature for 4 hours. Most of the solvents were removed in vacuo, water was added, then the mixture was acidified with 3N hydrochloric acid and extracted with dichloromethane-tetrahydrofuran (1:1). The dried (MgSO₄) extracts were evaporated and the crude oil was purified by flash chromatography over silica gel (toluene-ethyl acetate-acetic acid; 75:25:0.5 increasing to 74:25:1) and crystallized from diethyl ether-hexane to provide 0.13g of 4-[3-[4-acetyl-3-methoxy-2-(propylphenoxy)]propoxy]-alpha-oxobenzeneacetic acid, mp 94-96 °C.

| | | |
|---|---|---|
| Analysis Calculated for C₂₃H₂₆O₇: | C, 66.65; | H, 6.32 |
| Found: | C, 66.45; | H, 6.42 |

### Example 272

### Preparation of 4-[3-(4-acetyl-3-hydroxyphenoxy)propoxy]-alpha-oxobenzeneacetic acid ethyl ester.

4-Hydroxy-alpha-oxobenzeneacetic acid ethyl ester (1.2 g) in dimethylformamide (20 ml) under argon was stirred with 55% sodium hydride (0.299 g) for 15 minutes and then was treated with 1-[4-(3-bromopropoxy)-2-hydroxyphenyl]ethanone (1.7 g) in dimethylformamide (4 ml). The mixture was heated at 50 °C for 5 hours and worked up as in Example 20. The material was purified by HPLC (ethyl acetate-hexane; 1:39) to provide 0.8 g of an oil that was triturated with hexane to give 0.7 g of 4-[3-(4-acetyl-3-hydroxyphenoxy)propoxy]-alpha-oxobenzeneacetic acid ethyl ester as a colorless solid, mp 63-65 °C.

| | | |
|---|---|---|
| Analysis Calculated for C₂₁H₂₂O₇: | C, 65.28; | H, 5.74 |
| Found: | C, 65.23; | H, 5.68 |

### Example 273

### Preparation of 4-[3-(4-acetyl-3-hydroxyphenoxy)propoxy]-alpha-oxobenzeneacetic acid.

As in example 10, 4-[3-(4-acetyl-3-hydroxyphenoxy)propoxy]-alpha-oxobenzeneacetic acid ethyl ester (0.7 g) in methanol (70 ml) was treated with a solution of sodium carbonate (0.42 g) in water and brought just to reflux temperature, then was allowed to cool to room temperature. After 1 hour, most of the methanol was removed in vacuo, then the mixture was diluted with water, acidified with 3N hydrochloric acid and extracted twice with dichloromethane-tetrahydrofuran (1:1). Evaporation of the dried (MgSO₄) extracts and crystallization of the solid residue from dichloromethane-hexane afforded 0.58 g of 4-[3-(4-acetyl-3-hydroxyphenoxy)propoxy]-alpha-oxobenzeneacetic acid, mp 133-135 °C.

| | | |
|---|---|---|
| Analysis Calculated for C₁₉H₁₈O₇: | C, 63.68; | H, 5.06 |
| Found: | C, 63.11; | H, 5.03 |

### Example 274

### Preparation of 4-[3-[[3,5-bis(1,1-dimethylethyl)-4-hydroxyphenyl]thio]propoxy]-alpha-oxobenzeneacetic acid ethyl ester.

A mixture of 4-hydroxy-alpha-oxobenzeneacetic acid ethyl ester (0.33 g) in dimethylformamide (6 ml) under argon was treated with 55% sodium hydride (0.082 g), stirred for 15 minutes then 3,5-bis(1,1-dimethylethyl)-4-[(3-bromopropyl)thio] phenol (0.61 g) was added. The mixture was heated at 50 °C for 20 hours and worked up as in Example 20. The residual oil was purified by flash chromatography over silica gel (ethyl acetate-hexane; 1:19) to provide 0.8 g of an oil that was triturated with hexane to give 0.331 g of 4-[3-[[3,5-bis(1,1-dimethylethyl)-4-hydroxyphenyl]thio]propoxy]-alpha-oxobenzeneacetic acid ethyl ester.

### Example 275

### Preparation of 4-[3-[[3,5-bis(1,1-dimethylethyl)-4-hydroxyphenyl]thio]propoxy]-alpha-oxobenzeneacetic acid.

As in Example 19, a solution of 4-[3-[[3,5-bis(1,1-dimethylethyl)-4-hydroxyphenyl] thio]propoxy]-alpha-oxobenzeneacetic acid ethyl ester (0.32 g) in methanol (15 ml) was treated with 2N sodium hydroxide (0.75 ml) then stirred at room temperature. After 3 hours, most of the methanol was removed in vacuo, then the mixture was acidified with 3N hydrochloric acid and extracted with dichloromethane-tetrahydrofuran (1:1). The dried (MgSO₄) extracts were evaporated and the crude product was purified by flash chromatography over silica gel (toluene-ethyl acetate-acetic acid; 80:20:0.25 increasing to 74:25:1) to give 4-[3-[[3,5-bis(1,1-dimethylethyl)-4-hydroxyphenyl] thio]propoxy]-alpha-oxobenzeneacetic acid as an oil.

| | | | |
|---|---|---|---|
| Analysis Calculated for C₂₅H₃₂O₅S: | C, 67.54; | H, 7.25; | N, 7.21 |
| Found: | C, 67.32; | H, 7.09; | N, 7.31 |

### Example 276

### Preparation of rac-delta-hydroxy-2-naphthalenebutanol

A solution of diborane in tetrahydrofuran (1M; 120 ml) was added dropwise with stirring to a cooled (-10 °C) solution of gamma-oxo-2-naphthalenebutanoic acid (10 g) in dry tetrahydrofuran (120 ml). The stirred mixture was maintained at ice bath temperature for 3 hours before the slow addition of a 10% solution of acetic acid in ethyl acetate (100 ml). The solvents were removed in vacuo and the residue partitioned between ethyl acetate (150 ml) and 1N hydrochloric acid (100 ml). The separated aqueous layer was extracted with ethyl acetate (50 ml), then the organic layers were washed in turn with 1N hydrochloric acid (2 x 100 ml) and brine. Evaporation of the combined, dried (MgSO₄) extracts gave 7.3 g of crude diol that was first purified by HPLC (ethyl acetate-hexane; 4:1) and then crystallized from ethyl acetate-hexane to provide 4.11 g of rac-delta-hydroxy-2-naphthalenebutanol as a colorless solid.

### Example 277

### Preparation of rac.alpha-(3-mesyloxypropyl)-2-naphthalenemethanol.

Methanesulfonyl chloride (0.77 g was added slowly with stirring to a cooled (-40 °C) solution of rac-delta-hydroxy-2-naphthalenebutanol (1.45 g) in dry pyridine (6 ml). The mixture was stirred at -40 °C for 5 hours, then was poured into water (20 ml) and extracted with ethyl acetate (3 x 10 ml). The combined organic layers were washed in turn with water (2 x 10 ml), saturated magnesium sulfate solution (3 x 15 ml) and brine, then were dried (MgSO₄) and evaporated. The resulting oil was purified by HPLC (ethyl acetate-hexane; 2:3) to provide 1.25 g of rac-alpha-(3-mesyloxypropyl)-2-naphthalenemethanol as an oil.

### Example 278

### Preparation of rac-4-[4-[[(1,1-dimethylethyl)dimethylsilyl]oxy]-4-(2-naphthalenyl)butoxy]-alpha-oxobenzeneacetic acid methyl ester.

A mixture of rac-alpha-(3-mesyloxypropyl)-2-naphthalenemethanol (1.25 g), t-butyldimethylsilyl chloride (0.768 g) and imidazole (0.723 g) in dry dimethylformamide (14 ml) was stirred overnight at room temperature. The solvents were evaporated off and the residue was taken up in water (30 ml) and extracted with diethyl ether (3 x 8 ml). The combined organic layers were washed with brine (1 x 5 ml), then were dried (MgSO₄) and evaporated to give 1.5 g of crude product. Purification of the material by HPLC (ethyl acetate-hexane; 3:17) furnished 1.25 g of rac-alpha-(3-mesyloxypropyl)-2-naphthalenemethanol protected as its t-butyldimethylsilyl ether derivative.
4-Hydroxy-alpha-oxobenzeneacetic acid methyl ester (0.611 g) in dimethylformamide (10 ml) under argon was treated with 55% sodium hydride (0.149 g), stirred for 30 minutes and then the above t-butyldimethylsilyl ether derivative (1.25 g) in dimethylformamide (5 ml). The mixture was stirred at 60 °C overnight and worked up as in Example 20 and the isolated material (1.25 g) was purified by flash chromatography over silica gel (ethyl acetate-hexane; 1:9) to provide of 1.11 g of rac-4-[4-[[(1,1-dimethylethyl)dimethylsilyl]oxy]-4-(2-naphthalenyl)butoxy]-alpha-oxobenzeneacetic acid methyl ester as an oil.

| | | |
|---|---|---|
| Analysis Calculated for C₂₉H₃₆O₅Si: | C, 70.70; | H, 7.37 |
| Found: | C, 70.69; | H, 7.23 |

### Example 279

### Preparation of rac-4-[4-hydroxy-4-(2-naphthalenyl)butoxy]-alpha-oxobenzeneacetic acid methyl ester.

A solution of rac-4-[4-[[(1,1-dimethylethyl)dimethylsilyl]oxy]-4-(2-naphthalenyl) butoxy]-alpha-oxobenzeneacetic acid methyl ester (1.0 g) in acetonitrile (22.5 ml) containing 48% aqueous hydrofluoric acid solution (2.5 ml) was stirred at room temperature for 1 hour. The reaction mixture was poured into saturated sodium bicarbonate solution and extracted with ethyl acetate (3 x 10 ml). The combined organic extracts were dried (MgSO₄) and evaporated to provide 0.76 g of crude product that was purified by flash chromatography over silica gel (ethyl acetate-hexane; 7:13) to yield 0.554 g of rac-4-[4-hydroxy-4-(2-naphthalenyl)butoxy]-alpha-oxobenzeneacetic acid methyl ester as an oil.

| | | |
|---|---|---|
| Analysis Calculated for C₂₃H₂₂O₅: | C, 73.00; | H, 5.86 |
| Found: | C, 72.89; | H, 5.70 |

### Example 280

### Preparation of rac-4-[4-hydroxy-4-(2-naphthalenyl)butoxy]-alpha-oxobenzeneacetic acid.

As in Example 19, a stirred solution of rac-4-[4-hydroxy-4-(2-naphthalenyl)butoxy]-alpha-oxobenzeneacetic acid methyl ester (0.172 g) in warm methanol (2.5 ml) was treated with 1N sodium hydroxide (1 ml) and then water (25 ml) was added. After the methanol was removed in vacuo, the mixture was acidified with 1N hydrochloric acid (1.2 ml) and extracted with dichloromethane (1 x 50 ml, 1 x 10 ml). The dried (MgSO₄) extracts were concentrated and the residual gummy solid (∼0.17 g) was crystallized from acetone-hexane to give 0.06 g of rac-4-[4-hydroxy-4-(2-naphthalenyl) butoxy]-alpha-oxobenzeneacetic acid as a colorless solid, mp 108-110 °C.

| | | |
|---|---|---|
| Analysis Calculated for C₂₂H₂₀O₅: | C, 72.51; | H, 5.53 |
| Found: | C, 73.21; | H, 6.09 |

### Example 281

### Preparation of 4-[[4-(2-naphthalenyl)-4-oxobutyl]oxy]-alpha-oxobenzeneacetic acid methyl ester.

A solution of dimethylsulfoxide (0.079 g) in dichloromethane (2 ml) was added over 20 minutes to a stirred solution of oxalyl chloride (0.123 g) in dry dichloromethane (4 ml) maintained throughout at -78 °C by using an acetone-dry ice bath. After 30 minutes at -78 °C, a solution of rac-4-[4-hydroxy-4-(2-naphthalenyl)butoxy]-alpha-oxobenzeneacetic acid methyl ester (0.356 g) in dichloromethane (3 ml) was added over 30 minutes and the reaction mixture was stirred for 30 minutes in the acetone-dry ice bath, then triethylamine (0.144 ml) was added. The cooling bath was removed and the reaction was allowed to equilibrate to room temperature, then water was added and the phases were separated. After the aqueous layer was extracted with dichloromethane (2 x 7 ml), the combined organic layers were washed with brine, then were dried (MgSO₄) and evaporated. The residue was purified by flash chromatography over silica gel (ethyl acetate-hexane; 1:3) and then crystallized from ethyl acetate-hexane to furnish in two crops, 0.299 g of 4-[[4-(2-naphthalenyl)-4-oxobutyl]oxy]-alpha-oxobenzeneacetic acid methyl ester, mp 86-87 °C.

| | | |
|---|---|---|
| Analysis Calculated for C₂₃H₂₂O₅: | C, 73.39; | H, 5.36 |
| Found: | C, 73.49; | H, 5.33 |

### Example 282

### Preparation of 4-[[4-(2-naphthalenyl)-4-oxobutyl]oxy]-alpha-oxobenzeneacetic acid.

As in Example 19, a stirred solution of 4-[[4-(2-naphthalenyl)-4-oxobutyl]oxy]-alpha-oxobenzeneacetic acid methyl ester (0.299 g) in hot methanol (2.5 ml) was treated with 1N sodium hydroxide (1.75 ml) and then water (25 ml) was added. After the methanol was removed in vacuo, the mixture was acidified with 1N hydrochloric acid (2.1 ml) and extracted with dichloromethane (1 x 40 ml, 1 x 20 ml). The dried (Na₂SO₄) extracts were evaporated and the residue was crystallized from acetone-hexane to give 0.179 g of 4-[[4-(2-naphthalenyl)-4-oxobutyl]oxy]-alpha-oxobenzeneacetic acid as a colorless solid, mp 133-135 °C.

| | | |
|---|---|---|
| Analysis Calculated for C₂₂H₂₀O₅: | C, 72.92; | H, 5.01 |
| Found: | C, 73.95; | H, 6.05 |

### Example 283

### Preparation of 4-[2-(1-naphthalenyl)-2-oxoethoxy]-alpha-oxobenzeneacetic acid methyl ester (4:1) molar hydrate.

Sodium hydride (55%; 0.238 g) and 4-hydroxy-alpha-oxobenzeneacetic acid methyl ester (0.978 g) in dimethylformamide (12 ml) were stirred at room temperature for 30 minutes and then 2-chloro-1-(1-naphthalenyl)ethanone (1 g) in dimethylformamide (6 ml) was added. The reaction mixture was stirred at 60 °C overnight and worked up as in Example 20. The crude product (1.7 g) was purified by flash chromatography over silica gel (ethyl acetate-hexane-dichloromethane; 2.5:37.5:60) to afford of 0.277 g of 4-[2-(1-naphthalenyl)-2-oxoethoxy]-alpha-oxobenzeneacetic acid methyl ester. Crystallization of the ester from ethyl acetate-hexane gave the analytical specimen, mp 99-100.5 °C.

| | | |
|---|---|---|
| Analysis Calculated for C₂₁H₁₆O₅.4:1H₂O: | C, 71.48; | H, 4.71 |
| Found: | C, 71.49; | H, 4.74 |

### Example 284

### Preparation of 4-[2-(1-naphthalenyl)-2-oxoethoxy]-alpha-oxobenzeneacetic acid

To a hot solution of 4-[2-(1-naphthalenyl)-2-oxoethoxy]-alpha-oxobenzeneacetic acid methyl ester (0.128 g) in methanol (3 ml) and tetrahydrofuran (0.5 ml) was added 1N sodium hydroxide (0.8 ml) followed by water (15 ml). The solvents were removed under reduced pressure, then the solution was acidified with 1N hydrochloric acid (1 ml) and extracted with dichloromethane (1 x 20 ml, 1 x 10 ml). The combined organic layers were washed with water, dried (MgSO₄) and evaporated and the resulting yellow solid (0.107 g) was crystallized from ethyl acetate-hexane to provide 0.063 g of 4-[2-(1-naphthalenyl)-2-oxoethoxy]-alpha-oxobenzeneacetic acid, mp 141-143 °C.

| | | |
|---|---|---|
| Analysis Calculated for C₂₀H₁₄O₅: | C, 71.85; | H, 4.22 |
| Found: | C, 72.01; | H, 4.30 |

### Example 285

### Preparation of 4-[2-[4-(1,1-dimethylethyl)phenyl]-2-oxoethoxy]-alpha-oxobenzeneacetic acid methyl ester.

A mixture of sodium hydride (55%; 0.224 g) and 4-hydroxy-alpha-oxobenzeneacetic acid methyl ester (0.918 g) in dimethylformamide (12 ml) was stirred at room temperature for 30 minutes and then 2-chloro-1-[4-(1,1-dimethylethyl)phenyl]ethanone (1 g) in dimethylformamide (6 ml) was added. The reaction mixture was stirred at 60 °C overnight and worked up as in Example 20. The crude product (1.97 g) was purified initially by HPLC (ethyl acetate-hexane; 1:3) to give 0.79 g of material that was further purified by flash chromatography over silica gel (ethyl acetate-hexane-dichloromethane; 3:37:60) to afford of 0.535 g of 4-[2-[4-(1,1-dimethylethyl)phenyl]-2-oxoethoxy]-alpha-oxobenzeneacetic acid methyl ester as an oil.

| | | |
|---|---|---|
| Analysis Calculated for C₂₁H₂₂O₅: | C, 71.17; | H, 6.36 |
| Found: | C, 70.89; | H, 6.36 |

### Example 286

### Preparation of 4-[2-[4-(1,1-dimethylethyl)phenyl]-2-oxoethoxy]-alpha-oxobenzeneacetic acid.

A solution of 4-[2-[4-(1,1-dimethylethyl)phenyl-2-oxoethoxy]-alpha-oxobenzeneacetic acid methyl ester (0.354 g) in hot methanol (10 ml) was treated with 1N sodium hydroxide (2.2 ml) and then diluted with water (30 ml). After the methanol was removed in vacuo, the solution was acidified with 1N hydrochloric acid (2.7 ml) and extracted with dichloromethane (1 x 70 ml, 1 x 15 ml). The combined organic layers were washed with water, dried (MgSO₄) and evaporated and the residual material (0.296 g) was crystallized from ethyl acetate-hexane to furnish 0.217 g of 4-[2-[4-(1,1-dimethylethyl)phenyl]-2-oxoethoxy]-alpha-oxobenzeneacetic acid, mp 132-134 °C.

| | | |
|---|---|---|
| Analysis Calculated for C₂₀H₂₀O₅: | C, 70.58; | H, 5.92 |
| Found: | C, 71.12; | H, 2.92 |

### Example 287

### Preparation of 4-[2-[[1,1'-biphenyl]-2-yl]-2-oxoethoxy]-alpha-oxobenzeneacetic acid methyl ester.

4-Hydroxy-alpha-oxobenzeneacetic acid methyl ester (0.721 g) in dimethylformamide (8 ml) under argon was treated with 55% sodium hydride (0.175 g), stirred for 30 minutes and then 1-[1,1'-biphenyl]-2-yl-2-bromoethanone (1.1 g) in dimethylformamide (4 ml) was added. The mixture was stirred at room temperature overnight and worked up as in Example 20. The residual oil (1.5 g) was purified by flash chromatography over silica gel (dichloromethane) to afford of 0.62 g of 4-[2-[[1,1'-biphenyl]-2-yl]-2-oxoethoxy]-alpha-oxobenzeneacetic acid methyl ester as an oil.

| | | |
|---|---|---|
| Analysis Calculated for C₂₃H₁₈O₅: | C, 73.79; | H, 4.85 |
| Found: | C, 74.04; | H, 4.82 |

### Example 288

### Preparation of 4-[2-[[1,1'-biphenyl]-2-yl]-2-oxoethoxy]-alpha-oxobenzeneacetic acid.

A solution of 4-[-2-[[1,1'-biphenyl]-2-yl]-2-oxoethoxy]-alpha-oxobenzeneacetic acid methyl ester (0.563 g) in warm methanol (5 ml) was treated with 1N sodium hydroxide (3.3 ml). Within 5 minutes water (30 ml) was added, then after the methanol was removed in vacuo, the mixture was acidified with 1N hydrochloric acid (4.1 ml) and extracted with dichloromethane (1 x 60 ml, 2 x 10 ml). The combined organic layers were washed with water, then dried (MgSO₄) and evaporated. The resulting yellow foam was taken up in diethyl ether, and after the mixture was filtered to remove some insoluble material, the filtrate was evaporated and the residue was lyophilized from benzene to furnish 0.36 g of 4-[2-[[1,1'-biphenyl]-2-yl]-2-oxoethoxy]-alpha-oxobenzeneacetic acid.

| | | |
|---|---|---|
| Analysis Calculated for C₂₂H₁₆O₅: | C, 73.33; | H, 4.48 |
| Found: | C, 73.54; | H, 4.53 |

### Example 289

### Preparation of 4-(2-cyclooctyl-2-oxoethoxy)-alpha-oxobenzeneacetic acid ethyl ester.

55% Sodium hydride (0.14 g) was stirred with 4-hydroxy-alpha-oxobenzeneacetic acid ethyl ester (0.622 g) in dimethylformamide (8 ml) under argon for 30 minutes and then the solution was treated with 2-chloro-1-cyclooctylethanone (0.53 g) in dimethylformamide (3 ml). The mixture, stirred at room temperature overnight and then at 60°C for 1 hour, was worked up as in Example 20. The residual oil was purified by flash chromatography over silica gel (3% ethyl acetate in dichloromethane-hexane 1:1) to provide of 0.399 g of 4-(2-cyclooctyl-2-oxoethoxy)-alpha-oxobenzeneacetic acid ethyl ester as an oil.

| | | |
|---|---|---|
| Analysis Calculated for C₂₀H₂₆O₅: | C, 69.34; | H, 7.57 |
| Found: | C, 69.02.; | H, 7.60 |

### Example 290

### Preparation of 4-[2-(cyclooctyl)-2-oxoethoxy]-alpha-oxobenzeneacetic acid.

As in example 19, a stirred solution of 4-(2-cyclooctyl-2-oxoethoxy)-alpha-oxobenzeneacetic acid ethyl ester (0.318 g) in warm methanol (5 ml) was treated with 1N sodium hydroxide (2 ml). Within 5 minutes water (20 ml) was added, then after the methanol was removed in vacuo, the mixture was acidified with 1N hydrochloric acid (2.5 ml) and extracted with dichloromethane (1 x 40 ml, 1 x 10 ml). The dried (MgSO₄) extracts were evaporated and the resulting solid (0.255 g) was crystallized from diethyl ether-hexane to give 0.178 g of 4-[2-(cyclooctyl)-2-oxoethoxy]-alpha-oxobenzeneacetic acid as a colorless solid, mp 109-110 °C.

| | | |
|---|---|---|
| Analysis Calculated for C₁₈H₂₂O₅: | C, 67.91; | H, 6.97 |
| Found: | C, 67.75; | H, 6.97 |

### Example 291

### Preparation of 4-[2-oxo-2-(2,4,6-trimethylphenyl)ethoxy]-alpha-oxobenzeneacetic acid ethyl ester.

4-Hydroxy-alpha-oxobenzeneacetic acid ethyl ester (0.583 g) in dimethylformamide (6 ml) under argon was treated with 55% sodium hydride (0.131 g), stirred for 30 minutes and then 2-bromo-(2,4,6-triphenyl)ethanone (0.723 g) in dimethylformamide (2 ml) was added. The reaction mixture was stirred at room temperature overnight and worked up as in Example 20. The isolated material (1.15 g) was purified by flash chromatography over silica gel (dichloromethane) to yield of 0.468 g of 4-[2-oxo-2-(2,4,6-trimethylphenyl)ethoxy]-alpha-oxobenzeneacetic acid ethyl ester as an oil which solidified on standing, mp 68.5-70 °C.

| | | |
|---|---|---|
| Analysis Calculated for C₂₁H₂₂O₅: | C, 71.17; | H, 6.26 |
| Found: | C, 70.88; | H, 6.17 |

### Example 292

### Preparation of 4-[2-oxo-2-(2,4,6-trimethylphenyl)ethoxy]-alpha-oxobenzeneacetic acid.

A solution of 4-[2-oxo-2-(2,4,6-trimethylphenyl]ethoxy]-alpha-oxobenzeneacetic acid ethyl ester (0.357 g) in hot methanol (5 ml) was treated with 1N sodium hydroxide (2 ml). Water (20 ml) was added, then after the methanol was removed in vacuo, the solution was acidified with 1N hydrochloric acid (2.5 ml) and extracted with dichloromethane (1 x 40 ml, 1 x 7 ml). The combined organic layers were washed with brine, then dried (MgSO₄) and evaporated. The resulting residue was crystallized from diethyl ether-hexane to furnish 0.242 g of 4-[2-oxo-2-(2,4,6-trimethyl-phenyl)ethoxy]-alpha-oxobenzeneacetic acid, mp 144-145 °C

| | | |
|---|---|---|
| Analysis Calculated for C₁₉H₁₈O₅: | C, 69.93; | H, 5.56 |
| Found: | C, 70.01; | H, 5.66 |

### Example 293

### Preparation of 4-[3-[4-(2-chlorophenyl)-9-methyl-6H-thieno[3,2-f][1,2,4]triazolo[4,3-a][1,4]diazepin-2-yl]-2-propynyloxy]-alpha-oxobenzeneacetic acid ethyl ester (4:1) hydrate.

As in Example 15, 4-(-2-chlorophenyl)-2-(3-hydroxy-1-propynyl)-9-methyl-6H-thieno[3,2-f][1,2,4]triazolo[4,3-a][1,4]diazepine (0.369 g) was reacted with 4-hydroxyphenylglyoxylic acid ethyl ester (0.195 g) in the presence of diethyl azodicarboxylate (0.175 g) and triphenylphosphine (0.263 g) in dichloromethane (30 ml). The crude reaction product, isolated in the usual manner, was purified by flash chromatography over silica gel (120 g; ethanol-dichloromethane; 3.5:96.5) to furnish 0.398 g of 4-[3-[4-(2-chlorophenyl)-9-methyl-6H-thieno[3,2-f][1,2,4]triazolo[4,3-a][1,4]diazepin-2-yl]-2-propynyloxy]-alpha-oxobenzeneacetic acid-ethyl-ester (4:1) hydrate as a white foam.

| | | | | | |
|---|---|---|---|---|---|
| Analysis Calculated for C₂₈H₂₁ClN₄O₄S.4:1H₂O: | C, 61.20; | H, 3.94; | Cl, 6.45; | N, 10.20; | S, 5.83 |
| Found: | C, 61.13; | H, 3.94; | Cl, 6.66; | N, 10.24; | S, 5.65 |

### Example 294

### Preparation of 4-[3-[4-(2-chlorophenyl)-9-methyl-6H-thieno[3,2-f][1,2,4]triazolo[4,3-a][1,4]diazepin-2-yl]-2-propynyloxy]-alpha-oxobenzeneacetic acid (20:9) dichloromethane solvate.

A solution of 4-[3-[4-(2-chlorophenyl)-9-methyl-6H-thieno[3,2-f][1,2,4]triazolo[4,3-a][1,4]diazepin-2-yl]-2-propynyloxy]-alpha-oxobenzeneacetic acid ethyl ester (4:1) hydrate (0.306 g) in hot methanol (4 ml) was treated with 1N sodium hydroxide (1.8 ml) and then diluted with water (18 ml). After the methanol was removed in vacuo, the solution was acidified with 1N hydrochloric acid (2.2 ml) and extracted with dichloromethane (1 x 40 ml, 1 x 10 ml). The combined organic layers were washed with brine, dried (MgSO₄) and evaporated to furnish 0.276 g of 4-[3-[4-(2-chlorophenyl)-9-methyl-6H-thieno[3,2-f][1,2,4]triazolo[4,3-a][1,4]diazepin-2-yl]-2 propoxy]-alpha-oxobenzeneacetic acid (20:9) dichloromethane solvate as a light yellow powder.

| | | | | | |
|---|---|---|---|---|---|
| Analysis Calculated for C₂₆H₁₇ClN₄O₄S.20:9CH₂Cl₂: | C, 57.22; | H, 3.43; | Cl, 12.13; | N, 10.09; | S, 5.77 |
| Found: | C, 56.97; | H, 3.37; | Cl, 11.84; | N, 9.95; | S, 5.24 |

### Example 295

### Preparation of 4-[3-[4-(2-chlorophenyl)-9-methyl-6H-thieno[3,2-f][1,2,4]triazolo[4,3-a][1,4]diazepin-2-yl]propoxy]-alpha-oxobenzeneacetic acid ethyl ester.

As in Example 15, 4-(-2-chlorophenyl)-2-(3-hydroxy-1-propyl)-9-methyl-6H-thieno[3,2-f][1,2,4]triazolo[4,3-a][1,4]diazepine (0.322 g) was treated with 4-hydroxyphenylglyoxylic acid ethyl ester (0.168 g) in the presence of diethyl azodicarboxylate (0.155 g) and triphenylphosphine (0.227 g) in dichloromethane (23 ml). The crude ester, isolated in the usual manner, was purified by flash chromatography over silica gel (150 g; ethanol-dichloromethane; 3.5:96.5) to furnish 0.354 g of 4-[3-[4-(2-chlorophenyl)-9-methyl-6H-thieno[3,2-f][1,2,4]triazolo[4,3-a][1,4]diazepin-2-yl]propoxy]-alpha-oxobenzeneacetic acid ethyl ester as a white foam.

| | | | | | |
|---|---|---|---|---|---|
| Analysis Calculated for C₂₈H₂₅ClN₄O₄S: | C, 61.25; | H, 4.59; | Cl, 6.46; | N, 10.20; | S, 5.84 |
| Found: | C, 61.04; | H, 4.63; | Cl, 6.18; | N, 10.01; | S, 5.63 |

### Example 296

### Preparation of 4-[3-[4-(2-chlorophenyl)-9-methyl-6H-thieno[3,2-f][1,2,4]triazolo[4,3-a][1,4]diazepin-2-yl]-2-propoxy]-alpha-oxobenzeneacetic acid (10:7) hydrate(25:2) dichloromethane solvate.

A solution of 4-[3-[4-(2-chlorophenyl)-9-methyl-6H-thieno[3,2-f][1,2,4]triazolo[4,3-a][1,4]diazepin-2-yl]propoxy]-alpha-oxobenzeneacetic acid ethyl ester (0.3 g) in hot methanol (3 ml) was treated with 1N sodium hydroxide (1.1 ml) and then diluted with water (30 ml). After the methanol was removed in vacuo, the solution was acidified with 1N hydrochloric acid (1.2 ml) and extracted with dichloromethane (3 x 15 ml). The combined organic layers were washed with brine, dried (MgSO₄) and evaporated to yield 0.2 g 4-[3-[4-(2-chlorophenyl)-9-methyl-6H-thieno[3,2-f][1,2,4] triazolo[4,3-a][1,4]diazepin-2-yl]-2-propoxy]-alpha-oxobenzeneacetic acid (10:7) hydrate (25:2) dichloromethane solvate as a light yellow powder.

| | | | | | |
|---|---|---|---|---|---|
| Analysis Calculated for C₂₆H₂₁ClN₄O₄S.4:1H₂O.25:2CH₂Cl₂: | C, 57.97; | H, 4.21; | Cl, 7.61; | N, 10.37; | S, 5.93 |
| Found: | C, 57.76; | H, 4.17; | Cl, 7.81; | N, 10.13; | S, 5.83 |

### Example 297

### Preparation of (S)-alpha-[[(1,1-dimethylethoxy)carbonyl] amino]-4-[2-[2-[(2,2-dimethyl-1-oxobutoxy)methyl] -6-methylphenoxy] ethoxy]benzeneacetic acid benzyl ester.

As described in Example 15, a solution of diethyl azodicarboxylate (1.79 g) in dry tetrahydrofuran (15 ml) was added to a chilled (0-5 °C) solution of (S)-alpha-amino-N-[[(1,1-dimethyethyl)oxy]carbonyl]-4-hydroxybenzeneacetic acid benzyl ester (2.93 g), 2-[2-[(2,2-dimethyl-1-oxobutoxy)methyl]-6-methylphenoxy]ethanol (2.3 g) and triphenylphosphine (2.69 g) in dry tetrahydrofuran (50 ml). After the reaction mixture was stirred at 0-5 °C for 4 hours then at room temperature overnight, the reaction was worked up as previously described. Purification of the crude product by using HPLC (ethyl acetate-hexane; 3:17) furnished 3.56 g of (S)-alpha-[[(1,1-dimethylethoxy) carbonyl]amino]-4-[2-[2-[(2,2-dimethyl-1-oxobutoxy)methyl]-6-methylphenoxy] ethoxy]benzeneacetic acid benzyl ester as a colorless oil, [α]_{D} +31.52° (c, 1.21, methanol).

### Example 298

### Preparation of (S)-alpha-amino-4-[2-[2-[(2,2-dimethyl-1-oxobutoxy)methyl]-6-methylphenoxy]ethoxy]benzeneacetic acid.

As in Example 17, a solution of (S)-alpha-[[(1,1-dimethylethoxy)carbonyl]amino]-4-[2-[2-[(2,2-dimethyl-1-oxobutoxy)methyl]-6-methylphenoxy]ethoxy]benzeneacetic acid benzyl ester (2.59 g) in dichloromethane (8 ml) containing trifluoroacetic acid (8 ml) was stirred at room temperature for 45 minutes. The crude product was isolated in the previously described manner, was purified by HPLC (ethyl acetate-hexane; 1:1) to furnish 1.97 g of (S)-alpha-amino-4-[2-[2-[(2,2-dimethyl-1-oxobutoxy)methyl]-6-methylphenoxy]ethoxy] benzeneacetic acid benzyl ester as an oil.
A solution of the benzyl ester (1.48 g) in methanol (50 ml) containing one drop of acetic acid was hydrogenated over 10% Palladium on carbon (0.15 g) at ambient temperature and pressure for 1 hour. The reaction was diluted with methanol (100 ml) to dissolve the precipitated solids, then the catalyst was filtered off through a bed of Celite. The filtrates were evaporated and the resulting off-white solid was slurried with several portions of diethyl ether, then filtered and dried in vacuo to provide 0.9 g of (S)-alpha-amino-4-[2-[2-[(2,2-dimethyl-1-oxobutoxy)methyl]-6-methylphenoxy] ethoxy]benzeneacetic acid as a white solid, mp 170-172 °C, [α]_{D} +36.26° (c, 0.95, methanol).

| | | | |
|---|---|---|---|
| Analysis Calculated for C₂₄H₃₁NO₆: | C, 67.11; | H, 7.27; | N, 3.26 |
| Found: | C, 66.86; | H, 7.22; | N, 3.18 |

### Example 299

### Preparation of N-hydroxy-N-methyl-4-[2-(2-naphthalenyloxy)ethoxy]-alpha-oxobenzeneacetamide.

A solution of 4-[2-(2-naphthalenyloxy)ethoxy]-alpha-oxobenzeneacetyl chloride, derived from 4-[2-(2-naphthalenyloxy)ethoxy]-alpha-oxobenzeneacetic acid (0.312 g) in the previously described manner (Example 6), in dichloromethane was treated with a solution of N-methylhydroxylamine hydrochloride (0.094 g) in pyridine (1.5 ml). After the reaction solution was stirred at room temperature for 18 hours, it was concentated in vacuo and then diluted with water. The resulting fine yellow precipitate was filtered off, dried and crystallized from methanol-water to afford 0.129 g of N-hydroxy-N-methyl-4-[2-(2-naphthalenyloxy)ethoxy]-alpha-oxobenzeneacetamide, mp 161-165 °C.

| | | | |
|---|---|---|---|
| Analysis Calculated for C₂₁H₁₉NO₅: | C, 69.03; | H, 5.24; | N, 3.83 |
| Found: | C, 69.26; | H, 5.14; | N, 3.62 |

### Example 300

### Preparation of N-hydroxy-5-[2-(2-naphthalenyloxy)ethoxy]-alpha-oxo-2-thiopheneacetamide.

A solution of (E)-5-[2-(2-naphthalenyloxy)ethoxy]-alpha-oxo-2-thiopheneacetic acid ethyl ester (1.48 g) in a mixture of ethanol (20 ml) and tetrahydrofuran (10 ml) was treated in turn with hydroxylamine hydrochloride (0.77 g) and triethylamine (1.825 ml). After the reaction solution was stirred 50 °C for 18 hours, it was concentated in vacuo and was then taken up in dichloromethane (100 ml) and 0.1N hydrochloric acid (120 ml). The resulting dense precipitate was filtered off, then was washed in turn with dichloromethane and water to yield 1.1g N-hydroxy-4-[2-(2-naphthalenyloxy)ethoxy]-alpha-oxo-2-thiopheneacetamide as an off-white solid. A portion (0.1 g) was crystallized from tetrahydrofuran-2-propanol to give the analytical specimen, mp 165-167 °C.

| | | | | |
|---|---|---|---|---|
| Analysis Calculated for C₁₈H₁₅NO₅S: | C, 60.49; | H, 4.23; | N, 3.92; | S, 8.97 |
| Found: | C, 60.59; | H, 4.53; | N, 3.74; | S, 8.38 |

### Example 301

### Preparation of (Z)-alpha-(hydroxyimino)-4-[2-(2-naphthalenyloxy)ethoxy]benzeneacetic acid.

A solution of hydroxylamine hydrochloride (0.089 g) in 1N sodium hydroxide solution (1.3 ml) was added to a solution of 4-[2-(2-naphthalenyloxy)ethoxy]-alpha-oxobenzeneacetic acid (0.291 g) in dimethylformamide and the mixture was heated on a steam bath till all the solids dissolved. The reactants were maintained in solution by intermittent heating over 2 hours, then the mixture was stirred at room temperature overnight. The formed precipitate was filtered off, then the filtrate was diluted with water and adjusted to pH 2 by the addition of dilute hydrochloric acid solution. The resulting solid was recovered by filtration and dried in vacuo to furnish 0.164 g of (Z)-alpha-(hydroxyimino)-4-[2-(2-naphthalenyloxy)ethoxy]benzeneacetic acid, mp 178-179 °C.

| | | | |
|---|---|---|---|
| Analysis Calculated for C₂₀H₁₇NO₅: | C, 68.37; | H, 4.88; | N, 3.99 |
| Found: | C, 68.09; | H, 4.85; | N, 3.95 |

### Example 302

### Preparation of (Z)-alpha-(hydroxyimino)-5-[2-(2-naphthalenyloxy)ethoxy]-2-thiopheneacetic acid.

A solution of (E)-5-[2-(2-naphthalenyloxy)ethoxy]-alpha-oxo-2-thiopheneacetic acid (0.637 g) in dimethylformamide (10 ml) was treated in turn with hydroxylamine hydrochloride (0.17 g) and triethylamine (0.675 ml). After the reaction solution was stirred at room temperature for 18 hours, a second portion of hydroxylamine hydrochloride (0.17 g) and triethylamine (0.675 ml) was added and the reaction was allowed to proceed at ambient temperature. After 22 hours, the solvent was removed in vacuo and the residual material was taken up in dichloromethane (50 ml) and 0.25N hydrochloric acid (25 ml). The separated aqueous layer was extracted with dichloromethane (3 x 50 ml), then the dichloromethane phase and extracts were backwashed in turn with brine (25 ml). Concentration of the combined, dried (MgSO₄) organic layers furnished 0.625 g of a dark oil that was crystallized two times from chloroform to give (Z)-alpha-(hydroxyimino)-5-[2-(2-naphthalenyloxy)ethoxy]-2-thiopheneacetic acid as an off white solid, mp 138-140 °C;
MS (C₁₈H₁₅NO₅S), m/z 358 (M+1).

### Example 303

### Preparation of (Z)-4-[2-[2-[(2,2-dimethyl-1-oxobutoxy)methyl]-6-methylphenyloxy]ethoxy]-alpha-methoxyiminobenzeneacetic acid methyl ester.

As described in Example 15, (E)-2,2-dimethylbutanoic acid [2-(2-hydroxyethoxy)-3-methylphenyl]methyl ester (0.56 g) was reacted with 4-hydroxy-alpha-methoxyiminobenzeneacetic acid methyl ester (0.42 g) in the presence of diethyl azodicarboxylate (0.44 g) and triphenylphosphine (0.655 g) in tetrahydrofuran (20 ml). After the previously described work up, the methoxime was purified by chromatography over silica gel (75 g; diethyl ether-hexane; 1:4) to afford 0.405 g of (Z)-4-[2-[2-[(2,2-dimethyl-1-oxobutoxy)methyl]-6-methylphenyloxy]ethoxy]-alpha-methoxyiminobenzeneacetic acid methyl ester as an oil.

| | | | |
|---|---|---|---|
| Analysis Calculated for C₂₆H₃₃NO₇: | C, 66.62; | H, 7.05; | N, 2.97 |
| Found: | C, 66.30; | H, 7.04: | N, 2.79 |

### Example 304

### Preparation of (Z)-4-[2-[2-[(2,2-Dimethyl-1-oxobutoxy)methyl]-6-methylphenyloxy]ethoxy]-alpha-methoxyiminobenzeneacetic acid.

As in Example 19, (Z)-4-[2-[2-[(2,2-dimethyl-1-oxobutoxy)methyl]-6-methylphenyloxy]ethoxy]-alpha-methoxyiminobenzeneacetic acid methyl ester (0.231 g) in methanol (5 ml) was treated with 1N sodium hydroxide (0.54 ml) and the mixture was stirred overnight at room temperature. The usual work up gave 0.188 g of an oil which was purified by flash chomatography over silica gel (formic acid-diethyl ether 1:49) to yield 0.07 g of recovered starting ester along with 0.055 g of (Z)-4-[2-[2-[(2,2-dimethyl-1-oxobutoxy)methyl]-6-methylphenyloxy]ethoxy]-alpha-methoxyiminobenzene acetic acid as an oil.

| | | | |
|---|---|---|---|
| Analysis Calculated for C₂₅H₃₁NO₇ : | C, 65.63; | H, 6.83; | N 3.06 |
| Found: | C, 65.89; | H, 7.12; | N 2.82 |

### Example 305

### Preparation of (E)-alpha-(methoxyimino)-5-[2-(2-naphthalenyloxy)ethoxy]-2-thiopheneacetic acid ethyl ester and (Z)-alpha-(methoxyimino)-5-[2-(2-naphthalenyloxy)ethoxy]-2-thiopheneacetic acid ethyl ester.

5-[2-(2-naphthalenyloxy)ethoxy]-alpha-oxo-2-thiopheneacetic acid (0.741 g) was combined with methoxylamine hydrochloride (0.2 g) in pyridine (10 ml) and stirred for 48 hours at room temperature, whereupon another 0.2 g of methoxylamine hydrochloride was added and the mixture was stirred for an additional 72 hours. After the solvent was removed under reduced pressure, the residue was partitioned between dichloromethane and water and the separated aqueous phase was extracted with dichloromethane. The combined organic layers were washed with water, dried (MgSO₄) and evaporated to yield 0.7 g of a yellow solid. Flash chromatography of the solid over silica gel (75 g: dichloromethane-hexane; 4:1) resulted in the isolation of two isomeric compounds.
The less polar isomer was crystallized from dichloromethane-hexane to give 0.265 g of (Z)-alpha-(methoxyimino)-5-[2-(2-naphthalenyloxy)ethoxy]-2-thiopheneacetic acid ethyl ester as pale yellow crystals, mp 152-153.5 °C.

| | | | | |
|---|---|---|---|---|
| Analysis Calculated for C₂₁H₂₁NO₅S: | C, 63.14; | H, 5.30; | N, 3.51; | S, 8.03 |
| Found: | C, 62.91; | H, 5.36; | N, 3.48; | S, 7.89 |

The more polar compound (0.3 g) was crystallized from dichloromethane-hexane to give 0.248 g (E)-alpha-(methoxyimino)-5-[2-(2-naphthalenyloxy)ethoxy]-2-thiopheneacetic acid ethyl ester as yellow crystals, mp 132-133.5 °C.

| | | | | |
|---|---|---|---|---|
| Analysis Calculated for C₂₁H₂₁NO₅S: | C, 63.14; | H, 5.30; | N, 3.51; | S, 8.03 |
| Found: | C, 62.92; | H, 5.24; | N, 3.31; | S, 8.06 |

### Example 306

### Preparation of (Z)-alpha-(methoxyimino)-5-[2-(2-naphthalenyloxy)ethoxy]-2-thiopheneacetic acid (2:1) hydrate.

(Z)-alpha-(methoxyimino)-5-[2-(2-naphthalenyloxy)ethoxy]-2-thiopheneacetic acid ethyl ester (0.2 g) in methanol (2 ml) and tetrahydrofuran (2 ml) was treated with 3N sodium hydroxide (0.5 ml) and the stirred mixture was heated at 50 °C for 30 minutes. After the solvents were removed in vacuo, the concentrate was diluted with water (10 ml), acidified with 1N hydrochloric acid (3 ml) and extracted with dichloromethane (2 x 10 ml). The combined organic layers were washed with brine, dried (MgSO₄) and evaporated. The resulting solid was crystallized from dichloromethane-hexane to provide 0.157 g of (Z)-alpha-(methoxyimino)-5-[2-(2-naphthalenyloxy)ethoxy]-2-thiopheneacetic acid as its hemihydrate, mp118-120 °C.

| | | | | |
|---|---|---|---|---|
| Analysis Calculated for C₁₉H₁₇NO₅S.H₂O: | C, 59.99; | H, 4.77; | N, 3.68; | S, 8.43 |
| Found: | C, 60.18; | H, 4.57; | N, 3.50; | S, 8.52 |

### Example 307

### Preparation of (E)-alpha-(methoxyimino)-5-[2-(2-naphthalenyloxy)ethoxy]-2-thiopheneacetic acid.

As in Example 306, a solution of (E)-alpha-(methoxyimino)-5-[2-(2-naphthalenyloxy) ethoxy]-2-thiopheneacetic acid ethyl ester (0.2 g) in methanol (2 ml) and tetrahydrofuran (1 ml) was treated with 3N sodium hydroxide (0.5 ml) and the stirred mixture was heated at 53 °C for 30 minutes. After the normal work up, the product was crystallized from dichloromethane-hexane to furnish 0.142 g of (E)-alpha-(methoxyimino)-5-[2-(2-naphthalenyloxy)ethoxy]-2-thiopheneacetic acid, mp 118-120 °C.

| | | | | |
|---|---|---|---|---|
| Analysis Calculated for C₁₉H₁₇NO₅S: | C, 61.44; | H, 4.61; | N, 3.77; | S, 8.63 |
| Found: | C, 61.17; | H, 4.56; | N, 3.62; | S, 8.41 |

### Example 308

### Preparation of (E)-alpha-(ethoxyimino)-5-[2-(2-naphthalenyloxy)ethoxy]-2-thiopheneacetic acid ethyl ester and (Z)-alpha-(ethoxyimino)-5-[2-(2-naphthalenyloxy)ethoxy]-2-thiopheneacetic acid ethyl ester.

As described in example 305, 5-[2-(2-naphthalenyloxy)ethoxy]-alpha-oxo-2-thiopheneacetic acid (0.34 g) was combined with ethoxylamine hydrochloride (0.130 g) in pyridine (5 ml) and stirred for 10 days at room temperature. The mixture of isomers, obtained after work up of the reaction in the usual manner, were separated by flash chromatography over silica gel (70 g: dichloromethane-hexane; 4:1). Fractions containing the less polar isomer were evaporated to give 0.151 g of (Z)-alpha-(ethoxyimino)-5-[2-(2-naphthalenyloxy)ethoxy]-2-thiopheneacetic acid ethyl ester, mp 114-115.5 °C.

| | | | | |
|---|---|---|---|---|
| Analysis Calculated for C₂₂H₂₃NO₅S: | C, 63.91; | H, 5.61; | N, 3.39; | S, 7.75 |
| Found: | C, 63.81; | H, 5.30; | N, 3.37; | S, 7.82 |

Fractions containing the more polar isomer were evaporated to give 0.158 g of (E)-alpha-(ethoxyimino)-5-[2-(2-naphthalenyloxy)ethoxy]-2-thiopheneacetic acid ethyl ester, mp 102.5-104 °C.

| | | | | |
|---|---|---|---|---|
| Analysis Calculated for C₂₂H₂₃NO₅S: | C, 63.91; | H, 5.61; | N, 3.39; | S, 7.75 |
| Found: | C, 63.66; | H, 5.29; | N, 3.12; | S, 7.79 |

### Example 309

### Preparation of (Z)-alpha-(ethoxyimino)-5-[2-(2-naphthalenyloxy)ethoxy]-2-thiopheneacetic acid.

As in Example 306, a solution of (Z)-alpha-(ethoxyimino)-5-[2-(2-naphthalenyloxy) ethoxy]-2-thiopheneacetic acid ethyl ester (0.141 g) in methanol (2 ml) and tetrahydrofuran (1 ml) was treated with 4N sodium hydroxide (0.5 ml) and the stirred mixture was heated at 55 °C for 30 minutes. After the normal work up, the product was crystallized from ethyl acetate-hexane to furnish 0.07 g of (Z-alpha-(ethoxyimino)-5-[2-(2-naphthalenyloxy)ethoxy]-2-thiopheneacetic acid, mp 114 °C (dec.).

| | | | | |
|---|---|---|---|---|
| Analysis Calculated for C₂₀H₁₉NO₅S: | C, 62.32; | H, 4.97; | N, 3.65; | S, 8.32 |
| Found: | C, 62.05; | H, 4.82; | N, 3.45; | S, 8.25 |

### Example 310

### Preparation of (E)-alpha-(ethoxyimino)-5-[2-(2-naphthalenyloxy)ethoxy]-2-thiopheneacetic acid.

As in Example 306, a solution of (E)-alpha-(ethoxyimino)-5-[2-(2-naphthalenyloxy) ethoxy]-2-thiopheneacetic acid ethyl ester (0.131 g) in methanol (2 ml) and tetrahydrofuran (2 ml) was treated with 4N sodium hydroxide (0.5 ml) and the stirred mixture was heated at 55 °C for 45 minutes. After the usual work up, the isolated product was crystallized from ethyl acetate-hexane to afford 0.062 g of (E)-alpha-(ethoxyimino)-5-[2-(2-naphthalenyloxy)ethoxy]-2-thiopheneacetic acid, mp 17-108 °C (dec.).

| | | | | |
|---|---|---|---|---|
| Analysis Calculated for C₂₀H₁₉NO₅S: | C, 62.32; | H, 4.97; | N, 3.65; | S, 8.32 |
| Found: | C, 63.23; | H, 4.39; | N, 3.62; | S, 8.55 |

### Example 311

### Preparation of (E)-5-[2-(2-naphthalenyloxy)ethoxy]-alpha-[(2-propenyloxy)imino]-2-thiopheneacetic acid ethyl ester and (Z)-5-[2-(2-naphthalenyloxy)ethoxy]-alpha-[(2-propenyloxy)imino]-2-thiopheneacetic acid ethyl ester.

As described in Example 305, 5-[2-(2-naphthalenyloxy)ethoxy]-alpha-oxo-2-thiopheneacetic acid (0.34 g) was combined with O-(2-propenyl)hydroxylamine hydrochloride (0.145 g) in pyridine (5 ml) and stirred for 72 hours at room temperature. The mixture of isomers, obtained after work up of the reaction were separated by flash chromatography over silica gel (70 g: dichloromethane-hexane; 4:1). Fractions containing the less polar isomer were evaporated to give 0.114 g of (Z)-5-[2-(2-naphthalenyloxy)ethoxy]-alpha-[(2-propenyloxy)imino]-2-thiopheneacetic acid ethyl ester, mp 104-105 °C.

| | | | | |
|---|---|---|---|---|
| Analysis Calculated for C₂₃H₂₃NO₅S: | C, 64.92; | H, 5.45; | N, 3.29; | S, 7.53 |
| Found: | C, 65.79; | H, 5.67; | N, 2.99; | S, 7.00 |

Fractions containing the more polar isomer were evaporated to give 0.075 g of (E)-5-[2-(2-naphthalenyloxy)ethoxy]-alpha-[(2-propenyloxy)imino]-2-thiopheneacetic acid ethyl ester, mp 75-77 °C.

| | | | | |
|---|---|---|---|---|
| Analysis Calculated for C₂₃H₂₃NO₅S: | C, 64.92; | H, 5.45; | N, 3.29; | S, 7.53 |
| Found: | C, 64.78; | H, 5.42; | N, 3.54; | S, 7.37 |

### Example 312

### Preparation of (Z)-5-[2-(2-naphthalenyloxy)ethoxy]-alpha-[(2-propenyloxy)imino]-2-thiopheneacetic acid.

As in Example 306, a solution of (Z)-5-[2-(2-naphthalenyloxy)ethoxy]-alpha-[(2-propenyloxy)imino]-2-thiopheneacetic acid ethyl ester (0.102 g) in methanol (2 ml) and tetrahydrofuran (1 ml) was treated with 4N sodium hydroxide (0.5 ml) and the stirred mixture was heated at 50 °C for 30 minutes. After the normal work up, the product was crystallized from ethyl acetate-hexane to furnish 0.057 g of (Z)-5-[2-(2-naphthalenyloxy)ethoxy]-alpha-[(2-propenyloxy)imino]-2-thiopheneacetic acid, mp 114-115 °C.

| | | | | |
|---|---|---|---|---|
| Analysis Calculated for C₂₁H₁₉NO₅S: | C, 63.46; | H, 4.82; | N, 3.52; | S, 8.07 |
| Found: | C, 63.26; | H, 4.65; | N, 3.43; | S, 7.76 |

### Example 313

### Preparation of (E)-5-[2-(2-naphthalenyloxy)ethoxy]-alpha-[(2-propenyloxy)imino]-2-thiopheneacetic acid.

As in Example 306 a solution of (E)-5-[2-(2-naphthalenyloxy)ethoxy]-alpha-[(2 propenyloxy)imino]-2-thiopheneacetic acid ethyl ester (0.064 g) in methanol (2 ml) and tetrahydrofuran (2 ml) was treated with 4N sodium hydroxide (0.5 ml) and the stirred mixture was heated at 55 °C for 45 minutes. After the normal work up, the product was crystallized from ethyl acetate-hexane to furnish 0.038 g of (E)-5-[2-(2-naphthalenyloxy)ethoxy]-alpha-[(2-propenyloxy)imino]-2-thiopheneacetic acid, mp 106-108 °C.

| | | | | |
|---|---|---|---|---|
| Analysis Calculated for C₂₁H₁₉NO₅S: | C, 63.46; | H, 4.82; | N, 3.52; | S, 8.07 |
| Found: | C, 63.72; | H, 4.64; | N, 3.49; | S, 8.13 |

### Example 314

### Preparation of (E)-5-[2-(2-naphthalenyloxy)ethoxy]-alpha-[(phenylmethoxy)imino]-2-thiopheneacetic acid ethyl ester and (Z)-5-[2-(2-naphthalenyloxy)ethoxy]-alpha-[(phenylmethoxy)imino]-2-thiopheneacetic acid ethyl ester.

As described in example 305, 5-[2-(2-naphthalenyloxy)ethoxy]-alpha-oxo-2-thiopheneacetic acid (0.34 g) was combined with O-benzylhydroxylamine hydrochloride (0.214 g) in pyridine (5 ml) and stirred for 72 hours at room temperature. The mixture of isomers, obtained from the usual work up, were separated by flash chromatography over silica gel (75 g: dichloromethane-hexane; 4:1). Fractions containing the less polar isomer were evaporated to give 0.197 g of (Z)-5-[2-(2-naphthalenyloxy)ethoxy]-alpha-[(phenylmethoxy)imino]-2-thiopheneacetic acid ethyl ester, mp 140-141.5 °C.

| | | | | |
|---|---|---|---|---|
| Analysis Calculated for C₂₇H₂₅NO₅S: | C, 68.18; | H, 5.30; | N, 2.95; | S, 6.74 |
| Found: | C, 67.07; | H, 5.03; | N, 2.78; | S, 6.70 |

Fractions containing the more polar isomer were evaporated to give 0.152 g of (E)-5-[2-(2-naphthalenyloxy)ethoxy]-alpha-[(phenylmethoxy)imino]-2-thiopheneacetic acid ethyl ester, mp 111-112 °C.

| | | | | |
|---|---|---|---|---|
| Analysis Calculated for C₂₇H₂₅NO₅S: | C, 68.18; | H, 5.30; | N, 2.95; | S, 6.74 |
| Found: | C, 67.35; | H, 5.22; | N, 2.81; | S, 6.71 |

### Example 315

### Preparation of (Z)-5-[2-(2-naphthalenyloxy)ethoxy]-alpha-[(phenylmethoxy)imino]-2-thiopheneacetic acid.

As in Example 306, a solution of (Z)-5-[2-(2-naphthalenyloxy)ethoxy]-alpha-[(phenylmethoxy)imino]-2-thiopheneacetic acid ethyl ester (0.163 g) in methanol (2 ml) and tetrahydrofuran (1 ml) was treated with 4N sodium hydroxide (0.5 ml) and the stirred mixture was heated at 50 °C for 30 minutes. After the normal work up, the product was crystallized from diethyl ether to provide 0.11 g of (Z)-5-[2-(2-naphthalenyloxy)ethoxy]-alpha-[(phenylmethoxy)imino]-2-thiopheneacetic acid, mp 117-118 °C.

| | | | | |
|---|---|---|---|---|
| Analysis Calculated for C₂₅H₂₁NO₅S: | C, 67.10; | H, 4.73; | N, 3.13; | S, 7.16 |
| Found: | C, 67.03; | H, 4.46; | N, 3.28; | S, 7.33 |

### Example 316

### Preparation of (E)-5-[2-(2-naphthalenyloxy)ethoxy]-alpha-[(phenylmethoxy)imino]-2-thiopheneacetic acid.

As in Example 306, a solution of (E)-5-[2-(2-naphthalenyloxy)ethoxy]-alpha-[(phenylmethoxy)imino]-2-thiopheneacetic acid ethyl ester (0.102 g) in methanol (2 ml) and tetrahydrofuran (2 ml) was treated with 4N sodium hydroxide (0.5 ml) and the stirred mixture was heated at 55 °C for 45 minutes. After the normal work up, the product was crystallized from diethyl ether-hexane to furnish 0.063 g of (E)-5-[2-(2-naphthalenyloxy)ethoxy]-alpha-[(phenylmethoxy)imino]-2-thiopheneacetic acid, mp 103-104 °C.

| | | | | |
|---|---|---|---|---|
| Analysis Calculated for C₂₅H₂₁NO₅S: | C, 67.10; | H, 4.73; | N, 3.13; | S, 7.16 |
| Found: | C, 67.01; | H, 4.59; | N, 3.17; | S, 7.32 |

### Example 317

### Preparation of (E)-alpha-[(aminocarbonyl)hydrazono]-4-[2-(2-naphthalenyloxy)ethoxy]benzeneacetic acid methyl ester and (Z)-alpha-[(aminocarbonyl)hydrazono]-4-[2-(2-naphthalenyloxy)ethoxy]benzeneacetic acid methyl ester.

A mixture of 4-[2-(2-naphthalenyloxy)ethoxy]-alpha-oxobenzeneacetic acid methyl ester (1.75 g) in pyridine (25 ml) was warmed to dissolve and added semicarbazide hydrochloride (0.7 g). The stirred mixture was heated at 50 °C for 4 hours, cooled, acidified with hydrochloric acid and extracted twice with dichloromethane. The organic layers were washed with dilute hydrochloric acid, combined, dried (Na₂SO₄), filtered and evaporated to provide a crude mixture of (E) and (Z) isomers. The crude mixture was separated by HPLC (dichloromethane-tetrahydrofuran; 9:1) and the more polar component was crystallized from dichloromethane-methanol to provide 0.6 g of (E)-alpha-[(aminocarbonyl)hydrazono]-4-[2-(2-naphthalenyloxy)ethoxy]benzeneacetic acid methyl ester as a colorless solid, mp 180-181 °C.

| | | | |
|---|---|---|---|
| Analysis Calculated for C₂₂H₂₁N₃O₅: | C, 64.86; | H, 5.20; | N, 10.31 |
| Found: | C, 64.72; | H, 5.21; | N, 10.20 |

The less polar component was crystallized from dichloromethane-methanol to provide 0.75 g of (Z)-alpha-[(aminocarbonyl)hydrazono]-4-[2-(2-naphthalenyloxy)ethoxy] benzeneacetic acid methyl ester as a colorless solid, mp 132-134 °C.

| | | | |
|---|---|---|---|
| Analysis Calculated for C₂₂H₂₁N₃O₅: | C, 64.86; | H, 5.20; | N, 10.31 |
| Found: | C, 64.60; | H, 5.14; | N, 10.13 |

### Example 318

### Preparation of (E)-alpha-[(aminocarbonyl)hydrazono]-4-[2-(2-naphthalenyloxy)ethoxy]benzeneacetic acid

A mixture of (E)-alpha-[(aminocarbonyl)hydrazono]-4-[2-(2-naphthalenyloxy) ethoxy]benzeneacetic acid methyl ester (0.45 g) in warm methanol (3 ml) and tetrahydrofuran (10 ml) was treated with 1N sodium hydroxide (1.25 ml) and after 30 minutes the mixture was diluted with water and concentrated to remove the organic solvents. The residue was acidified with excess hydrochloric acid and extracted with a dichloromethane-tetrahydrofuran mixture. The organic layer was washed with water, dried (Na₂SO₄), filtered and evaporated to give crude product. Trituration from dichloromethane provided 0.242 g of pure (E)-alpha-[(aminocarbonyl)hydrazono]-4-[2-(2-naphthalenyloxy)ethoxy]benzeneacetic acid as a colorless solid, mp 204 °C with decomposition.

| | | | |
|---|---|---|---|
| Analysis Calculated for C₂₁H₁₉N₃O₅: | C, 64.12; | H, 4.87; | N, 10.68 |
| Found: | C, 64.11; | H, 4.80; | N, 10.67 |

### Example 319

### Preparation of (E)-alpha-(dimethylhydrazono)-4-[2-(2-naphthalenyloxy)ethoxy]benzeneacetic acid methyl ester and (Z)-alpha-(dimethylhydrazono)-4-[2-(2-naphthalenyloxy)ethoxy]benzeneacetic acid methyl ester.

A mixture of 4-[2-(2-naphthalenyloxy)ethoxy]-alpha-oxobenzeneacetic acid methyl ester (3.5 g), 1,1-dimethylhydrazine (2.28 ml) in methanol (40 ml), tetrahydrofuran (20 ml) and one drop of glacial acetic acid was refluxed for 48 hours and evaporated to dryness. The crude mixture was separated by HPLC (dichloromethane-ether; 49:1) and the more polar component was crystallized from dichloromethane-methanol to provide 0.9 g of (E)-alpha-(dimethylhydrazono)-4-[2-(2-naphthalenyloxy)ethoxy]benzeneacetic acid methyl ester as a colorless solid, mp 137-138 °C.

| | | | |
|---|---|---|---|
| Analysis Calculated for C₂₃H₂₄N₂O₄: | C, 70.39; | H, 6.16; | N, 7.14 |
| Found: | C, 70.27; | H, 6.16; | N, 7.19 |

The less polar component was crystallized from dichloromethane-methanol to provide 0.75 g of (Z)-alpha-(dimethylhydrazono)-4-[2-(2-naphthalenyloxy)ethoxy] benzeneacetic acid methyl ester as a yellow solid, mp 109-111°C.

| | | | |
|---|---|---|---|
| Analysis Calculated for C₂₃H₂₄N₂O₄: | C, 70.39; | H, 6.16; | N, 7.14 |
| Found: | C, 70.27; | H, 6.17; | N, 7.18 |

### Example 320

### Preparation of (E)-alpha-(dimethylhydrazono)-4-[2-(2-naphthalenyloxy)ethoxy]benzeneacetic acid.

A mixture of (E)-alpha-(dimethylhydrazono)-4-[2-(2-naphthalenyloxy)ethoxy] benzeneacetic acid methyl ester (0.45 g) in warm methanol (3 ml) and tetrahydrofuran (10 ml) was treated with 1N sodium hydroxide (2 ml), heated on the steam bath for one hour, and the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.38 g of pure (E)-alpha-(dimethylhydrazono)-4-[2-(2-naphthalenyloxy)ethoxy]benzeneacetic acid as a colorless solid, mp 135 °C with decomposition.

| | | | |
|---|---|---|---|
| Analysis Calculated for C₂₂H₂₂N₂O₄: | C, 69.83; | H, 5.86; | N, 7.40 |
| Found: | C, 69.55; | H, 5.90; | N, 7.35 |

### Example 321

### Preparation of (Z)-alpha-(dimethylhydrazono)-4-[2-(2-naphthalenyloxy)ethoxy]benzeneacetic acid.

A mix of (Z)-alpha-(dimethylhydrazono)-4-[2-(2-naphthalenyloxy)ethoxy] benzeneacetic acid methyl ester (0.45 g) in warm methanol (3 ml) and tetrahydrofuran (10 ml) was treated with 1N sodium hydroxide (2 ml), heated on the steam bath for 2 hours, and the mixture was diluted with water and concentrated to remove the organic solvents. The residue was acidified with excess hydrochloric acid and extracted with dichloromethane containing a little tetrahydrofuran. The organic layer was washed with water, dried (Na₂SO₄), filtered and evaporated to give crude product. Crystallization from dichloromethane-hexane provided 0.3 g of pure (Z)-alpha-(dimethylhydrazono)-4-[2-(2-naphthalenyloxy)ethoxy]benzeneacetic acid as a colorless solid, mp 136 °C with decomposition.

| | | | |
|---|---|---|---|
| Analysis Calculated for C₂₂H₂₂N₂O₄: | C, 69.83; | H, 5.86; | N, 7.40 |
| Found: | C, 69.84; | H, 5.74; | N, 7.13 |

### Example 322

| TABLET FORMULATION (Wet Granulation) | | | | | |
|---|---|---|---|---|---|
| Item | Ingredient | 25 mg | 100mg | 500mg | 1000 mg |
| 1. | 4-[2-(2-Naphthyloxy)ethoxy] -alpha-oxo-benzeneacetic acid | 25 | 100 | 500 | 1000 |
| 2. | Lactose | 143 | 132 | - | - |
| 3. | Pregelatinized Starch | 10 | 16 | 30 | 50 |
| 4. | Modified Starch | 20 | 30 | 40 | 50 |
| 5. | Magnesium Stearate | 2 | 2 | 6 | 8 |
| | Total | 200 | 280 | 576 | 1108 |
| Manufacturing Procedure: 1. Mix Items 1, 2, 3 and 4 and granulate with water. 2. Dry the granulation at 50° C. 3. Pass the granulation through suitable milling equipment. 4. Add Item 5 and mix for three minutes; compress on a suitable press. | | | | | |

### Example 323

| CAPSULE FORMULATION | | | | | |
|---|---|---|---|---|---|
| Item | Ingredient | mg/tablet | | | |
| 1. | 4-[2-(2-Naphthyloxy)ethoxy] -alpha-oxo-benzeneacetic acid | 25 | 50 | 100 | 500 |
| 2. | Lactose Hydrous | 143 | 168 | 148 | - |
| 3. | Corn Starch | 20 | 20 | 40 | 70 |
| 4. | Talc | 10 | 10 | 10 | 25 |
| 5. | Magnesium Stearate | 2 | 2 | 2 | 5 |
| | Total | 200 | 250 | 300 | 600 |
| Manufacturing Procedure: 1. Mix Items 1, 2 and 3 in a suitable mixer for 30 minutes. 2. Add Items 4 and 5 and mix for 3 minutes. 3. Fill into suitable capsules. | | | | | |

## Claims (Claims for the following Contracting State(s): AT, BE, CH, DE, DK, FR, GB, IT, LI, LU, MC, NL, PT, SE)

1. Compounds of the general formula 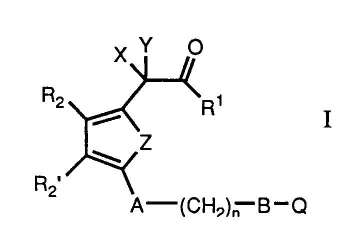 wherein R₁ is hydroxy, OR₃ or 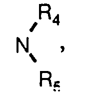 wherein one of R₄ and R₅ is hydrogen, C₁-C₇ alkyl or hydroxy-C₁-C₇ alkyl, and the other is hydrogen, hydroxy, C₁-C₇ alkyl or C₁-C₇ alkoxy, and wherein R₃ is (̵CH₂CH₂O)̵ₘH, 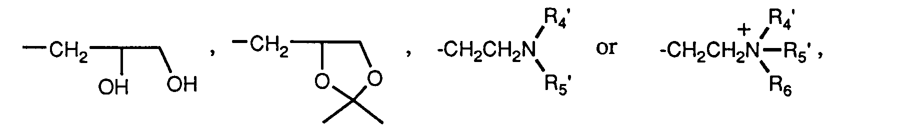 and wherein R₄' R₅', and R₆ are, independently, hydrogen , C₁-C₇ alkyl or hydroxy-C₁-C₇ alkyl, and wherein m is an integer from 1 to 4;
R₂ and R₂'_{,} independently, are hydrogen, C₁-C₇ alkyl, aryl, aryl-C₁-C₇ alkyl, C₁-C₇ alkoxy, hydroxy, amino, C₁-C₇ alkylamino, di-C₁-C₇ alkylamino, cyano, halogen, mercapto, C₁-C₇ alkylthio, C₁-C₇ alkylsulfinyl, C₁-C₇ alkylsulfonyl, trihalo-C₁-C₇ alkyl, acyl or nitro;
A is a bond,
―O―, ―NR₇―, ―S―, 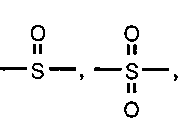 ―C≡C―, ―HC=CH―, ―CH₂CH₂―, 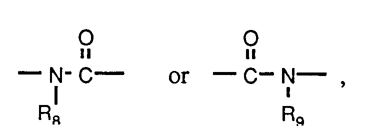 wherein R₇ is hydrogen, C₁-C₇ alkyl or acyl and R₈ and R₉ are, independently, hydrogen or C₁-C₇ alkyl; n is an integer from 0 to 10;
B is a bond, ―O―, ―NR₇―,―S―, 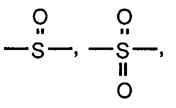 ―C≡C―, ―HC=CH―, 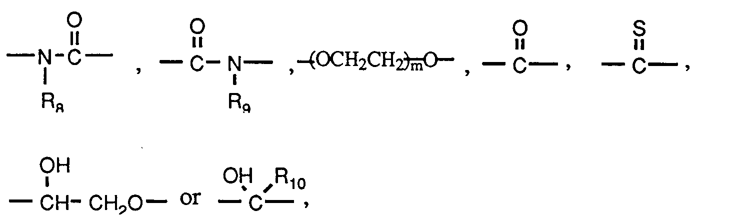 wherein R₇, R₈, R₉ and m are as defined above and R₁₀ is hydrogen or C₁-C₇ alkyl;
Z is ―O―, ―S―, ―CR₂ = CR₂'―, ―N = CR₂―, ―CR₂ = N― or 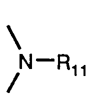 wherein R₁₁ is hydrogen or C₁-C₇ alkyl;
X and Y taken together are 0= , S=, hydroxyimino, alkyloxyimino, alkenyloxyimino, arylalkoxyimino, hydrazono, mono-C₁-C₇ alkyl hydrazono, di-C₁-C₇ alkyl hydrazono or semicarbazono, or, independently, when one of X and Y is halogen, the other is hydrogen, halogen, C₁-C₇ alkyl or aryl-C₁-C₇ alkyl; or when one of X and Y is amino, C₁-C₇ alkylamino or di-C₁-C₇ alkylamino, the other is hydrogen, C₁-C₇ alkyl or aryl-C₁-C₇ alkyl; or when one of X and Y is hydroxy, alkoxy or aryl-C₁-C₇ alkoxy, the other is hydrogen, hydroxy, C₁-C₇ alkyl, C₁-C₇ alkoxy or aryl-C₁-C₇ alkoxy; Q is a cycloalkyl, aryl or heterocyclic radical; provided that, when A is oxygen (O) and B is a bond, sulfur (S) or oxygen (O), then n is 2-10; and, when appropriate, in the form of enantiomers, racemates, diastereomers or mixtures thereof or geometric isomers or mixtures thereof, and, when a basic or acidic group is present, pharmaceutically acceptable salts thereof.

2. Compounds in accordance with claim 1, wherein one of R₄ and R₅ is hydrogen, C₁-C₇ alkyl or hydroxy-C₁-C₇ alkyl and the other is hydrogen or C₁-C₇ alkyl, X and Y taken together are O=, S= or, independently, when one of X and Y is halogen, the other is hydrogen, halogen, C₁-C₇ alkyl or aryl-C₁-C₇ alkyl; or when one of X and Y is amino, C₁-C₇ alkylamino or di-C₁-C₇ alkylamino, the other is hydrogen, C₁-C₇ alkyl or aryl-C₁-C₇ alkyl; or when one of X and Y is hydroxy, alkoxy or aryl-C₁-C₇ alkoxy, the other is hydrogen, hydroxy, C₁-C₇ alkyl, C₁-C₇ alkoxy or aryl-C₁-C₇ alkoxy.

3. Compounds in accordance with claim 1, wherein R₁ is as previously described; R₂ and R₂', independently, are hydrogen, C₁-C₇ alkyl, aryl, C₁-C₇ alkoxy, hydroxy, halogen, acyl or nitro;
A is a bond,
―O―, ―NR₇―, ―S―, 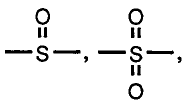 ―C≡C― , ―HC=CH―, ―CH₂CH₂―, 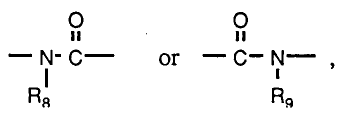 wherein R₇ is hydrogen, C₁-C₇ alkyl or acyl and R₈ and R₉ are, independently, hydrogen or C₁-C₇ alkyl;
B is a bond, ―O―, ―NR₇―, ―S―, ―C≡C―, ―HC=CH―, 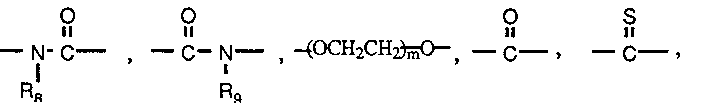 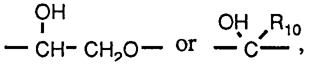 wherein R₇, R₈, R₉ and m are as previously described and R₁₀ is hydrogen;
n is an integer from 1 to 6, whereby when A is a bond or one of the following groups,
―O―, ―NR₇―, ―S―, 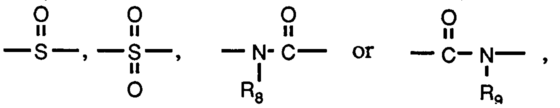 and B is a bond or one of the following groups
―O―, ―NR₇―, ―S―, 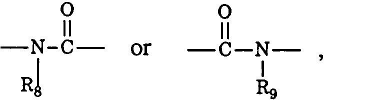 then n is different from the integer 1;
Z is ―S―, ―CR₂ = CR₂'― ,―N=CR₂― or ―CR₂ = N―;
X and Y taken together are 0=, hydroxyimino, alkyloxyimino, alkenyloxyimino, arylalkoxyimino, hydrazono, mono-C₁-C₇ alkyl hydrazono, di-C₁-C₇ alkyl hydrazono or semicarbazono, or, independently, when one of X and Y is halogen, the other is hydrogen or halogen; or when one of X and Y is amino, the other is hydrogen; or when one of X and Y is hydroxy or alkoxy, the other is hydrogen or hydroxy;
Q is a cycloalkyl, aryl or heterocyclic radical, which radical can be substituted by one or more of the following groups: alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl, carboxycarbonyl or alkoxalyl.

4. Compounds in accordance with claim 3, wherein R₁ is as previously described; R₂ and R₂', independently, are hydrogen, C₁-C₇ alkyl, C₁-C₇ alkoxy, hydroxy or halogen;
A is a bond,
―O―, ―NR₇―, ―S―, 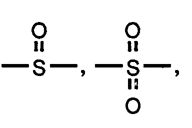 ―C≡C―, ―HC=CH―, ―CH₂CH₂―, 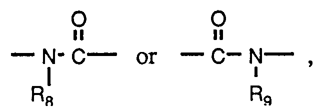 wherein R₇ is hydrogen or C₁-C₇ alkyl and R₈ and R₉ are hydrogen ;
B is a bond,
―O―, ―S―, ―C≡C―, ―HC=CH―, 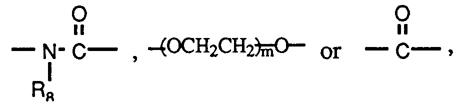 wherein m is 1 or 2 and R₈ is hydrogen;
n is an integer from 1 to 6, whereby when A is a bond or one of the following groups,
―O―, ―NR₇―, ―S―, 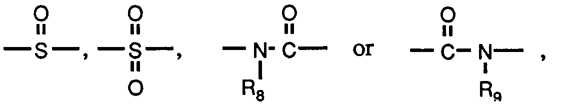 and B is a bond or one of the following groups
―O―, ―S― or 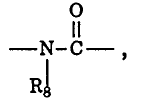 then n is different from the integer 1;
Z is ―S―, or ―CR₂ = CR₂'―,
X and Y taken together are 0= , hydroxyimino, alkyloxyimino or alkenyloxyimino, or, independently, when one of X and Y is halogen, the other is halogen; or when one of X and Y is amino, the other is hydrogen; or when one of X and Y is hydroxy , the other is hydrogen or hydroxy;
Q is phenyl, cyclohexyl, cyclooctyl, pyridinyl, adamantyl, 1,1'-biphenyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, which radical can be substituted by one or more of the following groups; alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl carboxycarbonyl or alkoxalyl.

5. Compounds in accordance with claim 4, wherein R₁ is hydroxy or 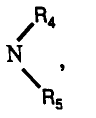 wherein one of R₄ and R₅ is hydrogen, C₁-C₇ alkyl or hydroxy-C₁-C₇ alkyl, and the other is hydroxy;
R₂ and R₂' are hydrogen;
A is ―O―, ―NR₇― or ―S―, wherein R₇ is hydrogen ;
B is a bond,
―O―, ―S―, ―C≡C―, ―HC=CH―, 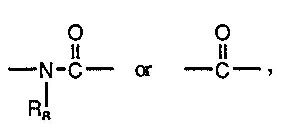 wherein R₈ is hydrogen;
n is an integer from 1 to 4, whereby when B is a bond or one of the following groups,
―O―, ―S― or 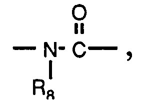 then n is different from the integer 1;
Z is ―S― or ―CR₂ = CR'₂―;
X and Y taken together are 0=, hydroxyimino, alkyloxyimino or alkenyloxyimino, or, independently, when one of X and Y is fluoro, the other is fluoro; or when one of X and Y is amino, the other is hydrogen; or when one of X and Y is hydroxy , the other is hydroxy; Q is phenyl, cyclohexyl, cyclooctyl, adamantyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, of which phenyl or naphthalenyl can be substituted by one or more of the following groups, C₁-C₇ alkyl, phenyl, acyloxy, acyloxyalkyl or hydroxyalkyl.

6. (S)-alpha-Amino-4-[[2-(cyclooctyloxy)ethyl]oxy]benzeneacetic acid hydrochloride.

7. 3-Fluor-4-[2-(2-naphthalenyloxy)ethoxy]-alpha-oxobenzeneacetic acid.

8. 4-[2-(1-Naphthalenyl)-2-oxoethoxy]-alpha-oxobenzeneacetic acid

9. 4-[[2-(2-Naphthalenyloxy)ethyl]oxy]-alpha-oxobenzeneacetic acid 2-(dimethylamino)ethyl ester.

10. (E)-4-[3-[2-[(2,2-Dimethyl-1-oxobutoxy)methyl]-6-methylphenyl]-2-propenyloxy]-alpha-oxobenzeneacetic acid(1:1) morpholine salt.

11. 4-[[2-(2-Naphthalenyloxy)ethyl]oxy]-alpha-oxobenzeneacetic acid.

12. 4-[[2-(2-Naphthalenyloxy)ethyl]thio]-alpha-oxobenzeneacetic acid.

13. 4-[[2-(2-Naphthalenylthio)ethyl]oxy]-alpha-oxobenzeneacetic acid.

14. 4-[[2-Cyclooctyloxy)ethyl]oxy]-alpha-oxobenzeneacetic acid.

15. 4-[3-(4-Acetyl-3-hydroxy-2-propylphenoxy)propoxy]-alpha-oxobenzeneacetic acid.

16. N-Hydroxy-N-methyl-4-[2-(2-naphthalenyloxy)ethoxy]-alpha-oxobenzeneacetamide.

17. (Z)-alpha-(Hydroxyimino)-5-[2-(2-naphthalenyloxy)ethoxy]-2-thiopheneacetic acid.

18. 5-[[2-(2-Naphthalenyloxy)ethyl]oxy]-alpha-oxo-2-thiopheneacetic acid.

19. alpha-Oxo-4-[[2-(phenoxy)ethyl]oxy]benzeneacetic acid.

20. (Z)-alpha-(Ethoxyimino)-5-[2-(2-naphthalenyloxy)ethoxy]-2-thiopheneacetic acid.

21. rac.-5-[[2-(2-Naphthalenyloxy)ethyl]oxy]-alpha-oxo-2-thiopheneacetic acid 2,3-dihydroxypropyl ester.

22. (S)-alpha-Amino-4-[2-[2-[(2,2-dimethyl-1-oxobutoxy)methyl]-6-methylphenoxy]-ethoxy]benzeneacetic acid.

23. (E)-4-[[3-(2-Naphthalenyl)-2-propenyl]oxy]-alpha-oxobenzeneacetic acid.

24. 4-[[2-[2-[(2,2-Dimethyl-1-oxobutoxy)methyl]-6-methylphenoxy]ethyl]oxy]-alpha-oxobenzeneacetic acid (2:1) hydrate.

25. 4-[[4-(2-Naphthalenyloxy)butyl]oxy]-alpha-oxobenzeneacetic acid.

26. 4-[2-(6-Hydroxy-2-naphthalenyloxy)ethoxy]-alpha-oxobenzeneacetic acid;
4-[2-(8-hydroxy-2-naphthalenyloxy)ethoxy]-alpha-oxobenzeneacetic acid;
6-[2-(2-naphthalenyloxy)ethoxy]-alpha-oxo-3-pyridineacetic acid;
4-[2-[2,4-dichloro-6-[(2,2-dimethyl-1-oxobutoxy)methyl]phenoxy]ethoxy]-alpha-oxobenzeneacetic acid;
4-[2-[2,4-dimethyl-6-[(2,2-dimethyl-1-oxobutoxy)methyl]phenoxy]ethoxy]-alpha-oxobenzeneacetic acid;
4-[2[2-[[[(4-fluorophenyl)carbonyl]oxy]methyl]phenoxy]ethoxy]-alpha-oxobenzeneacetic acid;
5-[2-[2-[[[(4-fluorophenyl)carbonyl]oxy]methyl]phenoxy]ethoxy]-alpha-oxo-2-thiopheneacetic acid; or
5-[2-[2-[(methoxycarbonyl)-6-methyl]phenoxy]ethoxy]-alpha-oxo-2-thiopheneacetic acid.

27. (E)-4-[[3-(4-Bromophenyl)-2-propenyl]oxy]-alpha-oxo-3-pyridineacetic acid;
(E)-4-[[4-(4-fluorophenyl)-2-butenyl]oxy]-alpha-oxobenzeneacetic acid;
(E)-alpha-oxo-4-[(3-phenyl-2-propenyl)oxy]thiopheneacetic acid;
(S)-alpha-amino-4-[[2-(2-naphthalenyloxy)ethyl]oxy]benzeneacetic acid;
(S)-alpha-amino-4-[[2-(phenoxy)ethyl]oxy]benzeneacetic acid hydrochloride;
(S)-alpha-amino-alpha-methyl-4-[[2-(2-naphthalenyloxy)ethyl]oxy]benzeneacetic acid;
(Z)-alpha-oxo-5-[(3-phenyl-2-propenyl)oxy]-2-thiopheneacetic acid;
(E)-4-[[3-(1-naphthalenyl)-2-propenyl]oxy]-alpha-oxobenzeneacetic acid;
alpha-oxo-5-[4-(3-pyridinyl)butoxy]-2-thiopheneacetic acid;
alpha-oxo-4-[2-(4-pyridinyl)ethoxy]benzeneacetic acid;
alpha-oxo-4-[[7-(phenoxy)heptyl]oxy]benzeneacetic acid;
alpha-oxo-4-[[8-(phenoxy)octyl]oxy]benzeneacetic acid;
alpha-oxo-5-[[2-(4-phenoxyphenoxy)ethyl]oxy]-2-furanacetic acid;
alpha-oxo-4-[[3-(4-quinolyloxy)propyl]oxy]benzeneacetic acid;
2-[[4-[[4-(1-naphthalenyloxy)butyl]oxy]-alpha-oxobenzeneacetyl]oxy]-N,N,N-trimethylethanaminium iodide;
5-[3-(2-naphthalenyloxy)propyl]-alpha-oxo-2-thiopheneacetic acid;
4-[[4-(1-naphthalenyloxy)butyl]oxy]-alpha-oxobenzeneacetic acid;
4-[[2-(2-chlorophenoxy)ethyl]oxy]-alpha-oxobenzeneacetic acid;
4-[[2-(2-fluorophenoxy)ethyl]oxy]-alpha-oxo-3-pyridineacetic acid;
4-[[2-(2-naphthalenyl)ethyl]amino]-alpha-oxobenzeneacetic acid;
5-[[2-(2-naphthalenyloxy)ethyl]oxy]-alpha-oxo-2-thiopheneacetamide;
4-[[3-(2-naphthalenyloxy)propyl]sulfonyl]-alpha-oxobenzeneacetic acid;
4-[[3-(2-naphthalenylthio)propyl]oxy]-alpha-oxobenzeneacetic acid;
5-[[2-(3,4,5-trimethoxyphenoxy)ethyl]oxy]-alpha-oxo-2-furanacetic acid;
4-[[2-(6-methoxy-2-naphthalenyloxy)ethyl]oxy]-alpha-oxobenzeneacetic acid;
4-[[2-(3-naphtho[2,3-b]thienyloxy)ethyl]oxy]-alpha-oxobenzeneacetic acid;
4-[[2-(7-isoquinolyl)ethyl]oxy]-alpha-oxobenzeneacetic acid;
4-[[3-(cyclohexyl)propyl]oxy]-alpha-oxobenzeneacetic acid;
4-[[3-(cyclooctyloxy)propyl]oxy]-alpha-oxobenzeneacetic acid;
4-[[2-[2-(hydroxymethyl)phenoxy]ethyl]oxy]-alpha-oxobenzeneacetic acid;
4-[[2-[2-[(2,2-dimethyl-1-oxopropoxy)methyl]-6-methylphenoxy] ethyl]oxy]-alpha-oxobenzeneacetic acid;
4-[[2-[4-(methylaminosulfonyl)phenoxy]ethyl]oxy]-alpha-oxobenzeneacetic acid
4-[[2-[8-(2,2-dimethyl-1-oxopropoxy)-2-naphthalenyloxy]ethyl]oxy]-alpha-oxobenzeneacetic acid;
4-[[3-(2-dimethylaminophenyl)propyl]oxy]-alpha-oxobenzeneacetic acid:
5-[[4-(2-naphthalenyl)butyl]oxy]-alpha-oxo-2-thiopheneacetic acid;
4-[[3-(4-cyanophenyl)propyl]oxy]-alpha-oxobenzeneacetic acid
4-[[3-(4-nitrophenoxy)propyl]oxy]-alpha-oxobenzeneacetic acid;
4-[[3-[2-(trifluoromethyl)phenyl]propyl]oxy]-alpha-oxobenzeneacetic acid;
4-[[3-[4-(N,N-dimethylsulfamoyl)phenoxy]ethyl]oxy]-alpha-oxobenzeneacetic acid;
4-[[3-[4-(N-ethylsulfamoyl)phenoxy]ethyl]oxy]-alpha-oxobenzeneacetic acid;
alpha-oxo-4-[[4-(phenylthio)butyl]oxy]benzeneacetic acid;
alpha-oxo-4-[[4-(2-pyrimidyl)butyl]oxy]benzeneacetic acid;
4-[[4-(3-fluorophenoxy)butyl]oxy]-alpha-oxobenzeneacetic acid;
4-[[5-(4-fluorophenoxy)pentyl]oxy]-alpha-oxobenzeneacetic acid;
4-methyl-5-[[2-(2-naphthalenyloxy)ethyl]oxy]-alpha-oxo-2-thiopheneacetic acid;
5-[(phenylmethyl)oxy]-alpha-oxo-2-furanacetic acid;
5-[[2-(2-naphthalenyloxy)ethyl]oxy]-alpha-oxo-2-thiopheneacetic acid 2-(dimethylamino)ethyl ester;
5-[[2-(3-carbazolyloxy)ethyl]oxy]-alpha-oxo-2-thiopheneacetic acid;
5-[[2-(cyclooctyloxy)ethyl]thio]-alpha-oxo-2-thiopheneacetic acid;
5-[[3-(cycloheptyloxy)propyl]oxy]-alpha-oxo-2-thiopheneacetic acid potassium salt;
alpha, alpha-difluoro-4-[[2-(3-methylphenoxy)ethyl]oxy]benzeneacetic acid;
alpha, alpha-difluoro-4-[[2-(3-naphtho[2,3,-b]thienyloxy)ethyl]oxy]benzeneacetic acid;
alpha, alpha-dimethoxy-4-[[2-(phenoxy)ethyl]oxy]benzeneacetic acid
alpha-oxo-4-[(3-phenyl-2-propynyl)oxy]thiopheneacetic acid;
alpha-oxo-4-[[2-(2-xanthenyloxy)ethyl]oxy]benzeneacetic acid (1:1) diethanolamine salt;
alpha-oxo-4-[[2-(4-trifluoromethylphenoxy)ethyl]oxy]-2-furanacetic acid;
alpha-oxo-4-[[6-(phenoxy)hexyl]oxy]benzeneacetic acid;
4-[[2-(3-benzo[b]thienyloxy)ethyl]oxy]-alpha-oxobenzeneacetic acid
rac.-5-[[2-(3-indolyloxy)ethyl]oxy]-alpha-oxo-2-thiopheneacetic acid 2,3-dihydroxypropyl ester;
rac.-alpha-chloro-alpha-methyl-4-[[2-(phenoxy)ethyl]oxy]benzeneacetic acid;
rac.-alpha-ethoxy-4-[(2-phenylethyl)oxy]benzeneacetic acid; or
rac.-alpha-oxo-4-[[2-(1,2,3,4-tetrahydro-2-naphthalenyloxy)ethyl]oxy]benzeneacetic acid.

28. Compounds in accordance with any one of claims 1-27 for use as therapeutically active substances.

29. Compounds in accordance with any one of claims 1-27 for use in the control or prevention of injury to ischemic tissue and arrhythmias during and following a myocardial infarction.

30. A process for the manufacture of a compound in accordance with any one of claims 1-27, which comprises
(a) for the manufacture of a compound of formula I wherein R₁ is hydroxy and the remaining symbols are as described in claim 1, saponifying a compound of the general formula 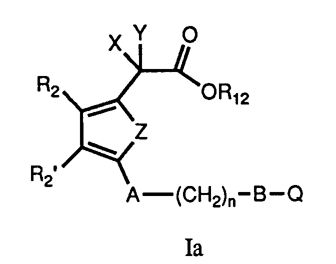 wherein A, B, Q, R₂, R₂', X, Y, Z and n are as described in claim 1 and R₁₂ is C₁-C₇ alkyl,
or
(b) for the manufacture of a compound of formula I wherein R₁ is OR₃ and the remaining symbols are as described in claim 1, treating a compound of the general formula 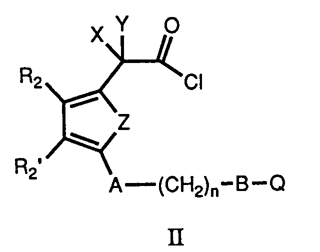 wherein A, B, Q, R₂, R₂', X, Y, Z and n are as described in claim 1, with an alcohol of the general formula
HO-R₃ IV
wherein R₃ is as described in claim 1,
or
(c) for the manufacture of a compound of formula I wherein R₁ is 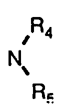 and the remaining symbols are as described in claim 1, treating a compound of the general formula 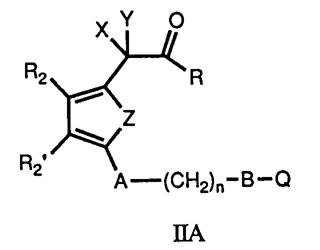 wherein A, B, Q, R₂, R₂', X, Y, Z and n are as described in claim 1 and R is chloro or C₁-C₇ alkoxy,
with an amine of the general formula 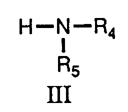 wherein R₄ and R₅ are as described in claim 1,
or
(d) for the manufacture of a compound of formula I wherein R₁ is hydroxy, A is -O- or -S-, X and Y taken together are O= and Z is -CR₂=CR₂'- and the remaining symbols are as described in claim 1, reacting a compound of the general formula 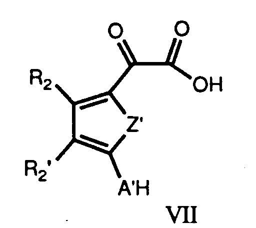 wherein R₂ and R₂' are as described in claim 1 and A' is -O- or -S- and Z' is
-CR₂=CR₂'-
with a compound of the general formula 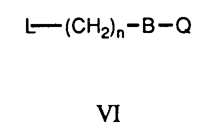 wherein B, Q and n are as described in claim 1 and L is a leaving group,
or
(e) for the manufacture of a compound of formula I wherein R₁ is hydroxy, A is -O- or -S-, X and Y taken together are O= and Z is -CR₂=CR₂'- and the remaining symbols are as described in claim 2, oxidizing a compound of the general formula 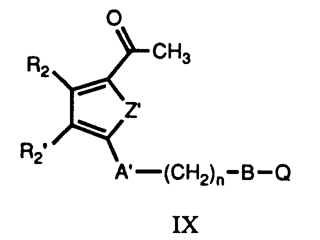 wherein B, Q, R₂, R₂' and n are as described in claim 1 and A' and Z' are as described above,
or
(f) for the manufacture of a compound of formula I wherein R₁ is hydroxy, A is 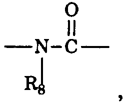 X and Y taken together are O= and Z is -CR₂=CR₂'- and the remaining symbols are as described in claim 1, oxidizing a compound of the general formula 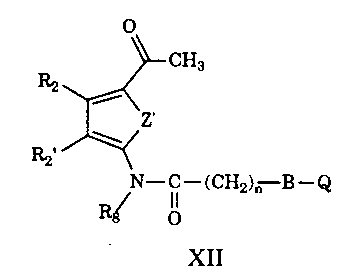 wherein B, Q, R₂, R₂', R₈ and n are as described in claim 1 and Z' is as described above,
or
(g) for the manufacture of a compound of formula I wherein R₁ is hydroxy, A is -O-, X is amino and Z is -CR₂=CR₂'- and the remaining symbols are as described in claim 1, splitting off the amino acid O-protecting group from a compound of the general formula 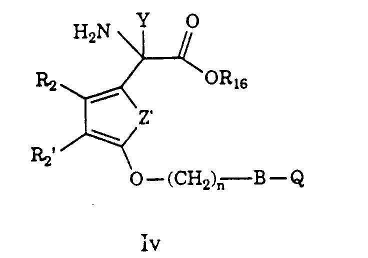 wherein B, Q, R₂, R₂', Y and n are as described in claim 1 and R₁₆ is an amino acid O-protecting group,
and, if desired,
(h) converting a compound obtained into a pharmaceutically acceptable salt.

31. A medicament containing a compound in accordance with any one of claim 1-27 and a therapeutically inert excipient.

32. A medicament for the control or prevention of injury to ischemic tissue and arrhythmias during and following a myocardial infarction, containing a compound in accordance with any one of claims 1-27 and a therapeutically inert excipient.

33. The use of compound in accordance with any one of claims 1-27 for the manufacture of medicaments for the control or prevention of illnesses.

34. The use of a compound in accordance with any one of claims 1-27 for the manufacture of medicaments against injury to ischemic tissue and/or arrhythmias during and following a myocardial infarction.

## Claims (Claims for the following Contracting State(s): ES, GR)

1. A process for the manufacture of compounds of the general formula 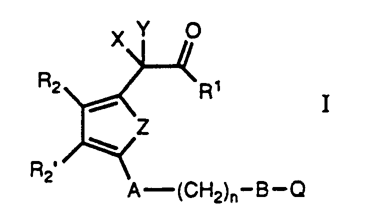 wherein R₁ is hydroxy, OR₃ or 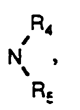 wherein one of R₄ and R₅ is hydrogen, C₁-C₇ alkyl or hydroxy-C₁-C₇ alkyl, and the other is hydrogen, hydroxy, C₁-C₇ alkyl or C₁-C₇ alkoxy, and wherein R₃ is (̵CH₂CH₂O)̵ₘH, 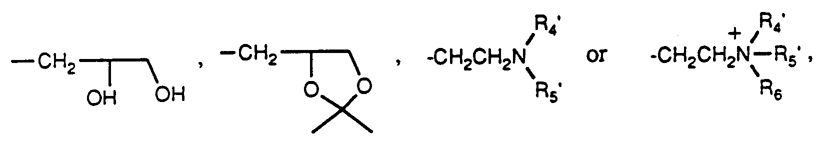 and
wherein R₄', R₅', and R₆ are, independently, hydrogen , C₁-C₇ alkyl or hydroxy C₁-C₇ alkyl, and wherein m is an integer from 1 to 4;
R₂ and R₂', independently, are hydrogen, C₁-C₇ alkyl, aryl, aryl-C₁-C₇ alkyl, C₁-C₇ alkoxy, hydroxy, amino, C₁-C₇ alkylamino, di-C₁-C₇ alkylamino, cyano, halogen, mercapto, C₁-C₇ alkylthio, C₁-C₇ alkylsulfinyl, C₁-C₇ alkylsulfonyl, trihalo-C₁-C₇ alkyl, acyl or nitro;
A is a bond,
―O―, ―NR₇―, ―S―, 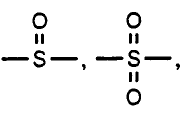 ―C≡C―, ―HC=CH―, ―CH₂CH₂―, 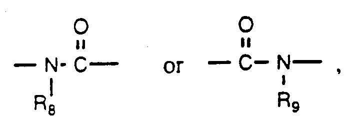 wherein R₇ is hydrogen, C₁-C₇ alkyl or acyl and R₈ and R₉ are, independently, hydrogen or C₁-C₇ alkyl; n is an integer from 0 to 10;
B is a bond, ―O―, ―NR₇, ―S―, 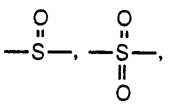 ―C≡C―, ―HC=CH―, 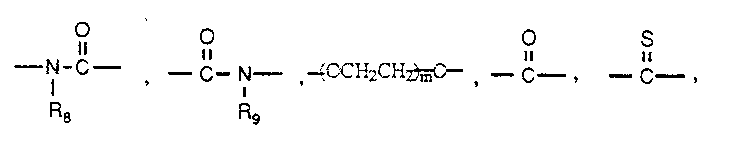 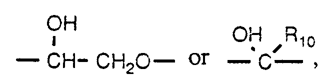 wherein R₇, R₈, R₉ and m are as defined above and R₁₀ is hydrogen or C₁-C₇ alkyl;
Z is ―O―, ―S―, ―CR₂ = CR₂'―, ―N = CR₂―, ―CR₂ = N― or 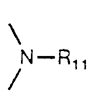 wherein R₁₁ is hydrogen or lower alkyl;
X and Y taken together are 0=, S=, hydroxyimino, alkyloxyimino alkenyloxyimino, arylalkoxyimino, hydrazono, mono-C₁-C₇ alkyl hydrazono, di-C₁-C₇ alkyl hydrazono or semicarbazono, or, independently, when one of X and Y is halogen, the other is hydrogen, halogen, C₁-C₇ alkyl or aryl-C₁-C₇ alkyl; or when one of X and Y is amino, C₁-C₇ alkylamino or di-C₁-C₇ alkylamino, the other is hydrogen, C₁-C₇ alkyl or aryl-C₁-C₇ alkyl; or when one of X and Y is hydroxy, alkoxy or aryl-C₁-C₇ alkoxy, the other is hydrogen, hydroxy, C₁-C₇ alkyl, C₁-C₇ alkoxy or aryl-C₁-C₇ alkoxy; Q is a cycloalkyl, aryl or heterocyclic radical; provided that, when A is oxygen (O) and B is a bond, sulfur (S) or oxygen (O), then n is 2-10; when appropriate, in the form of enantiomers, racemates, diastereomers or mixtures thereof or geometric isomers or mixtures thereof, and, when a basic or acidic group is present, pharmaceutically acceptable salts thereof, which comprises
(a) for the manufacture of compounds of formula I wherein R₁ is hydroxy and the remaining symbols are as previously described, saponifying a compound of the general formula 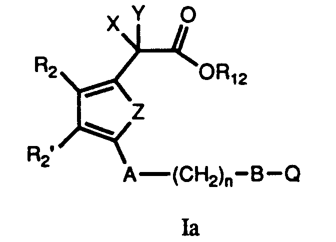 wherein A, B, Q, R₂, R₂', X, Y, Z and n are as previously described and R₁₂ is lower alkyl,
or
(b) for the manufacture of compounds of formula I wherein R₁ is OR₃ and the remaining symbols are as previously described, treating a compound of the general formula 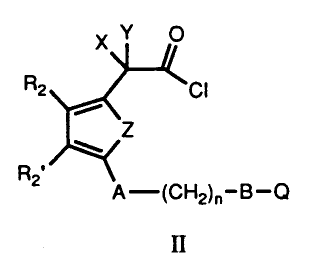 wherein A, B, Q, R₂, R₂', X, Y, Z and n are as previously described, with an alcohol of the general formula
HO-R₃ IV
wherein R₃ is as previously described,
or
(c) for the manufacture of compounds of formula I wherein R₁ is 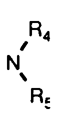 and the remaining symbols are as previously described, treating a compound of the general formula 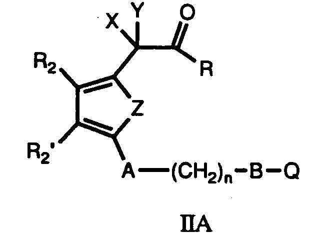 wherein A, B, Q, R₂, R₂', X, Y, Z and n are as previously described and R is chloro or C₁-C₇ alkoxy,
with an amine of the general formula 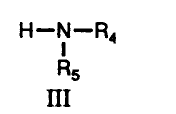 wherein R₄ and R₅ are as previously described,
or
(d) for the manufacture of compounds of formula I wherein R₁ is hydroxy, A is -O- or -S-, X and Y taken together are O= and Z is -CR₂=CR₂'- and the remaining symbols are as previously described, reacting a compound of the general formula 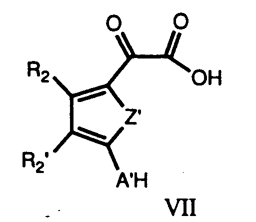 wherein R₂ and R₂' are as previously described and A' is -O- or -S- and Z' is
-CR₂=CR₂'-
with a compound of the general formula 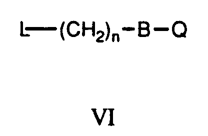 wherein B, Q and n are as previously described and L is a leaving group,
or
(e) for the manufacture of compounds of formula I wherein R₁ is hydroxy, A is -O- or -S-, X and Y taken together are O= and Z is -CR₂=CR₂'- and the remaining symbols are as previously described, oxidizing a compound of the general formula 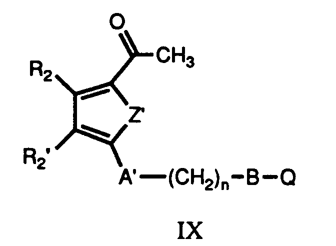 wherein A', B, Q, R₂, R₂', Z' and n are as previously described,
or
(f) for the manufacture of compounds of formula I wherein R₁ is hydroxy, A is 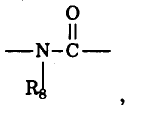 X and Y taken together are O= and Z is -CR₂=CR₂'- and the remaining symbols are as previously described, oxidizing a compound of the general formula 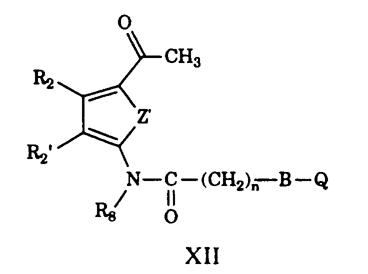 wherein B, Q, R₂, R₂', R₈, Z' and n are as previously described,
or
(g) for the manufacture of compounds of formula I wherein R₁ is hydroxy, A is -O-, X is amino and Z is -CR₂=CR₂'- and the remaining symbols are as previously described, splitting off the amino acid O-protecting group from a compound of the general formula 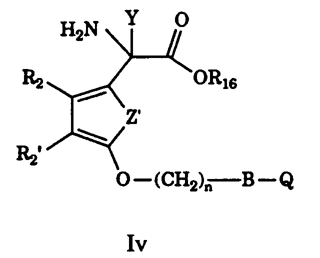 wherein B, Q, R₂, R₂', Y and n are as previously described and R₁₆ is an amino acid O-protecting group,
and, if desired,
(h) converting a compound obtained into a pharmaceutically acceptable salt..

2. A process in accordance with claim 1, wherein one of R₄ and R₅ is hydrogen, C₁-C₇ alkyl or hydroxy-C₁-C₇ alkyl and the other is hydrogen or C₁-C₇ alkyl, X and Y taken together are O=, S= or, independently, when one of X and Y is halogen, the other is hydrogen, halogen, C₁-C₇ alkyl or C₁-C₇-lower alkyl; or when one of X and Y is amino, C₁-C₇ alkylamino or di-C₁-C₇ alkylamino, the other is hydrogen, C₁-C₇ alkyl or aryl-C₁-C₇ alkyl; or when one of X and Y is hydroxy, alkoxy or aryl-C₁-C₇ alkoxy, the other is hydrogen, hydroxy, C₁-C₇ alkyl, C₁-C₇ alkoxy or aryl-C₁-C₇ alkoxy.

3. A process in accordance with claim 1, wherein R₁ is as previously described; R₂ and R₂', independently, are hydrogen, C₁-C₇ alkyl, aryl, C₁-C₇ alkoxy, hydroxy, halogen, acyl or nitro;
A is a bond,
―O―, ―NR₇―, ―S―, 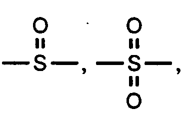 ―C≡C―, ―HC=CH―, ―CH₂CH₂―, 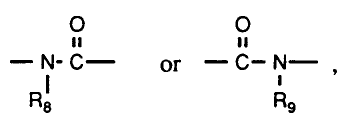 wherein R₇ is hydrogen, C₁-C₇ alkyl or acyl and R₈ and R₉ are, independently, hydrogen or C₁-C₇ alkyl;
B is a bond, ―O―, ―NR₇―, ―S―, ―C≡C―, ―HC=CH―, 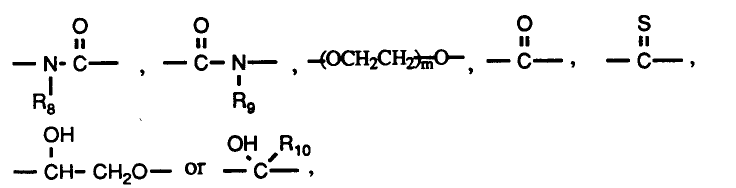 wherein R₇, R₈, R₉ and m are as previously described and R₁₀ is hydrogen;
n is an integer from 1 to 6, whereby when A is a bond or one of the following groups,
―O―, ―NR₇―, ―S―, 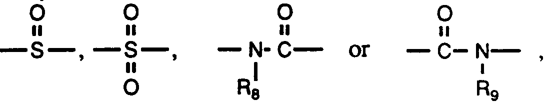 and B is a bond or one of the following groups
―O―, ―NR₇―, ―S―, 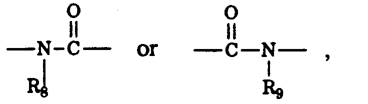 then n is different from the integer 1;
Z is ―S―, ―CR₂ = CR₂'―, ―N = CR₂― or ―CR₂ = N―;
X and Y taken together are 0=, hydroxyimino, alkyloxyimino, alkenyloxyimino, arylalkoxyimino, hydrazono, mono-C₁-C₇ alkyl hydrazono, di-C₁-C₇ alkyl hydrazono or semicarbazono, or, independently, when one of X and Y is halogen, the other is hydrogen or halogen; or when one of X and Y is amino, the other is hydrogen; or when one of X and Y is hydroxy or alkoxy, the other is hydrogen or hydroxy;
Q is a cycloalkyl, aryl or heterocyclic radical, which radical can be substituted by one or more of the following groups: alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl, carboxycarbonyl or alkoxalyl.

4. A process in accordance with claim 3, wherein R₁ is as previously described; R₂ and R₂', independently, are hydrogen, C₁-C₇ alkyl, C₁-C₇ alkoxy, hydroxy or halogen;
A is a bond,
―O―, ―NR₇―, ―S―, 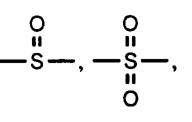 ―C≡C―, ―HC=CH―, ―CH₂CH₂―, 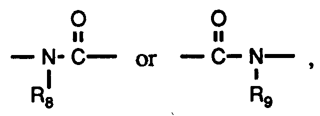 wherein R₇ is hydrogen or C₁-C₇ alkyl and R₈ and R₉ are hydrogen;
B is a bond,
―O―, ―S―, ―C≡C―, ―HC=CH―, 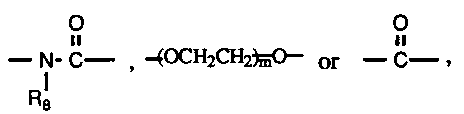 wherein m is 1 or 2 and R₈ is hydrogen;
n is an integer from 1 to 6, whereby when A is a bond or one of the following groups,
―O―, ―NR₇―, ―S―, 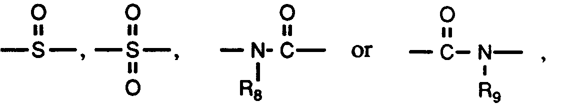 and B is a bond or one of the following groups
―O―, ―S― or 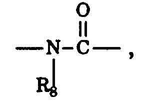 then n is different from the integer 1;
Z is ―S―, or ―CR₂ = CR₂'―,
X and Y taken together are 0= , hydroxyimino, alkyloxyimino or alkenyloxyimino, or, independently, when one of X and Y is halogen, the other is halogen; or when one of X and Y is amino, the other is hydrogen; or when one of X and Y is hydroxy , the other is hydrogen or hydroxy;
Q is phenyl, cyclohexyl, cyclooctyl, pyridinyl, adamantyl, 1,1'-biphenyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, which radical can be substituted by one or more of the following groups; alkyl, alkoxy, acyloxy, halogen, acyloxyalkyl, alkoxyalkyl, aryloxyalkoxy, hydroxy, hydroxyalkyl, hydroxyalkoxy, phenyl, trihaloalkyl, sulfamoyl carboxycarbonyl or alkoxalyl.

5. A process in accordance with claim 4, wherein R₁ is hydroxy or 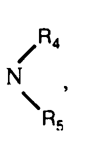 wherein one of R₄ and R₅ is hydrogen, C₁-C₇ alkyl or hydroxy-C₁-C₇ alkyl, and the other is hydroxy;
R₂ and R₂' are hydrogen;
A is ―O―, ―NR₇― or ―S―, wherein R₇ is hydrogen;
B is a bond,
―O―, ―S―, ―C≡C―, ―HC=CH―, 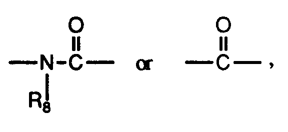 wherein R₈ is hydrogen;
n is an integer from 1 to 4, whereby when B is a bond or one of the following groups,
―O―, ―S― or 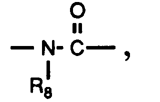 then n is different from the integer 1;
Z is ―S― or ―CR₂ = CR'₂―;
X and Y taken together are 0=, hydroxyimino, alkyloxyimino or alkenyloxyimino, or, independently, when one of X and Y is fluoro, the other is fluoro; or when one of X and Y is amino, the other is hydrogen; or when one of X and Y is hydroxy , the other is hydroxy; Q is phenyl, cyclohexyl, cyclooctyl, adamantyl, anthracenyl, phenanthrenyl, naphthalenyl, 5,6,7,8-tetrahydro-1-naphthalenyl, 5,6,7,8-tetrahydro-2-naphthalenyl, 1,2,3,4-tetrahydro-1-naphthalenyl, 1,2,3,4-tetrahydro-2-naphthalenyl, quinolyl or isoquinolyl, of which phenyl or naphthalenyl can be substituted by one or more of the following groups, lower alkyl, phenyl, acyloxy, acyloxyalkyl or hydroxyalkyl.

6. A process in accordance with claim 2, wherein (S)-alpha-amino-4-[[2-(cyclooctyloxy)ethyl]oxy]benzeneacetic acid hydrochloride is prepared.

7. A process in accordance with claim 1, wherein 3-fluoro-4-[2-(2-naphthalenyloxy)ethoxy]-alpha-oxobenzeneacetic acid is prepared.

8. A process in accordance with claim 1, wherein 4-[2-(1-naphthalenyl)-2-oxoethoxy]-alpha-oxobenzeneacetic acid is prepared.

9. A process in accordance with claim 2, wherein 4-[[2-(2-naphthalenyloxy)ethyl]oxy]-alpha-oxobenzeneacetic acid 2-(dimethylamino)ethyl ester is prepared.

10. A process in accordance with claim 1, wherein (E)-4-[3-[2-[(2,2-dimethyl-1-oxobutoxy)methyl]-6-methylphenyl]-2-propenyloxy]-alpha-oxobenzeneacetic acid(1:1) morpholine salt is prepared.

11. A process in accordance with claim 2, wherein 4-[[2-(2-naphthalenyloxy)ethyl]oxy]-alpha-oxobenzeneacetic acid is prepared.

12. A process in accordance with claim 2, wherein 4-[[2-(2-naphthalenyloxy)ethyl]thio]-alpha-oxobenzeneacetic acid is prepared.

13. A process in accordance with claim 2, wherein 4[[2-(2-naphthalenylthio)ethyl]oxy]-alpha-oxobenzeneacetic acid is prepared.

14. A process in accordance with claim 2, wherein 4-[[2-cyclooctyloxy)ethyl]oxy]-alpha-oxobenzeneacetic acid is prepared.

15. A process in accordance with claim 1, wherein 4-[3-(4-acetyl-3-hydroxy-2-propylphenoxy)propoxy]-alpha-oxobenzeneacetic acid is prepared.

16. A process in accordance with claim 1, wherein N-hydroxy-N-methyl-4-[2-(2-naphthalenyloxy)ethoxy]-alpha-oxobenzeneacetamide is prepared.

17. A process in accordance with claim 1, wherein (Z)-alpha-(hydroxyimino)-5-[2-(2-naphthalenyloxy)ethoxy]-2-thiopheneacetic acid is prepared.

18. A process in accordance with claim 2, wherein 5-[[2-(2-naphthalenyloxy)ethyl]oxy]-alpha-oxo-2-thiopheneacetic acid is prepared.

19. A process in accordance with claim 2, wherein alpha-oxo-4-[[2-(phenoxy)ethyl]oxy]benzeneacetic acid is prepared.

20. A process in accordance with claim 1, wherein (Z)-alpha-(ethoxyimino)-5-[2-(2-naphthalenyloxy)ethoxy]-2-thiopheneacetic acid is prepared.

21. A process in accordance with claim 2, wherein rac.-5-[[2-(2-naphthalenyloxy)ethyl]oxy]-alpha-oxo-2-thiopheneacetic acid 2,3-dihydroxypropyl ester is prepared.

22. A process in accordance with claim 1, wherein (S)-alpha-smino-4-[2-[2-[(2,2-dimethyl-1-oxobutoxy)methyl]-6-methylphenoxy]-ethoxy]benzeneacetic acid is prepared.

23. A process in accordance with claim 2, wherein (E)-4-[[3-(2-naphthalenyl)-2-propenyl]oxy]-alpha-oxobenzeneacetic acid is prepared.

24. A process in accordance with claim 2, wherein 4-[[2-[2-[(2,2-dimethyl-1-oxobutoxy)methyl]-6-methylphenoxy]ethyl]oxy]-alpha-oxobenzeneacetic acid (2:1) hydrate is prepared.

25. A process in accordance with claim 2, wherein 4-[[4-(2-naphthalenyloxy)butyl]oxy]-alpha-oxobenzeneacetic acid is prepared.

26. A process for the manufacture of a medicament, particularly to be used in the control or prevention of injury to ischemic tissue and arrhythmias during and following a myocardial infarction, which process comprises bringing a compound of formula I set forth in claim 1 in the form of an enantiomer, a racemate, a diastereomer or a mixture thereof or a geometric isomer or a mixture thereof or, when a basic or acidic group is present, a pharmaceutically acceptable salt of such a compound into a galenical dosage form.

27. The use of a compound of formula I set forth in claim 1 in the form of an enantiomer, a racemate, a diastereomer or a mixture thereof, or a geometric isomer or a mixture thereof or, when a basic or acidic group is present, of a pharmaceutically acceptable salt of such a compound for the manufacture of medicaments against injury to ischemic tissue and/or arrhythmias during and following a myocardial infarction.

## Patentansprüche (Patentansprüche für folgende(n) Vertragsstaat(en): AT, BE, CH, DE, DK, FR, GB, IT, LI, LU, MC, NL, PT, SE)

1. Verbindungen der allgemeinen Formel 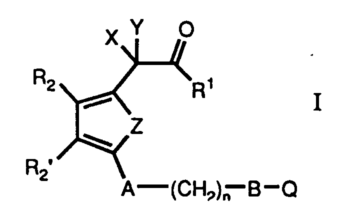 worin
R₁ Hydroxy, OR₃ oder 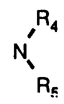 bedeutet, wobei einer der Reste R₄ und R₅ Wasserstoff, C₁-C₇-Alkyl oder Hydroxy-C₁-C₇-alkyl und der andere Wasserstoff, Hydroxy, C₁-C₇-Alkyl oder C₁-C₇-Alkoxy und wobei R₃ (̵CH₂CH₂O)̵ₘH 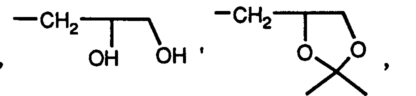 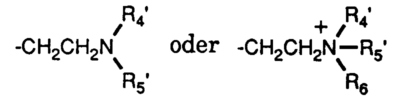 bedeutet, wobei
R₄', R₅', und R₆ unabhängig voneinander Wasserstoff, C₁-C₇-Alkyl oder Hydroxy-C₁-C₇-Alkyl bedeutet und m eine ganze Zahl von 1-4 bedeutet;
R₂ und R₂' unabhängig voneinander Wasserstoff, C₁-C₇-Alkyl, Aryl, Aryl-C₁-C₇-alkyl, C₁-C₇-Alkoxy, Hydroxy, Amino, C₁-C₇-Alkylamino, di-C₁-C₇-Alkylamino, Cyano, Halogen, Mercapto, C₁-C₇-Alkylthio, C₁-C₇-Alkylsulfinyl, C₁-C₇-Alkylsulfonyl, Trihalogen-C₁-C₇-Alkyl, Acyl oder Nitro bedeuten;
A eine Bindung,―O―, ―NR₇―, ―S―, 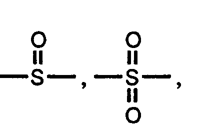 ―C≡C―, ―HC=CH―, ―CH₂CH₂―, 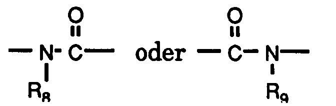 bedeutet, worin R₇ Wasserstoff, C₁-C₇-Alkyl oder Acyl und R₈ und R₉ unabhängig voneinander Wasserstoff oder C₁-C₇-Alkyl bedeutet;
n eine ganze Zahl von 1-10 bedeutet;
B eine Bindung,―O―, ―NR₇―, ―S―, 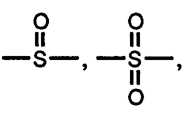 ―C≡C―, ―HC=CH―, 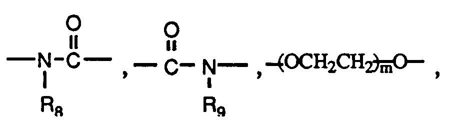 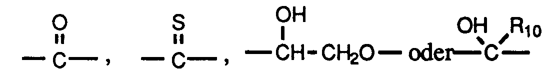 bedeutet, worin R₇, R₈, R₉ und m die oben angegebene Bedeutung haben und R₁₀ Wasserstoff oder C₁-C₇-Alkyl bedeutet;
Z ―O―, ―S―, ―CR₂ = CR'₂―, ―N=CR₂―, ―CR₂ = N― oder 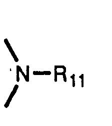 bedeutet, worin R₁₁ Wasserstoff oder C₁-C₇-Alkyl bedeutet;
X und Y zusammen 0= , S=, Hydroxyimino, Alkyloxyimino, Alkenyloxyimino, Arylalkoxyimino, Hydrazono, mono-C₁-C₇-Alkylhydrazono, di-C₁-C₇-Alkylhydrazono oder Semicarbazono bedeuten, oder unabhängig voneinander, wenn einer der Reste X und Y Halogen ist, ist der andere Rest Wasserstoff, Halogen, C₁-C₇-Alkyl oder Aryl-C₁-C₇-Alkyl; oder wenn einer der Reste X und Y Amino, C₁-C₇-Alkylamino oder di-C₁-C₇-Alkylamino ist, ist der andere Rest Wasserstoff, C₁-C₇-Alkyl oder Aryl-C₁-C₇-Alkyl; oder wenn einer der Reste X und Y Hydroxy, Alkoxy oder Aryl-C₁-C₇-Alkoxy ist, ist der andere Rest Wasserstoff, Hydroxy, C₁-C₇-Alkyl, C₁-C₇-Alkoxy oder Aryl-C₁-C₇-alkoxy;
Q Cycloalkyl, Aryl oder ein heterocyclisches Radical bedeutet; mit der Massgabe, dass falls A Sauerstoff (O) und B eine Bindung, Schwefel, (S) oder Sauerstoff (O) ist, dann ist n 2-10;
sowie gegebenenfalls Enantiomere, Racemate, Diastereomere und Mischungen davon oder geometrische Isomere und Mischungen davon und falls eine basische oder saure Gruppe vorhanden ist pharmazeutisch anwendbare Salze der Verbindungen.

2. Verbindungen gemäss Anspruch 1, wobei einer der Reste R₄ und R₅ Wasserstoff, C₁-C₇-Alkyl oder Hydroxy-C₁-C₇-alkyl und der andere Wasserstoff oder C₁-C₇-Alkyl bedeutet; X und Y zusammen 0= , S= bedeuten, oder unabhängig voneinander, wenn einer der Reste X und Y Halogen ist, ist der andere Rest Wasserstoff, Halogen, C₁-C₇-Alkyl oder Aryl-C₁-C₇-alkyl; oder wenn einer der Reste X und Y Amino, C₁-C₇-Alkylamino oder di-C₁-C₇-Alkylamino ist, ist der andere Rest Wasserstoff, C₁-C₇-Alkyl oder Aryl-C₁-C₇-alkyl; oder wenn einer der Reste X und Y Hydroxy, Alkoxy oder Aryl- C₁-C₇-alkoxy ist, ist der andere Rest Wasserstoff, Hydroxy, C₁-C₇-Alkyl, C₁-C₇-Alkoxy oder Aryl-C₁-C₇-alkoxy.

3. Verbindungen gemäss Anspruch 1, worin R₁ die oben angegebene Bedeutung hat; R₂ und R₂'unabhängig voneinander Wasserstoff, C₁-C₇-Alkyl, Aryl, C₁-C₇-Alkoxy, Hydroxy, Halogen, Acyl oder Nitro bedeuten;
A eine Bindung
―O―, ―NR₇―, ―S―, 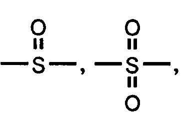 ―C≡C―, ―HC=CH―, ―CH₂CH₂―, 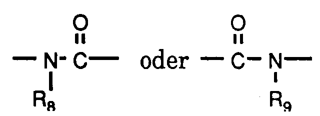 bedeutet, worin R₇ Wasserstoff, C₁-C₇-Alkyl oder Acyl und R₈ und R₉ unabhängig voneinander Wasserstoff oder C₁-C₇-Alkyl bedeuten;
B eine Bindung, ―O―, ―NR₇―, ―S―, ―C≡C―, ―HC=CH―, 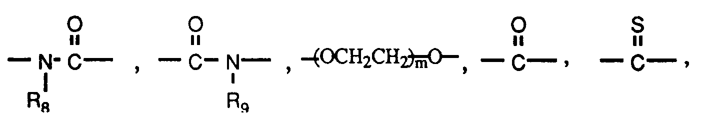 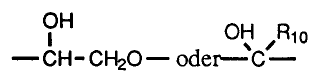 bedeutet, worin R₇, R₈, R₉ und m die in Anspruch 1 angegebene Bedeutung haben und R₁₀ Wasserstoff ist;
n eine ganze Zahl von 1-6 bedeutet, mit der Massgabe, dass falls A eine Bindung oder eine der folgenden Gruppen
―O―, ―NR₇―, ―S―_{,} 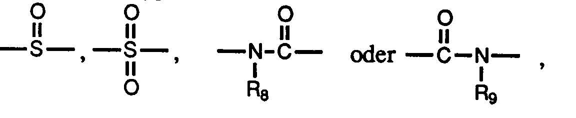 und B eine Bindung oder eine der folgenden Gruppen
―O―, ―NR₇―, ―S―, 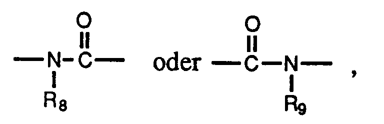 bedeutet, n ungleich 1 ist;
Z ―S―, ―CR₂ = CR₂'―, ―N = CR₂ ― oder ―CR₂ = N―
bedeutet;
X und Y zusammen 0=, S=, Hydroxyimino, Alkyloxyimino, Alkenyloxyimino, Arylalkoxyimino, Hydrazono, mono-C₁-C₇-Alkylhydrazono, di-C₁-C₇-Alkylhydrazono oder Semicarbazono bedeuten, oder unabhängig voneinander, wenn einer der Reste X und Y Halogen ist, ist der andere Rest Wasserstoff oder Halogen; oder wenn einer der Reste X und Y Amino ist, ist der andere Rest Wasserstoff; oder wenn einer der Reste X und Y Hydroxy oder Alkoxy ist, ist der andere Rest Wasserstoff oder Hydroxy;
Q Cycloalkyl, Aryl oder ein heterocyclisches Radical bedeutet; wobei das heterocyclische Radical durch eine oder mehrere der folgenden Gruppen substituiert sein kann: Alkyl, Alkoxy, Acyloxy, Halogen, Acyloxyalkyl, Alkoxyalkyl, Aryloxyalkoxy, Hydroxy, Hydroxyalkyl, Hydroxyalkoxy, Phenyl, Trihalogenalkyl, Sulfamoyl, Carboxycarbonyl oder Alkoxalyl.

4. Verbindungen gemäss Anspruch 3, worin R₁ die in Anspruch 1 angegebene Bedeutung hat; R₂ und R₂'unabhängig voneinander Wasserstoff, C₁-C₇-Alkyl, C₁-C₇-Alkoxy, Hydroxy oder Halogen bedeuten;
A eine Bindung,
―O―, ―NR₇―, ―S―, 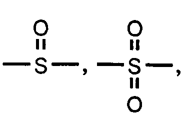 ―C≡C―, ―HC=CH―, ―CH₂CH₂―, 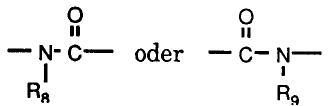 bedeutet, worin R₇ Wasserstoff oder C₁-C₇-Alkyl und R₈ und R₉ Wasserstoff bedeuten; B eine Bindung
―O―, ―S―, ―C≡C―, ―HC=CH―, 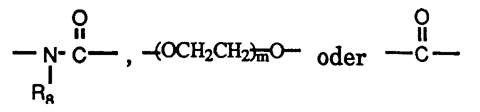 bedeutet, worin m 1 oder 2 und R₈ Wasserstoff bedeutet ;
n eine ganze Zahl von 1-6 bedeutet, mit der Massgabe, dass falls A eine Bindung oder eine der folgenden Gruppen
―O―, ―NR₇―, ―S―, 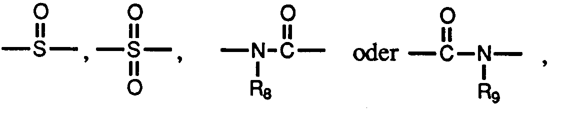 und B eine Bindung oder eine der folgenden Gruppen bedeutet
―O―, ―S― oder 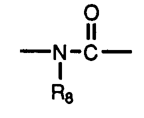
n ungleich 1 ist;
Z ―S― oder ―CR₂ = CR'₂― bedeutet;
X und Y zusammen 0= , Hydroxyimino, Alkyloxyimino oder Alkenyloxyimino bedeuten, oder unabhängig voneinander, wenn einer der Reste X und Y Amino ist, ist der andere Rest Wasserstoff, oder wenn einer der Reste X und Y Hydroxy ist, ist der andere Rest Wasserstoff oder Hydroxy;
Q Phenyl, Cyclohexyl, Cyclooctyl, Pyridyl, Adamantyl, 1,1'-Biphenyl, Anthryl, Phenanthryl, Naphthyl, 5,6,7,8-Tetrahydro-1-naphthyl, 5,6,7,8-Tetrahydro-2-naphthyl, 1,2,3,4-Tetrahydro-1-naphthyl, 1,2,3,4-Tetrahydro-2-naphthyl, Chinolyl oder Isochinolyl, wobei das heterocyclische Radical durch eine oder mehrere der folgenden Gruppen substituiert sein kann: Alkyl, Alkoxy, Acyloxy, Halogen, Acyloxyalkyl, Alkoxyalkyl, Aryloxyalkoxy, Hydroxy, Hydroxyalkyl, Hydroxyalkoxy, Phenyl, Trihalogenalkyl, Sulfamoyl, Carboxycarbonyl oder Alkoxalyl.

5. Verbindungen gemäss Anspruch 4, worin R₁ Hydroxy oder 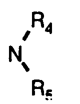 bedeutet, wobei einer der Reste R₄ und R₅ Wasserstoff, C₁-C₇-Alkyl oder Hydroxy-C₁-C₇-alkyl und der andere Hydroxy bedeutet;
R₂ und R₂' Wasserstoff bedeuten;
A ―O―, ―NR₇― oder ―S― bedeutet, worin R₇ Wasserstoff bedeutet;
B eine Bindung,
―O―, ―S―, ―C≡C―, ―HC=CH―, 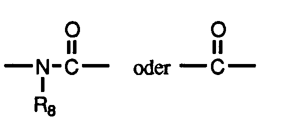 bedeutet, worin R₈ Wasserstoff bedeutet; n eine ganze Zahl von 1-4 bedeutet, mit der Massgabe, dass falls B eine Bindung oder eine der folgenden Gruppen bedeutet,
―O―, ―S― oder 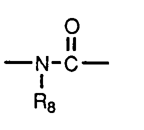 n ungleich 1 ist ;
Z ―S― oder ―CR₂ = CR'₂― bedeutet;
X und Y zusammen 0= , Hydroxyimino, Alkyloxyimino oder Alkenyloxyimino bedeuten, oder unabhängig voneinander, wenn einer der Reste X und Y Fluor ist, ist der andere Rest Fluor, oder wenn einer der Reste X und Y Amino ist, ist der andere Rest Wasserstoff, oder wenn einer der Reste X und Y Hydroxy ist, ist der andere Rest Hydroxy;
Q Phenyl, Cyclohexyl, Cyclooctyl, Adamantyl, Anthryl, Phenanthryl, Naphthyl, 5,6,7,8-Tetrahydro-1-naphthyl, 5,6,7,8-Tetrahydro-2-naphthyl, 1,2,3,4-Tetrahydro-1-naphthyl, 1,2,3,4-Tetrahydro-2-naphthyl, Chinolyl oder Isochinolyl, wobei der Phenyl- oder Naphthyl Rest durch eine oder mehrere der folgenden Gruppen substituiert sein kann: C₁-C₇-Alkyl, Phenyl, Acyloxy, Acyloxyalkyl oder Hydroxyalkyl.

6. (S)-alpha-Amino-4-[[2-(cyclooctyloxy)ethyl]oxy]phenylessigsäure hydrochlorid.

7. 3-Fluor-4-[2-(2-naphthyloxy)ethoxy]-alpha-oxophenylessigsäure.

8. 4-[2-(1-Naphthyl)-2-oxoethoxy]-alpha-oxophenylessigsäure.

9. 4-[[2-(2-Naphthyloxy)ethyl]oxy]-alpha-oxophenylessigsäure 2-(dimethylamino)ethylester.

10. (E)-4-[3-[2-[(2,2-Dimethyl-1-oxobutoxy)methyl]-6-methylphenyl]-2-propenyloxy]-alpha-oxophenylessigsäure(1:1) morpholiniumsalz.

11. 4-[[2-(2-Naphthyloxy)ethyl]oxy]-alpha-oxophenylessigsäure.

12. 4-[[2-(2-Naphthyloxy)ethyl]thio]-alpha-oxophenylessigsäure.

13. 4-[[2-(2-Naphthylthio)ethyl]oxy]-alpha-oxophenylessigsäure.

14. 4-[[2-Cyclooctyloxy)ethyl]oxy]-alpha-oxophenylessigsäure.

15. 4-[3-(4-Acetyl-3-hydroxy-2-propylphenoxy)propoxy]-alpha-oxophenyl essigsäure.

16. N-Hydroxy-N-methyl-4-[2-(2-naphthyloxy)ethoxy]-alpha-oxophenylacetamid.

17. (Z)-alpha-(Hydroxyimino)-5-[2-(2-naphthyloxy)ethoxy]-2-thienyl essigsäure.

18. 5-[[2-(2-Naphthyloxy)ethyl]oxy]-alpha-oxo-2-thienylessigsäure.

19. alpha-Oxo-4-[[2-(phenoxy)ethyl]oxy]phenylessigsäure.

20. (Z)-alpha-(Ethoxyimino)-5-[2-(2-naphthyloxy)ethoxy]-2-thienyl essigsäure.

21. rac.-5-[[2-(2-Naphthyloxy)ethyl]oxy]-alpha-oxo-2-thienylessigsäure-2,3-dihydroxypropylester.

22. (S)-alpha-Amino-4-[2-[2-[(2,2-dimethyl-1-oxobutoxy)methyl]-6-methylphenoxy]-ethoxy]phenylessigsäure.

23. (E)-4-[[3-(2-Naphthyl)-2-propenyl]oxy]-alpha-oxophenylessigsäure.

24. 4-[[2-[2-[(2,2-Dimethy-1-oxobutoxy)methyl]-6-methylphenoxy]ethyl]oxy]-alpha-oxophenylessigsäure (2:1) hydrat.

25. 4-[[4-(2-Naphthyloxy)butyl]oxy]-alpha-oxophenylessigsäure.

26. 4-[2-(6-Hydroxy-2-naphthyloxy)ethoxy]-alpha-oxophenylessigsäure;
4-[2-(8-Hydroxy-2-naphthyloxy)ethoxy]-alpha-oxophenylessigsäure;
6-[2-(2-Naphthyloxy)ethoxy]-alpha-oxo-3-pyridylessigsäure;
4-[2-[2,4-Dichlor-6-[(2,2-dimethyl-1-oxobutoxy)methyl]phenoxy]ethoxy]-alpha-oxophenylessigsäure;
4-[2-[2,4-Dimethyl-6-[(2,2-dimethyl-1-oxobutoxy)methyl]phenoxy]ethoxy]-alpha-oxophenylessigsäure;
4-[2-[2-[[[(4-Fluorphenyl)carbonyl]oxy]methyl]phenoxy]ethoxy]-alpha-oxophenylessigsäure;
5-[2-[2-[[[(4-Fluorphenyl)carbonyl]oxy]methyl]phenoxy]ethoxy]-alpha-oxo-2-thienylessigsäure oder 5-[2-[2-[(Methoxycarbonyl)-6-methyl]phenoxy] ethoxy]-alpha-oxo-2-thienylessigsäure.

27. (E)-4-[[3-(4-Bromphenyl)-2-propenyl]oxy]-alpha-oxo-3-pyridylphenylessigsäure;
(E)-4-[[4-(4-Fluorphenyl)-2-butenyl]oxy]-alpha-oxophenylessigsäure;
(E)-alpha-Oxo-4-[(3-phenyl-2-propenyl)oxy]thienylessigsäure;
(S)-alpha-Amino-4-[[2-(2-naphthyloxy)ethyl]oxy]phenylessigsäure;
(S)-alpha-Amino-4-[[2-(phenoxy)ethyl]oxy]phenylessigsäure hydrochlorid;
(S)-alpha-Amino-alpha-methyl-4-[[2-(2-naphthyloxy)ethyl]oxy]phenyl essigsäure;
(Z)-alpha-Oxo-5-[(3-phenyl-2-propenyl)oxy]-2-thienylessigsäure;
(E)-4-[[3-(1-Naphthyl)-2-propenyl]oxy]-alpha-oxophenylessigsäure;
alpha-Oxo-5-[4-(3-pyridyl)butoxy]-2-thienylessigsäure;
alpha-Oxo-4-[2-(4-pyridyl)ethoxy]phenylessigsäure;
alpha-Oxo-4-[[7-(phenoxy)heptyl]oxy]phenylessigsäure;
alpha-Oxo-4-[[8-(phenoxy)octyl]oxy]phenylessigsäure;
alpha-Oxo-5-[[2-(4-phenoxy)ethyl]oxy]-2-furylphenylessigsäure;
alpha-Oxo-4-[[3-(4-chinolyloxy)propyl]oxy]phenylessigsäure;
2-[[4-[[4-(1-Naphthyloxy)butyl]oxy]-alpha-oxophenylacetyl]oxy]-N,N,N-trimethylethylamonium iodid;
5-[3-(2-Naphthyloxy)propyl]-alpha-oxo-2-thienylessigsäure;
4-[[4-(1-Naphthyloxy)butyl]oxy]-alpha-oxophenylessigsäure;
4-[[2-(2-Chlorphenoxy)ethyl]oxy]-alpha-oxophenylessigsäure;
4-[[2-(2-Fluorphenoxy)ethyl]oxy]-alpha-oxo-3-pyridylessigsäure;
4-[[2-(2-Naphthyl)ethyl]amino]-alpha-oxophenylessigsäure;
5-[[2-(2-Naphthyloxy)ethyl]oxy]-alpha-oxo-2-thienylacetamid;
4-[[3-(2-Naphthyloxy)propyl]sulfonyl]-alpha-oxophenylessigsäure;
4-[[3-(2-Naphthylthio)propyl]oxy]-alpha-oxophenylessigsäure;
5-[[2-(3,4,5-Trimethoxyphenoxy)ethyl]oxy]-alpha-oxo-2-furylessigsäure;
4-[[2-(6-Methoxy-2-naphthyloxy)ethyl]oxy]-alpha-oxophenylessigsäure;
4-[[2-(3-Naphtho[2,3-b]thienyloxy)ethyl]oxy]-alpha-oxophenylessigsäure;
4-[[2-(7-Isochinolyl)ethyl]oxy]-alpha-oxophenylessigsäure;
4-[[3-(Cyclohexyl)propyl]oxy]-alpha-oxophenylessigsäure;
4-[[3-(Cyclooctyloxy)propyl]oxy]-alpha-oxophenylessigsäure;
4-[[2-[2-(Hydroxymethyl)phenoxy]ethyl]oxy]-alpha-oxophenylessigsäure;
4-[[2-[2-[(2,2-Dimethyl-1-oxopropoxy)methyl]-6-methylphenoxy]ethyl]oxy]-alpha-oxophenylessigsäure;
4-[[2-[4-(Methylaminosulfonyl)phenoxy]ethyl]oxy]-alpha-oxophenylessigsäure;
4-[[2-[8-(2,2-Dimethyl-1-oxopropoxy)-2-naphthyloxy]ethyl]oxy]-alpha-oxophenylessigsäure;
4-[[3-(2-Dimethylaminophenyl)propyl]oxy]-alpha-oxophenylessigsäure:
5-[[4-(2-Naphthyl)butyl]oxy]-alpha-oxo-2-thienylessigsäure;
4-[[3-(4-Cyanophenyl)propyl]oxy]-alpha-oxophenylessigsäure;
4-[[3-(4-Nitrophenoxy)propyl]oxy]-alpha-oxophenylessigsäure;
4-[[3-[2-(Trifluormethyl)phenyl]propyl]oxy]-alpha-oxophenylessigsäure;
4-[[3-[4-(N,N-Dimethylsulfamoyl)phenoxy]ethyl]oxy]-alpha-oxophenylessigsäure;
4-[[3-[4-(N-Ethylsulfamoyl)phenoxy]ethyl]oxy]-alpha-oxophenylessigsäure;
alpha-Oxo-4-[[4-(phenylthio)butyl]oxy]phenylessigsäure;
alpha-Oxo-4-[[4-(2-pyrimidyl)butyl]oxy]phenylessigsäure;
4-[[4-(3-Fluorphenoxy)butyl]oxy]-alpha-oxophenylessigsäure;
4-[[5-(4-Fluorphenoxy)pentyl]oxy]-alpha-oxophenylessigsäure;
4-Methyl-5-[[2-(2-naphthyloxy)ethyl]oxy]-alpha-oxo-2-thienylessigsäure;
5-[(Phenylmethyl)oxy]-alpha-oxo-2-furylessigsäure;
5-[[2-(2-Naphthyloxy)ethyl]oxy]-alpha-oxo-2-thienylphenylessigsäure 2-(dimethylamino)ethyl ester;
5-[[2-(3-Carbazolyloxy)ethyl]oxy]-alpha-oxo-2-thienylessigsäure;
5-[[2-(Cyclooctyloxy)ethyl]thio]-alpha-oxo-2-thienylessigsäure;
5-[[3-(Cycloheptyloxy)propyl]oxy]-alpha-oxo-2-thienyl-Kaliumacetat;
alpha, alpha-Difluor-4-[[2-(3-methylphenoxy)ethyl]oxy]phenylessigsäure;
alpha, alpha-Difluor-4-[[2-(3-naphtho[2,3-b]thienyloxy)ethyl]oxy]phenyl essigsäure;
alpha, alpha-Dimethoxy-4-[[2-(phenoxy)ethyl]oxy]phenylessigsäure ;
alpha-Oxo-4-[(3-phenyl-2-propynyl)oxy]thienylessigsäure;
alpha-Oxo-4-[[2-(2-xanthenyloxy)ethyl]oxy]phenylessigsäure (1:1) diethanylamoniumsalz;
alpha-Oxo-4-[[2-(4-trifluormethylphenoxy)ethyl]oxy]-2-furylessigsäure;
alpha-Oxo-4-[[6-(phenoxy)hexyl]oxy]phenylessigsäure;
4-[[2-(3-Benzo[b]thienyloxy)ethyl]oxy]-alpha-oxophenylessigsäure;
rac.-5-[[2-(3-Indolyloxy)ethyl]oxy]-alpha-oxo-2-thienylessigsäure 2,3-dihydroxypropylester;
rac.-alpha-Chlor-alpha-methyl-4-[[2-(phenoxy)ethyl]oxy]phenylessigsäure ;
rac.-alpha-Ethoxy-4-[(2-phenylethyl)oxy]phenylessigsäure oder
rac.-alpha-Oxo-4-[[2-(1,2,3,4-tetrahydro-2-naphthyloxy)ethyl]oxy] phenylessigsäure.

28. Verbindungen gemäss einem der Ansprüche 1-27 zur Anwendung als therapeutische Wirkstoffe.

29. Verbindungen gemäss einem der Ansprüche 1-27 zur Anwendung bei der Bekämpfung oder Verhütung von Verletzungen von ischämischem Gewebe und Arrhythmien während und nach Myokardinfarkten.

30. Verfahren zur Herstellung einer Verbindung gemäss einem der Ansprüche 1-27, dadurch gekennzeichnet, dass man
(a) zur Herstellung von Verbindungen der Formel I, worin R₁ Hydroxy bedeutet und die übrigen Symbole die in Anspruch 1 angegebene Bedeutung besitzen, eine Verbindung der allgemeinen Formel Ia verseift, 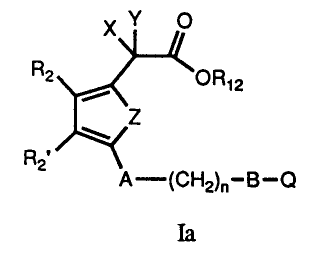 worin A, B, Q, R₂, R₂', X, Y, Z und n die in Anspruch 1 angegebene Bedeutung haben und R₁₂ niederes Alkyl bedeutet;
oder
(b) zur Herstellung von Verbindungen der Formel I, worin R₁ OR₃ bedeutet und die übrigen Symbole die in Anspruch 1 angegebene Bedeutung besitzen, eine Verbindung der allgemeinen Formel II 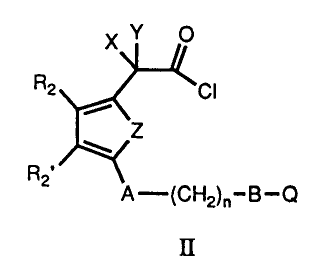 worin A, B, Q, R₂, R₂', X, Y, Z und n die in Anspruch 1 angegebene Bedeutung haben
mit einem Alkohol der allgemeinen Formel IV umsetzt,
HO-R₃ IV
worin R₃ die in Anspruch 1 angegebene Bedeutung hat;
oder
(c) zur Herstellung von Verbindungen der Formel I, worin R₁ 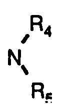 bedeutet und die übrigen Symbole die in Anspruch 1 angegebene Bedeutung besitzen, eine Verbindung der allgemeinen Formel IIA 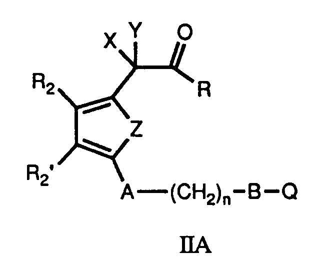 worin A, B, Q, R₂, R₂', X, Y, Z und n die in Anspruch 1 angegebene Bedeutung haben und R Chlor oder C₁-C₇-Alkoxy bedeutet
mit einem Amin der allgemeinen Formel III behandelt 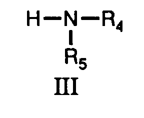 worin R₄ und R₅ die in Anspruch 1 angegebene Bedeutung haben,
oder
(d) zur Herstellung von Verbindungen der Formel I, worin R₁ Hydroxy; A -O- oder -S-; X und Y zusammen 0= und Z -CR₂=CR₂'- bedeutet und die übrigen Symbole die in Anspruch 1 angegebene Bedeutung haben, eine Verbindung der allgemeinen Formel VII 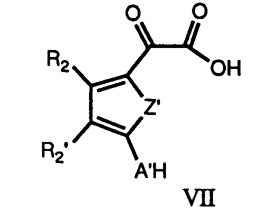 worin R₂ und R₂' die in Anspruch 1 angegebene Bedeutung haben und A' -O- oder -S- und Z' -CR₂=CR₂'- bedeutet
mit einer Verbindung der allgemeinen Formel VI umsetzt, 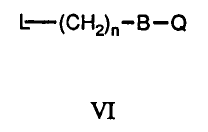 worin B, Q und n die in Anspruch 1 angegebene Bedeutung haben und L eine Abgangsgruppe ist,
oder
(e) zur Herstellung von Verbindungen der Formel I, worin R₁ Hydroxy; A -O- oder -S-; X und Y zusammen 0= und Z -CR₂=CR₂'- bedeutet und die übrigen Symbole die in Anspruch 1 angegebene Bedeutung haben, eine Verbindung der allgemeinen Formel IX oxidiert, 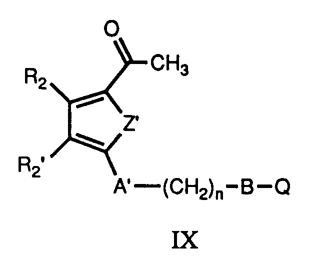 worin B, Q, R₂, R₂' und n die in Anspruch 1 angegebene Bedeutung haben und A' und Z' die oben beschriebene Bedeutung haben;
oder
(f) zur Herstellung von Verbindungen der Formel I, worin R₁ Hydroxy, A 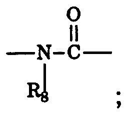 X und Y zusammen 0= und Z -CR₂=CR₂'- bedeuten und die übrigen Symbole die in Anspruch 1 angegebene Bedeutung haben, eine Verbindung der allgemeinen Formel XII oxidiert, 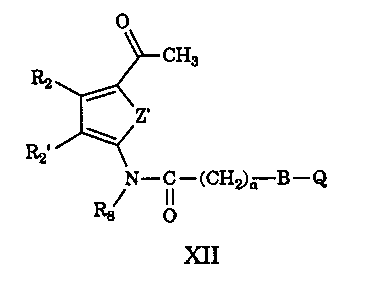 worin B, Q, R₂, R₂', R₈ und n die in Anspruch 1 angegebene Bedeutung haben und Z' die oben beschriebene Bedeutung hat;
oder
(g) zur Herstellung von Verbindungen der Formel I, worin R₁ Hydroxy; A -O; X Amino und Z -CR₂=CR₂'- bedeuten und die übrigen Symbole die in Anspruch 1 angegebene Bedeutung haben, von einer Verbindung der allgemeinen Formel IV 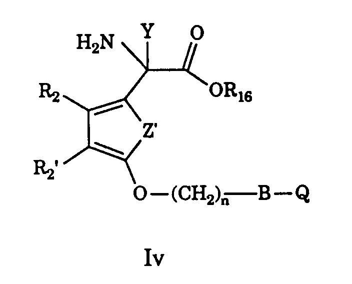 worin B, Q, R₂, R₂', Y und n die in Anspruch 1 angegebene Bedeutung haben und R₁₆ eine Aminosäure-O-Schutzgruppe ist, die Aminosäure-O-Schutzgruppe abspaltet
und
(h) gegebenenfalls die erhaltenen Verbindungen in ein pharmazeutisch anwendbares Salz überführt.

31. Ein pharmazeutisches Präparat enthaltend eine Verbindung der Ansprüche 1-27 und therapeutisch inerte Excipientien.

32. Ein pharmazeutisches Präparat zur Bekämpfung oder Verhütung von Verletzungen von ischämischem Gewebe und Arrhythmien während und nach Myokardinfarkten enthaltend eine Verbindung gemäss einem der Ansprüche 1-27 und therapeutisch inerte Excipientien.

33. Verwendung von Verbindungen gemäss einem der Ansprüche 1-27 zur Herstellung eines Medikaments zur Bekämpfung oder Verhütung von Krankheiten.

34. Verwendung von Verbindungen gemäss einem der Ansprüche 1-27 zur Herstellung eines Medikaments zur Bekämpfung oder Verhütung von Verletzungen von ischämischem Gewebe und Arrhythmien während und nach Myokardinfarkten

## Patentansprüche (Patentansprüche für folgende(n) Vertragsstaat(en): ES, GR)

1. Verfahren zur Herstellung von Verbindungen der allgemeinen Formel I 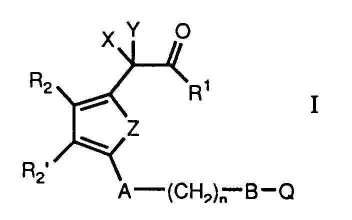 worin
R₁ Hydroxy, OR₃ oder 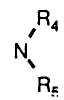 bedeutet, wobei einer der Reste R₄ und R₅ Wasserstoff, C₁-C₇-Alkyl oder Hydroxy-C₁-C₇-alkyl und der andere Wasserstoff, Hydroxy, C₁-C₇-Alkyl oder C₁-C₇-Alkoxy und wobei R₃ (̵CH₂CH₂O)̵ₘH, 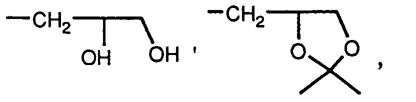 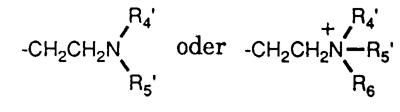 bedeutet, wobei
R₄', R₅', und R₆ unabhängig voneinander Wasserstoff, C₁-C₇-Alkyl oder Hydroxy-C₁-C₇-Alkyl bedeuten und m eine ganze Zahl von 1-4 bedeutet;
R₂ und R₂' unabhängig voneinander Wasserstoff, C₁-C₇-Alkyl, Aryl, Aryl-C₁-C₇-alkyl, C₁-C₇-Alkoxy, Hydroxy, Amino, C₁-C₇-Alkylamino, di-C₁-C₇-Alkylamino, Cyano, Halogen, Mercapto, C₁-C₇-Alkylthio, C₁-C₇-Alkylsulfinyl, C₁-C₇-Alkylsulfonyl, Trihalogen-C₁-C₇-Alkyl, Acyl oder Nitro bedeuten;
A eine Bindung,―O―, ―NR₇―, ―S―, 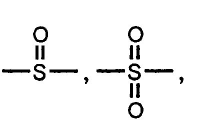 ―C≡C―, ―HC=CH―, -CH₂CH₂―, 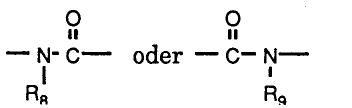 bedeutet, worin R₇ Wasserstoff, C₁-C₇-Alkyl oder Acyl und R₈ und R₉ unabhängig voneinander Wasserstoff oder C₁-C₇-Alkyl bedeutet;
n eine ganze Zahl von 1-10 bedeutet;
B eine Bindung,―O―, ―NR₇―, ―S―, 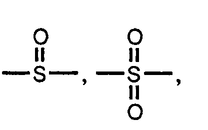 ―C≡C―, ―HC=CH―, 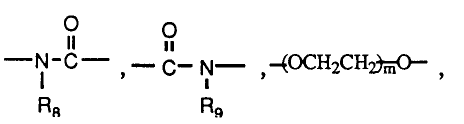 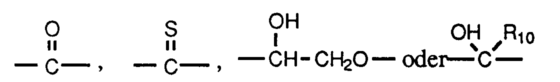 bedeutet, worin R₇, R₈, R₉ und m die oben angegebene Bedeutung haben und R₁₀ Wasserstoff oder C₁-C₇-Alkyl bedeutet;
Z ―O―, ―S―, ―CR₂ = CR₂'―, ―N = CR₂―, ―CR₂ = N― oder 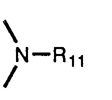 bedeutet, worin R₁₁ Wasserstoff oder C₁-C₇-Alkyl bedeutet;
X und Y zusammen 0= , S=, Hydroxyimino, Alkyloxyimino, Alkenyloxyimino, Arylalkoxyimino, Hydrazono, mono-C₁-C₇-Alkylhydrazono, di-C₁-C₇-Alkylhydrazono oder Semicarbazono bedeuten, oder unabhängig voneinander, wenn einer der Reste X und Y Halogen ist, ist der andere Rest Wasserstoff, Halogen, C₁-C₇-Alkyl oder Aryl-C₁-C₇-Alkyl; oder wenn einer der Reste X und Y Amino, C₁-C₇-Alkylamino oder di-C₁-C₇-Alkylamino ist, ist der andere Rest Wasserstoff, C₁-C₇-Alkyl oder Aryl-C₁-C₇-Alkyl; oder wenn einer der Reste X und Y Hydroxy, Alkoxy oder Aryl-C₁-C₇-Alkoxy ist, ist der andere Rest Wasserstoff, Hydroxy, C₁-C₇-Alkyl, C₁-C₇-Alkoxy oder Aryl-C₁-C₇-alkoxy;
Q Cycloalkyl, Aryl oder ein heterocyclisches Radical bedeutet; mit der Massgabe, dass falls A Sauerstoff (O) und B eine Bindung, Schwefel, (S) oder Sauerstoff (O) ist, dann ist n 2-10;
sowie gegebenenfalls Enantiomere, Racemate, Diastereomere und Mischungen davon oder geometrische Isomere und Mischungen davon und falls eine basische oder saure Gruppe vorhanden ist pharmazeutisch anwendbare Salze der Verbindungen, wobei das Verfahren dadurch gekennzeichnet ist, dass
(a) zur Herstellung von Verbindungen der Formel I, worin R₁ Hydroxy bedeutet und die übrigen Symbole die in Anspruch 1 angegebene Bedeutung besitzen, eine Verbindung der allgemeinen Formel Ia verseift, 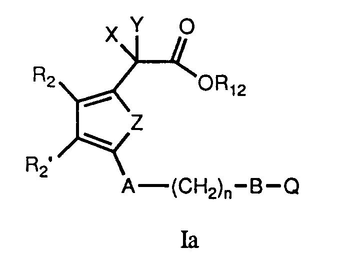 worin A, B, Q, R₂, R₂', X, Y, Z und n die in Anspruch 1 angegebene Bedeutung haben und R₁₂ niederes Alkyl bedeutet;
oder
(b) zur Herstellung von Verbindungen der Formel I, worin R₁ OR₃ bedeutet und die übrigen Symbole die in Anspruch 1 angegebene Bedeutung besitzen, eine Verbindung der allgemeinen Formel II 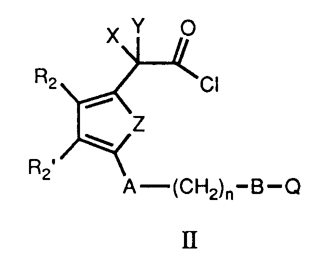 worin A, B, Q, R₂, R₂', X, Y, Z und n die in Anspruch 1 angegebene Bedeutung haben
mit einem Alkohol der allgemeinen Formel IV umsetzt,
HO-R₃ IV
worin R₃ die in Anspruch 1 angegebene Bedeutung hat;
oder
(c) zur Herstellung von Verbindungen der Formel I, worin R₁ 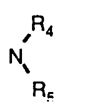 bedeutet und die übrigen Symbole die in Anspruch 1 angegebene Bedeutung besitzen, eine Verbindung der allgemeinen Formel IIA 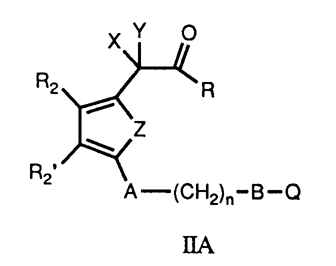 worin A, B, Q, R₂, R₂', X, Y, Z und n die in Anspruch 1 angegebene Bedeutung haben und R Chlor oder C₁-C₇-Alkoxy bedeutet
mit einem Amin der allgemeinen Formel III behandelt 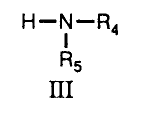 worin R₄ und R₅ die in Anspruch 1 angegebene Bedeutung haben, oder
(d) zur Herstellung von Verbindungen der Formel I, worin R₁ Hydroxy; A -O- oder -S-; X und Y zusammen 0= und Z -CR₂=CR₂'- bedeutet und die übrigen Symbole die in Anspruch 1 angegebene Bedeutung haben, eine Verbindung der allgemeinen Formel VII 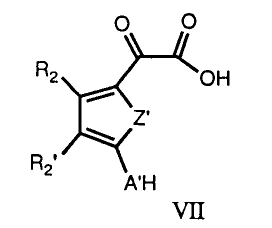 worin R₂ und R₂' die in Anspruch 1 angegebene Bedeutung haben und A' -O- oder -S- und Z' -CR₂=CR₂'- bedeutet
mit einer Verbindung der allgemeinen Formel VI umsetzt, 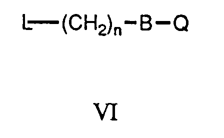 worin B, Q und n die in Anspruch 1 angegebene Bedeutung haben und L eine Abgangsgruppe ist,
oder
(e) zur Herstellung von Verbindungen der Formel I, worin R₁ Hydroxy; A -O- oder -S-; X und Y zusammen 0= und Z -CR₂=CR₂'- bedeutet und die übrigen Symbole die in Anspruch 1 angegebene Bedeutung haben, eine Verbindung der allgemeinen Formel IX oxidiert, 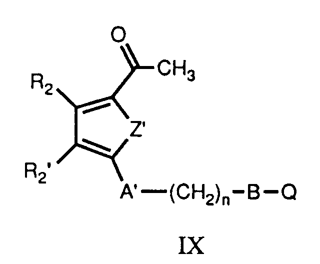 worin B, Q, R₂, R₂' und n die in Anspruch 1 angegebene Bedeutung haben und A' und Z' die oben beschriebene Bedeutung haben;
oder
(f) zur Herstellung von Verbindungen der Formel I, worin R₁ Hydroxy, A 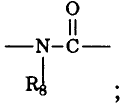 X und Y zusammen 0= und Z -CR₂=CR₂'- bedeuten und die übrigen Symbole die in Anspruch 1 angegebene Bedeutung haben, eine Verbindung der allgemeinen Formel XII oxidiert, 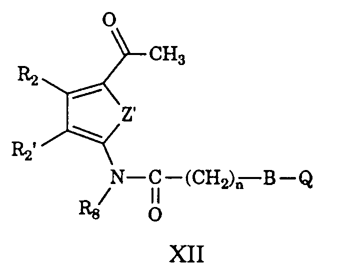 worin B, Q, R₂, R₂', R₈ und n die in Anspruch 1 angegebene Bedeutung haben und Z' die oben beschriebene Bedeutung hat;
oder
(g) zur Herstellung von Verbindungen der Formel I, worin R₁ Hydroxy; A -O; X Amino und Z -CR₂=CR₂'- bedeuten und die übrigen Symbole die in Anspruch 1 angegebene Bedeutung haben, von einer Verbindung der allgemeinen Formel IV 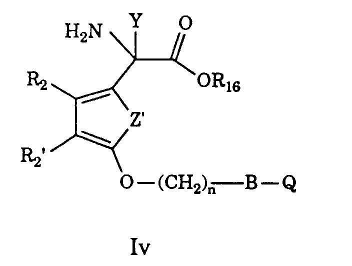 worin B, Q, R₂, R₂', Y und n die in Anspruch 1 angegebene Bedeutung haben und R₁₆ eine Aminosäure-O-Schutzgruppe ist, die Aminosäure-O-Schutzgruppe abspaltet
und
(h) gegebenenfalls die erhaltenen Verbindungen in ein pharmazeutisch anwendbares Salz überführt.

2. Verfahren gemäss Anspruch 1, wobei einer der Reste R₄ und R₅ Wasserstoff, C₁-C₇-Alkyl oder Hydroxy-C₁-C₇-alkyl und der andere Wasserstoff oder C₁-C₇-Alkyl bedeutet; X und Y zusammen 0= , S= bedeuten, oder unabhängig voneinander, wenn einer der Reste X und Y Halogen ist, ist der andere Rest Wasserstoff, Halogen, C₁-C₇-Alkyl oder Aryl-C₁-C₇-alkyl; oder wenn einer der Reste X und Y Amino, C₁-C₇-Alkylamino oder di-C₁-C₇-Alkylamino ist, ist der andere Rest Wasserstoff, C₁-C₇-Alkyl oder Aryl-C₁-C₇-alkyl; oder wenn einer der Reste X und Y Hydroxy, Alkoxy oder Aryl- C₁-C₇-alkoxy ist, ist der andere Rest Wasserstoff, Hydroxy, C₁-C₇-Alkyl, C₁-C₇-Alkoxy oder Aryl-C₁-C₇-alkoxy.

3. Verfahren gemäss Anspruch 1, worin R₁ die oben angegebene Bedeutung hat; R₂ und R₂'unabhängig voneinander Wasserstoff, C₁-C₇-Alkyl, Aryl, C₁-C₇-Alkoxy, Hydroxy, Halogen, Acyl oder Nitro bedeuten;
A eine Bindung
―O―, ―NR₇―, ―S―, 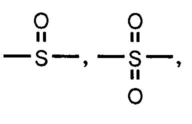 ―C≡C―, ―HC=CH―, ―CH₂CH₂-, 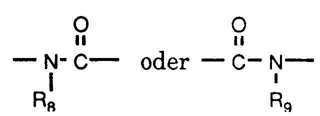 bedeutet, worin R₇ Wasserstoff, C₁-C₇-Alkyl oder Acyl und R₈ und R₉ unabhängig voneinander Wasserstoff oder C₁-C₇-Alkyl bedeuten;
B eine Bindung, ―O―, ―NR₇―, ―S―, ―C≡C―, ―HC=CH―, 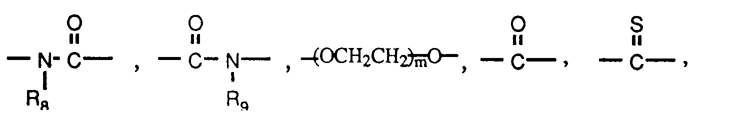 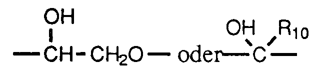 bedeutet, worin R₇, R₈, R₉ und m die in Anspruch 1 angegebene Bedeutung haben und R₁₀ Wasserstoff ist;
n eine ganze Zahl von 1-6 bedeutet, mit der Massgabe, dass falls A eine Bindung oder eine der folgenden Gruppen
―O―, ―NR₇―, ―S―, 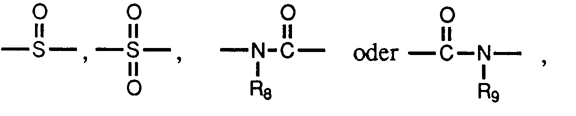 und B eine Bindung oder eine der folgenden Gruppen
―O―, ―NR₇―, ―S―, 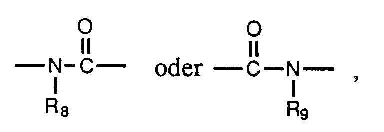 bedeutet, n ungleich 1 ist;
Z ―S―, ―CR₂ = CR'₂―, ―N = CR₂ ― oder ―CR₂ = N―
bedeutet;
X und Y zusammen 0= , S=, Hydroxyimino, Alkyloxyimino, Alkenyloxyimino, Arylalkoxyimino, Hydrazono, mono-C₁-C₇-Alkylhydrazono, di-C₁-C₇-Alkylhydrazono oder Semicarbazono bedeuten, oder unabhängig voneinander, wenn einer der Reste X und Y Halogen ist, ist der andere Rest Wasserstoff oder Halogen; oder wenn einer der Reste X und Y Amino ist, ist der andere Rest Wasserstoff; oder wenn einer der Reste X und Y Hydroxy oder Alkoxy ist, ist der andere Rest Wasserstoff oder Hydroxy;
Q Cycloalkyl, Aryl oder ein heterocyclisches Radical bedeutet; wobei das heterocyclische Radical durch eine oder mehrere der folgenden Gruppen substituiert sein kann: Alkyl, Alkoxy, Acyloxy, Halogen, Acyloxyalkyl, Alkoxyalkyl, Aryloxyalkoxy, Hydroxy, Hydroxyalkyl, Hydroxyalkoxy, Phenyl, Trihalogenalkyl, Sulfamoyl, Carboxycarbonyl oder Alkoxalyl.

4. Verfahren gemäss Anspruch 3, worin R₁ die in Anspruch 1 angegebene Bedeutung hat; R₂ und R₂'unabhängig voneinander Wasserstoff, C₁-C₇-Alkyl, C₁-C₇-Alkoxy, Hydroxy oder Halogen bedeuten;
A eine Bindung,
―O―, ―NR₇―, ―S―, 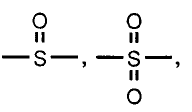 ―C≡C―, ―HC=CH―, ―CH₂CH₂―, 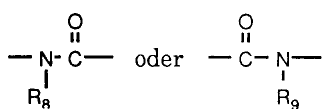 bedeutet, worin R₇ Wasserstoff oder C₁-C₇-Alkyl und R₈ und R₉ Wasserstoff bedeuten; B eine Bindung
―O―, ―S―, ―C≡C―, ―HC=CH―, 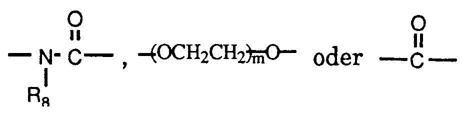 bedeutet, worin m 1 oder 2 und R₈ Wasserstoff bedeutet;
n eine ganze Zahl von 1-6 bedeutet, mit der Massgabe, dass falls A eine Bindung oder eine der folgenden Gruppen
―O―, ―NR₇―, ―S―, 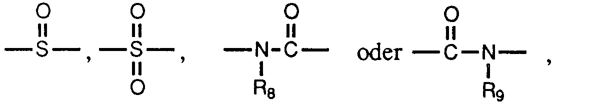 und B eine Bindung oder eine der folgenden Gruppen bedeutet
―O―, ―S― oder 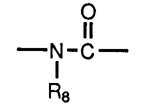 n ungleich 1 ist;
Z ―S― oder ―CR₂ = CR'₂― bedeutet;
X und Y zusammen 0= , Hydroxyimino, Alkyloxyimino oder Alkenyloxyimino bedeuten, oder unabhängig voneinander, wenn einer der Reste X und Y Amino ist, ist der andere Rest Wasserstoff, oder wenn einer der Reste X und Y Hydroxy ist, ist der andere Rest Wasserstoff oder Hydroxy;
Q Phenyl, Cyclohexyl, Cyclooctyl, Pyridyl, Adamantyl, 1,1'-Biphenyl, Anthryl, Phenanthryl, Naphthyl, 5,6,7,8-Tetrahydro-1-naphthyl, 5,6,7,8-Tetrahydro-2-naphthyl, 1,2,3,4-Tetrahydro-1-naphthyl, 1,2,3,4-Tetrahydro-2-naphthyl, Chinolyl oder Isochinolyl, wobei das heterocyclische Radical durch eine oder mehrere der folgenden Gruppen substituiert sein kann: Alkyl, Alkoxy, Acyloxy, Halogen, Acyloxyalkyl, Alkoxyalkyl, Aryloxyalkoxy, Hydroxy, Hydroxyalkyl, Hydroxyalkoxy, Phenyl, Trihalogenalkyl, Sulfamoyl, Carboxycarbonyl oder Alkoxalyl.

5. Verfahren gemäss Anspruch 4, worin R₁ Hydroxy oder 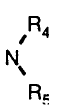 bedeutet, wobei einer der Reste R₄ und R₅ Wasserstoff, C₁-C₇-Alkyl oder Hydroxy-C₁-C₇-alkyl und der andere Hydroxy bedeutet;
R₂ und R₂' Wasserstoff bedeuten; A ―O―, ―NR₇― oder ―S― bedeutet, worin R₇ Wasserstoff bedeutet; B eine Bindung,
―O―, ―S―, ―C≡C―, ―HC=CH―, 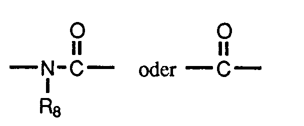 bedeutet, worin R₈ Wasserstoff bedeutet; n eine ganze Zahl von 1-4 bedeutet mit der Massgabe, dass falls B eine Bindung oder eine der folgenden Gruppen bedeutet,
―O―, ―S― oder 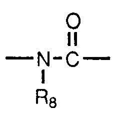 n ungleich 1 ist;
Z ―S― oder ―CR₂ = CR'₂― bedeutet;
X und Y zusammen 0= , Hydroxyimino, Alkyloxyimino oder Alkenyloxyimino bedeuten, oder unabhängig voneinander, wenn einer der Reste X und Y Fluor ist, ist der andere Rest Fluor, oder wenn einer der Reste X und Y Amino ist, ist der andere Rest Wasserstoff, oder wenn einer der Reste X und Y Hydroxy ist, ist der andere Rest Hydroxy;
Q Phenyl, Cyclohexyl, Cyclooctyl, Adamantyl, Anthryl, Phenanthryl, Naphthyl, 5,6,7,8-Tetrahydro-1-naphthyl, 5,6,7,8-Tetrahydro-2-naphthyl, 1,2,3,4-Tetrahydro-1-naphthyl, 1,2,3,4-Tetrahydro-2-naphthyl, Chinolyl oder Isochinolyl, wobei der Phenyl oder Naphthyl Rest durch eine oder mehrere der folgenden Gruppen substituiert sein kann: C₁-C₇-Alkyl, Phenyl, Acyloxy, Acyloxyalkyl oder Hydroxyalkyl.

6. Verfahren gemäss Anspruch 2, wobei (S)-alpha-Amino-4-[[2-(cyclooctyloxy)ethyl]oxy]phenylessigsäure hydrochlorid hergestellt wird.

7. Verfahren gemäss Anspruch 1, wobei 3-Fluor-4-[2-(2-naphthyloxy)ethoxy]-alpha-oxophenylessigsäure hergestellt wird.

8. Verfahren gemäss Anspruch 1, wobei 4-[2-(1-Naphthyl)-2-oxoethoxy]-alpha-oxophenylessigsäure hergestellt wird.

9. Verfahren gemäss Anspruch 2, wobei 4-[[2-(2-Naphthyloxy)ethyl] oxy]-alpha-oxophenylessigsäure 2-(dimethylamino)ethylester hergestellt wird.

10. Verfahren gemäss Anspruch 1, wobei (E)-4-[3-[2-[(2,2-Dimethyl-1-oxobutoxy)methyl]-6-methylphenyl]-2-propenyloxy]-alpha-oxophenylessigsäure(1:1) morpholiniumsalz hergestellt wird.

11. Verfahren gemäss Anspruch 2, wobei 4-[[2-(2-Naphthyloxy) ethyl]oxy]-alpha-oxophenylessigsäure hergestellt wird.

12. Verfahren gemäss Anspruch 2, wobei 4-[[2-(2-Naphthyloxy) ethyl]thio]-alpha-oxophenylessigsäure hergestellt wird.

13. Verfahren gemäss Anspruch 2, wobei 4-[[2-(2-Naphthylthio) ethyl]oxy]-alpha-oxophenylessigsäure hergestellt wird.

14. Verfahren gemäss Anspruch 2, wobei 4-[[2-Cyclooctyloxy)ethyl]oxy]-alpha-oxophenylessigsäure hergestellt wird.

15. Verfahren gemäss Anspruch 1, wobei 4-[3-(4-Acetyl-3-hydroxy-2-propylphenoxy)propoxy]-alpha-oxophenyl essigsäure hergestellt wird.

16. Verfahren gemäss Anspruch 1, wobei N-Hydroxy-N-methyl-4-[2-(2-naphthyloxy)ethoxy]-alpha-oxophenylacetamid hergestellt wird.

17. Verfahren gemäss Anspruch 1, wobei (Z)-alpha-(Hydroxyimino)-5-[2-(2-naphthyloxy)ethoxy]-2-thienylessigsäure hergestellt wird.

18. Verfahren gemäss Anspruch 2, wobei 5-[[2-(2-Naphthyloxy) ethyl]oxy]-alpha-oxo-2-thienylessigsäure hergestellt wird.

19. Verfahren gemäss Anspruch 2, wobei alpha-Oxo-4-[[2-(phenoxy) ethyl]oxy]phenylessigsäure hergestellt wird.

20. Verfahren gemäss Anspruch 1, wobei (Z)-alpha-(Ethoxyimino)-5-[2-(2-naphthyloxy)ethoxy]-2-thienylessigsäure hergestellt wird.

21. Verfahren gemäss Anspruch 2, wobei rac.-5-[[2-(2-Naphthyloxy) ethyl]oxy]-alpha-oxo-2-thienylessigsäure-2,3-dihydroxypropylester hergestellt wird.

22. Verfahren gemäss Anspruch 1, wobei (S)-alpha-Amino-4-[2-[2-[(2,2-dimethyl-1-oxobutoxy)methyl]-6-methylphenoxy]-ethoxy]phenylessigsäure hergestellt wird.

23. Verfahren gemäss Anspruch 2, wobei (E)-4-[[3-(2-Naphthyl)-2-propenyl]oxy]-alpha-oxophenylessigsäure hergestellt wird.

24. Verfahren gemäss Anspruch 2, wobei 4-[[2-[2-[(2,2-Dimethyl-1-oxobutoxy)methyl]-6-methylphenoxy]ethyl]oxy]-alpha-oxophenylessigsäure (2:1) hydrat hergestellt wird.

25. Verfahren gemäss Anspruch 2, wobei 4-[[4-(2-Naphthyloxy) butyl]oxy]-alpha-oxophenylessigsäure hergestellt wird.

26. Verfahren zur Herstellung eines Arzneimittels, insbesondere zur Bekämpfung oder Verhütung von Verletzungen von ischämischem Gewebe und Arrhythmien während und nach Myokardinfarkten, dadurch gekennzeichnet, dass man eine Verbindung der in Anspruch 1 definierten Formel I in Form eines Enantiomeren, Racemats, Diastereomeren oder einer Mischung davon oder eines geometrischen Isomeren oder einer Mischung davon oder falls eine basische oder saure Gruppe vorhanden ist ein pharmazeutisch anwendbares Salz dieser Verbindung in eine galenische Darreichungsform bringt.

27. Verwendung einer Verbindungen der in Anspruch 1 definierten Formel I in Form eines Enantiomeren, Racemats, Diastereomeron oder einer Mischung davon oder eines geometrischen Isomeren oder einer Mischung davon oder falls eine basische oder saure Gruppe vorhanden ist eines pharmazeutisch anwendbaren Salzes dieser Verbindung zur Herstellung von Arzneimitteln zur Bekämpfung oder Verhütung von Verletzungen von ischämischem Gewebe und Arrhythmien während und nach Myokardinfarkten.

## Revendications (Revendications pour l'(les) Etat(s) contractant(s) suivant(s): AT, BE, CH, DE, DK, FR, GB, IT, LI, LU, MC, NL, PT, SE)

1. Composés de formule générale 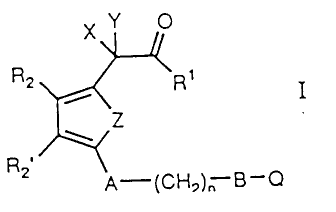 dans laquelle R₁ est un groupe hydroxy, OR₃ ou 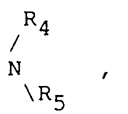 où l'un des radicaux R₄ et R₅ est un atome d'hydrogène, un groupe alkyle en C₁-C₇ ou hydroxyalkyle en C₁-C₇, et l'autre est un atome d'hydrogène, un groupe hydroxy, alkyle en C₁-C₇ ou alcoxy en C₁-C₇, et R₃ est -(CH₂CH₂O)ₘ-H, 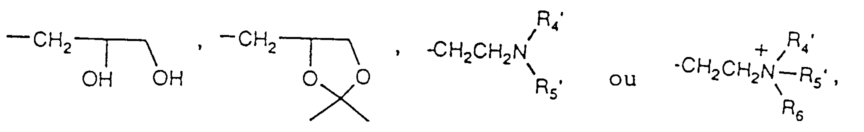 et où R₄', R₅' et R₆ sont, indépendamment un atome d'hydrogène, un groupe alkyle en C₁-C₇ ou hydroxyalkyle en C₁-C₇, et où m est un nombre entier de 1 à 4;
R₂ et R₂' sont, indépendamment un atome d'hydrogène, un groupe alkyle en C₁-C₇, aryle, aryl(alkyle en C₁-C₇), alcoxy en C₁-C₇, hydroxy, amino, alkylamino en C₁-C₇, dialkylamino en C₁-C₇, cyano, halogéno, mercapto, alkylthio en C₁-C₇, alkylsulfinyle en C₁-C₇, alkylsulfonyle en C₁-C₇, trihalogénoalkyle en C₁-C₇, acyle ou nitro;
A est une liaison,
-O-, -NR₇-, -S-, 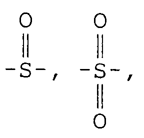 -C≡C-, -HC=CH-, -CH₂CH₂-, 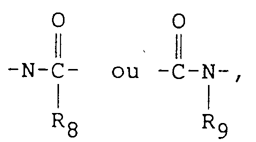 où R₇ est un atome d'hydrogène, un groupe alkyle en C₁-C₇ ou acyle et R₈ et R₉ sont, indépendamment, un atome d'hydrogène ou une groupe alkyle en C₁-C₇, n est un nombre entier de 0 à 10;
B est une liaison, -O-, -NR₇-, -S-, 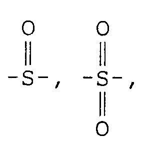 -C≡C-, -HC=CH-, 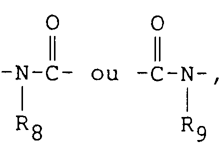 -(OCH₂CH₂)ₘ-O-, 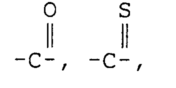 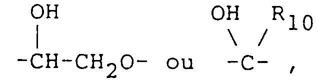 où R₇, R₈, R₉ et m sont tels que définis ci-dessus et R₁₀ est un atome d'hydrogène ou un groupe alkyle en C₁-C₇;
Z est -O-, -S-, -CR₂=CR₂'-, -N=CR₂-, -CR₂=N- ou 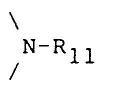 où R₁₁ est un atome d'hydrogène ou un groupe alkyle en C₁-C₇;
X et Y, pris conjointement, sont des groupes O=, S=, hydroxyimino, alkyloxyimino, alcényloxyimino, arylalcoxyimino, hydrazono, mono(alkylhydrazono en C₁-C₇), di(alkylhydrazono en C₁-C₇) ou semicarbazono, ou bien, indépendamment, lorsque l'un des radicaux X et Y est un atome d'halogène, l'autre est un atome d'hydrogène, un atome d'halogène, un groupe alkyle en C₁-C₇ ou aryl(alkyle en C₁-C₇); ou bien, lorsque l'un des radicaux X et Y est un groupe amino, alkylamino en C₁-C₇ ou dialkylamino en C₁-C₇, l'autre est un atome d'hydrogène, un groupe alkyle en C₁-C₇ ou aryl(alkyle en C₁-C₇); ou bien, lorsque l'un des radicaux X et Y est un groupe hydroxy, alcoxy ou aryl(alcoxy en C₁-C₇), l'autre est un atome d'hydrogène, un groupe hydroxy, alkyle en C₁-C₇, alcoxy en C₁-C₇ ou aryl(alcoxy en C₁-C₇); Q est un radical cycloalkyle, aryle, ou hétérocyclique; à condition que, lorsque A est un atome d'oxygène (O) et que B est une liaison, un atome de soufre (S) ou un atome d'oxygène (O), n ait une valeur de 2 à 10; et, le cas échéant, sous forme d'énantomières, de racémates, de diastéréoisomères, ou de mélanges de ceux-ci, ou d'isomères géométriques, ou de mélanges de ceux-ci, et, lorsqu'un groupe basique ou acide est présent, les sels pharmaceutiquement acceptables de ces composés.

2. Composés selon la revendication 1, dans lesquels l'un des radicaux R₄ et R₅ est un atome d'hydrogène, un groupe alkyle en C₁-C₇ ou hydroxyalkyle en C₁-C₇ et l'autre est un atome d'hydrogène ou un groupe alkyle en C₁-C₇, X et Y, pris conjointement, sont O=, S=, ou bien, indépendamment, lorsque l'un des radicaux X et Y est un atome d'halogène, l'autre est un atome d'hydrogène, un atome d'halogène, un groupe alkyle en C₁-C₇ ou aryl(alkyle en C₁-C₇); ou bien, lorsque l'un des radicaux X et Y est un groupe amino, alkylamino en C₁-C₇ ou dialkylamino en C₁-C₇, l'autre est un atome d'hydrogène, un groupe alkyle en C₁-C₇ ou aryl(alkyle en C₁-C₇); ou bien, lorsque l'un des radicaux X et Y est un groupe hydroxy, alcoxy ou aryl(alcoxy en C₁-C₇), l'autre est un atome d'hydrogène, un groupe hydroxy, alkyle en C₁-C₇, alcoxy en C₁-C₇ ou aryl(alcoxy en C₁-C₇).

3. Composés selon la revendication 1, dans lesquels R₁ est tel que décrit ci-dessus, R₂ et R₂' sont, indépendamment, un atome d'hydrogène, un groupe alkyle en C₁-C₇, aryle, alcoxy en C₁-C₇, hydroxy, halogéno, acyle ou nitro;
A est une liaison,
-O-, -NR₇-, -S-, 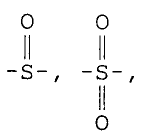 -C≡C-, -HC=CH-, -CH₂CH₂-, 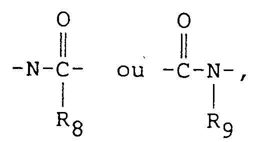 où R₇ est un atome d'hydrogène, un groupe alkyle en C₁-C₇ ou acyle, et R₈ et R₉ sont, indépendamment, un atome d'hydrogène ou un groupe alkyle en C₁-C₇;
B est une liaison -O-, -NR₇-, -S-, -C≡C-,
―HC=CH―, 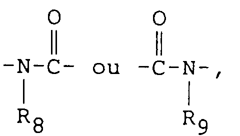 -(OCH₂CH-2)ₘ-O-, 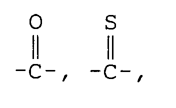 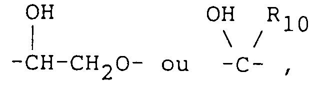 où R₇, R₈, R₉ et m sont tels que définis ci-dessus et R₁₀ est un atome d'hydrogène;
n est un nombre entier de 1 à 6, de sorte que lorsque A est une liaison ou l'un des groupes suivants
-O-, -NR₇-, -S-, 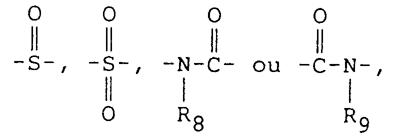 et que B est une liaison ou l'un des groupes suivants
-O-, -NR₇-, -S-, 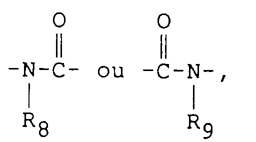 n soit différent du nombre entier 1;
Z est -S-, -CR₂=CR'₂-, -N=CR₂- ou -CR₂=N-;
X et Y, pris conjointement, sont des groupes O=, hydroxyimino, alkyloxyimino, alcényloxyimino, arylalcoxyimino, hydrazono, mono(alkylhydrazono en C₁-C₇), di(alkylhydrazono en C₁-C₇) ou semicarbazono, ou bien, indépendamment, lorsque l'un des radicaux X et Y est un atome d'halogène, l'autre est un atome d'hydrogène ou d'halogène; ou bien, lorsque l'un des radicaux X et Y est un groupe amino, l'autre est un atome d'hydrogène; ou bien, lorsque l'un des radicaux X et Y est un groupe hydroxy ou alcoxy, l'autre est un atome d'hydrogène ou un groupe hydroxy;
Q est un radical cycloalkyle, aryle, ou hétérocyclique, ce radical pouvant être substitué par un ou plusieurs des groupes suivants: alkyle, alcoxy, acyloxy, halogéno, acyloxyalkyle, alcoxyalkyle, aryloxyalcoxy, hydroxy, hydroxyalkyle, hydroxyalcoxy, phényle, trihalogénoalkyle, sulfamoyle, carboxycarbonyle ou alcoxyalyle.

4. Composés selon la revendication 3, dans lesquels R₁ est tel que décrit ci-dessus; R₂ et R₂', sont, indépendamment, un atome d'hydrogène, un groupe alkyle en C₁-C₇, alcoxy en C₁-C₇, hydroxy ou halogéno;
A est une liaison,
-O-, -NR₇-, -S-, 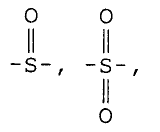 -C≡C-, -HC=CH-, -CH₂CH₂-, 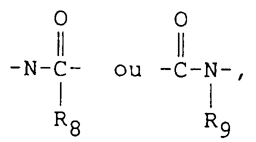 où R₇ est un atome d'hydrogène, un groupe alkyle en C₁-C₇ et R₈ et R₉ sont des atomes d'hydrogène;
B est une liaison,
-O-, -S-, -C≡C-, -HC=HC-, 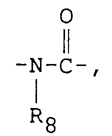 -(OCH₂CH₂)ₘ-O- ou 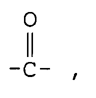 où m est égal à 1 ou 2 et R₈ est un atome d'hydrogène;
n est un nombre entier de 1 à 6, de sorte que, lorsque A est une liaison ou l'un des groupes suivants,
-O-, -NR₇-, -S-, 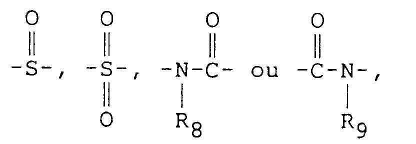 et que B est une liaison ou l'un des groupes suivants
-O-, -S-, ou 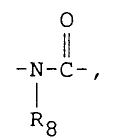 n soit différent du nombre entier 1;
Z est -S-, ou -CR₂=CR₂'-,
X et Y pris conjointement, sont un groupe O=, hydroxyimino, alkyloxyimino ou alcényloxyimino, ou bien, indépendamment, lorsque l'un des radicaux X et Y est un atome d'halogène, l'autre est un atome d'halogène; ou bien, lorsque l'un des radicaux X et Y est un groupe amino, l'autre est un atome d'hydrogène; ou bien, lorsque l'un des radicaux X et Y est un groupe hydroxy, l'autre est un atome d'hydrogène ou un groupe hydroxy;
Q est un radical phényle, cyclohexyle, cyclooctyle, pyridinyle, adamantyle, 1,1'-biphényle, anthracényle, phénanthrényle, naphtalényle, 5,6,7,8-tétrahydro-1-naphtalényle, 5,6,7,8-tétrahydro-2-naphtalényle, 1,2,3,4-tétrahydro-1-naphtalényle, 1,2,3,4-tétrahydro-2-naphtalényle, quinoléyle ou isoquinoléyle, ce radical pouvant être substitué par un ou plusieurs des groupes suivants: alkyle, alcoxy, acyloxy, halogéno, acyloxyalkyle, alcoxyalkyle, aryloxyalcoxy, hydroxy, hydroxyalkyle, hydroxyalcoxy, phényle, trihalogénoalkyle, sulfamoyle, carboxycarbonyle ou alcoxalyle.

5. Composés selon la revendication 4, dans lesquels R₁ est un groupe hydroxy ou 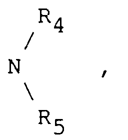 où l'un des radicaux R₄ et R₅ est un atome d'hydrogène, un groupe alkyle en C₁-C₇ ou hydroxyalkyle en C₁-C₇, et l'autre est un groupe hydroxy;
R₂ et R₂' sont des atomes d'hydrogène;
A est -O-, -NR₇- ou -S-, où R₇ est un atome d'hydrogène;
B est une liaison,
-O-, -S-, -C≡C-, -HC=HC-, 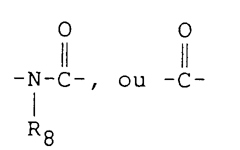 où R₈ est un atome d'hydrogène;
n est un nombre entier de 1 à 4, de sorte que lorsque B est une liaison ou l'un des groupes suivants, -O-, -S-, ou 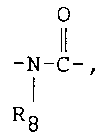 n soit différent du nombre entier 1;
Z est -S- ou -CR₂ = CR₂'-;
X et Y pris conjointement, sont un groupe O=, hydroxyimino, alkyloxyimino ou alcényloxyimino, ou bien, indépendamment, lorsque l'un des radicaux X et Y est un groupe fluoro, l'autre est un fluoro; ou bien, lorsque l'un des radicaux X et Y est un groupe amino, l'autre est un atome d'hydrogène; ou bien, lorsque l'un des radicaux X et Y est un groupe hydroxy, l'autre est un groupe hydroxy;
Q est un radical phényle, cyclohexyle, cyclooctyle, adamantyle, anthracényle, phénanthrényle, naphtalényle, 5,6,7,8-tétrahydro-1-naphtalényle, 5,6,7,8-tétrahydro-2-naphtalényle, 1,2,3,4-tétrahydro-1-naphtalényle, 1,2,3,4-tétrahydro-2-naphtalényle, quinoléyle ou isoquinoléyle, dont le radical phényle ou le radical naphtalényle peut être substitué par un ou plusieurs des groupes suivants: alkyle en C₁-C₇, phényle, acyloxy, acyloxyalkyle ou hydroxyalkyle.

6. Chlorhydrate d'acide (S)-alpha-amino-4-[[2-(cyclooctyloxy)éthyl]oxy]benzèneacétique.

7. Acide 3-fluoro-4-[2-(2-naphtalényloxy)éthoxy]-alpha-oxobenzèneacétique.

8. Acide 4-[2-(1-naphtalényl)-2-oxoéthoxy]-alpha-oxobenzèneacétique.

9. Ester 2-(diméthylamino)éthylique d'acide 4-[[2-(2-naphtalényloxy)éthyl]oxy]-alpha-oxobenzèneacétique.

10. Sel de morpholine de l'acide (E)-4-[3-[2-[(2,2-diméthyl-1-oxobutoxy)méthyl]-6-méthylphényl]-2-propényloxy]-alpha-oxobenzèneacétique (1:1).

11. Acide 4-[[2-(2-naphtalényloxy)éthyl]oxy]-alpha-oxobenzèneacétique.

12. Acide 4-[[2-(2-naphtalényloxy)éthyl]thio]-alpha-oxobenzèneacétique.

13. Acide 4-[[2-(2-naphtalénylthio)éthyl]oxy]-alpha-oxobenzèneacétique.

14. Acide 4-[[2-cyclooctyloxy)éthyl]oxy]-alpha-oxobenzèneacétique.

15. Acide 4-[3-(4-acétyl-3-hydroxy-2-propylphénoxy)propoxy]-alpha-oxobenzèneacétique.

16. N-hydroxy-N-méthyl-4-[2-(2-naphtalényloxy)éthoxy]-alpha-oxobenzèneacétamide.

17. Acide (Z)-alpha-(hydroxyimino)-5-[2-(2-naphtalényloxy)éthoxy]-2-thiophèneacétique.

18. Acide 5-[[2-(2-napthalényloxy)éthyl]oxy]-alpha-oxo-2-thiophèneacétique.

19. Acide alpha-oxo-4-[[2-(phénoxy)éthyl]oxy]benzèneacétique.

20. Acide (Z)-alpha-(éthoxyimino)-5-[2-(2-naphtalényloxy)éthoxy]-2-thiophèneacétique.

21. Ester 2,3-dihydroxypropylique de l'acide 5-[[2-(2-naphtalényloxy)éthyl]oxy]-alpha-oxo-2-thiophèneacétique racémique.

22. Acide (S)-alpha-amino-4-[2-[2-[(2,2-diméthyl-1-oxobutoxy)méthyl]-6-méthylphénoxy]éthoxy]benzèneacétique.

23. Acide (E)-4-[[3-(2-naphtalényl)-2-propényl]oxy]-alpha-oxobenzèneacétique.

24. Hydrate d'acide 4-[[2-[2-[(2,2-diméthyl-1-oxobutoxy)méthyl]-6-méthylphénoxy]éthyl]oxy]alpha-oxobenzèneacétique (2:1).

25. Acide 4-[[4-(2-napthalényloxy)butyl]oxy]-alpha-oxobenzèneacétique.

26. Acide 4-[2-(6-hydroxy-2-naphtalényloxy)éthoxy]-alpha-oxobenzèneacétique;
acide 4-[2-(8-hydroxy-2-naphtalényloxy)éthoxy]-alpha-oxobenzèneacétique;
acide 6-[2-(2-naphtalényloxy)éthoxy]-alpha-oxo-3-pyridineacétique;
acide 4-[2-[2,4-dichloro-6-[(2,2-diméthyl-1-oxobutoxy)méthyl]phénoxy]éthoxy]-alpha-oxobenzèneacétique;
acide 4-[2-[2,4-diméthyl-6-[(2,2-diméthyl-1-oxobutoxy)méthyl]phénoxy]éthoxy]-alpha-oxobenzèneacétique;
acide 4-[2-[2-[[[(4-fluorophényl)carbonyl]oxy]méthyl]phénoxy]éthoxy]-alpha-oxobenzèneacétique;
acide 5-[2-[2-[[[(4-fluorophényl)carbonyl]oxy]méthyl]phénoxy]éthoxy]-alpha-oxo-2-thiophèneacétique; ou
acide 5-[2-[2-[(méthoxycarbonyl)-6-méthyl]phénoxy]éthoxy]-alpha-oxo-2-thiophèneacétique.

27. Acide (E)-4-[[3-(4-bromophényl)-2-propényl]oxy]-alpha-oxo-3-pyridineacétique;
acide (E)-4-[[4-(4-fluorophényl)-2-butényl]oxy]-alpha-oxobenzèneacétique;
acide (E)-alpha-oxo-4-[(3-phényl-2-propényl)oxy]thiophèneacétique;
acide (S)-alpha-amino-4-[[2-(2-naphtalényloxy)éthyl]oxy]benzèneacétique;
chlorhydrate d'acide (S)-alpha-amino-4-[[2-(phénoxy)éthyl]oxy]benzèneacétique;
acide (S)-alpha-amino-alpha-méthyl-4-[[2-(2-naphtalényloxy)éthyl]oxy]benzèneacétique;
acide (Z)-alpha-oxo-5-[(3-phényl-2-propényl)oxy]-2-thiophèneacétique;
acide (E)-4-[[3-(1-naphtalényl)-2-propényl]oxy]-alpha-oxobenzèneacétique;
acide alpha-oxo-5-[4-(3-pyridinyl)butoxy]-2-thiophèneacétique;
acide alpha-oxo-4-[2-(4-pyridinyl)éthoxy]benzèneacétique;
acide alpha-oxo-4-[[7-(phénoxy)heptyl]oxy]benzèneacétique;
acide alpha-oxo-4-[[8-(phénoxy)octyl]oxy]benzèneacétique;
acide alpha-oxo-5-[[2-(4-phénoxyphénoxy)éthyl]oxy]-2-furanacétique;
acide alpha-oxo-4-[[3-(4-quinoléyloxy)propyl]oxy]benzèneacétique;
iodure de 2-[[4-[[4-(1-naphtalényloxy)butyl]oxy]-alpha-oxobenzèneacétyl]oxy]-N,N,N-triméthyléthanaminium;
acide 5-[3-(2-naphtalényloxy)propyl]-alpha-oxo-2-thiophèneacétique;
acide 4-[[4-(1-naphtalényloxy)butyl]oxy]-alpha-oxobenzèneacétique;
acide 4-[[2-(2-chlorophénoxy)éthyl]oxy]-alpha-oxobenzèneacétique;
acide 4-[[2-(2-fluorophénoxy)éthyl]oxy]-alpha-oxo-3-pyridineacétique;
acide 4-[[2-(2-naphtalényl)éthyl]amino]-alpha-oxobenzèneacétique;
5-[[2-(2-naphtalényloxy)éthyl]oxy]-alpha-oxo-2-thiophèneacétamide;
acide 4-[[3-(2-naphtalényloxy)propyl]sulfonyl]-alpha-oxobenzèneacétique;
acide 4-[[3-(2-naphtalénylthio)propyl]oxy]-alpha-oxobenzèneacétique;
acide 5-[[2-(3,4,5-triméthoxyphénoxy)éthyl]oxy]-alpha-oxo-2-furanacétique;
acide 4-[[2-(6-méthoxy-2-naphtalényloxy)éthyl]oxy]-alpha-oxobenzèneacétique;
acide 4-[[2-(3-naphto[2,3-b]thiényloxy)éthyl]oxy]-alpha-oxo-benzèneacétique;
acide 4-[[2-(7-isoquinoléyl)éthyl]oxy]-alpha-oxobenzèneacétique;
acide 4-[[3-(cyclohexyl)propyl]oxy]-alpha-oxobenzèneacétique;
acide 4-[[3-(cyclooctyl)propyl]oxy]-alpha-oxobenzèneacétique;
acide 4-[[2-[2-(hydroxyméthyl)phénoxy]éthyl]oxy]-alpha-oxobenzèneacétique;
acide 4-[[2-[2-(2,2-diméthyl-1-oxopropoxy)méthyl]-6-méthylphénoxy]éthyl]oxy]-alpha-oxobenzèneacétique;
acide 4-[[2-[4-(méthylaminosulfonyl)phénoxy]éthyl]oxy]alpha-oxobenzèneacétique;
acide 4-[[2-[8-(2,2-diméthyl-1-oxopropoxy)-2-naphtalényloxy]éthyl]oxy]-alpha-oxobenzèneacétique;
acide 4-[[3-(2-diméthylaminophényl)propyl]oxy]-alpha-oxobenzèneacétique;
acide 5-[[4-(2-naphtalényl)butyl]oxy]-alpha-oxo-2-thiophèneacétique;
acide 4-[[3-(4-cyanophényl)propyl]oxy]-alpha-oxobenzèneacétique;
acide 4-[[3-(4-nitrophénoxy)propyl]oxy]-alpha-oxobenzèneacétique;
acide 4-[[3-[2-(trifluorométhyl)phényl]propyl]oxy]-alpha-oxobenzèneacétique;
acide 4-[[3-[4-(N,N-diméthylsulfamoyl)phénoxy]éthyl]oxy]-alpha-oxobenzèneacétique;
acide 4-[[3-[4-(N-éthylsulfamoyl)phénoxy]éthyl]oxy]-alpha-oxobenzèneacétique;
acide alpha-oxo-4-[[4-(phénylthio)butyl]oxy]benzèneacétique;
acide alpha-oxo-4-[[4-(2-pyrimidyl)butyl]oxy]benzèneacétique;
acide 4-[[4-(3-fluorophénoxy)butyl]oxy]-alpha-oxobenzèneacétique;
acide 4-[[5-(4-fluorophénoxy)pentyl]oxy]-alpha-oxobenzèneacétique;
acide 4-méthyl-5-[[2-(2-naphtalényloxy)éthyl]oxy]-alpha-oxo-2-thiophèneacétique;
de l'acide 5-[(phénylméthyl)oxy]-alpha-oxo-2-furanacétique;
de l'ester 2-(diméthylamino)éthylique de l'acide 5-[[2-(2-naphtalényloxy)éthyl]oxy]-alpha-oxo-2-thiophèneacétique;
de l'acide 5-[[2-(3-carbazolyloxy)éthyl]oxy]-alpha-oxo-2-thiophèneacétique;
de l'acide 5-[[2-(cyclooctyloxy)éthyl]thio]-alpha-oxo-2-thiophèneacétique;
du sel de potassium de l'acide 5-[[3-(cycloheptyloxy)propyl]oxy]-alpha-oxo-2-thiophèneacétique;
de l'acide alpha,alpha-difluoro-4-[[2-(3-méthylphénoxy)éthyl]oxy]benzèneacétique;
acide alpha,alpha-difluoro-4-[[2-(3-naphto[2,3-b]thiényloxy)éthyl]oxy]benzèneacétique;
acide alpha,alpha-diméthoxy-4-[[2-(phénoxy)éthyl]oxy]benzèneacétique;
acide alpha-oxo-4-[(3-phényl-2-propynyl)oxy]thiophèneacétique;
sel de diéthanolamine d'acide alpha-oxo-4-[[2-(2-xanthényloxy)éthyl]oxy]benzèneacétique (1:1);
acide alpha-oxo-4-[[2-(4-trifluorométhylphénoxy)éthyl]oxy]-2-furanacétique;
acide alpha-oxo-4-[[6-(phénoxy)hexyl]oxy]benzèneacétique;
acide 4-[[2-(3-benzo[b]thiényloxy)éthyl]oxy]-alpha-oxobenzèneacétique;
ester 2,3-dihydroxypropylique d'acide 5-[[2-(3-indolyloxy)éthyl]oxy]-alpha-oxo-2-thiophèneacétique racémique;
acide alpha-chloro-alpha-méthyl-4-[[2-(phénoxy)éthyl]oxy]benzèneacétique racémique;
acide alpha-éthoxy-4-[(2-phényléthyl)oxy]benzèneacétique racémique; ou
acide alpha-oxo-4-[[2-(1,2,3,4-tétrahydro-2-naphtalényloxy)éthyl]oxy]benzèneacétique.

28. Composés selon l'une quelconque des revendications 1-27, à utiliser comme substances à activité thérapeutique.

29. Composés selon l'une quelconque des revendications 1-27, à utiliser pour combattre ou empêcher les lésions du tissu ischémique et les arythmies pendant et après un infarctus du myocarde.

30. Procédé de fabrication d'un composé selon l'une quelconque des revendications 1-27, qui comprend
(a) pour la fabrication d'un composé de formule I, dans laquelle R₁ est un groupe hydroxy et les symboles restants sont tels que décrits dans la revendication 1, la saponification d'un composé de formule générale 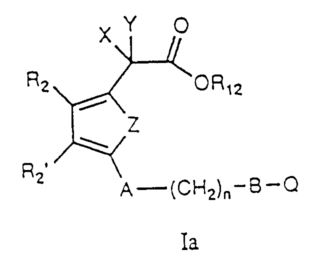 dans laquelle A, B, Q, R₂, R₂', X, Y, Z et n sont tels que décrits dans la revendication 1 et R₁₂ est un groupe alkyle en C₁-C₇,
ou
(b) pour la fabrication d'un composé de formule I, dans laquelle R₁ est OR₃ et les symboles restants sont tels que décrits dans la revendication 1, le traitement d'un composé de formule générale 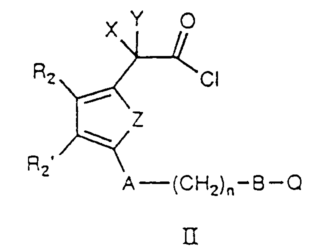 dans laquelle A, B, Q, R₂, R₂', X, Y, Z et n sont tels que décrits dans la revendication 1, avec un alcool de formule générale
HO-R₃ IV
dans laquelle R₃ est tel que décrit dans la revendication 1,
ou
(c) pour la fabrication d'un composé de formule I, dans laquelle R₁ est 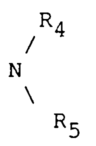 et les symboles restants sont tels que décrits dans la revendication 1, le traitement d'un composé de formule générale 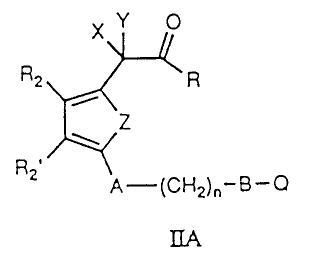 dans laquelle A, B, Q, R₂, R₂', X, Y, Z et n sont tels que décrits dans la revendication 1 et R est un groupe chloro ou alcoxy en C₁-C₇,
avec une amine de formule générale 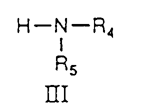 dans laquelle R₄ et R₅ sont tels que décrit dans la revendication 1,
ou
(d) pour la fabrication d'un composé de formule I, dans laquelle R₁ est un groupe hydroxy, A est -O- ou -S-, X et Y, pris conjointement, sont O= et Z est -CR₂=CR₂'- et les symboles restants sont tels que décrits dans la revendication 1, la réaction d'un composé de formule générale 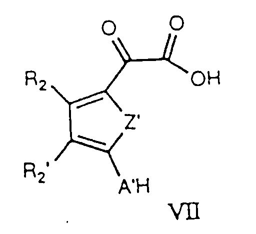 dans laquelle R₂ et R₂' sont tels que décrits dans la revendication 1 et A' est -O- ou -S-, et Z' est -CR₂=CR₂'-, avec un composé de formule générale 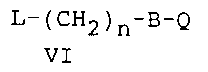 dans laquelle B, Q et n sont tels que décrits dans la revendication 1 et L est un groupe partant,
ou
(e) pour la fabrication d'un composé de formule I, dans laquelle R₁ est un groupe hydroxy, A est -O- ou -S-, X et Y, pris conjointement, sont O= et Z est -CR₂=CR₂'- et les symboles restants sont tels que décrits dans la revendication 1, l'oxydation d'un composé de formule générale 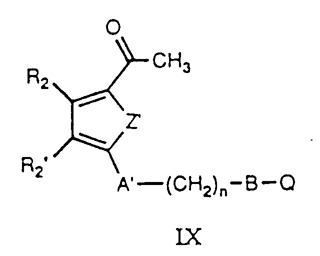 dans laquelle B, Q, R₂, R₂' et n sont tels que décrits dans la revendication 1 et A' et Z' sont tels que décrits ci-desssus,
ou
(f) pour la fabrication d'un composé de formule I, dans laquelle R₁ est un groupe hydroxy, A est 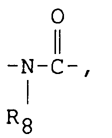 X et Y, pris conjointement, sont O= et Z est -CR₂=CR₂'- et les symboles restants sont tels que décrits dans la revendication 1, l'oxydation d'un composé de formule générale 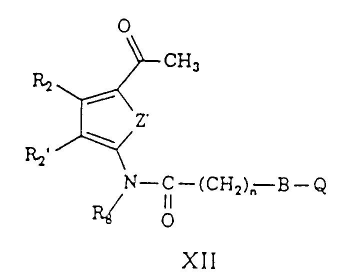 dans laquelle B, Q, R₂, R₂', R₈ et n sont tels que décrits dans la revendication 1 et Z' est tel que décrit ci-dessus, ou
(g) pour la fabrication d'un composé de formule I, dans laquelle R₁ est un groupe hydroxy, A est -O-, X est un groupe amino et Z est -CR₂=CR₂'- et les symboles restants sont tels que décrits dans la revendication 1, l'élimination du groupe O-protecteur de l'acide aminé d'un composé de formule générale 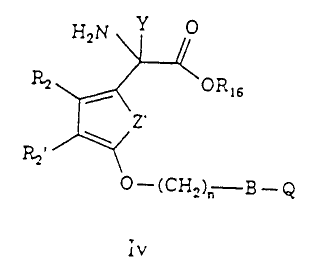 dans laquelle B, Q, R₂, R₂', Y et n sont tels que décrits dans la revendication 1 et R₁₆ est un groupe O-protecteur d'acide aminé,
et, le cas échéant,
(h) la conversion d'un composé obtenu en un sel pharmaceutiquement acceptable.

31. Médicament contenant un composé selon l'une quelconque des revendications 1-27 et un excipient inerte en thérapeutique.

32. Médicament pour combattre ou empêcher les lésions du tissu ischémique et les arythmies pendant et après un infarctus du myocarde, contenant un composé selon l'une quelconque des revendications 1-27 et un excipient inerte en thérapeutique.

33. Utilisation d'un composé selon l'une quelconque des revendications 1-27, pour fabriquer des médicaments pour combattre ou empêcher les maladies.

34. Utilisation d'un composé selon l'une quelconque des revendications 1-27 pour fabriquer des médicaments contre les lésions au tissu ischémique et/ou les arythmies pendant et après un infarctus du myocarde.

## Revendications (Revendications pour l'(les) Etat(s) contractant(s) suivant(s): ES, GR)

1. Procédé de fabrication de composés de formule générale 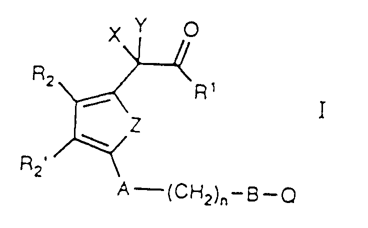 dans laquelle R₁ est un groupe hydroxy, OR₃ ou 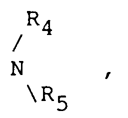 où l'un des radicaux R₄ et R₅ est un atome d'hydrogène, un groupe alkyle en C₁-C₇ ou hydroxyalkyle en C₁-C₇, et l'autre est un atome d'hydrogène, un groupe hydroxy, alkyle en C₁-C₇ ou alcoxy en C₁-C₇, et R₃ est -(CH₂CH₂O)ₘ-H, 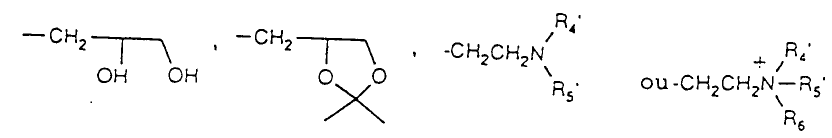 et où R₄', R₅' et R₆ sont, indépendamment un atome d'hydrogène, un groupe alkyle en C₁-C₇ ou hydroxyalkyle en C₁-C₇, et où m est un nombre entier de 1 à 4;
R₂ et R₂' sont, indépendamment un atome d'hydrogène, un groupe alkyle en C₁-C₇, aryle, aryl(alkyle en C₁-C₇), alcoxy en C₁-C₇, hydroxy, amino, alkylamino en C₁-C₇, dialkylamino en C₁-C₇, cyano, halogéno, mercapto, alkylthio en C₁-C₇, alkylsulfinyle en C₁-C₇, alkylsulfonyle en C₁-C₇, trihalogénoalkyle en C₁-C₇, acyle ou nitro;
A est une liaison,
-O-, -NR₇-, -S-, 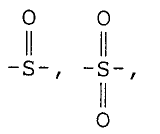 -C≡C-, -HC=CH-, -CH₂CH₂-, 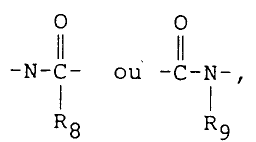 où R₇ est un atome d'hydrogène, un groupe alkyle en C₁-C₇ ou acyle et R₈ et R₉ sont, indépendamment, un atome d'hydrogène ou un groupe alkyle en C₁-C₇, n est un nombre entier de 0 à 10;
B est une liaison, -O-, -NR₇-, -S-, 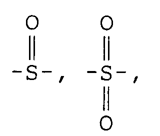 -C≡C-,
―HC=CH―, 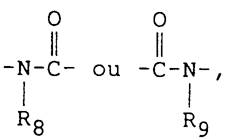 -(OCH₂CH-2)ₘ-O-, 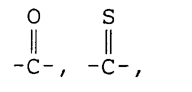 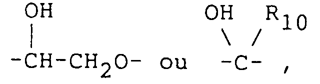 où R₇, R₈, R₉ et m sont tels que définis ci-dessus et R₁₀ est un atome d'hydrogène ou un groupe alkyle en C₁-C₇;
Z est -O-, -S-, -CR₂=CR₂'-, -N=CR₂-, -CR₂=N- ou 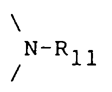 où R₁₁ est un atome d'hydrogène ou un groupe alkyle en C₁-C₇;
X et Y, pris conjointement, sont des groupes O=, S=, hydroxyimino, alkyloxyimino, alcényloxyimino, arylalcoxyimino, hydrazono, mono(alkylhydrazono en C₁-C₇), di(alkylhydrazono en C₁-C₇) ou semicarbazono, ou bien, indépendamment, lorsque l'un des radicaux X et Y est un atome d'halogène, l'autre est un atome d'hydrogène, un atome d'halogène, un groupe alkyle en C₁-C₇ ou aryl(alkyle en C₁-C₇); ou bien, lorsque l'un des radicaux X et Y est un groupe amino, alkylamino en C₁-C₇ ou dialkylamino en C₁-C₇, l'autre est un atome d'hydrogène, un groupe alkyle en C₁-C₇ ou aryl(alkyle en C₁-C₇); ou bien, lorsque l'un des radicaux X et Y est un groupe hydroxy, alcoxy ou aryl(alcoxy en C₁-C₇), l'autre est un atome d'hydrogène, un groupe hydroxy, alkyle en C₁-C₇, alcoxy en C₁-C₇ ou aryl(alcoxy en C₁-C₇); Q est un radical cycloalkyle, aryle, ou hétérocyclique; à condition que, lorsque A est un atome d'oxygène (O) et que B est une liaison, un atome de soufre (S) ou un atome d'oxygène (O), n ait une valeur de 2 à 10; le cas échéant, sous forme d'énantomières, de racémates, de diastéréoisomères, ou de mélanges de ceux-ci, ou d'isomères géométriques, ou de mélanges de ceux-ci, et, lorsqu'un groupe basique ou acide est présent, des sels pharmaceutiquement acceptables de ces composés, qui comprend
(a) pour la fabrication d'un composé de formule I, dans laquelle R₁ est un groupe hydroxy et les symboles restants sont tels que décrits ci-dessus, la saponification d'un composé de formule générale 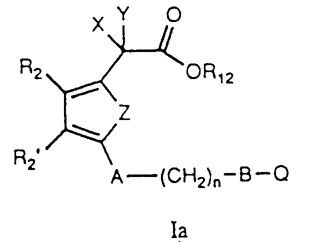 dans laquelle A, B, Q, R₂, R_{2'}, X, Y, Z et n sont tels que décrits ci-dessus et R₁₂ est un groupe alkyle inférieur,
ou
(b) pour la fabrication d'un composé de formule I, dans laquelle R₁ est OR₃ et les symboles restants sont tels que décrits ci-dessus, le traitement d'un composé de formule générale 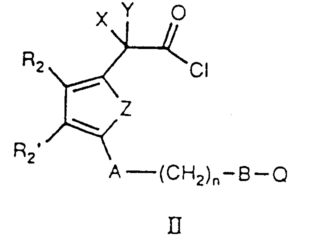 dans laquelle A, B, Q, R₂, R₂', X, Y, Z et n sont tels que décrits ci-dessus, avec un alcool de formule générale
HO-R₃ IV
dans laquelle R₃ est tel que décrit ci-dessus,
ou
(c) pour la fabrication d'un composé de formule I, dans laquelle R₁ est 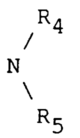 et les symboles restants sont tels que décrits ci-dessus, le traitement d'un composé de formule générale 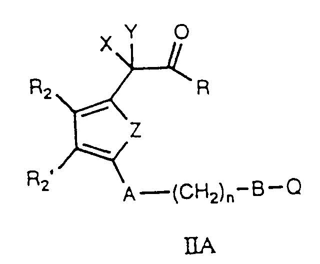 dans laquelle A, B, Q, R₂, R₂', X, Y, Z et n sont tels que décrits ci-dessus et R est un groupe chloro ou alcoxy en C₁-C₇,
avec une amine de formule générale 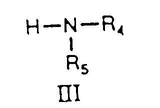 dans laquelle R₄ et R₅ sont tels que décrit ci-dessus,
ou
(d) pour la fabrication d'un composé de formule I, dans laquelle R₁ est un groupe hydroxy, A est -O- ou -S-, X et Y, pris conjointement, sont O= et Z est -CR₂=CR₂'- et les symboles restants sont tels que décrits ci-dessus, la réaction d'un composé de formule générale 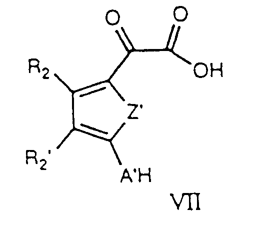 dans laquelle R₂ et R₂' sont tels que décrits ci-dessus et A' est -O- ou -S-, et Z' est -CR₂=CR₂'-,
avec un composé de formule générale 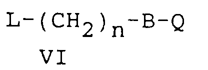 dans laquelle B, Q et n sont tels que décrits ci-dessus et L est un groupe partant,
ou
(e) pour la fabrication d'un composé de formule I, dans laquelle R₁ est un groupe hydroxy, A est -O- ou -S-, X et Y, pris conjointement, sont O= et Z est -CR₂=CR₂'- et les symboles restants sont tels que décrits ci-dessus, l'oxydation d'un composé de formule générale 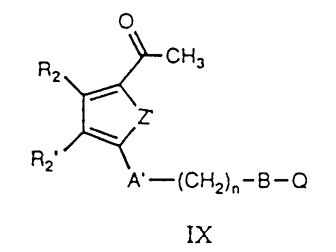 dans laquelle A', B, Q, R₂, R₂', Z' et n sont tels que décrits ci-dessus,
ou
(f) pour la fabrication d'un composé de formule I, dans laquelle R₁ est un groupe hydroxy, A est 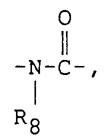 X et Y, pris conjointement, sont O= et Z est -CR₂=CR₂'- et les symboles restants sont tels que décrits ci-dessus, l'oxydation d'un composé de formule générale 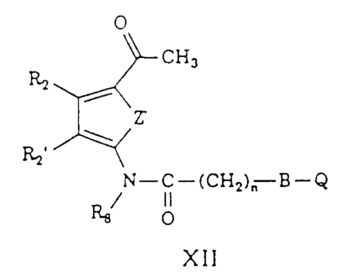 dans laquelle B, Q, R₂, R₂', R₈, Z' et n sont tels que décrits ci-dessus,
ou
(g) pour la fabrication d'un composé de formule I, dans laquelle R₁ est un groupe hydroxy, A est -O-, X est un groupe amino et Z est -CR₂=CR₂'- et les symboles restants sont tels que décrits ci-dessus, l'élimination du groupe O-protecteur de l'acide aminé d'un composé de formule générale 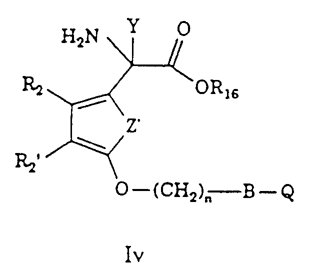 dans laquelle B, Q, R₂, R₂', Y et n sont tels que décrits ci-dessus et R₁₆ est un groupe O-protecteur d'acide aminé,
et, le cas échéant,
(h) la conversion d'un composé obtenu en un sel pharmaceutiquement acceptable.

2. Procédé selon la revendication 1, dans lequel l'un des radicaux R₄ et R₅ est un atome d'hydrogène, un groupe alkyle en C₁-C₇ ou hydroxyalkyle en C₁-C₇ et l'autre est un atome d'hydrogène ou un groupe alkyle en C₁-C₇, X et Y, pris conjointement, sont O=, S=, ou bien, indépendamment, lorsque l'un des radicaux X et Y est un atome d'halogène, l'autre est un atome d'hydrogène, un atome d'halogène, un groupe alkyle en C₁-C₇ ou aryl(alkyle en C₁-C₇); ou bien, lorsque l'un des radicaux X et Y est un groupe amino, alkylamino en C₁-C₇ ou dialkylamino en C₁-C₇, l'autre est un atome d'hydrogène, un groupe alkyle en C₁-C₇ ou aryl(alkyle en C₁-C₇); ou bien, lorsque l'un des radicaux X et Y est un groupe hydroxy, alcoxy ou aryl(alcoxy en C₁-C₇), l'autre est un atome d'hydrogène, un groupe hydroxy, alkyle en C₁-C₇, alcoxy en C₁-C₇ ou aryl(alcoxy en C₁-C₇).

3. Procédé selon la revendication 1, dans lequel R₁ est tel que décrit ci-dessus, R₂ et R₂' sont, indépendamment, un atome d'hydrogène, un groupe alkyle en C₁-C₇, aryle, alcoxy en C₁-C₇, hydroxy, halogéno, acyle ou nitro;
A est une liaison, -O-, -NR₇-, -S-, 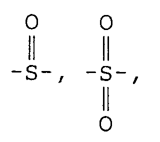 -C≡C-, -HC=CH-, -CH₂CH₂-, 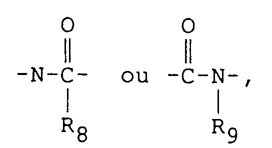 où R₇ est un atome d'hydrogène, un groupe alkyle en C₁-C₇ ou acyle, et R₈ et R₉ sont, indépendamment, un atome d'hydrogène ou un groupe alkyle en C₁-C₇;
B est une liaison,
-O-, NR₇-, -S-, -C≡C-, -HC=HC-, 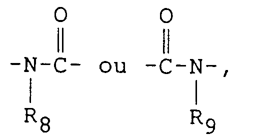 -(OCH₂CH₂)ₘ-O-, 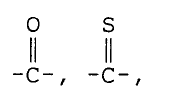 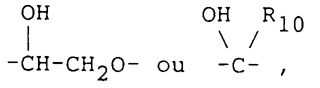 où R₇, R₈, R₉ et ni sont tels que définis ci-dessus et R₁₀ est un atome d'hydrogène;
n est un nombre entier de 1 à 6, de sorte que lorsque A est une liaison ou l'un des groupes suivants
-O-, NR₇-, -S-, 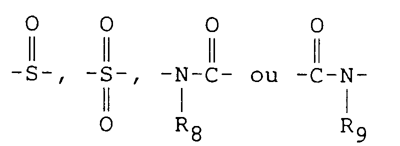 et que B est une liaison ou l'un des groupes suivants
-O-, -NR₇-, -S-, 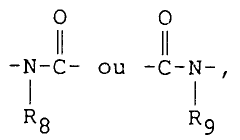 n soit différent du nombre entier 1;
Z est -S-, -CR₂=CR₂'-, -N=CR₂- ou -CR₂=N-;
X et Y, pris conjointement, sont des groupes O=, hydroxyimino, alkyloxyimino, alcényloxyimino, arylalcoxyimino, hydrazono, mono(alkylhydrazono en C₁-C₇), di(alkylhydrazono en C₁-C₇) ou semicarbazono, ou bien, indépendamment, lorsque l'un des radicaux X et Y est un atome d'halogène, l'autre est un atome d'hydrogène ou d'halogène; ou bien, lorsque l'un des radicaux X et Y est un groupe amino, l'autre est un atome d'hydrogène; ou bien, lorsque l'un des radicaux X et Y est un groupe hydroxy ou alcoxy, l'autre est un atome d'hydrogène ou un groupe hydroxy;
Q est un radical cycloalkyle, aryle, ou hétérocyclique, ce radical pouvant être substitué par un ou plusieurs des groupes suivants: alkyle, alcoxy, acyloxy, halogéno, acyloxyalkyle, alcoxyalkyle, aryloxyalcoxy, hydroxy, hydroxyalkyle, hydroxyalcoxy, phényle, trihalogénoalkyle, sulfamoyle, carboxycarbonyle ou alcoxalyle.

4. Procédé selon la revendication 3, dans lesquel R₁ est tel que décrit ci-dessus; R₂ et R₂', sont, indépendamment, un atome d'hydrogène, un groupe alkyle en C₁-C₇, alcoxy en C₁-C₇, hydroxy ou halogéno;
A est une liaison,
-O-, -NR₇-, -S-, 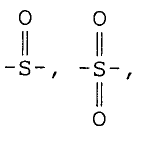 -C≡C-, -HC=CH-, -CH₂CH₂-, 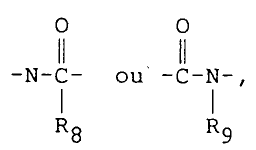 où R₇ est un atome d'hydrogène, un groupe alkyle en C₁-C₇ et R₈ et R₉ sont des atomes d'hydrogène;
B est une liaison,
-O-, -S-, -C≡C-, -HC=HC-, 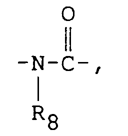 -(OCH₂CH₂)ₘ-O- ou 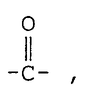 où m est égal à 1 ou 2 et R₈ est un atome d'hydrogène;
n est un nombre entier de 1 à 6, de sorte que, lorsque A est une liaison ou l'un des groupes suivants,
-O-, -NR₇-, -S-, 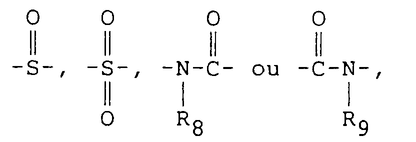 et que B est une liaison ou l'un des groupes suivants
-O-, -S-, ou 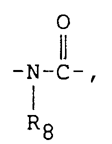 n soit différent du nombre entier 1;
Z est -S-, ou -CR₂=CR₂'-,
X et Y pris conjointement, sont un groupe O=, hydroxyimino, alkyloxyimino ou alcényloxyimino, ou bien, indépendamment, lorsque l'un des radicaux X et Y est un atome d'halogène, l'autre est un atome d'halogène; ou bien, lorsque l'un des radicaux X et Y est un groupe amino, l'autre est un atome d'hydrogène; ou bien, lorsque l'un des radicaux X et Y est un groupe hydroxy, l'autre est un atome d'hydrogène ou un groupe hydroxy;
Q est un radical phényle, cyclohexyle, cyclooctyle, pyridinyle, adamantyle, 1,1'-biphényle, anthracényle, phénanthrényle, naphtalényle, 5,6,7,8-tétrahydro-1-naphtalényle, 5,6,7,8-tétrahydro-2-naphtalényle, 1,2,3,4-tétrahydro-1-naphtalényle, 1,2,3,4-tétrahydro-2-naphtalényle, quinoléyle ou isoquinoléyle, ce radical pouvant être substitué par un ou plusieurs des groupes suivants: alkyle, alcoxy, acyloxy, halogéno, acyloxyalkyle, alcoxyalkyle, aryloxyalcoxy, hydroxy, hydroxyalkyle, hydroxyalcoxy, phényle, trihalogénoalkyle, sulfamoyle, carboxycarbonyle ou alcoxalyle.

5. Procédé selon la revendication 4, dans lequel R₁ est un groupe hydroxy ou 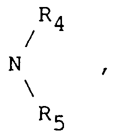 où l'un des radicaux R₄ et R₅ est un atome d'hydrogène, un groupe alkyle en C₁-C₇ ou hydroxyalkyle en C₁-C₇, et l'autre est un groupe hydroxy;
R₂ et R₂' sont des atomes d'hydrogène;
A est -O-, -NR₇- ou -S-, où R₇ est un atome d'hydrogène;
B est une liaison,
-O-, -S-, -C≡C-, -HC=HC-, 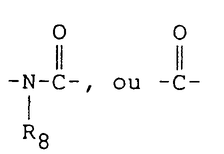 où R₈ est un atome d'hydrogène;
n est un nombre entier de 1 à 4, de sorte que lorsque B est une liaison ou l'un des groupes suivants, -O-, -S-, ou 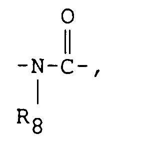 n soit différent du nombre entier 1;
Z est -S- ou -CR₂ = CR₂'-;
X et Y pris conjointement, sont un groupe O=, hydroxyimino, alkyloxyimino ou alcényloxyimino, ou bien, indépendamment, lorsque l'un des radicaux X et Y est un groupe fluoro, l'autre est un fluoro; ou bien, lorsque l'un des radicaux X et Y est un groupe amino, l'autre est un atome d'hydrogène; ou bien, lorsque l'un des radicaux X et Y est un groupe hydroxy, l'autre est un groupe hydroxy;
Q est un radical phényle, cyclohexyle, cyclooctyle, adamantyle, anthracényle, phénanthrényle, naphtalényle, 5,6,7,8-tétrahydro-1-naphtalényle, 5,6,7,8-tétrahydro-2-naphtalényle, 1,2,3,4-tétrahydro-1-naphtalényle, 1,2,3,4-tétrahydro-2-naphtalényle, quinoléyle ou isoquinoléyle, dont le radical phényle ou le radical naphtalényle peut être substitué par un ou plusieurs des groupes suivants: alkyle inférieur, phényle, acyloxy, acyloxyalkyle ou hydroxyalkyle.

6. Procédé selon la revendication 2, dans lequel on prépare le chlorhydrate d'acide (S)-alpha-amino-4-[[2-cyclooctyloxy)éthyl]oxy]benzèneacétique.

7. Procédé selon la revendication 1, dans lequel on prépare l'acide 3-fluoro-4-[2-(2-naphtalényloxy)éthoxy]-alpha-oxobenzèneacétique.

8. Procédé selon la revendication 1, dans lequel on prépare l'acide 4-[2-(1-naphtalényl)-2-oxoéthoxy]-alpha-oxobenzèneacétique.

9. Procédé selon la revendication 2, dans lequel on prépare l'ester 2-(diméthylamino)éthylique d'acide 4-[[2-(2-naphtalényloxy)éthyl]oxy]-alpha-oxobenzèneacétique.

10. Procédé selon la revendication 1, dans lequel on prépare le sel de morpholine de l'acide (E)-4-[3-[2-[(2,2-diméthyl-1-oxobutoxy)méthyl]-6-méthylphényl]-2-propényloxy]-alpha-oxobenzèneacétique (1:1).

11. Procédé selon la revendication 2, dans lequel on prépare l'acide 4-[[2-(2-naphtalényloxy)éthyl]oxy]-alpha-oxobenzèneacétique.

12. Procédé selon la revendication 2, dans lequel on prépare l'acide 4-[[2-(2-naphtalényloxy)éthyl]thio]-alpha-oxobenzèneacétique.

13. Procédé selon la revendication 2, dans lequel on prépare l'acide 4-[[2-(2-naphtalénylthio)éthyl]oxy]-alpha-oxobenzèneacétique.

14. Procédé selon la revendication 2, dans lequel on prépare l'acide 4-[[2-cyclooctyloxy)éthyl]oxy]-alpha-oxobenzèneacétique.

15. Procédé selon la revendication 1, dans lequel on prépare l'acide 4-[3-(4-acétyl-3-hydroxy-2-propylphénoxy)propoxy]-alpha-oxobenzèneacétique.

16. Procédé selon la revendication 1, dans lequel on prépare le N-hydroxy-N-méthyl-4-[2-(2-naphtalényloxy)éthoxy]-alpha-oxobenzèneacétamide.

17. Procédé selon la revendication 1, dans lequel on prépare l'acide (Z)-alpha-(hydroxyimino)-5-[2-(2-naphtalényloxy)éthoxy]-2-thiophèneacétique.

18. Procédé selon la revendication 2, dans lequel on prépare l'acide 5-[[2-(2-napthalényloxy)éthyl]oxy]-alpha-oxo-2-thiophèneacétique.

19. Procédé selon la revendication 2, dans lequel on prépare l'acide alpha-oxo-4-[[2-(phénoxy)éthyl]oxy]benzèneacétique.

20. Procédé selon la revendication 1, dans lequel on prépare l'acide (Z)-alpha-(éthoxyimino)-5-[2-(2-naphtalényloxy)éthoxy]-2-thiophèneacétique.

21. Procédé selon la revendication 2, dans lequel on prépare l'ester 2,3-dihydroxypropylique de l'acide 5-[[2-(2-naphtalényloxy)éthyl]oxy]-alpha-oxo-2-thiophèneacétique racémique.

22. Procédé selon la revendication 1, dans lequel on prépare l'acide (S)-alpha-amino-4-[2-[2-[(2,2-diméthyl-1-oxobutoxy)méthyl]-6-méthylphénoxy]éthoxy]benzèneacétique.

23. Procédé selon la revendication 2, dans lequel on prépare l'acide (E)-4-[[3-(2-naphtalényl)-2-propényl]oxy]-alpha-oxobenzèneacétique.

24. Procédé selon la revendication 2, dans lequel on prépare l'hydrate d'acide 4-[[2-[2-[(2,2-diméthyl-1-oxobutoxy)méthyl]-6-méthylphénoxy]éthyl]oxy]-alpha-oxobenzèneacétique (2:1).

25. Procédé selon la revendication 2, dans lequel on prépare l'acide 4-[[4-(2-napthalényloxy)butyl]oxy]-alpha-oxobenzèneacétique.

26. Procédé de fabrication d'un médicament, en particulier à utiliser pour combattre ou empêcher les lésions au tissu ischémique et les arythmies pendant et après un infarctus du myocarde, ce procédé comprenant le fait de mettre un composé de formule I, selon la revendication 1, sous forme d'un énantiomère, d'un racémate, d'un diastéréoisomère, ou d'un mélange de ceux-ci, d'un isomère géométrique, ou d'un mélange de ceux-ci, ou bien, lorsqu'un groupe basique ou acide est présent, un sel pharmaceutiquement acceptable de ce composé, sous la forme d'une dose galénique.

27. Utilisation d'un composé de formule I selon la revendication 1, sous forme d'un énantiomère, d'un racémate, d'un diastéréoisomère, ou d'un mélange de ceux-ci, ou d'un isomère géométrique, ou d'un mélange de ceux-ci, ou bien, lorsqu'un groupe basique ou acide est présent, d'un sel pharmaceutiquement acceptable de ce composé, pour fabriquer des médicaments contre les lésions au tissu ischémique et/ou les arythmies pendant et après un infarctus du myocarde.
